(19) Europäisches Patentamt
European Patent Office
Office européen des brevets

(11) EP 3 065 721 B1

(12) **EUROPEAN PATENT SPECIFICATION**

(45) Date of publication and mention of the grant of the patent:
**13.03.2024 Bulletin 2024/11**

(21) Application number: **14795634.6**

(22) Date of filing: **07.11.2014**

(51) International Patent Classification (IPC):
***A61K 9/50*** *(2006.01)* ***A61P 1/00*** *(2006.01)*
***A61P 1/04*** *(2006.01)* ***A61P 1/12*** *(2006.01)*
***A61P 15/00*** *(2006.01)* ***A61K 38/13*** *(2006.01)*
***A61P 17/00*** *(2006.01)* ***A61P 19/02*** *(2006.01)*
***A61P 21/04*** *(2006.01)* ***A61P 17/06*** *(2006.01)*
***A61P 29/00*** *(2006.01)* ***A61P 35/00*** *(2006.01)*
***A61P 37/08*** *(2006.01)* ***A61P 37/06*** *(2006.01)*

(52) Cooperative Patent Classification (CPC):
**A61K 38/13; A61K 9/5047; A61K 9/5073; A61P 1/00; A61P 1/04; A61P 1/12; A61P 15/00; A61P 17/00; A61P 17/06; A61P 19/02; A61P 21/04; A61P 29/00; A61P 35/00; A61P 37/06; A61P 37/08**

(86) International application number:
**PCT/EP2014/074054**

(87) International publication number:
**WO 2015/067762 (14.05.2015 Gazette 2015/19)**

(54) **FORMULATIONS COMPRISING CYCLOSPORIN A**

FORMULIERUNGEN MIT CYCLOSPORIN A

FORMULATIONS CONTENANT DE LA CYCLOSPORINE A

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.11.2013 GB 201319792**

(43) Date of publication of application:
**14.09.2016 Bulletin 2016/37**

(73) Proprietor: **Sublimity Therapeutics Limited**
**Dublin D11 KXN4 (IE)**

(72) Inventors:
- **COULTER, Ivan**
  **Mount Merrion**
  **Co. Dublin (IE)**
- **AVERSA, Vincenzo**
  **Swords**
  **Co. Dublin (IE)**
- **ROSA, Monica**
  **Dublin 7 (IE)**

(74) Representative: **HGF**
**HGF Limited**
**1 City Walk**
**Leeds LS11 9DX (GB)**

(56) References cited:
**WO-A1-93/13753**
**WO-A1-2014/128233**
**WO-A2-2008/122965**
**WO-A2-2010/133609**
**WO-A2-2012/069658**

- **LEI Y ET AL: "Solid self-nanoemulsifying cyclosporine A pellets prepared by fluid-bed coating: stability and bioavailability study", J BIOMED NANOTECHNOL, vol. 8, no. 3, 2012, pages 515-521, XP008174329,**
- **MALAEKEH-NIKOUEI B ET AL: "Preparation, characterization, and mucoadhesive properties of chitosan-coated microspheres encapsulated with cyclosporine A", DRUG DEVELOPMENT AND INDUSTRIAL PHARMACY, vol. 34, no. 5, May 2008 (2008-05), pages 492-498, XP002734890,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 065 721 B1

Remarks:

The file contains technical information submitted after the application was filed and not included in this specification

## Description

This invention relates to an orally administered modified release composition comprising cyclosporin A and to this composition for use in the treatment or prevention of disorders of the gastrointestinal tract. Also disclosed are methods for preparing such compositions.

## BACKGROUND

Cyclosporin A is a cyclic polypeptide which has immunosuppressive and anti-inflammatory properties. The compound has been approved for the prevention of organ rejection following kidney, liver, heart, combined heart-lung, lung or pancreas transplantation, for the prevention of rejection following bone marrow transplantation; the treatment and prophylaxis of Graft Versus Host Disease (GVHD); psoriasis; atopic dermatitis, rheumatoid arthritis and nephrotic syndrome (Neoral™ Summary of Product Characteristics 24/02/2012). Cyclosporin A may also be useful for the treatment of a range of other diseases including for the treatment severe recalcitrant plaque psoriasis Bechet's disease, anemia, myasthenia gravis and various conditions affecting the GI tract, including irritable bowel syndrome, Crohn's disease, colitis, including ulcerative colitis, diverticulitis, pouchitis, proctitis, Gastro-Intestinal Graft Versus Host Disease (GI-GVHD), colorectal carcinoma and adenocarcinoma. A range or other diseases may benefit from treatment with cyclosporin A (Landford et al. (1998) Ann Intern Med;128: 1021-1028). Cyclosporin A has been used to treat a number of gastrointestinal conditions including inflammatory bowel disease (Sandborn WJ, a critical review of cyclosporin therapy in inflammatory bowel disease, Inflamm Bowel Dis. 1995;1 :48-63), including ulcerative colitis (Lichtiger et al, preliminary report (cyclosporine in the treatment of severe ulcerative colitis), Lancet. 1990;336:16-19; Cohen et al, Intravenous cyclosporine in ulcerative colitis (a five-year experience), Am J Gastroenterol. 1999;94:1587-1592).

However cyclosporin A has a number of undesirable side effects including hypertension, impaired renal function, and neurotoxicity (Feutren et al, Risk factors for cyclosporine-induced nephropathy in patients with auto-immune diseases, International kidney biopsy registry of cyclosporine for autoimmune diseases, N Engl J Med. 1992;326:1654-1660; Wijdicks et al., Neurotoxicity in liver transplant recipients with cyclosporine immunosuppression, Neurology. 1995;45:1962-1964; and Porter et al, Cyclosporine-associated hypertension, National High Blood Pressure Education Program. Arch Intern Med. 1990;150:280-283).

Cyclosporin A is available as an intravenous formulation; Sandimmun™, which is a solution of 50 mg/ml of cyclosporin A in ethanol and polyethoxylated castor oil (for example Kolliphor™ EL). The product is also available as orally administered formulations, including a soft gelatin capsule containing a solution of cyclosporin A in ethanol, corn oil and lineoyl macrogolglycerides (Sandimmune™ Soft Gelatin capsules) and as an orally administered solution containing the cyclosporin dissolved in olive oil, ethanol, and labrafil M 1944 CS (polyethoxylated oleic glycerides) (Sandimmune™ Oral Solution). More recently a microemulsion concentrate formulation has been approved containing cyclosporin A dissolved in DL-$\alpha$-tocopherol, absolute ethanol, propylene glycol, corn oil-mono-di-triglycerides, polyoxyl 40 hydrogenated castor oil (Neoral™). Following oral administration the Neoral™ formulation results in the formation of a microemulsion and is stated to have an improved bioavailability compared to orally administered Sandimmune™. These orally administered cyclosporin A compositions are all instant release compositions and cyclosporin A will be present at high concentration in the stomach and small intestine from where it is systemically absorbed.

Sandborn et al.(J Clin Pharmacol. 1991 ; 31 :76-80) determined the relative systemic absorption of cyclosporin following oral and intravenous as well as oil- and water-based enemas. Based on negligible plasma cyclosporin concentrations observed following enema administration, it was suggested that cyclosporin, even when solubilised, is poorly absorbed from the colon. The enemas however demonstrated considerable efficacy in the treatment of inflammatory bowel disease (Ranzi T, et al, Lancet 1989;2:97). Intravenous or orally administered cyclosporin efficacy in the treatment of inflammatory bowel disease is dose dependent, requiring high doses to ensure adequate concentration reaches the colon. Systemic toxicity is known to be dose and duration dependent.

WO 2008/122965 discloses oral cyclosporin minicapsule compositions which release cyclosporin in at least the colon. WO2010/133609 discloses compositions comprising a water-soluble polymer matrix in which are dispersed droplets of oil. The disclosed compositions also contain an active principle.

There remains a need for orally administered cyclosporin A compositions which provide high levels of cyclosporin A in the lower GI tract, particularly in the colon and absorption of the cyclosporin A from the luminal contents into the tissues of the GI tract, particularly into the colonic tissue, for the treatment of conditions of the lower GI tract such as ulcerative colitis. Such compositions desirably minimise the systemic blood exposure to cyclosporin A thereby minimising the undesirable side effect associated with systemic exposure to cyclosporin A. Particularly there is a need for orally administered compositions which have a low exposure/ area under the curve (AUC) and/or low peak blood concentration (Cmax) compared to the orally administered product Neoral and/or cyclosporin A administered intravenously as for example Sandimmune™.

## BRIEF SUMMARY OF THE DISCLOSURE

In accordance with a first aspect of the present invention, there is provided a modified release composition for oral administration comprising a core having the form of a solid colloid, the colloid comprising a continuous phase comprising a hydrogel forming polymer and a disperse phase comprising cyclosporin A and an oil phase, the oil phase comprising an oil and one or more surfactants, wherein:

the oil and surfactant have an HLB of up to 10;
the oil comprises medium chain triglyceride;
the surfactant comprises a surfactant selected from: fatty acid glycerides, polyethylene glycol fatty acid esters, propylene glycol fatty acid esters, fatty acid lactic acid ester, sucrose fatty acid esters, sorbitan fatty acid esters, polyethylene glycol fatty alcohol ethers, ethylene oxide-propylene oxide block co-polymers and polyoxyethylene ethers;
the composition comprises a first coating and a second coating outside the first coating; and wherein

(i) the first coating comprises a water-soluble cellulose ether or a water-soluble derivative of a cellulose ether, wherein the first coating is present in an amount corresponding to a weight gain of the composition due to the first coating of from 1% to 20% by weight based upon the weight of the composition prior to applying the first coating; and
(ii) the second coating comprises a modified release coating to control or modulate release of the cyclosporin A from the composition selected from a controlled release polymer, a sustained release polymer, an enteric polymer, a pH independent polymer, a pH dependent polymer and a polymer specifically susceptible to degradation by bacterial enzymes in the gastrointestinal tract, or a combination of two or more such polymers.

In accordance with a second aspect of the present invention, there is provided a modified release composition for oral administration comprising a core having the form of a solid colloid, the colloid comprising a continuous phase comprising a hydrogel forming polymer and a disperse phase comprising cyclosporin A and an oil phase, the oil phase comprising an oil and one or more surfactants, wherein:

the oil and surfactant have an HLB of up to 10;
the weight ratio of surfactant: oil is from 5:1 to 1.5:1;
the surfactant comprises a surfactant selected from: fatty acid glycerides, polyethylene glycol fatty acid esters, propylene glycol fatty acid esters, fatty acid lactic acid ester, sucrose fatty acid esters, sorbitan fatty acid esters, polyethylene glycol fatty alcohol ethers, ethylene oxide-propylene oxide block co-polymers and polyoxyethylene ethers;
the composition comprises a first coating and a second coating outside the first coating; and wherein

(i) the first coating comprises a water-soluble cellulose ether or a water-soluble derivative of a cellulose ether, wherein the first coating is present in an amount corresponding to a weight gain of the composition due to the first coating of from 1% to 20% by weight based upon the weight of the composition prior to applying the first coating; and
(ii) the second coating comprises a modified release coating to control or modulate release of the cyclosporin A from the composition selected from a controlled release polymer, a sustained release polymer, an enteric polymer, a pH independent polymer, a pH dependent polymer and a polymer specifically susceptible to degradation by bacterial enzymes in the gastrointestinal tract, or a combination of two or more such polymers.

In accordance with a third aspect of the present invention, there is provided a composition according to the first or second aspect for use:

(A) as a medicament;
(B) in the treatment of an inflammatory bowel disease, Crohn's disease, ulcerative colitis, graft-versus-host disease, gastrointestinal graft-versus-host disease, myasthenia gravis, irritable bowel syndrome, celiac disease, stomach ulcers, diverticulitis, pouchitis, proctitis, mucositis, chemotherapy-associated enteritis, radiation-associated enteritis, short bowel disease, chronic diarrhea, gastroenteritis, duodenitis, jejunitis, peptic ulcer, Curling's ulcer, appendicitis, colitis, diverticulosis, endometriosis, colorectal carcinoma, adenocarcinoma, diversion colitis, ischemic colitis, infectious colitis, chemical colitis, microscopic colitis (including collagenous colitis and lymphocytic colitis), atypical colitis, pseudomembraneous colitis, fulminant colitis, autistic enterocolitis, indeterminate colitis, jejunoiletis, ileitis, ileocolitis or granulomatous colitis, prevention of rejection following bone marrow transplantation, psoriasis, atopic dermatitis,

rheumatoid arthritis, nephrotic syndrome, primary sclerosing cholangitis, familial adenomatous polyposis, or perianal Crohn's, including perianal fistulae;

(C) in the treatment of an inflammatory condition of the GIT; or

(D) in the treatment of a condition of the GIT selected from irritable bowel syndrome, celiac disease, stomach ulcers, diverticulitis, pouchitis, proctitis, mucositis, radiation-associated enteritis, short bowel disease, or chronic diarrhea, gastroenteritis, duodenitis, jejunitis, peptic ulcer, Curling's ulcer, appendicitis, colitis, diverticulosis, endometriosis, colorectal carcinoma, adenocarcinoma, inflammatory disorders such as diversion colitis, ischemic colitis, infectious colitis, chemical colitis, microscopic colitis (including collagenous colitis and lymphocytic colitis), atypical colitis, pseudomembraneous colitis, fulminant colitis, autistic enterocolitis, indeterminate colitis, jejunoiletis, ileitis, ileocolitis, granulomatous colitis, fibrosis, graft-versus-host disease, gastrointestinal graft-versus-host disease, HIV or enteropathies.

In an embodiment there is provided a modified release composition comprising cyclosporin A, wherein the composition provides a mean whole blood $AUC_{0\text{-}inf}$ of less than about 900 ng.hr/ml, less than about 650 ng.hr/ml, less than about 550 ng.hr/ml, less than about 450 ng.hr/ml, less than about 350 ng.hr/ml, or less than about 300 ng.hr/ml after oral administration of the composition as a single dose containing 75 mg cyclosporin A to a human in a fasted state, or an $AUC_{0\text{-}inf}$ directly proportional thereto for a total dose other than 75mg. Suitably the composition provides a mean whole blood $AUC_{0\text{-}inf}$ of from about 100 to about 900 ng.hr/ml, for example from about 200 to about 900 ng.hr/ml, or about 350 to about 750 ng.hr/ml, or about 150 to about 450 ng.hr/ml, about 140 to about 420 ng.hr/ml, about 150 to about 300 ng.hr/ml, about 160 to about 350 ng.hr/ml, or about 200 to about 400 ng.hr/ml after oral administration of the composition as a single dose containing 75 mg cyclosporin A to a human in a fasted state, or an $AUC_{0\text{-}inf}$ directly proportional thereto for a total dose other than 75mg.

In an embodiment there is provided a modified release composition comprising cyclosporin A, wherein the composition provides a mean whole blood $AUC_{0\text{-}inf}$ of 672 $\pm$ 296 ng.hr/ml after oral administration of the composition as a single dose containing 75 mg cyclosporin A to a human in a fasted state, or an $AUC_{0\text{-}inf}$ directly proportional thereto for a total dose other than 75mg.

Another embodiment provides a modified release composition comprising cyclosporin A, wherein the composition provides a mean whole blood $AUC_{0\text{-}inf}$ of 474 $\pm$ 247 ng.hr/ml after oral administration of the composition as a single dose containing 75 mg cyclosporin A to a human in a fasted state, or an $AUC_{0\text{-}inf}$ directly proportional thereto for a total dose other than 75mg.

The composition according to the invention provides a lower $AUC_{0\text{-}24}$ following oral administration compared to oral administration of Neoral™ at the same dose of cyclosporin A. According to one embodiment there is provided a modified release composition comprising cyclosporin A, wherein the composition provides a mean whole blood $AUC_{0\text{-}24hr}$ of less than about 850 ng.hr/ml, less than about 650 ng.hr/ml, less than about 550 ng.hr/ml, less than about 450 ng.hr/ml, less than about 350 ng.hr/ml, or less than about 300 ng.hr/ml after oral administration of the composition as a single dose containing 75 mg cyclosporin A to a human in a fasted state, or an $AUC_{0\text{-}24hr}$ directly proportional thereto for a total dose other than 75mg. Suitably the composition provides a mean whole blood $AUC_{0\text{-}24hr}$ of from about 100 to about 500 ng.hr/ml, from about 150 to about 500 ng.hr/ml, from about 150 to about 850 ng.hr/ml, for example about 300 to about 700 ng.hr/ml, about 140 to 420 ng.hr/ml, about 150 to about 400 ng.hr/ml, about 160 to about 380 ng.hr/ml, about 140 to about 380 ng.hr/ml about 150 to about 350 ng.hr/ml, about 180 to about 380 ng.hr/ml, about 200 to about 400 ng.hr/ml, about 150 to about 380 ng.hr/ml or about 150 to about 300 ng.hr/ml after oral administration of the composition as a single dose containing 75 mg cyclosporin A to a human in a fasted state, or an $AUC_{0\text{-}24hr}$ directly proportional thereto for a total dose other than 75mg.

A further embodiment provides a modified release composition comprising cyclosporin A, wherein the composition provides a mean whole blood $AUC_{0\text{-}24}$ of 610 $\pm$ 280 ng.hr/ml after oral administration of the composition as a single dose containing 75 mg cyclosporin A to a human in a fasted state, or an $AUC_{0\text{-}24}$ directly proportional thereto for a total dose other than 75mg.

In another embodiment there is provided a modified release composition comprising cyclosporin A, wherein the composition provides a mean whole blood $AUC_{0\text{-}24}$ of 408 $\pm$ 231 ng.hr/ml after oral administration of the composition as a single dose containing 75 mg cyclosporin A to a human in a fasted state, or an $AUC_{0\text{-}24}$ directly proportional thereto for a total dose other than 75mg.

A high peak blood concentration of cyclosporin A and associated AUC may result in undesirable side effects and potentially reduced the therapeutic window available for a composition containing cyclosporin A. In one embodiment there is provided a modified release composition comprising cyclosporin A, wherein the composition provides a mean maximum whole blood concentration of cyclosporin A (Cmax) of less than about 250 ng/ml after oral administration of the composition as a single dose containing 75 mg of cyclosporin A to a human in a fasted state, or a Cmax directly proportional thereto for a total dose other than 75mg. In one particular embodiment the Cmax is from about 10 to about 220 ng/ml. In another particular embodiment the Cmax is from about 20 to about 220 ng/ml. In another particular

embodiment the Cmax is from about 75 to about 150 ng/ml. In another particular embodiment the Cmax is from about 20 to about 50 ng/ml (in these embodiments the Cmax is the Cmax after oral administration of the composition as a single dose containing 75 mg of cyclosporin A to a human in a fasted state, or a Cmax directly proportional thereto for a total dose other than 75mg).

In another particular embodiment there is provided a modified release composition comprising cyclosporin A, wherein the composition provides a Cmax of 138 $\pm$ 63 ng/ml after oral administration of the composition as a single dose containing 75 mg cyclosporin A to a human in a fasted state, or a Cmax directly proportional thereto for a total dose other than 75mg.

In another embodiment there is provided a modified release composition comprising cyclosporin A, wherein the composition provides a Cmax of 83 $\pm$ 48 ng/ml after oral administration of the composition as a single dose containing 75 mg cyclosporin A to a human in a fasted state, or a Cmax directly proportional thereto for a total dose other than 75mg.

Following the oral administration of a pharmaceutical composition to a subject drug is released from the composition and is absorbed systemically from the stomach and/or GI tract. The time taken to reach the peak concentration of the drug in the blood is Tmax. The modified release compositions according to the invention provide a longer Tmax compared to the Tmax of Neoral. The Examples show that a longer Tmax of the composition of the invention results in a lower blood exposure (lower AUC) and also results in a lower Cmax compared to Neoral™ orally administered at the same dose of cyclosporin A.

One embodiment provides a modified release composition comprising cyclosporin A wherein the time taken to reach maximum whole blood concentration (Tmax) of the cyclosporin A occurs between about 3 and about 10 hours after oral administration of the composition as a single dose to a human in a fasted state. In certain embodiments Tmax occurs between about 4 hours and about 10 hours, or between about 4 hours and about 8 hours, or between about 5 and about 6 hours. In another embodiment Tmax occurs at about 5 hours following oral administration of a single dose of the composition. In these embodiments wherein the time taken to reach maximum whole blood concentration ($T_{max}$) of the cyclosporin A occurs between about 3 and about 10 hours after oral administration of the composition as a single dose containing from about 25 to about 250 mg of cyclosporin A, for example about 37.5mg, about 75 mg or about 150 mg of cyclosporin A (optionally about 75 mg cyclosporin A) to a human in a fasted state. Suitably in these embodiments the corresponding mean Cmax is less than about 250 ng/ml, for example about 20 to about 220 ng/ml, about 75 to about 150 ng/ml, about 15 to about 60 ng/ml, or about 20 to about 50 ng/ml after oral administration of the composition as a single dose containing 75 mg of cyclosporin A to a human in a fasted state, or a Cmax directly proportional thereto for a total dose other than 75mg.

The compositions of the invention provide a low blood exposure (AUC) compared to oral administration of Neoral, accordingly the ratio of the mean AUC of a composition of the invention : to mean AUC of Neoral™ is less than 1, reflecting the relatively low systemic blood exposure to cyclosporin A provided by the compositions of the invention compared to Neoral.

Accordingly in an embodiment there is provided a modified release composition comprising cyclosporin A, wherein after oral administration of a single dose of the composition to a human in a fasted state the % ratio of the mean whole blood $AUC_{0\text{-}inf}$ of the composition : the mean whole blood $AUC_{0\text{-}inf}$ of Neoral™ when administered orally as a single dose of cyclosporin A of the same mass is less than 60% calculated using least-squares means. Suitably the ratio is from 15 to 50% calculated using least-squares means. In a further embodiment the ratio is from 25 to 50% calculated using least-squares means. In a further embodiment the ratio is from 15 to 35% calculated using least-squares means.

Another embodiment provides a modified release composition comprising cyclosporin A, wherein after oral administration of a single dose of the composition to a human in a fasted state the % ratio of the mean whole blood $AUC_{0\text{-}24}$ of the composition : the mean whole blood $AUC_{0\text{-}24}$ of Neoral™ when administered orally as a single dose of cyclosporin A of the same mass is less than 55% calculated using least-squares means. Suitably the ratio of the mean $AUC_{0\text{-}24}$ values is from 10% to 50% calculated using least-squares means.

The relative maximum blood concentration of cyclosporin A (Cmax) of the compositions according to the invention compared to the administration of Neoral™ is also low. Accordingly, in another embodiment there is provided a modified release composition comprising cyclosporin A, wherein after oral administration of a single dose of the composition to a human in a fasted state the % ratio of the mean Cmax of the composition : the mean Cmax of Neoral™ when administered orally as a single dose of cyclosporin A of the same mass is less than 40% calculated using least-squares means. Suitably the ratio of the mean Cmax values is from 5% to 30% calculated using least-squares means. In another embodiment the ratio of the mean Cmax values is from 10% to 30% calculated using least-squares means. In another embodiment the ratio of the mean Cmax values is from 5% to 20% calculated using least-squares means.

The modified release compositions according to the invention result in low systemic blood exposure relative to Neoral™ as described above and shown by the relatively low PK parameters such as Cmax and AUC mentioned above. The compositions of the invention also provide relatively high levels of cyclosporin A in the lower GI tract (particularly in the colon) compared to the oral administration of Neoral at the same cyclosporin A dose. The local release of cyclosporin A in the colon provides cyclosporin A in an active form, for example in a solubilised form, where it is expected to be

beneficial in the treatment or prevention of a number of conditions of the lower GI tract, particularly those that affect the colon, for example Crohn's disease and ulcerative colitis. Systemic cyclosporin A absorption to the blood from the colon is low compared to the upper GI tract, however, the composition according the invention releases the cyclosporin A in a form which is readily absorbed from the luminal contents into the mucosa and sub-mucosa of the colon. The relatively high levels of active cyclosporin A in the colon is illustrated by analysis of faecal samples from subjects dosed with cyclosporin A. The concentration of cyclosporin A in faecal samples following administration of a composition according to the invention is significantly higher than that in subjects orally administered with Neoral. Oral administration of Neoral™ capsules requires emulsification with bile salts before active cyclosporin is released and available for absorption. Therefore, the bioavailability of Cyclosporin A following administration of Neoral™is dependent on food intake, bile flow and gastrointestinal mobility. The major pathways of cyclosporin A metabolism in humans are via cytochrome P450 3A4 (CYP 3A4) and cytochrome P450 2J2 (CYP 2J2), with three major metabolites being formed (two hydroxylated metabolites AM1, AM9 and one N-demethylated AM4N). These metabolites have minimal, if any immunosuppressive activity. The primary metabolism is via CYP 3A4, which is mainly found in the liver and the small intestine. CYP 3A4 expression in the colon is lower. Similarly, the expression of CYP 2J2 is greatest in small intestine, followed by the liver and then the colon.

Analysis of the concentration of cyclosporin A metabolites, for example the AM1, AM4N and AM9 metabolites, in faecal samples show that compositions according to the invention are absorbed in the local tissue and are metabolised. However, as mentioned above whole blood exposure (systemic exposure) to cyclosporin A is significantly lower than the whole blood exposure resulting from oral administration of Neoral™. Faecal analysis therefore provides a measure of the relative local availability of cyclosporin A in the colon, and a measure of the local tissue absorption of the cyclosporin A following oral administration of a composition of the invention. Oral administration of the compositions according to the invention provide high ratios of cyclosporin A: cyclosporin A metabolites in faeces samples compared to the oral administration of Neoral™.

Accordingly in one embodiment there is provided a modified release composition comprising cyclosporin A, wherein after oral administration of a single dose of the composition to a human in a fasted state the ratio of the mean concentration of cyclosporin A : the concentration of cyclosporin A metabolites in a faecal sample collected from 12 to 28 hours after dosing the composition is greater than 1:1, for example, from 2:1 to 100:1, from 4:1 to 80 :1, from 20:1 to 40:1, from 20:1 to 35:1, from 20:1 to 30:1, from 30:1 to 50:1 or from 2:1 to 12:1, for example: from 3:1 to 12:1; from 4:1 to 12:1; from 4:1 to 10:1, from 5:1 to 12:1, from 5:1 to 8:1 or from 9:1 to 12:1. Suitably the cyclosporin metabolites are the major metabolites of cyclosporin A for example the AM1, AM4N and AM9 metabolites of cyclosporin A. The ratios above may be the ratio of cyclosporin A: sum of the concentrations of the AM4N and AM9 metabolites. Optionally the ratio above may be the ratio of cyclosporin A: sum of the concentrations of the AM1, AM4N and AM9 metabolites. The ratio of cyclosporin A: metabolite concentration may be measured in a faecal sample collected 12 to 28 hours after orally administering a single dose of 75 mg cyclosporin A. Optionally the ratio may be measured after oral administration of doses of the cyclosporin A other than a 75 mg single dose, for example 37.5 mg or 150 mg. Optionally the ratio of cyclosporin A:metabolite concentrations may be measured in faecal samples collected at different time points to the 12 to 28 hour period stated above, provided sufficient time has elapsed from administration of the composition to allow transit of the cyclosporin through the GI tract. In these embodiments, the composition suitably provides a Tmax in whole blood which occurs between about 3 and about 10 hours, or about 4 to about 10 hours, about 4 to about 8 hours or about 5 to about 6 hours, for example at about 5 hours after oral administration of a single dose of the composition to a human in a fasted state. The composition results in low systemic blood exposure to cyclosporin A and may provide a mean whole blood $AUC_{0\text{-}inf}$ of, for example, from about 350 to about 750 ng.hr/ml after oral administration of the composition as a single dose containing 75 mg cyclosporin A to a human in a fasted state, or an $AUC_{0\text{-}inf}$ directly proportional thereto for a total dose other than 75mg.

The modified release compositions according to the invention are expected to provide relatively high concentrations of cyclosporin A in the lower GI tract (particularly in the colon) compared to the intravenous administration cyclosporin A as for example Sandimmun™ when administered at an equivalent therapeutic dose of cyclosporin A. An IV dose of 2 to 4 mg/kg/day cyclosporin is known to be efficacious in the treatment of ulcerative colitis patients (Lichtiger et al N. Engl J Med 1994; 330: 1841-1845). A comparison of a modified release composition of the invention at a dose of 75 mg is expected to show higher cyclosporin concentration in the colon compared to IV administration of cyclosporin at a dose of 2 mg/kg.

IV administration of cyclosporin such as Sandimmun™ avoids metabolism in the intestine and therefore the concentration of cyclosporin metabolites in the colon following IV administration is lower than that observed following oral administration of Neoral™. However, systemic exposure to the drug is high when an IV route of administration is used. The modified release compositions according to the invention are expected to provide similar or lower concentrations cyclosporin metabolites in the colon compared to the use of Sandimmun™ IV, but with lower the systemic exposure to cyclosporin.

The modified release compositions of the invention are expected to provide similar or higher levels of cyclosporin

A in the colonic tissue compared to IV administration of Sandimmun™, but with a higher faecal concentration of cyclosporin A as a result of the local release of the cyclosporin in the colon. The relatively high local concentration of cyclosporin in the colon is expected to provide beneficial therapeutic effects.

Also provided is a modified release composition comprising cyclosporin A, wherein the ratio of the mean concentration of cyclosporin A present in intracolonic faeces : the mean concentration of cyclosporin A present in colonic tissue in an adult human patient after oral administration of the composition is from about 50:1 to about 500:1, optionally from about 80:1 to about 300:1, or optionally about 100:1 to about 250:1; wherein the concentration of the cyclosporin A is measured in samples of the intracolonic faeces and the colonic tissue taken substantially simultaneously 4 to 6 hours after oral administration of the last dose of a once daily oral dosing regimen of the composition, the dosing regimen comprising once daily oral administration of the composition for seven days.

Suitably the once daily oral dosing regimen of the composition provides a single daily dose of 75 mg cyclosporin A. However, other doses may be administered for example 37.5 mg or 150 mg once per day. Optionally the dosage regimen may be a twice daily dosage regimen for seven days, for example 37.5 mg twice a per day, 75 mg twice per day or 150 mg twice per day.

Reference herein to a sample being taken "substantially simultaneously" means that the samples are obtained close to the same time point, for example the colonic tissue and/or intracolonic faeces and/or blood samples are taken within about 2 hours, 1 hour or 30 minutes of each other, suitably the samples are all taken at the same time point.

Also provided is a modified release composition comprising cyclosporin A, wherein the ratio of the mean concentration of cyclosporin A present in colonic tissue: the mean whole blood concentration of cyclosporin A in an adult human patient after oral administration of the composition is from about 10:1 to about 200:1, for example about 20:1 to about 100:1, or from about 20:1 to about 40:1,

wherein the concentration of the cyclosporin A in the tissue is measured in a sample of colonic tissue taken 4 to 6 hours after oral administration of the last dose of a once daily oral dosing regimen of the composition, the dosing regimen comprising once daily oral administration of the composition for seven days; and

wherein the concentration of cyclosporin A in the blood is measured in a sample of whole blood taken from the patient substantially simultaneously with tissue sample.

Suitably the once daily oral dosing regimen of the composition provides a single daily dose of 75 mg cyclosporin A. However, other doses may be administered for example 37.5 mg or 150 mg once per day. Optionally the dosage regimen may be a twice daily dosage regimen for seven days, for example 37.5 mg twice a per day, 75 mg twice per day or 150 mg twice per day.

The blood sample and tissue sample are taken from the patient substantially simultaneously. Therefore the ratio of the concentration of cyclosporin in the tissue to that in the blood replicates the concentration gradient between the colonic tissue and the blood at the same time point. Taking the blood and tissue samples at 4 to 6 hours after the last dose of the seven day dosing regimen is expected to coincide with the Tmax of the last dose of the composition. Additionally the seven day dosing regimen is expected to provide steady state conditions in the colon and therefore reduce variability in the measured data.

In contrast to the compositions according to the invention, IV administration cyclosporin as Sandimmun™ results in a lower ratio of concentration of cyclosporin A present in colonic tissue: the whole blood concentration of cyclosporin A. As illustrated in the Examples when patients were treated with Sandimmun® IV (2mg/kg) administered as an infusion over 24 hours (2mg/kg/day) had a tissue : whole blood ratio of about 5.75, wherein the tissue and blood samples were obtained substantially simultaneously during the last hour of the IV infusion. The concentration of cyclosporin A in the tissue is expected to be similar to those observed following oral administration of the composition of the invention at a once daily dose of 75 mg cyclosporin. However, due to the high systemic exposure resulting from the IV infusion the tissue : blood ratio is significantly lower than that according to the invention. Accordingly, the compositions of the invention are expected to provide a therapeutically active concentration on cyclosporin in the colonic tissue, but with a significantly lower systemic exposure compared to Sandimmun™ IV.

The low systemic exposure to cyclosporin A resulting from the oral administration of the composition of the invention may also be illustrated by the ratio of whole blood $AUC_{0-24hr}$ :cyclosporin A concentration in colonic tissue. The compositions according to the invention are expected to provide a low ratio of AUC: cyclosporin concentration in colonic tissue. In contrast IV administration of cyclosporin will result in a significantly higher ratio due to the high blood levels and hence systemic exposure resulting from the IV administration of the cyclosporin, The composition of the invention thus provides a low AUC and a high tissue concentration of cyclosporin A.

Accordingly also provided is a modified release composition comprising cyclosporin A, wherein the ratio of the mean whole blood $AUC_{0-24hr}$ : the mean concentration of cyclosporin A present in colonic tissue is from about 0.05 to about 1, for example from about 0.05 to about 0.5 or from about 0.05 to about 0.25;

wherein the mean concentration of cyclosporin A present in colonic tissue is measured in a sample of colonic tissue taken 4 to 6 hours after oral administration of the last dose of a once daily oral dosing regimen of the composition, the dosing regimen comprising once daily oral administration of the composition for seven days; and

wherein the mean whole blood $AUC_{0-24hr}$ is determined after oral administration of the last dose of the composition administered in the dosing regimen.

Suitably the once daily oral dosing regimen of the composition provides a single daily dose of 75 mg cyclosporin A. However, other doses may be administered for example 37.5 mg or 150 mg once per day. Optionally the dosage regimen may be a twice daily dosage regimen for seven days, for example 37.5 mg twice per day, 75 mg twice per day or 150 mg twice per day.

The local release of cyclosporin A from a composition of the invention may also be determined by measurement of cyclosporin concentration in the luminal contents at specific locations along the GI tract and/or by analysis of cyclosporin A concentration in tissue biopsies from the GI tract. The Examples illustrate the analysis of cyclosporin A concentration in the lumen contents and tissue of the lower GI tract in a pig model 24 hours after administration of cyclosporin A. The composition according to the invention provide higher peak levels of cyclosporin A in the luminal contents and GI tract tissue compared to administration of Neoral™ 24 hours after oral administration of the cyclosporin A.

According to one embodiment there is provided a modified release composition comprising cyclosporin A, wherein 24 hours after oral administration of a single dose of the composition to a male pig weighing about 18kg the ratio of the mean peak cyclosporin A concentration in the gastrointestinal luminal contents : the mean peak cyclosporin A concentration in the gastrointestinal luminal contents 24 hours after oral administration of Neoral™ as a single dose of cyclosporin A of the same mass is from 2.5:1 to 12:1; for example: from 3.5:1 to 12:1; from 4:1 to 12:1; from 4.5:1 to 6:1; from 3.5:1 to 8:1; from 3.5:1 to 7:1, from 3.5:1 to 6:1; from 3.5:1 to 12:1; from 2.5:1 to 8:1; from 2.5:1 to 7:1.

Another embodiment provides a modified release composition comprising cyclosporin A, wherein 24 hours after oral administration of a single dose of the composition to a male pig weighing about 18kg the ratio of the mean peak cyclosporin A concentration in colonic tissue : the mean peak cyclosporin A concentration in colonic tissue 24 hours after oral administration of Neoral™ as a single dose of cyclosporin A of the same mass is from 2:1 to 12:1, for example: from 3:1 to 12:1, for example from 5:1 to 12:1; from 5.5:1 to 12:1; from 5.5:1 to 8:1; from 2:5:1 to 10:1; from 2.5:1 to 8:1; from 2.5:1 to 6:1; from 3:1 to 10:1; from 3:1 to 8:1; from 3:1 to 6:1.

In another embodiment there is provided a modified release composition comprising cyclosporin A, wherein 24 hours after oral administration of a single dose of the composition to a male pig weighing about 18kg the ratio of the mean peak cyclosporin A concentration in the gastrointestinal luminal contents : the mean peak cyclosporin A concentration in the colonic tissue is from 3.5:1 to 15:1, for example: from 3.5:1 to 12:1; from 4:1 to 12:1; from 4:1 to 10:1; from 6:1 to 12:1; from 7:1 to 12:1; from 7.5:1 to 10:1; or about 8:1. Suitably in this embodiment the ratios are measured after administration of a single dose containing 2 mg/kg cyclosporin A.

Particularly high levels of cyclosporin A is observed in the inner mucosa and sub-mucosa tissue in the colon following oral administration of a composition according to the invention compared to the administration of Neoral.

According to another embodiment there is provided a modified release composition comprising cyclosporin A, wherein 24 hours after oral administration of a single dose of the composition to a male pig weighing about 18kg the ratio of the mean cyclosporin A concentration in the mucosa : the mean cyclosporin A concentration in the muscularis externa of transverse colonic tissue is from 1:1 to 5:1, for example from 2:1 to 4:1, particularly from 2:1 to 3:1. Suitably in this embodiment the ratios are measured after administration of a single dose containing 2 mg/kg cyclosporin A.

According to another embodiment there is provided a modified release composition comprising cyclosporin A, wherein 24 hours after oral administration of a single dose of the composition to a male pig weighing about 18kg the ratio of the mean cyclosporin A concentration in the mucosa of transverse colonic tissue : the mean cyclosporin A concentration in the mucosa of transverse colonic tissue 24 hours after oral administration of Neoral™ as a single dose of cyclosporin A of the same mass is from 5:1 to 10:1, for example from 6:1 to 9:1, suitably from 6.5:1 to 8.5:1.

It is to be understood that the individual embodiments described above may be combined with one or more of the other embodiments described to provide further embodiments of the invention defined by a for example a combination of one or more of the embodiments to the AUC; Cmax; Tmax; concentration of cyclosporin A in the luminal contents, concentration of cyclosporin A in GI tract tissue; and ratio of cyclosporin A in luminal contents: GI tract tissue.

The compositions according to the invention provide a specific rate and extent of release of cyclosporin A following oral administration which provides the cyclosporin A in an active form (for example a solubilised form as discussed below in relation to the composition) at the required location within the lower GI tract, particularly in the colon.

In one embodiment there is provided a modified release composition comprising cyclosporin A wherein the composition releases less than 20% of the cyclosporin A after 2 hours; and releases at least 50% of the cyclosporin A after 12 hours, when measured in a two stage dissolution test using a USP Apparatus II with a paddle speed of 75 rpm and a dissolution medium temperature of 37°C; wherein for the first 2 hours of the dissolution test the dissolution medium

is 750 ml of 0.1 N HCl, and at 2 hours 250 ml of 0.2M tribasic sodium phosphate containing 2% SDS is added to the dissolution medium and the pH is adjusted to pH 6.8 (i.e. volume of dissolution medium in the second part of the test is 1000 ml). In one embodiment the composition releases 0 to 10% of the cyclosporin A after 2 hours; and releases from 60 to 100% of the cyclosporin A after 12 hours, when measured in the two stage dissolution test. In another embodiment the composition releases less than 20% of the cyclosporin A after 2 hours; releases 10 to 40% of the cyclosporin A at 4 hours and releases at least 50% of the cyclosporin A at 12 hours, when measured in the two stage dissolution test. In a further embodiment the composition releases less than 20% of the cyclosporin A after 2 hours; releases 15 to 40% of the cyclosporin A at 4 hours; and releases at least 75% of the cyclosporin A at 12 hours, when measured in the two stage dissolution test. In a further embodiment the composition releases less than 10% of the cyclosporin A after 2 hours; releases 10 to 30% of the cyclosporin A at 4 hours; and releases at least 70% of the cyclosporin A at 12 hours, when measured in the two stage dissolution test. In a further embodiment the composition releases from about 50 to about 75% of the cyclosporin A between 4 hours and 12 hours in the two stage dissolution test, for example the composition releases from about 55 to about 75%, particularly from about 55 to 70% of the cyclosporin A between 4 hours and 12 hours in the two stage dissolution test.

In another embodiment the composition releases 0 to 10% of the cyclosporin A after 2 hours; and releases from 50 to 100% of the cyclosporin A after 12 hours, when measured in the two stage dissolution test. In another embodiment the composition releases less than 20% of the cyclosporin A after 2 hours; releases 5 to 40% of the cyclosporin A at 4 hours and releases at least 50% of the cyclosporin A at 12 hours, when measured in the two stage dissolution test.

In a further embodiment the composition releases less than 20% of the cyclosporin A after 2 hours; releases 10 to 40% of the cyclosporin A at 4 hours; and releases at least 60% of the cyclosporin A at 12 hours, when measured in the two stage dissolution test. In a further embodiment the composition releases less than 10% of the cyclosporin A after 2 hours; releases 10 to 30% of the cyclosporin A at 4 hours; and releases at least 50% of the cyclosporin A at 12 hours, when measured in the two stage dissolution test. In a further embodiment the composition releases from about 30 to about 75% of the cyclosporin A between 4 hours and 12 hours in the two stage dissolution test, for example the composition releases from about 40 to about 75%, particularly from about 45 to 70% of the cyclosporin A between 4 hours and 12 hours in the two stage dissolution test.

In another embodiment the composition of the invention releases less than 15% (for example 0 to 10%) of the cyclosporin A after 2 hours; releases 10% to 40% (for example 10% to 35%, or suitably 15% to 35%) of the cyclosporin A at 4 hours; and releases from about 25% to 70% (for example 40% to 70%) of the cyclosporin A between 4 hours and 12 hours in the two stage dissolution test.

In one embodiment the composition of the invention releases less than 15% (for example 0 to 10%) of the cyclosporin A after 2 hours; releases 15% to 40% (for example 20% to 35%, or suitably 25% to 35%) of the cyclosporin A at 4 hours; and releases from about 30% to 70% (for example 55% to 70%) of the cyclosporin A between 4 hours and 12 hours in the two stage dissolution test.

Suitably the composition releases at least 80%, at least 85%, at least 90% or at least 95% of the cyclosporin A within 24 hours, when measured in the two stage dissolution test. Accordingly in all of the dissolution profiles above the composition releases at least 80%, at least 85%, at least 90% or at least 95% of the cyclosporin A within 24 hours, when measured in the two stage dissolution test.

It is to be understood that the in-vitro release profiles described in the embodiment above are applicable to each of the embodiments described above or below, for example those embodiments relating to any one or a combination of AUC; Cmax; Tmax; cyclosporin A concentration in the luminal contents; cyclosporin A concentration in the GI tract tissue; the ratio of cyclosporin A in the luminal contents : cyclosporin A in GI tract tissues; the ratio of the concentration of cyclosporin A: the concentration of cyclosporin A metabolites in faeces; the concentration of cyclosporin A in intracolonic faeces: the concentration of cyclosporin A in colonic tissue; or the concentration of cyclosporin A in colonic tissue: the concentration of cyclosporin A in whole blood.

Accordingly in one embodiment there is provided a modified release composition comprising cyclosporin A wherein the composition releases less than 15% (for example 0 to 10%) of the cyclosporin A after 2 hours; releases 15% to 40% (for example 20% to 35%, or suitably 25% to 35%) of the cyclosporin A at 4 hours; and releases from about 30% to 70% (for example 55% to 70%) of the cyclosporin A between 4 hours and 12 hours in the two stage dissolution test; and wherein

after oral administration of a single dose of the composition to a human in a fasted state the ratio of the mean concentration of cyclosporin A : the concentration of cyclosporin A metabolites in a faecal sample collected from 12 to 28 hours after dosing the composition is greater than 1:1. Suitably the ratio is from 2:1 to 12:1, for example: from 3:1 to 12:1; from 4:1 to 12:1; from 4:1 to 10:1, from 5:1 to 12:1, from 5:1 to 8:1 or from 9:1 to 12:1. Suitably the cyclosporin metabolite concentration is the total of the AM4N and AM9 metabolite concentrations in the faeces. Suitably the concentrations of cyclosporin and metabolites in a faecal sample collected 12 to 28 hours after orally administering a single dose of 75 mg cyclosporin A In this embodiment, the composition suitably provides a Tmax in whole blood which occurs between

about 3 and about 10 hours, for example 4 to 10 hours or particularly at about 5 hours after oral administration of a single dose of the composition to a human in a fasted state. Suitably in this embodiment the composition provides a mean whole blood $AUC_{0-inf}$ of from about 350 to about 750 ng.hr/ml after oral administration of the composition as a single dose containing 75 mg cyclosporin A to a human in a fasted state, or an $AUC_{0-inf}$ directly proportional thereto for a total dose other than 75mg. Suitably in this embodiment the composition provides a Cmax of from about 75 to about 150 ng/ml after oral administration of the composition as a single dose containing 75 mg of cyclosporin A to a human in a fasted state, or a Cmax directly proportional thereto for a total dose other than 75mg.

In another embodiment there is provided a modified release composition comprising cyclosporin A wherein the composition releases less than 15% (for example 0 to 10%) of the cyclosporin A after 2 hours; releases 10% to 40% (for example 10% to 35%, or suitably 15% to 35%) of the cyclosporin A at 4 hours; and releases from about 30% to 70% (for example 40% to 70%) of the cyclosporin A between 4 hours and 12 hours in the two stage dissolution test; and wherein after oral administration of a single dose of the composition to a human in a fasted state the ratio of the mean concentration of cyclosporin A : the concentration of cyclosporin A metabolites in a faecal sample collected from 12 to 28 hours after dosing the composition is 2:1 to 100:1, from 4:1 to 80 :1, from 20:1 to 40:1, or from 20:1 to 35:1, Suitably in this embodiment the cyclosporin metabolite concentration is the total of the AM1, AM4N and AM9 metabolite concentrations in the faeces. Suitably the concentrations of cyclosporin and metabolites are measured in a faecal sample collected 12 to 28 hours after orally administering a single dose of 75 mg cyclosporin A. Suitably in this embodiment the composition provides a Cmax of from about 20 to about 220 ng/ml, for example from about 20 to about 50 ng/ml. Suitably in this embodiment the composition provides a provides a mean whole blood $AUC_{0-24hr}$ of from about 150 to about 550 ng.hr/ml, for example, about 150 to about 400 ng.hr/ml, about 160 to about 380 ng.hr/ml, about 140 to about 380 ng.hr/ml about 150 to about 350 ng.hr/ml, about 180 to about 380 ng.hr/ml, about 200 to about 400 ng.hr/ml, about 150 to about 380 ng.hr/ml or about 150 to about 300 ng.hr/ml after oral administration of the composition as a single dose containing 75 mg cyclosporin A to a human in a fasted state, or an $AUC_{0-24hr}$ directly proportional thereto for a total dose other than 75mg. Suitably in this embodiment the composition provides a Tmax in whole blood which occurs between about 4 to about 10 hours, for example, about 4.5 to about 6.5 hours or about 5 to about 6 hours after oral administration of a single dose of the composition to a human in a fasted state.

In another embodiment there is provided a modified release composition comprising cyclosporin A wherein the composition releases less than 15% (for example 0 to 10%) of the cyclosporin A after 2 hours; releases 15% to 40% (for example 20% to 35%, or suitably 25% to 35%) of the cyclosporin A at 4 hours; and releases from about 30% to 70% (for example 55% to 70%) of the cyclosporin A between 4 hours and 12 hours in the two stage dissolution test; and wherein 24 hours after oral administration of a single dose of the composition to a male pig weighing about 18kg the ratio of the mean peak cyclosporin A concentration in the gastrointestinal luminal contents : the mean peak cyclosporin A concentration in the colonic tissue is from 3.5:1 to 15:1, for example: from 3.5:1 to 12:1; from 4:1 to 12:1; from 4:1 to 10:1; from 6:1 to 12:1; from 7:1 to 12:1; from 7.5:1 to 10:1; or about 8:1. Suitably in this embodiment the ratios are measured after administration of a single dose containing 2 mg/kg cyclosporin A.

In another embodiment there is provided a modified release composition comprising cyclosporin A wherein the composition releases less than 15% (for example 0 to 10%) of the cyclosporin A after 2 hours; releases 15% to 40% (for example 20% to 35%, or suitably 25% to 35%) of the cyclosporin A at 4 hours; and releases from about 30% to 70% (for example 55% to 70%) of the cyclosporin A between 4 hours and 12 hours in the two stage dissolution test; and wherein 24 hours after oral administration of a single dose of the composition to a male pig weighing about 18kg the ratio of the mean cyclosporin A concentration in the mucosa : the mean cyclosporin A concentration in the muscularis externa of transverse colonic tissue is from 1:1 to 5:1, for example from 2:1 to 4:1, particularly from 2:1 to 3:1. Suitably in this embodiment the ratios are measured after administration of a single dose containing 2 mg/kg cyclosporin A.

In another embodiment there is provided a modified release composition comprising cyclosporin A wherein the composition releases less than 15% (for example 0 to 10%) of the cyclosporin A after 2 hours; releases 10% to 40% (for example 10% to 35%, or suitably 15% to 35%) of the cyclosporin A at 4 hours; and releases from about 30% to 70% (for example 40% to 70%) of the cyclosporin A between 4 hours and 12 hours in the two stage dissolution test; and wherein the ratio of the mean concentration of cyclosporin A present in intracolonic faeces : the mean concentration of cyclosporin A present in colonic tissue in an adult human patient after oral administration of the composition is from 50:1 to about 500:1, optionally from about 80:1 to about 300:1, or optionally about 100:1 to about 250:1;

wherein the concentration of the cyclosporin A is measured in samples of the intracolonic faeces and the colonic tissue taken substantially simultaneously 4 to 6 hours after oral administration of the last dose of a once daily oral dosing regimen of the composition, the dosing regimen comprising once daily oral administration of the composition for seven days. Suitably the once daily oral dosing regimen of the composition provides a single daily dose of 75 mg cyclosporin A. However, other doses may be administered for example 37.5 mg or 150 mg once per day. Optionally the dosage regimen may be a twice daily dosage regimen for seven days, for example 37.5 mg twice a per day, 75 mg twice per day or 150 mg twice per day.

The composition comprises a matrix and cyclosporin A. The matrix may be or comprise a polymer matrix

comprising a polymer selected from a water-permeable polymer, a water-swellable polymer, a water-soluble polymer, a hydrogel forming polymer and a biodegradable polymer. In the present invention the matrix is or comprises a hydrogel forming polymer matrix.

The composition comprises a coating to control or modulate release of the cyclosporin A from the composition (a modified release coating). Advantageously the coating is a polymeric coating to provide delayed and/or sustained release of the cyclosporin form the composition. Suitable such modified release coatings are described in more detail below under "Modified Release Coatings) and include a coating which is or comprises a coating selected from a controlled release polymer, a sustained release polymer, an enteric polymer, a pH independent polymer, a pH dependent polymer and a polymer specifically susceptible to degradation by bacterial enzymes in the gastrointestinal tract, or a combination of two or more such polymers. In a particular embodiment the coating is or comprises a pH-independent polymer, for example a coating which is or comprises ethyl cellulose. In a further specific embodiment the coating is or comprises a pH-independent polymer, for example ethyl cellulose and a water-soluble polysaccharide, for example selected pectin or chitosan, or a combination thereof, particularly pectin.

It has surprisingly been found that compositions comprising cyclosporin A which are coated with a sub-coat which is or comprises a water-soluble cellulose ether or a water-soluble derivative of a cellulose ether prior to coating with a further modified release coating as described above provides advantageous properties. In particular it has been found that the presence of a sub-coating results in a higher total release of cyclosporin A from the composition and/or a greater rate of release of the cyclosporin A compared to a composition which does not have a sub-coat. The greater extent and/or rate of release of cyclosporin A resulting from the presence of a sub-coat provides a composition which has a novel in-vivo pharmacokinetic profile compared to compositions without a sub-coat. In vitro dissolution testing has also shown that the sub-coated compositions according to the invention reduce batch to batch variability in the in-vitro release profile. Accordingly, the sub-coated compositions are expected to demonstrate a reduced inter and/or intra-patient variability compared to non-sub-coated compositions.

Therefore, the composition of the invention comprises a first coating and a second coating outside the first coating, as in claims 1 and 2; and wherein

the first coating is or comprises a water-soluble cellulose ether or a water-soluble derivative of a cellulose ether; and the second coating is or comprises a coating, suitably a polymeric coating, as defined above to control or modulate release of cyclosporin A from the composition. The first and second coatings are suitably coatings on a core comprising cyclosporin A. Accordingly the first coating is a sub-coating as described herein and the second coating is suitably a modified release coating as described herein. The first and second coatings are suitably different polymers.

In this embodiment the first coating suitably is or comprises a water-soluble cellulose ether or a water-soluble ester of a cellulose ether. Particularly the first coating is or comprises a water-soluble cellulose ether. The water-soluble cellulose ether may for example be a water-soluble cellulose ether selected from an alkyl cellulose; a hydroxyalkyl cellulose; a hydroxyalkyl alkyl cellulose; and a carboxyalkyl cellulose. Suitably the first coating is or comprises one or more water-soluble cellulose ethers selected from methyl cellulose, hydroxyethyl cellulose, hydroxypropyl cellulose and hydroxypropylmethyl cellulose and combinations thereof. In particular embodiments the first coating is or comprises a water-soluble hydroxypropylmethyl cellulose. The water-soluble cellulose ethers and water-soluble derivatives thereof (e.g. water-soluble esters of a cellulose ether) present in the first coating (sub-coat) suitably form at least 40%, 50%, 60%, 70%, 80%, 85% or 90% by weight of the dry weight of the first coating.

The coating(s) described above is applied to a core comprising a hydrogel forming polymer and cyclosporin A. Accordingly the composition comprises a core and the coating is outside the core, wherein the core comprises hydrogel forming polymer matrix and cyclosporin A.

In the present invention, the composition comprises a core, a first coating outside the core, wherein the first coating is a water-soluble cellulose ether or a water-soluble derivative thereof as described above; and a second coating outside the first coating, wherein the core comprises a hydrogel forming polymer matrix and cyclosporin A as in claims 1 and 2. Suitably the first coating is or comprises a water-soluble cellulose ether, for example HPMC.

In the present invention the core has the form of a solid colloid, the colloid comprising a continuous phase and a disperse phase, wherein the continuous phase comprises a hydrogel forming polymer matrix. Suitable continuous phases and disperse phases which may be used to form the core are defined in more detail below and in the detailed description of the invention.

Suitably the continuous phase of the core is or comprises a hydrogel forming polymer matrix. In embodiments the hydrogel forming polymer matrix is or comprises a hydrocolloid, a non-hydrocolloid gum or chitosan. In a particular embodiment the a hydrogel forming polymer matrix is or comprises gelatin, agar, a polyethylene glycol, starch, casein, chitosan, soya bean protein, safflower protein, alginates, gellan gum, carrageenan, xanthan gum, phthalated gelatin, succinated gelatin, cellulose phthalate-acetate, oleoresin, polyvinylacetate, hydroxypropyl methyl cellulose, polymerisates of acrylic or methacrylic esters and polyvinylacetate-phthalate and any derivative of any of the foregoing; or a

mixture of two or more such polymers. In a further embodiment the hydrogel forming polymer matrix is or comprises a hydrocolloid selected from carrageenan, gelatin, agar and pectin, or a combination thereof optionally selected from gelatin and agar or a combination thereof. Particularly, the polymer of the hydrogel forming polymer matrix is or comprises gelatin.. In an embodiment, the hydrogel-forming polymer does not comprise a cellulose or a cellulose derivative, e.g. does not comprise a cellulose ether.

In embodiments cyclosporin A is or is comprised in the disperse phase of the core.

The disperse phase may be solid, semi-solid or liquid. In particular, the disperse phase may be liquid. In other particular instances the disperse phase may be semi-solid, for example it may be waxy.

The disperse phase may be a hydrophobic phase, for example a hydrophobic phase which is a solid, a semi-solid or a liquid. Suitably the disperse phase is or comprises a liquid lipid and optionally a solvent miscible therewith, optionally wherein the cyclosporin A is soluble in the disperse phase.

The cyclosporin A may be dissolved in the disperse phase. The cyclosporin A may be suspended in the disperse phase. The disperse phase of the present invention is or comprises cyclosporin A and an oil phase, as in claims 1 and 2.

Accordingly the disperse phase may further comprise a solvent, wherein the solvent is miscible with the disperse phase and water, optionally wherein the solvent is selected from 2-(2-ethoxyethoxy)ethanol and a polyethylene glycol), particularly wherein the solvent is 2-(2-ethoxyethoxy)ethanol. The solvent may also be or comprise a polyethylene glycol) selected from a PEG with an average molecular weight of from about 200 to about 400, for example PEG 200 or PEG 400.

In a particular embodiment the disperse phase is or comprises a liquid lipid and a solvent, wherein the solvent is miscible with the liquid lipid and water, optionally wherein the solvent is selected from 2-(2-ethoxyethoxy)ethanol and a polyethylene glycol), particularly wherein the solvent is 2-(2-ethoxyethoxy)ethanol. In a further embodiment not according to the invention, the disperse phase is or comprises an oil phase comprising a medium chain mono- di- or triglyceride (particularly a medium chain triglyceride), a polyethoxylated castor oil and 2-(ethoxyethoxy)ethanol.

In embodiments of the invention the composition further comprises one or more surfactants as in claims 1 and 2, suitable surfactants are described in more detail in the detailed description of the invention. In the present invention where the composition comprises a core in the form of a solid colloid, the colloid comprising a continuous phase and a disperse phase, wherein the continuous phase comprises a hydrogel forming polymer matrix, the surfactant is present in the disperse phase. Accordingly in one embodiment the core further comprises a surfactant present in at least the continuous phase, the surfactant having an HLB value of from about 1 to about 15. In another embodiment the core further comprises a surfactant present in at least the continuous phase, the surfactant having an HLB value of greater than 10, for example greater than 20, for example from about 10 to about 15. The surfactant of the disperse phase has an HLB value in the range of up to 10.

In one embodiment the composition comprises a core and a coating outside the core, wherein the core is in the form of a solid colloid, the colloid comprising a continuous phase and a disperse phase, wherein the disperse phase is or comprises:

cyclosporin A;
a medium chain mono- di- or tri-glyceride, for example caprylic/capric triglyceride;
a non-ionic surfactant (for example a polyethoxylated castor oil); and
a co-solvent (for example 2-(ethoxyethoxy)ethanol);

and wherein the continuous phase is or comprises:

a hydrogel forming polymer matrix which is or comprises a hydrocolloid selected from carrageenan, gelatin, agar and pectin, or a combination thereof optionally selected from gelatin and agar or a combination thereof, more optionally the polymer of the water-soluble polymer matrix is or comprises gelatin;
optionally a plasticiser, for example a plasticiser selected from glycerin, a polyol for example sorbitol, polyethylene glycol and triethyl citrate or a mixture thereof, particularly sorbitol; and
an anionic surfactant, for example at least one surfactant selected from fatty acid salts, alkyl sulphate salts and bile salts, particularly an alkyl sulphate salt, for example sodium dodecyl sulfate; and wherein the coating on the core is any of the coatings described herein. Suitably the coating comprises a first coating and a second coating outside the first coating; and wherein
the first coating is or comprises a water-soluble cellulose ether or a water-soluble derivative of a cellulose ether as described above (for example the first coating is or comprises a water soluble cellulose ether as described herein, particularly HPMC); and
the second coating is or comprises a coating, suitably a polymeric coating, as defined above to control or modulate release of cyclosporin A from the composition. In the above composition, only the embodiments falling within the scope of the claims are according to the invention.

In embodiments comprising a first coating and a second coating, for example as mentioned in the immediately preceding paragraph, a particular first coating is or comprises hydroxypropylmethyl cellulose and a particular second coating outside the first coating is or comprises a pH independent polymer, for example ethyl cellulose; more particularly the second coating is or comprises ethyl cellulose and optionally a polysaccharide selected from water-soluble and naturally occurring polysaccacharides, for example pectin or another water-soluble naturally occurring polysaccharide. The second coating may therefore contain pectin or another said polysaccharide or it may be substantially free of pectin and other said polysaccharides. There are therefore disclosed second coatings which comprise ethylcellulose as a modified release polymer and which further comprise pectin or another said polysaccharide as well as second coatings which comprise ethylcellulose as a modified release polymer and which do not further comprise pectin or another said polysaccharide.

The core of the composition described above may comprise a hydrogel forming polymer matrix and cyclosporin A and have the characteristics of a core obtained by a process comprising:

(i) dissolving a hydrogel forming polymer in an aqueous liquid to form a solution;
(ii) dissolving or dispersing cyclosporin A in a liquid to form a solution or dispersion (particularly a solution) of the cyclosporin A in the liquid (an oil phase);
(iii) mixing the aqueous solution (i) and the solution or dispersion (ii) to form a colloid;
(iv) ejecting the colloid through a nozzle to form droplets;
(v) causing or allowing the a hydrogel forming polymer to gel or solidify to form a hydrogel-forming polymer matrix; and
(vi) drying the solid.

Suitably the aqueous phase pre-mix (i) further comprises an anionic surfactant, e.g. as described elsewhere herein, for example sodium dodecyl sulfate (SDS).

The solution or dispersion (ii) (oil phase) may be prepared by dissolving or dispersing the cyclosporin A in a suitable hydrophobic liquid. The hydrophobic liquid may be for example, any of the oils or liquid lipids described herein. By way of example the hydrophobic liquid may be, or comprise, saturated or unsaturated fatty acids or a triglyceride, or an ester or ether thereof with polyethylene glycols. A particular oil for the oil phase is or comprises a triglyceride, for example an oil comprising a medium chain triglyceride, optionally wherein the oil comprises a triglyceride of at least one fatty acid selected from fatty acids having 6, 7, 8, 9, 10, 11 or 12 carbon atoms, e.g. $C_8$-$C_{10}$ fatty acids.

In one embodiment the core having the characteristics of a core obtained by the process above is a core comprising a hydrogel forming polymer matrix and a non-aqueous phase dispersed in the a hydrogel forming polymer matrix, wherein the core is or comprises gelatin, SDS, sorbitol, polyethoxylated castor oil, caprylic/capric triglyceride, 2-(ethoxyethoxy)ethanol; wherein the aqueous solution (i) is or comprises gelatin, sorbitol and SDS; and the solution or dispersion (ii) is or comprises polyethoxylated castor oil, caprylic/capric triglyceride, 2-(ethoxyethoxy)ethanol and cyclosporin A.

It has been found that the use of certain surfactants during the manufacture of the compositions is particularly effective in stabilising the colloid (for example emulsion), resulting from the mixing of the mixing the aqueous solution (i) and oil phase (ii) comprising the cyclosporin A. When the colloid comprises an oil-in-water emulsion, it has been found that the presence of a surfactant having an HLB of up to 10 (particularly up to 8) in the oil phase is particularly effective in stabilising the emulsion during the preparation of the composition. The presence of such surfactants has been found to inhibit the formation of cyclosporin A crystals after the formation of the colloid (oil-in-water emulsion). The presence of a surfactant with an HLB of up to 10 maintains the cyclosporin A in solution in the oil phase during manufacture and may also provide favourable release of the cyclosporin A in a solubilised form from the composition following oral administration of the composition to a subject. Compositions comprising a surfactant with an HLB of up to 10 in at least the oil phase may exhibit high rates of release and/or extent of release of cyclosporin A from the composition compared to the use of surfactants with a higher HLB value in the oil phase. The presence of a surfactant with an HLB of up to 10 in at least the oil phase in the composition may inhibit the precipitation of cyclosporin A after release of the cyclosporin from the composition thereby retaining higher levels of cyclosporin in a solubilised form within the GI tract, for example in the colon. The compositions described herein wherein the composition comprises an oil phase and a surfactant having an HLB of up to 10 form an independent aspect of the invention.

Accordingly provided is an orally administered modified release composition comprising a core having the form of a solid colloid, the colloid comprising a continuous phase being or comprising a hydrogel forming polymer and a disperse phase being or comprising cyclosporin A, and an oil phase, the oil phase comprising an oil and one or more surfactants, wherein the oil and the surfactant have an HLB of up to 10, for example an HLB in the range 0-10.

The surfactant present in the oil phase may be any of the surfactants described herein with an HLB value up to 10 The surfactant present in the oil phase may a HLB value selected from: up to 8, up to 7, 1-8, 1-7, 1-5, 2-5, 1-4, 1-3, 1-2, 2-4, 3-4, 5-8, 6-8 and 6-7. Suitably the surfactant present in the oil phase is a non-ionic surfactant having an HLB value above.

The oil may be any of the oils described herein. Suitably the oil is not itself a surfactant. However, certain oils, particularly those derived from natural sources will comprise components which may have surface active properties. For example many triglyceride oils also comprise mono and diglyceride components and may therefore exhibit some surfactant like properties. Accordingly the oil suitably has an HLB value of 0-10, however suitably the oil has an HLB which is close to 0 for example an HLB of 0 to 3, optionally about 0, about 1 or about 2.

In the present invention, the oil and the surfactant present in the oil phase both independently have an HLB value of 0 to 10. The oil may have an HLB of 1-5 and the surfactant may have an HLB of 2-8, optionally 3-7, 2-6, or 3-4. Suitably the oil and the surfactant are different.

The cyclosporin A may be soluble in the oil. The cyclosporin A may be soluble in the surfactant used in the oil phase. Suitably the cyclosporin A is soluble in both the oil and the surfactant. Suitably, substantially all of the cyclosporin A may be dissolved in the oil phase.

The oil phase may further comprises a solvent, wherein the solvent is miscible with the disperse phase and water, optionally wherein the solvent is selected from 2-(2-ethoxyethoxy)ethanol and a polyethylene glycol), particularly wherein the solvent is 2-(2-ethoxyethoxy)ethanol.

The hydrogel forming polymer of the core may be any of the hydrogel forming polymers described herein.

The composition may further comprise additional surfactants in addition to the surfactant present in the oil phase. In particular the continuous phase comprising the hydrogel forming polymer may further comprise one or more surfactants. Surfactants which may be present in the continuous phase are any of the surfactants described herein as being suitable for inclusion in the aqueous (continuous) phase of the composition. Suitably the continuous phase comprises one or more anionic surfactant, for example at least one surfactant selected from fatty acid salts, alkyl sulfates and bile salts, particularly the surfactant in the continuous phase is or comprises an alkyl sulfate, for example sodium dodecyl sulfate.

In the embodiments above the cores having the characteristics of cores obtained by the process and the orally administered modified release core compositions described above are coated. Suitably the core is coated with a first sub-coating, and with a coating to control or modify release, suitably a polymeric coating. The first sub-coating and modified release coatings may be any of the coatings described above and herein to provide the modified release composition according to the invention. The coated core may be obtained by applying to the core the coating, e.g. applying to the core the first and second coatings as described above. Before the coating is applied, the core may be made by a process having steps (i) to (vi) described above. Suitable methods for applying the coating(s) are described below and include applying the coatings by spray coating a coating composition onto the core.

In one particular embodiment the orally administered modified release composition described above provides a mean whole blood $AUC_{0-inf}$ of more than about 900 ng.hr/ml after oral administration of the composition as a single dose containing 75 mg cyclosporin A to a human in a fasted state, or an $AUC_{0-inf}$ directly proportional thereto for a total dose other than 75mg.

In another particular embodiment the an orally administered modified release composition described above provides a mean whole blood $AUC_{0-inf}$ of less than about 900 ng.hr/ml after oral administration of the composition as a single dose containing 75 mg cyclosporin A to a human in a fasted state, or an $AUC_{0-inf}$ directly proportional thereto for a total dose other than 75mg.

In an embodiment the composition is in the form of a minibead. Suitably the largest cross sectional dimension of the minibead is from 0.1 to 5 mm, for example from 1 mm to 5 mm as in the case of from 1mm to 3mm or 1mm to 2mm. The minibead may be spheroidal. The spheroidal minibead may have an aspect ratio of no more than 1.5, for example of from 1.1 to 1.5.

The composition may be formulated into a unit dosage form for oral administration comprising from 0.1 mg to 1000 mg, optionally from 1mg to 500 mg, for example 10mg to 300 mg, or 25 to 250 mg suitably about 25mg, 35 mg, about 75mg, about 180 mg, about 210 mg or about 250 mg cyclosporin A. Suitably the composition is in a multiple minibead unit dosage form selected from soft or hard gel capsules, gelatin capsules, HPMC capsules, compressed tablets or sachets. The minibeads may be as described elsewhere herein.

A further aspect of the invention provides a core prepared according to any of the processes described herein. The processes comprise coating the core, for example by applying the sub-coating and applying the modified release coating using any of the coating processes described herein.

A further aspect of the invention provides the modified release composition comprising cyclosporin A as claimed for use as a medicament. In particular there is provided such a composition for use in the treatment or prevention of a condition of the GIT. In particular embodiments the composition is for use in the treatment or prevention of any of inflammatory bowel diseases, Crohn's disease, ulcerative colitis, graft-versus- host disease, gastrointestinal graft-versus-host disease, myasthenia gravis, irritable bowel syndrome (e.g. with constipation, diarrhea and/or pain symptoms), celiac disease, stomach ulcers, diverticulitis, pouchitis, proctitis, mucositis, radiation-associated enteritis, short bowel disease, chronic diarrhea, gastroenteritis, duodenitis, jejunitis, peptic ulcer, Curling's ulcer, appendicitis, colitis, diverticulosis, endometriosis, colorectal carcinoma, adenocarcinoma, inflammatory disorders, for example diversion colitis, ischemic

colitis, infectious colitis, chemical colitis, microscopic colitis (including collagenous colitis and lymphocytic colitis), atypical colitis, pseudomembraneous colitis, fulminant colitis, autistic enterocolitis, indeterminate colitis, jejunoiletis, ileitis, ileocolitis or granulomatous colitis. The composition may be for use in the prevention of rejection following bone marrow transplantation. The composition may be for use in the prevention or treatment of primary sclerosing cholangitis, familial adenomatous polyposis, or perinanal Crohn's, including perianal fistulae. The composition may be for use in the treatment and prophylaxis of psoriasis, atopic dermatitis, rheumatoid arthritis or nephrotic syndrome. The composition may, for example, be for use in the treatment or prevention of Crohn's disease, ulcerative colitis, celiac disease, graft-versus-host disease, gastrointestinal graft-versus-host disease. Particularly the composition may be for use in the treatment or prevention of ulcerative colitis.

A further aspect provides the modified release composition described herein for use in the manufacture of a medicament for the treatment or prevention of a condition of the GIT. Conditions of the GI tract include those disclosed herein.

A further aspect provides a method for treating or preventing a condition of the GI tract in a subject, preferably a human, in need thereof comprising orally administering to the mammal a therapeutically effective amount of a modified release composition described herein. Conditions of the GI tract which may be treated or prevented and dosages include the conditions and dosages disclosed herein.

Included in this description by reference are the subject matters of the appended claims. The description is therefore to be read together with the claims and features mentioned in the claims are applicable to the subject matters of the description. For example, a feature described in a process claim is applicable also to products mentioned in the description, where the feature is manifested in the product. For example, a feature mentioned in a product claim is applicable also to relevant process subject matters contained in this description. Similarly, a feature mentioned in the description in the context of a process is applicable also to products mentioned in the description, where the feature is manifested in the product. Also, a feature mentioned in the description in the context of a product is applicable also to relevant process subject matters contained in this description.

## BRIEF DESCRIPTION OF THE DRAWINGS

Embodiments of the invention are further described hereinafter with reference to the accompanying drawings, in which:

**Figure 1** shows the mean whole blood cyclosporin A concentration - time profile following administration of 75 mg cyclosporin A of four treatment formulations used in a human PK study described in Example 2 herein. Test 1 refers to the Comparative Formulation; Test 2 is Formulation I (medium coating); Test 3 is Formulation II (high coating); and Neoral™ is a reference orally administered Neoral™ dose. a) is shows the data on a linear scale and b) shows the data on a log-scale.

**Figure 2** shows the relative % of cyclosporin A and the metabolites AMN4 and AM9 in faecal samples for each of the treatments used in a human PK study (see Example 2; faecal analysis). Fast refers to the Comparative Formulation; Medium refers to Formulation I; Slow refers to Formulation II and Neoral™ is the reference treatment with Neoral. The y-axis shows the relative % of cyclosporin A or the cyclosporin A metabolites AM9 and AM4N.

**Figure 3** shows the mean whole blood cyclosporin A (CsA) concentration - time profile in the pig study described in Example 3 comparing oral administration of Formulation III with orally administered Neoral™ and Sandimmun™ (i.v) dosed at 2 mg/kg cyclosporin A. The data represents mean ± Standard Error of the Mean (SEM) (n = 3).

**Figure 4** shows the mean concentration of cyclosporin A (ng/g) in gastrointestinal tissue sections at specific locations along the GI tract taken 24 hours after a single oral dose of 2 mg/kg cyclosporin A for each of Formulation III, Neoral™ and Sandimmun in the pig PK study described in Example 3. Data represents mean ± SEM (n = 3). In the figure DUM is the duodenum, ILM is the Ileum, CAC is the caecum, PCN is the proximal colon, TCN is the transverse colon, DCN is the descending colon and RTM the rectum. LOQ is the limit of quantification.

**Figure 5** shows the concentration of cyclosporin A (ng/g) in the gastrointestinal luminal contents at specific locations along the GI tract taken 24 hours after a single oral dose of 2 mg/kg cyclosporin A for each of Formulation III, Neoral™ and Sandimmun™ in a pig PK study. The data represents mean ± SEM (n = 3). In the figure DUM, ILM, CAS, PCN TCN, RTM and LOQ are as defined in Figure 4.

**Figure 6** shows the concentration of cyclosporin A in the mucosa, submucosa and muscularis externa of a section of transverse colon tissue measured 24 hours after dosing pigs with Formulation III according to the invention,

Neoral™ and Sandimmun at a dose of 2mg/kg cyclosporin A. The data represents mean ± SEM (n = 3).

**Figure 7** shows the transverse tissue layers in the transverse colon.

**Figure 8** shows dissolution profiles from minibeads with HPMC sub-coatings and an ethyl cellulose:pectin (Surelease™:pectin - S:P) modified release outer coating compared to minibeads with an ethyl cellulose:pectin modified release coating but without an HPMC sub-coating. The y-axis shows the % cyclosporin A released against time on the x-axis. E5 refers to Methocel E5 (HPMC coating).

**Figure 9** shows the batch to batch variability of the in-vitro dissolution profile for minibeads with an HPMC subcoat compared to minibeads without an HPMC subcoat for three separate batches of minibeads. Batches with the sub-coat had a 5% weight gain HPMC sub-coat (Opadry) and an 11.5% weight gain ethyl cellulose:pectin (Surelease™:pectin - S:P (98:2)) outer coat. The non-sub-coated batches had no HPMC sub-coat and a 9% weight gain ethyl cellulose:pectin (Surelease™:pectin - S:P (98:2)) outer coat. The y-axis shows the % cyclosporin A released against time on the x-axis. No 1, No 2 and No 3 refers to the first, second and third batches.

## DETAILED DESCRIPTION

Where the composition of the invention is used in the treatment of a patient with a disorder, treatment contemplates any one or more of: maintaining the health of the patient, e.g. the GIT (gastrointestinal tract); restoring or improving the health of the patient, e.g. of the GIT; and delaying the progression of the disorder, e.g. in the GIT. The term "treatment", and the therapies encompassed by this invention, include the following and combinations thereof: (1) reducing the risk of or inhibiting, e.g. delaying, initiation and/or progression of, a state, disorder or condition; (2) preventing, e.g. reducing the risk of, or delaying the appearance of clinical symptoms of a state, disorder or condition developing in a patient (e.g. human or animal) that may be afflicted with or predisposed to the state, disorder or condition but does not yet experience or display clinical or subclinical symptoms of the state, disorder or condition; (3) inhibiting the state, disorder or condition (e.g., arresting, reducing or delaying the development of the disease, or a relapse thereof in case of maintenance treatment, of at least one clinical or subclinical symptom thereof); and/or (4) relieving the condition (e.g. causing regression of the state, disorder or condition or at least one of its clinical or subclinical symptoms). Where the formulation of the invention is used in the treatment of a patient, treatment contemplates any one or more of: maintaining the health of the patient; restoring or improving the health of the patient; and delaying the progression of the disorder. The benefit to a patient to be treated may be either statistically significant or at least perceptible to the patient or to the physician. It will be understood that a medicament will not necessarily produce a clinical effect in every patient to whom it is administered, and this paragraph is to be understood accordingly. The formulations and methods described herein are of use for therapy and/or prophylaxis of disease.

The treatments may include maintenance therapy of patients who have suffered a GI tract disorder and whose condition has subsequently improved, e.g. because of treatment. Such patients may or may not suffer a symptomatic GIT disorder. Maintenance therapy aims to arrest, reduce or delay (re-)occurrence or progression of a GIT disorder.

"Effective amount" means an amount sufficient to achieve the desired treatment e.g. result in the desired therapeutic or prophylactic response. The therapeutic or prophylactic response can be any response that a user (e.g., a clinician) will recognize as an effective response to the therapy. It is further within the skill of one of ordinary skill in the art to determine appropriate treatment duration, appropriate doses, and any potential combination treatments, based upon an evaluation of therapeutic or prophylactic response.

The terms "dry" and "dried" as applied to compositions of the disclosure may each include reference to compositions containing less than 5% free water by weight, e.g. less than 1% free water by weight. Primarily, however, "dry" and "dried" as applied to compositions of the disclosure mean that the hydrogel present in the initial solidified composition has dried sufficiently to form a rigid composition.

Reference to "a % weight gain" of a coating applied to a composition is a % weight gain based upon the dry weight of the coating (i.e. the solids content of the coating applied to the composition).

Ingredients and excipients of the described formulations are suitable for the intended purpose. For example, pharmaceutical formulations comprise pharmaceutically acceptable ingredients.

If not otherwise stated, ingredients, components, excipients etc. of the composition of the invention are suitable for one or more of the intended purposes discussed elsewhere herein.

For the avoidance of doubt, it is hereby stated that the information disclosed earlier in this specification under the heading "Background" is relevant to the invention and is to be read as part of the disclosure of this specification.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses

the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive.

The reader's attention is directed to all papers and documents which are filed concurrently with or previous to this specification in connection with this application and which are open to public inspection with this specification.

The invention provides an orally administered modified release composition comprising cyclosporin A which has, amongst other features, a favourable pharmacokinetic profile compared to orally administered Neoral and/or to intravenously administered cyclosporin A as for example Sandimmun™.

Reference to "Neoral" is to the regulatory approved cyclosporin A formulation consisting of cyclosporin A in DL-$\alpha$-tocopherol, absolute ethanol, propylene glycol, corn oil-mono-di-triglycerides, polyoxyl 40 hydrogenated castor oil. The formulation is a pre-emulsion concentrate which forms a micro-emulsion upon contact with water, for example following oral administration. Neoral™ is currently approved in Europe as the following formulations: Neoral™ Soft Gelatin Capsules 10mg (marketing authorisation number PL 00101/0483; Neoral™ Soft Gelatin Capsules 25mg, 50mg and 100mg (marketing authorisation number: PL 00101/0387-0389; and Neoral™ Oral Solution: (marketing authorisation number PL 00101/0390). NEORAL Soft Gelatin Capsules contain:Ethanol: 11.8% v/v ethanol (9.4% m/v) (10mg, 25mg, 50 mg and 100 mg capsules).Propylene glycol: 10 mg/capsule (10 mg capsules); 25 mg/capsule (25 mg capsules); 50 mg/capsule (50 mg capsules); 100 mg/capsule (100 mg capsules).Macrogolglycerol hydroxystearate/Polyoxyl 40 hydrogenated castor oil: 40.5 mg/capsule (10 mg capsules), 101.25 mg/capsule (25 mg capsules), 202.5 mg/capsule (50 mg capsules), 405.0 mg/capsule (100 mg capsules).

Reference to "Sandimmun" is to the regulatory approved cyclosporin concentrate for infusion comprising ethanol and polyethoxylated castor oil. The product is commercially available as a concentrate containing 50mg/ml cyclosporin; 278 mg/ml ethanol and 650 mg/ml polyethoxylated castor oil and is described (Marketing authorisation number 00101/0153).

The compositions according to the invention provide lower mean whole blood exposure to cyclosporin A following oral administration compared to oral administration of Neoral™ at the same dose of cyclosporin A. The whole blood exposure to cyclosporin A may be determined by measuring the area under the Curve (AUC) of the whole blood cyclosporin A concentration-time curve following administration of a single dose of a composition containing cyclosporin A. The area under the concentration-time curve (AUC), calculated from the start of dosing (t=0) to the last measured concentration (t) is designated to be "$AUC_{0-t}$". Accordingly reference to "$AUC_{0-24hr}$" is the AUC between t= 0 and the last measurement point at 24 hours following administration. The $AUC_{0-t}$ may be calculated using well known methods for example by linear trapezoidal analysis. The area under the concentration-time curve extrapolated to infinity is "$AUC_{0-inf}$". The $AUC_{0-inf}$ is calculated using known methods as:

$$AUC_{0-t} + \frac{C_t}{K_{el}}$$

Where: $C_t$ = the fitted last non-zero concentration for that treatment, $AUC_{0-t}$ is as defined above; and $K_{el}$ = the elimination rate constant. $K_{el}$ is calculated by regression analysis of the natural log (Ln) of whole blood concentration values- time profile.

The term "Cmax" refers to the maximum concentration of cyclosporin in whole blood following administration of a single dose of a composition containing cyclosporin A.

The term "Tmax" refers to the time taken to reach Cmax following oral administration of a composition containing cyclosporin A.

For statistical analysis, the PK data is log-transformed prior to conducting statistical testing. In general, statistical tests are carried out using an analysis of variance procedure (ANOVA) and calculating a 90% confidence interval for each pharmacokinetic parameter (Cmax and AUC).

The measurement and analysis of AUC, Cmax and Tmax are well known in the art and can be carried out using methods and techniques described in further detail in the examples or by reference to standard textbooks such as Remington, The Science and Practice of Pharmacy 22nd edition, or Basic Pharmacokinetics and Pharmacodynamics: An integrated Textbook and Computer Simulations, Sara E. Rosenbaum, 2011 John Wiley& Sons. In all cases references

to AUC, Cmax and Tmax are the mean values measured following administration of a composition containing cyclosporin A to a human in a fasted state. Suitably the subjects used in the PK study are adult humans with weighing about 70 kg (for example 70kg ± 12 kg). Suitably the subjects have a body mass index of about 25 kg/$m^2$ (for example 25 kg/$m^2$ ± 2.5 kg/$m^2$).

In some embodiments the composition of the invention provides an AUC and/or a Cmax value as the AUC or Cmax "following oral administration of a single dose of 75 mg cyclosporin A". It is known that cyclosporin A exhibits an approximately linear pharmacokinetic profile EU HMA's Public Assessment Report on Ciclosporin "Docpharma" soft capsules DK/H/968/1-3/MR, page 4.

Accordingly reference to an "AUC or Cmax of a particular value after oral administration of the composition as a single dose containing 75 mg cyclosporin A to a human in a fasted state, or an AUC or Cmax directly proportional thereto for a total dose other than 75mg" is to be understood to mean that the AUC or Cmax value is directly proportional to mass of the cyclosporin A dose administered. By way of example, if a single dose of 150 mg cyclosporin A were to be administered the corresponding AUC and Cmax values will be approximately twice that obtained with a single dose of 75 mg cyclosporin A. Similarly administration of a single dose of 37.5mg of cyclosporin A would be expected to provide a AUC and Cmax values approximately half those observed following administration of 75 mg cyclosporin A. The dose proportionality for cyclosporin A is applicable over a broad range of dosages of cyclosporin A for example from 0.1 to 1000mg, suitably between about 1 mg and about 500 mg, more particularly between about 5 mg and about 350 mg.

$AUC_{0\text{-}INF}$

As described above the composition provides a mean whole blood $AUC_{0\text{-}inf}$ of less than about 900 ng.hr/ml after oral administration of the composition as a single dose containing 75 mg cyclosporin A to a human in a fasted state, or an $AUC_{0\text{-}inf}$ directly proportional thereto for a total dose other than 75mg. Suitably the $AUC_{0\text{-}inf}$ is less than: about 850, about 800, about 750, about 700, about 650, about 550, about 500, about 450, about 400, about 375, about 350, about 300, about 250 or about 200 ng.hr/ml after oral administration of the composition as a single dose containing 75 mg cyclosporin A to a human in a fasted state, or an $AUC_{0\text{-}inf}$ directly proportional thereto for a total dose other than 75mg.

Suitably the composition provides a mean whole blood $AUC_{0\text{-}inf}$ of from about 200 to about 900 ng.hr/ml after oral administration of the composition as a single dose containing 75 mg cyclosporin A to a human in a fasted state, or an $AUC_{0\text{-}inf}$ directly proportional thereto for a total dose other than 75mg. In embodiments the composition provides a mean whole blood $AUC_{0\text{-}inf}$ of from about 200 to about 900 ng.hr/ml; about 200 to about 850 ng.hr/ml; about 250 to about 800 ng.hr/ml; or about 400 to about 750 ng.hr/ml after oral administration of the composition as a single dose containing 75 mg cyclosporin A to a human in a fasted state, or an $AUC_{0\text{-}inf}$ directly proportional thereto for a total dose other than 75mg.

The composition may provide a mean whole blood $AUC_{0\text{-}inf}$ of from about 120 to about 450 ng.hr/ml after oral administration of the composition as a single dose containing 75 mg cyclosporin A to a human in a fasted state, or an $AUC_{0\text{-}inf}$ directly proportional thereto for a total dose other than 75mg. In embodiments the composition provides a mean whole blood $AUC_{0\text{-}inf}$ of from about 140 to about 350 ng.hr/ml, about 140 to about 420ng.hr/ml about 150 to about 350 ng.hr/ml, about 150 to about 300 ng.hr/ml about 180 to about 350 ng.hr/ml, about 200 to about 400 ng.hr/ml or about 180 to about 320 ng.hr/ml after oral administration of the composition as a single dose containing 75 mg cyclosporin A to a human in a fasted state, or an $AUC_{0\text{-}inf}$ directly proportional thereto for a total dose other than 75mg.

In a particular embodiment the composition provides a mean whole blood $AUC_{0\text{-}inf}$ of from about 350 to about 750 ng.hr/ml after oral administration of the composition as a single dose containing 75 mg cyclosporin A to a human in a fasted state, or an $AUC_{0\text{-}inf}$ directly proportional thereto for a total dose other than 75mg. Accordingly in this embodiment administration of a single dose containing 25 mg cyclosporin A provides a mean whole blood $AUC_{0\text{-}inf}$ of from about 117 to about 250 ng.hr/ml. Administration of a single dose containing 35 mg cyclosporin A provides a mean whole blood $AUC_{0\text{-}inf}$ of from about 163 to about 350 ng.hr/ml. Administration of a single dose containing 50 mg cyclosporin A provides a mean whole blood $AUC_{0\text{-}inf}$ of from about 233 to about 500 ng.hr/ml. Administration of a single dose containing 100 mg cyclosporin A provides a mean whole blood $AUC_{0\text{-}inf}$ of from about 467 to about 1000ng.hr/ml. Administration of a single dose containing 150mg cyclosporin A provides a mean whole blood $AUC_{0\text{-}inf}$ of from about 700 to about 1500 ng.hr/ml. Administration of a single dose containing 180mg cyclosporin A provides a mean whole blood $AUC_{0\text{-}inf}$ of from about 840 to about 1800 ng.hr/ml. Administration of a single dose containing 200 mg cyclosporin A provides a mean whole blood $AUC_{0\text{-}inf}$ of from about 933 to about 2000 ng.hr/ml Administration of a single dose containing 210mg cyclosporin A provides a mean whole blood $AUC_{0\text{-}inf}$ of from about 980 to about 2100 ng.hr/ml. Administration of a single dose containing 250 mg cyclosporin A provides a mean whole blood $AUC_{0\text{-}inf}$ of from about 1167 to about 2500 ng.hr/ml. Other doses of cyclosporin A (for example between 1 and 500 mg) would be expected to show the same linear proportionality in the $AUC_{0\text{-}inf}$ values described herein. The term "about" in relation to $AUC_{0\text{-}inf}$ means ± 50 ng.hr/ml.

$AUC_{0-24HR}$

As described above the composition provides a mean whole blood $AUC_{0-24hr}$ of less than about 850 ng.hr/ml after oral administration of the composition as a single dose containing 75 mg cyclosporin A to a human in a fasted state, or an $AUC_{0-24hr}$ directly proportional thereto for a total dose other than 75mg. In embodiments the composition provides a mean whole blood $AUC_{0-24hr}$ of less than: about 800, about 750, about 700, about 650, about 550, about 500, about 450, about 400, about 350, about 300, about 250, about 200 or about 150ng.hr/ml after oral administration of the composition as a single dose containing 75 mg cyclosporin A to a human in a fasted state, or an $AUC_{0-24hr}$ directly proportional thereto for a total dose other than 75mg.

Suitably the composition provides a mean whole blood $AUC_{0-24hr}$ of from about 150 to about 850 ng.hr/ml after oral administration of the composition as a single dose containing 75 mg cyclosporin A to a human in a fasted state, or an $AUC_{0-24hr}$ directly proportional thereto for a total dose other than 75mg. In embodiments the composition provides a mean whole blood $AUC_{0-24hr}$ of from about 200 to about 800 ng.hr/ml; about 250 to about 800 ng.hr/ml; about 300 to about 750 ng.hr/ml; about 375 to about 700 ng.hr/ml after oral administration of the composition as a single dose containing 75 mg cyclosporin A to a human in a fasted state, or an $AUC_{0-24hr}$ directly proportional thereto for a total dose other than 75mg.

The composition may provide a mean whole blood $AUC_{0-24hr}$ of from about 120 to about 450 ng.hr/ml after oral administration of the composition as a single dose containing 75 mg cyclosporin A to a human in a fasted state, or an $AUC_{0-24hr}$ directly proportional thereto for a total dose other than 75mg. In embodiments the composition provides a mean whole blood $AUC_{0-inf}$ of from about or from about 140 to about 420 ng.hr/ml ,140 to about 350 ng.hr/ml, about 140 to about 400ng.hr/ml about 150 to about 350 ng.hr/ml, about 150 to about 300 ng.hr/ml, about 180 to about 350 ng.hr/ml, about 200 to about 400 ng.hr/ml or about 180 to about 320 ng.hr/ml after oral administration of the composition as a single dose containing 75 mg cyclosporin A to a human in a fasted state, or an $AUC_{0-24hr}$ directly proportional thereto for a total dose other than 75mg.

In a particular embodiment the composition provides a mean whole blood $AUC_{0-24hr}$ of from about 300 to about 700 ng.hr/ml after oral administration of the composition as a single dose containing 75 mg cyclosporin A to a human in a fasted state, or an $AUC_{0-24hr}$ directly proportional thereto for a total dose other than 75mg. Accordingly in this embodiment administration of a single dose containing 25 mg cyclosporin A provides a mean whole blood $AUC_{0-24}$ of about 100 to about 233ng.hr/ml. Administration of a single dose containing 35 mg cyclosporin A provides a mean whole blood $AUC_{0-24hr}$ of about 140 to about 327ng.hr/ml. Administration of a single dose containing 150 mg cyclosporin A provides a mean whole blood $AUC_{0-24hr}$ of about 600 to about 1400 ng.hr/ml. Administration of a single dose containing 180 mg cyclosporin A provides a mean whole blood $AUC_{0-24}$ of about 720 to about 1680 ng.hr/ml. Administration of a single dose containing 210 mg cyclosporin A provides a mean whole blood $AUC_{0-24hr}$ of about 840 to about 1960 ng.hr/ml. Administration of a single dose containing 250 mg cyclosporin A provides a mean whole blood $AUC_{0-24hr}$ of about 1000 to about 2333 ng.hr/ml. Other doses of cyclosporin A (for example between 1 and 500 mg) would be expected to show the same linear proportionality in the $AUC_{0-24hr}$ values described herein. The term "about" in relation to $AUC_{0-24hr}$ means $\pm$ 50 ng.hr/ml.

As described above in one embodiment the composition provides a mean whole blood concentration of cyclosporin A (Cmax) of less than about 250 ng/ml after oral administration of the composition as a single dose containing 75 mg of cyclosporin A to a human in a fasted state, or a Cmax directly proportional thereto for a total dose other than 75mg. In embodiments the composition provides a mean whole blood Cmax of less than: about 225 ng/ml; about 200 ng/ml; about 175 ng/ml; about 150 ng/ml about 125 ng/ml; about 100 ng/ml; about 175 ng/ml; about 150 ng/ml; about 125 ng/ml; about 100 ng/ml; about 75 ng/ml; about 50 ng/ml; or about 20 ng/ml after oral administration of the composition as a single dose containing 75 mg of cyclosporin A to a human in a fasted state, or a Cmax directly proportional thereto for a total dose other than 75mg.

Suitably the composition provides a mean whole blood Cmax of from about 20 to about 220 ng/ml after oral administration of the composition as a single dose containing 75 mg cyclosporin A to a human in a fasted state, or a Cmax directly proportional thereto for a total dose other than 75mg. In embodiments the composition provides a mean whole blood Cmax of from about 30 to about 200 ng.ml; about 50 to about 180 ng/ml; about 70 to 175 ng/ml; about 10 to about 70 ng/ml; about 15 to about 60 ng/ml; about 20 to about 50 ng/ml; about 25 to about 55 ng/ml; about 25 to about 45 ng/ml; or about 25 to about 45 ng/ml after oral administration of the composition as a single dose containing 75 mg cyclosporin A to a human in a fasted state, or a Cmax directly proportional thereto for a total dose other than 75mg.

In a particular embodiment the composition provides a mean whole blood Cmax of from about 75 to about 150 ng/ml after oral administration of the composition as a single dose containing 75 mg cyclosporin A to a human in a fasted state. Accordingly in this embodiment administration of a single dose containing 25 mg cyclosporin A provides a mean whole blood Cmax of from about 25 ng/ml to about 50 ng/ml. Administration of a single dose containing 35 mg cyclosporin A provides a mean whole blood Cmax of from about 35 ng/ml to about 70 ng/ml. Administration of a single dose containing 150 mg cyclosporin A provides a mean whole blood Cmax of from about 150 ng/ml to about 300 ng/ml. Administration

of a single dose containing 180 mg cyclosporin A provides a mean whole blood Cmax of from about 180 ng/ml to about 360 ng/ml. Administration of a single dose containing 210 mg cyclosporin A provides a mean whole blood Cmax of from about 210 ng/ml to about 420 ng/ml. Administration of a single dose containing 250 mg cyclosporin A provides a mean whole blood Cmax of from about 250 ng/ml to about 500 ng/ml. Other doses of cyclosporin A (for example between 1 and 500 mg) would be expected to show the same linear proportionality in the Cmax values described herein. The term "about" in relation to Cmax means ± 10 ng/ml.

Tmax

The time taken to reach maximum whole blood concentration ($T_{max}$) of the cyclosporin A occurs between about 3 and about 10 hours after oral administration of the composition as a single dose. Suitably Tmax occurs between about 4 hours and about 10 hours; between about 4 hours and about 8 hours, between about 4.5 hours and about 8 hours; between about 4 hours and about 6 hours; between about 5 hours and about 10 hours, between about 6 hours and about 8 hours; or between about 4.5 hours and about 5.5 hours; or between about 4.5hours to about 6 hours; or between about 5 hours and about 6 hours.. Suitably Tmax occurs at a time selected from about 3 hours; about 4.5 hours; about 5 hours; about 5.5 hours; about 6 hours; about 6.5 hours; about 7 hours; about 8 hours or about 9 hours after oral administration as a single dose of the composition. Particularly Tmax occurs at about 5 hours after oral administration of a single dose of the composition. The term "about" in relation to Tmax means ± 15 minutes.

As will be realised the Tmax values stated here the times taken to reach Cmax. Accordingly any of the Tmax times here may be applied to any of the Cmax values disclosed herein.

Accordingly in an embodiment the composition provides a mean whole blood Cmax of from about 75 to about 150 ng/ml after oral administration of the composition as a single dose containing 75 mg cyclosporin A to a human in a fasted state, or a Cmax directly proportional thereto for a total dose other than 75mg; and the composition provides a Tmax of from about 4 hours to about 8 hours, suitably from about 4 hours to about 6 hours and particularly at about 5 hours; or about 5.5 hours; or about 6 hours.

In an embodiment the composition provides a mean whole blood Cmax of from about 10 to about 70 ng/ml; about 15 to about 60 ng/ml; about 20 to about 50 ng/ml; about 25 to about 55 ng/ml; about 25 to about 45 ng/ml; or about 25 to about 45 ng/ml after oral administration of the composition as a single dose containing 75 mg cyclosporin A to a human in a fasted state, or a Cmax directly proportional thereto for a total dose other than 75mg; and the composition provides a Tmax of from about 4 hours to about 8 hours, suitably from about 4 hours to about 6 hours and particularly at about 5 hours; or about 5.5 hours; or about 6 hours.

It is also to be understood that the individual AUC values quoted here are applicable to any of the Cmax values disclosed.

Accordingly in one embodiment the composition provides a mean whole blood $AUC_{0-inf}$ of from about 350 to about 750 ng.hr/ml and a mean whole blood Cmax of from about 75 to about 150 ng/ml after oral administration of the composition as a single dose containing 75 mg cyclosporin A to a human in a fasted state, or a $AUC_{0-inf}$ and Cmax directly proportional thereto for a total dose other than 75mg. Suitably the composition provides a Tmax of from about 4 hours to about 8 hours, suitably from about 4 hours to about 6 hours and particularly at about 5 hours; or about 5.5 hours; or about 6 hours.

In one embodiment the composition provides a mean whole blood $AUC_{0-inf}$ of from about 140 to about 420 ng.hr/ml, for example from about 140 to about 350 ng.hr/ml, about 140 to about 400ng.hr/ml about 150 to about 350 ng.hr/ml, about 150 to about 300 ng.hr/ml about 180 to about 350 ng.hr/ml, about 200 to about 400 ng.hr/ml or about 180 to about 320 ng.hr/ml and a mean whole blood Cmax of from about 25 to about 45 ng/ml after oral administration of the composition as a single dose containing 75 mg cyclosporin A to a human in a fasted state, or a $AUC_{0-inf}$ and Cmax directly proportional thereto for a total dose other than 75mg. Suitably the composition provides a Tmax of from about 4 hours to about 8 hours, suitably from about 4 hours to about 6 hours and particularly at about 5 hours; or about 5.5 hours; or about 6 hours.

According to a further embodiment the composition provides a mean whole blood $AUC_{0-24hr}$ of from about 300 to about 700 ng.hr/ml and a mean whole blood Cmax of from about 75 to about 150 ng/ml after oral administration of the composition as a single dose containing 75 mg cyclosporin A to a human in a fasted state, or a $AUC_{0-24hr}$ and Cmax directly proportional thereto for a total dose other than 75mg. Suitably the composition provides a Tmax of from about 4 hours to about 8 hours, suitably from about 4 hours to about 6 hours and particularly at about 5 hours.

In another embodiment the composition provides a mean whole blood $AUC_{0-24hr}$ of from about 140 to about 420 ng.hr/ml ,140 to about 350 ng.hr/ml, about 140 to about 400ng.hr/ml about 150 to about 350 ng.hr/ml, about 150 to about 300 ng.hr/ml, about 180 to about 350 ng.hr/ml, about 200 to about 400 ng.hr/ml or about 180 to about 320 ng.hr/ml and a mean whole blood Cmax of from about 25 to about 45 ng/ml after oral administration of the composition as a single dose containing 75 mg cyclosporin A to a human in a fasted state, or a $AUC_{0-24hr}$ and Cmax directly proportional thereto for a total dose other than 75mg. Suitably the composition provides a Tmax of from about 4 hours to about 8

hours, suitably from about 4 hours to about 6 hours and particularly at about 5 hours.

As mentioned above the AUC and Cmax values of the composition of the invention are lower than those obtained following oral administration of Neoral™. Accordingly the % ratio of the AUC of the composition according to the invention and the AUC of Neoral™ is less than 100% for a given dose of cyclosporin A. Similarly the % ratio of Cmax of the composition according to the invention and the Cmax of Neoral™ is also less than 100%.

The ratios described are the ratios calculated using least-squares means values. Reference to "least square means" is a well-known statistical tool which refers to a linear combination (sum) of the estimated effects (means) from a linear model (see for example http://www.uiweb.uidaho.edu/ag/statprog/sas/workshops/glm/lsmeans.htm )

**$AUC_{0\text{-}inf}$ Ratios**

After oral administration of a single dose of the composition of the invention to a human in a fasted state the % ratio of the mean whole blood $AUC_{0\text{-}inf}$ of the composition : the mean whole blood $AUC_{0\text{-}inf}$ of Neoral™ when administered orally as a single dose of cyclosporin A of the same mass is less than 60% calculated using least-squares means. Suitably the ratio is less than: about 55%; about 50%; about 45%; about 40%; about 35%; about 30%; about 25% or about 20% calculated using least-squares means. Suitably the mean whole blood $AUC_{0\text{-}inf}$ ratio is from about 15% to about 60%, for example a ratio of: about 15% to about 50%; from about 15% to about 45%; from about 20% to about 40%; about 25% to about 40%; about 30% to about 40%; about 10% to about 35%; or about 20% to about 30% calculated using least-squares means. For example about 25% or about 35% calculated using least-squares means. The term "about" in relation to the ratios of AUC values is suitably ± 5%.

**$AUC_{0\text{-}24hr}$ Ratios**

After oral administration of a single dose of the composition of the invention to a human in a fasted state the % ratio of the mean whole blood $AUC_{0\text{-}24hr}$ of the composition : the mean whole blood $AUC_{0\text{-}24hr}$ of Neoral™ when administered orally as a single dose of cyclosporin A of the same mass is less than 55% calculated using least-squares means. Suitably the ratio is less than: about 50%; about 45%; about 40%; about 35%; about 30%; about 25%;about 20% or about 15% calculated using least-squares means. Suitably the mean whole blood $AUC_{0\text{-}24hr}$ ratio is from about 10% to about 50%, for example a ratio of: about 10% to about 45%; from about 15% to about 45%; from about 20% to about 40%; about 30% to about 40%; about 10% to about 30%; or about 25% to about 40% calculated using least-squares means. For example about 20% or about 35% calculated using least-squares means. The term "about" in relation to the ratios of AUC values is suitably ± 5%.

**Cmax Ratios**

After oral administration of a single dose of the composition of the invention to a human in a fasted state the % ratio of the mean whole blood Cmax of the composition : the mean whole blood Cmax of Neoral™ when administered orally as a single dose of cyclosporin A of the same mass is less than 40% calculated using least-squares means. Suitably the ratio of the Cmax values is less than: about 35%; about 30%; about 25%; about 20%; about 15%; about 10% or about 5% calculated using least-squares means. Suitably the Cmax ratio is: from about 5% to about 35%; about 5% to about 25%; about 15% to about 25%; or about 5% to about 15% calculated using least-squares means. For example the Cmax ratio is about 20% or about 10% calculated using least-squares means. The term "about" in relation to the ratios of Cmax values is suitably ± 5%.

The Tmax for the AUC and Cmax ratios is any of the Tmax values described herein. Therefore in embodiments the composition provides a Tmax of from about 3 hours to about 10 hours, for example about 4 hours to about 8 hours, suitably from about 4 hours to about 6 hours and particularly at about 5 hours following oral administration of the composition.

**Cyclosporin A concentration in Faecal Samples**

The composition of the invention releases cyclosporin A (preferably in a solubilised form for example as a solution in an oil droplet or as micelles containing cyclosporin A) in the lower GI tract and particularly the colon. Accordingly, the composition provides high local cyclosporin A concentrations in the luminal contents and further results in absorption of cyclosporin A into the tissue of the GI tract. The luminal and tissue concentration of cyclosporin A following oral administration of a composition of the invention is higher relative to that resulting from oral administration of Neoral. However, as discussed above, the composition according to the invention results in a relatively low systemic blood exposure to the cyclosporin A. Without wishing to be bound by theory it is thought that the dissolution profile of the composition is such that the composition releases relatively low amounts of cyclosporin A during the first four hours

following oral administration. In the first four hours following oral administration to a fasted human, the composition is expected to pass from the stomach and through the duodenum, jejunum and ileum, which are the primary sites for systemic absorption of cyclosporin A in the GI tract, thus systemic absorption of cyclosporin is low compared to Neoral. The majority of the cyclosporin A in the composition is expected to be released in the time period between 4 and 20 hours as the composition passes through the colon. However, in the colon, systemic absorption of cyclosporin A is low compared to that in the small intestine. The composition therefore provides a high concentration of cyclosporin A in the colonic luminal contents. The presence of cyclosporin A (preferably in a solubilised form) in the luminal contents of the colon provides a relatively high drug concentration gradient between the luminal content and the colonic tissue and is expected to drive absorption of the lipophilic cyclosporin into the lamina propria (e.g. the epithelial cells, mucosa and submucosa) where it provides therapeutic effect to conditions affecting the colon. Providing the composition of the invention in the form of minibeads is expected to be particularly advantageous as the individual minibeads will disperse throughout the colon providing release of cyclosporin A throughout the colon. Such minibead forms are described in more detail below under Compositions.

A proxy ex-vivo measure of the relatively high local concentration in-vivo of cyclosporin A in the luminal contents and low systemic exposure resulting from the compositions according to the invention is provided by analysing faecal samples taken from patients that have been orally administered with a composition according to the invention. Analysis of faecal samples taken 12 to 28 hours after dosing the patient for cyclosporin A and the primary metabolites of cyclosporin A show that the composition of the invention has high levels of cyclosporin A and low levels of cyclosporin A metabolites compared to oral administration of an equivalent dose of Neoral. Cyclosporin A is metabolised into a number of metabolites, the main ones being AM1, AM9, and AM4N (Hermann et al.,Journal of Pharmaceutical and Biomedical Analysis,30 (2002) 1263-1276). The faecal samples may be analysed for the concentration of all cyclosporin A metabolites. However, the faecal sample is conveniently analysed for the concentration of the AM4N and AM9 metabolites. The concentration of cyclosporin A and the metabolites may be determined using routine methods, for example by liquid chromatography/mass spectrometry as described in the Examples. Although cyclosporin A is poorly absorbed systemically from the colon, the high concentration of cyclosporin A in the colon lumen provided by the composition of the invention is expected to drive absorption into the colonic tissue (e.g. the epithelial cells, mucosa and sub-mucosa). Although systemic absorbtion from the colon is poor, some metabolism of the cyclosporin A would be expected via, for example locally expressed CYP enzymes accordingly the presence of cyclosporin metabolites in faecal samples provides an indication of local tissue absorbtion of the cyclosporin A. The concentration of metabolites relative to the concentration of cyclosporin A in the faeces is much lower than that observed by highly systemically absorbed formulations such as Neoral.

The composition of the invention provides a ratio of the mean concentration of cyclosporin A : the concentration of cyclosporin A metabolites (for example the sum of the mean AM4N and AM9 metabolite concentrations or the sum of the mean AM1, AM9, and AM4N metabolite concentrations) in a faecal sample collected from 12 to 28 hours after dosing the composition is greater than 1:1, for example, from 2:1 to 12: 1. In another embodiment the ratio is from 2:1 to 100:1. Suitably the ratio of cyclosporin A : cyclosporin A metabolites in the faecal sample is from 4:1 to 80:1, from 4:1 to 35:1; from 20:1 to 30:1, from 10:1 to 90:1, from 10:1 to 80:1, from 20:1 to 60:1, from 30:1 to 50:1, from 3:1 to 12:1; from 4:1 to 12:1, from 5:1 to 12:1, from 5:1 to 8:1 or from 9:1 to 12:1. The ratios above are calculated based upon the relative concentration (ng/g) of cyclosporin metabolites to cyclosporin A in faecal samples. In embodiments the ratio is the ratio of cyclosporin A concentration : the concentration of the AM4N + AM9 metabolite concentrations in the faecal sample. In other embodiments the ratio is the ratio of cyclosporin A concentration : the concentration of the AM4N + AM9 + AM1 metabolite concentrations in the faecal sample. Suitably the metabolite concentration is measured as the sum of the mean concentration of each metabolite present in the faecal sample. In one embodiment "the concentration of cyclosporin A metabolites" refers to the sum of the mean concentrations of the AM4N + AM9 metabolites present in the sample. In another embodiment "the concentration of cyclosporin A metabolites" refers to the sum of the mean concentrations of the AM4N + AM9 + AM1 metabolites present in the sample. Accordingly In one embodiment ratio of the mean concentration of cyclosporin A : the concentration of AM1, AM9, and AM4N metabolites is greater than 1:1, for example, from 2:1 to 100:1, from 4:1 to 80 :1, from 20:1 to 40:1, from 20:1 to 35:1, Suitably the ratio of cyclosporin A: metabolite concentration in the faecal sample is determined after orally administering a single dose of 75 mg cyclosporin A. However, other doses and dose regimens such as twice daily dosing may also be used. As described above the concentrations may be determined in a faecal sample collected 12 to 28 hours after dosing the composition. However, the concentrations may be determined in faeces collected at other time points following oral administration of the composition provided sufficient time has elapsed after oral administration of the composition for transit through the gut such that cyclosporin and its metabolites to be present in the collected faecal sample. It is expected that the ratios of cyclosporin to metabolites measured in a collected faecal samples will be approximately the same irrespective of the specific time point at which the faeces is collected. Accordingly, reference herein to collection of a faecal sample at 12 to 28 hours is not intended to be limiting. Suitably, the ratios of cyclosporin to metabolites are measured in samples of faeces taken from subjects that have been exposed to a regular daily dose of the composition. After a prolonged period of daily dosing it is expected that steady-state concentrations of cyclosporin and metabolites will be achieved and as such there may

be less variability in the measured concentrations of cyclosporin and metabolites in the faeces. Accordingly, the concentration of cyclosporin:metabolites may, for example, be measured in a faecal sample collected 4 to 6 hours after oral administration of the last dose of a once daily oral dosing regimen of the composition, the dosing regimen comprising once daily oral administration of the composition (for example containing 75 mg cyclosporin A) for seven days.

By way of comparison to the compositions according to the invention, the examples herein show that oral administration of Neoral™ results in a ratio of cyclosporin A : Cyclosporin metabolites which is approximately 0.6:1, reflecting the relatively high systemic exposure and relatively low local tissue exposure in the lower GI tract, particularly in the colon.

**Cyclosporin A in Luminal Contents and GI Tissue**

The high concentration of cyclosporin A in the luminal contents of the lower GI tract and the concentration of cyclosporin A in the tissue of the GI tract may be determined by measuring cyclosporin A concentration in luminal content and tissue samples taken at specific points along the GI tract. Such analysis is suitably performed on an animal model, for example a pig as described in the Examples. Alternatively, cyclosporin A concentrations in intracolonic faeces and colonic tissue may also be measured in human patients as described in the protocols described in the Examples. As mentioned herein the composition provides high concentrations of cyclosporin A in the mucosa and sub-mucosa (i.e. the inner tissues) of for example the colon. The cyclosporin A concentration in the colonic tissues may be measured by taking a section of the colonic tissue, separating the layers of tissue (for example the mucosa, sub-mucosa and muscularis externa), and measuring the cyclosporin concentration in each of the respective tissue layers. Measurement of cyclosporin in a section of colonic tissue is illustrated in the Examples herein.

As discussed above, the presence of a high colonic luminal cyclosporin A concentration provided by the composition of the invention is expected to provide a concentration gradient which acts to promote absorption of the cyclosporin A (preferably in a solubilised form) into the lamina propria of the colon, where the main target dysregulated immune cells associates with many inflammatory diseases of the colon predominate. The compositions of the invention therefore provide a local topical treatment of diseased colonic tissue and are expected to be useful in the treatment of conditions such as ulcerative colitis and other inflammatory diseases affecting the at least the colon. In contrast oral administration of Neoral™ provides relatively low luminal concentration of cyclosporin A to the inner colonic tissues. Similarly, as discussed above, intravenous administration of cyclosporin A as Sandimmun™ reduces the metabolism in the intestine and is expected to give similar faecal metabolite concentrations as an orally administered composition according to the invention. However, the IV administration of cyclosporin will result in significantly higher systemic exposure and moreover, relatively high doses of IV cyclosporin may be required to provide therapeutic concentrations of cyclosporin in the colonic tissue compared to oral administration of a composition according to the invention.

The composition of the invention may therefore be expected to provide a therapeutic benefit at lower doses than Neoral and /or Sandimmun™, thus further minimising side effects associated with systemic exposure to cyclosporin A. Some release or cyclosporin A from the composition may occur as the composition passes through the GI tract and release of cyclosporin may not be exclusive to the colon. As such the composition of the invention may provide locally acting cyclosporin A in at least the colon and in other parts of the GI tract, for example the rectum and ileum, the composition may therefore provide therapeutic benefit in the treatment or prevention of conditions affecting not just the colon, but also other parts of the GI tract as described herein.

The concentration of cyclosporin A in the luminal content or in the tissue is suitably determined by measuring the peak concentration of cyclosporin A in the tissue and/or luminal content 24 hours after administering a composition to the pig as described in the Examples. The term "peak concentration" means the highest cyclosporin A concentration in the luminal contents and/or tissue measured along the GI tract of the pig. As will be realised the peak concentration may vary if the analysis is carried out at time points different to 24 hours, particularly with respect to the location along the GI tract where the peak luminal concentration is observed. For example measurement at a time point sooner than 24 hours, for example at 18 hours, would be expected to result in the peak luminal content being higher in the GI tract towards the proximal colon. Similarly measurement at a time point later than 24 hours, for example 28 hours would be expected to reveal the peak luminal content to be lower in the GI tract towards the distal colon or rectum.

Figures 4 and 5 show that in the pig model the composition of the invention provides high levels of cyclosporin A in colonic tissue and the luminal contents of the colon compared to either orally administered Neoral™ or intravenously administered Sandimmun™. The composition of the invention therefore provides cyclosporin A in a form which is absorbed in at least the colonic tissue, for example in the proximal colon, transverse colon or distal colon tissue.

Measurement of cyclosporin concentration in the colonic tissue and intracolonic faeces in humans may be performed as described in the Examples. Suitably samples of colonic tissue are obtained from a patient who has been orally treated with a composition containing cyclosporin by sigmoidoscopy using, for example pinch biopsy forceps, to obtain samples of colonic tissue. Suitably sigmoidoscopy is a flexible sigmoidoscopy. The sigmoidoscopy is preferably carried out in the unprepared bowel (except for air and water) such that the tissue samples obtained replicate as closely

as possible the in-vivo tissue status, which might otherwise be disturbed by extensive bowel preparation. Biopsies are suitably about 5mm in size and ideally at least 5 biopsies are taken approximately 1 cm apart from the subject. Preferably the biopsies are obtained as close to the splenic flexure as possible. Alternatively, biopsies may also be obtained from within the sigmoid colon. Each biopsy should be rinsed with saline, blot dried and then stored at low temperature, suitably at about -70°C, prior to analysis. The tissue samples may be analysed directly for the concentration of cyclosporin A present in the tissue. However, preferably the mucous layer present on the tissue surface is first removed from the sample such that the cyclosporin concentration measured is the concentration of cyclosporin present in the epithelial and musosal tissue. The mucous layer may be removed by washing with a suitable solvent such as of N-acetyl cysteine

Samples of intracolonic faeces are suitably collected from approximately the same location within the colon as the tissue biopsies such that the measurement of cyclosporin concentration in the tissue and intra-colonic faeces represents the concentrations present at approximately the same position within the colon.

The tissue biopsies and intra-colonic faecal samples should be obtained after a sufficient duration of cyclosporin dosing to reach steady-state concentrations in the colon. For example, the biopsies and faecal samples are suitably may be carried out after 7 days of daily oral dosing with the composition. The biopsies and intracolonic faecal samples are suitably obtained simultaneously within 4 to 6 hours after the last dose in the 7 day dosage regimen.

The ratio of the mean concentration of cyclosporin A present in intracolonic faeces : the mean concentration of cyclosporin A present in colonic tissue in an adult human patient after oral administration of the composition is expected to be greater than 30:1, for example, greater than about 40:1 or greater than about 50:1. The mean concentration of cyclosporin A present in intracolonic faeces : the mean concentration of cyclosporin A present in colonic tissue may be about 30:1 to about 500:1, about 50:1 to about 500:1, optionally from about 80:1 to about 300:1, or optionally about 100:1 to about 250:1. In contrast the Examples show that IV administration of Sandimmun results in an intracolonic faecal : tissue ratio of cyclosporin A of about 2:1.

**Dissolution Profile**

The compositions of the invention provide compositions with a specific in-vitro dissolution profile for the release cyclosporin A from the composition. The compositions show minimal release of cyclosporin A in the stomach and upper GI tract such as the duodenum and jejunum and higher release in at least the colon. The in-vivo release may be modelled using a two stage in-vitro dissolution test in which a composition is exposed to 0.1 N HCl for two hours to simulate pH of the gastric environment and is then exposed to pH 6.8 for twenty two hours (by adding a sufficient quantity of 0.2M tribasic sodium phosphate solution containing 2% sodium dodecyl sulfate (SDS)) to simulate pH in the small intestine and lower GI tract.

Reference to "a two stage dissolution test using a USP Apparatus II with a paddle speed of 75 rpm and a dissolution medium temperature of 37°C; wherein for the first 2 hours of the dissolution test the dissolution medium is 750 ml of 0.1 N HCl, and at 2 hours 250 ml of 0.2M tribasic sodium phosphate containing 2% SDS is added to the dissolution medium and the pH is adjusted to pH 6.8" is an in- vitro test carried out in accordance with the USP <711> Dissolution test using Apparatus II (paddle apparatus) operated with a paddle speed of 75 rpm and with the dissolution medium at a temperature of 37°C ± 0.5°C. At the start of the test (t=0) the sample is placed in the acidic dissolution medium. After 2 hours an aliquot of the medium is taken for subsequent analysis and immediately (suitably within 5 minutes) the second stage of the dissolution test is initiated. In the second stage of the dissolution test 250 ml of 0.2M tribasic sodium phosphate containing 2% sodium dodecyl sulfate (SDS) is added to the dissolution medium and the pH is adjusted to 6.8 ± 0.05 using 2N NaOH or 2N HCl as required. Samples of the dissolution medium are taken at time points during the second stage of the test, for example at 4, 6, 12 and 24 hours from the start of the test (i.e. from t=0 at the start of the first stage). The samples are analysed for cyclosporin A dissolved in the medium. The "% released" is the amount of cyclosporin A in solution in the respective dissolution medium at a particular time point relative to the amount of cyclosporin in the composition at the start of the test. The cyclosporin A concentrations in a sample may be measured using standard techniques, such as Reverse Phase HPLC as illustrated in the Examples. References to "two stage dissolution test" herein also refer to this test method.

The in-vitro dissolution profile of the composition according to the invention is described above under the Brief Summary. In further embodiments the composition provides an in-vitro release profile wherein after 4 hours in the two stage dissolution test (i.e. two hours after being placed in the pH 6.8 dissolution medium) the composition releases from about 15% to about 40% of the cyclosporin A. For example in one embodiment the composition releases from about 25% to about 35%, suitably about 30%, cyclosporin A after four hours in the two stage dissolution test. In a further embodiment the composition releases from about 15% to about 25%, suitably about 20% cyclosporin A after four hours in the two stage dissolution test.

In these and other embodiments, the composition suitably results in substantially all of the cyclosporin A being released after 12 hours in the two stage dissolution test. For example the composition releases more than 70%, for example more than 75%, suitably more than 80%, 85% or particularly more than 90% after twelve hours in the two stage

dissolution test.

In these and other embodiments, the modified release composition releases less than 15% (for example 0 to 10%) of the cyclosporin A after 2 hours; releases 10% to 40% (for example 10% to 35%, or suitably 15% to 35%) of the cyclosporin A at 4 hours; and releases from about 30% to 70% (for example 40% to 70%) of the cyclosporin A between 4 hours and 12 hours in the two stage dissolution test.

**Composition**

In the present invention, the modified release composition comprises a matrix and cyclosporin A. The matrix is formed with a hydrogel-forming polymer, and may contain additional excipient(s) to the polymer. The cyclosporin A is contained within the matrix. The cyclosporin A may be in solution or in suspension, or in a combination thereof; however the invention is not limited to formulations comprising a solution or suspension of the cyclosporin A and it includes, for example, cyclosporin A encapsulated in liposomes or cyclodextrin. The matrix may contain inclusions in which the cyclosporin A is comprised; for example, the inclusions may comprise a hydrophobic medium in which the cyclosporin A is dissolved or suspended. Cyclosporin A may therefore be directly dissolved or suspended in the matrix, or it may be dissolved or suspended indirectly in the matrix by way of inclusions in which the active ingredient is dissolved or suspended.

A composition may therefore comprise a matrix-forming polymer, in particular a hydrogel-forming polymer. The matrix of the composition may be or comprise a polymer matrix comprising a polymer selected from a water-permeable polymer, a water-swellable polymer and a biodegradable polymer. In the present invention, the matrix is or comprises a hydrogel-forming polymer described in more detail below.

**Modified Release Coatings**

In the invention modified release of cyclosporin A is achieved wholly or in part through the use of one or more suitable coatings on a core containing a cyclosporin A. The term "modified release" is intended to encompass controlled release, extended (or sustained) release and delayed release or any combination thereof, for example delayed and controlled release of cyclosporin A from the composition following oral administration of the composition. Reference to a coating "to control or modulate release" used herein therefore includes the modified release coatings described in this section and elsewhere

Thus according to the present invention, there is provided a modified release composition comprising a core, wherein the core comprises cyclosporin A, and the core bears a modified release coating outside the core (i.e. is coated) in order to modulate release of cyclosporin A from the core.

The modified release coating may be present in an amount described elsewhere in this specification.

The core is preferably in the form of a minibead as described hereafter in more detail. The modified release coating may be a film or it may be a membrane. The modified release coating, e.g. film or membrane, may serve to delay release until after the stomach; the coat may therefore be an enteric coat. The coat may comprise one or more substances of a polymeric nature (e.g. methacrylates etc; polysaccharides etc as described in more detail below) or combination of more than one such substance, optionally including other excipients, for example, plasticizers. Preferred plasticizers, if they are used, include hydrophilic plasticizers for example triethyl citrate (TEC) which is particularly preferred when using the Eudragit™ family of polymers as coatings as described below. Another preferred plasticiser, described in more detail below in relation to coating with ethyl cellulose, is dibutyl sebacate (DBS). Alternative or additional optionally included excipients are glidants. A glidant is a substance that is added to a powder or other medium to improve its flowability. A typical glidant is talc which is preferred when using the Eudragit™ family of polymers as coatings.

The modified release coating may be applied as described below and may vary as to thickness and density. The amount of modified release coating is defined by the additional weight added to (gained by) the dry composition (e.g. bead) to which it is applied. Weight gain due to the modified release coating is suitably in the range 0.1% to 50%, for example 5% to 40% or from 1% to 18%; or from 1 to 15% of the dry weight of the bead, more preferably in the range 3% to 10% or in the range 5-12% or in the range 7-12%.

The thickness of the modified release coating may be from 1μm to 1mm, but is suitably 1μm to 150 μm, for example for 1 to 100 μm. Suitably the modified release coating provides a coating thickness on the composition of from about 10μm to about 1mm, for example, from about 10 μm to about 500 μm, from about 50 μm to about 1 mm, or about from about 50 μm to about 500 μm. The thickness may therefore be from about 100 μm to about 1 mm, e.g. 100 μm to about 750 μm or about 100 μm to about 500 μm. The thickness may be from about 250 μm to about 1 mm, e.g. about 250 μm to about 750 μm or 250 μm to about 500 μm. . The thickness may be from about 500 μm to about 1 mm, e.g. about 750 μm to about 1 mm or about 500 μm to about 750 μm. The thickness may therefore be from about 10 μm to about 100 μm, e.g. from about 10 μm to about 50 μm or about 50 μm to about 100 μm.

The polymeric coating material of the modified release coating may comprise methacrylic acid co-polymers,

ammonio methacrylate co-polymers, or mixtures thereof. Methacrylic acid co-polymers such as, for example, EUDRAGIT™ S and EUDRAGIT™ L (Evonik) are particularly suitable. These polymers are gastroresistant and enterosoluble polymers. Their polymer films are insoluble in pure water and diluted acids. They may dissolve at higher pHs, depending on their content of carboxylic acid. EUDRAGIT™ S and EUDRAGIT™ L can be used as single components in the polymer coating or in combination in any ratio. By using a combination of the polymers, the polymeric material can exhibit solubility at a variety of pH levels, e.g. between the pHs at which EUDRAGIT™ L and EUDRAGIT™ S are separately soluble. In particular, the coating may be an enteric coating comprising one or more co-polymers described in this paragraph. A particular coating material to be mentioned is Eudragit L 30 D-55.

The trademark "EUDRAGIT" is used hereinafter to refer to methacrylic acid copolymers, in particular those sold under the EUDRAGIT™ by Evonik.

The modified release coating can comprise a polymeric material comprising a major proportion (e.g., greater than 50% of the total polymeric coating content) of at least one pharmaceutically acceptable water-soluble polymer, and optionally a minor proportion (e.g., less than 50% of the total polymeric content) of at least one pharmaceutically acceptable water insoluble polymer. Alternatively, the membrane coating can comprise a polymeric material comprising a major proportion (e.g., greater than 50% of the total polymeric content) of at least one pharmaceutically acceptable water insoluble polymer, and optionally a minor proportion (e.g., less than 50% of the total polymeric content) of at least one pharmaceutically acceptable water-soluble polymer.

Ammonio methacrylate co-polymers such as, for example, EUDRAGIT™ RS and EUDRAGIT™ RL (Evonik) are suitable for use in the present invention. These polymers are insoluble in pure water, dilute acids, buffer solutions, and/or digestive fluids over the entire physiological pH range. The polymers swell in water and digestive fluids independently of pH. In the swollen state, they are then permeable to water and dissolved active agents. The permeability of the polymers depends on the ratio of ethylacrylate (EA), methyl methacrylate (MMA), and trimethylammonioethyl methacrylate chloride (TAMCl) groups in the polymer. For example, those polymers having EA:MMA:TAMCl ratios of 1:2:0.2 (EUDRAGIT™ RL) are more permeable than those with ratios of 1:2:0.1 (EUDRAGIT™ RS). Polymers of EUDRAGIT™ RL are insoluble polymers of high permeability. Polymers of EUDRAGIT™ RS are insoluble films of low permeability. A diffusion-controlled pH-independent polymer in this family is RS 30 D which is a copolymer of ethyl acrylate, methyl methacrylate and a low content of methacrylic acid ester with quaternary ammonium groups present as salts to make the polymer permeable. RS 30 D is available as an aqueous dispersion.

The amino methacrylate co-polymers can be combined in any desired ratio, and the ratio can be modified to modify the rate of drug release. For example, a ratio of EUDRAGIT™ RS: EUDRAGIT™ RL of 90:10 can be used. Alternatively, the ratio of EUDRAGIT™ RS: EUDRAGIT™ RL can be about 100:0 to about 80:20, or about 100:0 to about 90:10, or any ratio in between. In such formulations, the less permeable polymer EUDRAGIT™ RS generally comprises the majority of the polymeric material with the more soluble RL, when it dissolves, permitting gaps to be formed through which solutes can come into contact with the core allowing for the active to escape in a controlled manner.

The amino methacrylate co-polymers can be combined with the methacrylic acid co-polymers within the polymeric material in order to achieve the desired delay in the release of the drug and/or poration of the coating and/or exposure of the composition within the coating to allow egress of drug and/or dissolution of the immobilization or water-soluble polymer matrix. Ratios of ammonio methacrylate co-polymer (e.g., EUDRAGIT™ RS) to methacrylic acid co-polymer in the range of about 99:1 to about 20:80 can be used. The two types of polymers can also be combined into the same polymeric material, or provided as separate coats that are applied to the beads.

Eudragit™ FS 30 D is an anionic aqueous-based acrylic polymeric dispersion consisting of methacrylic acid, methyl acrylate, and methyl methacrylate and is pH sensitive. This polymer contains fewer carboxyl groups and thus dissolves at a higher pH (> 6.5). The advantage of such a system is that it can be easily manufactured on a large scale in a reasonable processing time using conventional powder layering and fluidized bed coating techniques. A further example is EUDRAGIT™ L 30D-55 which is an aqueous dispersion of anionic polymers with methacrylic acid as a functional group. It is available as a 30% aqueous dispersion.

In addition to the EUDRAGIT™ polymers described above, a number of other such copolymers can be used to control drug release. These include methacrylate ester co-polymers such as, for example, the EUDRAGIT™ NE and EUDRAGIT™ NM ranges. Further information on the EUDRAGIT™ polymers can be found in "Chemistry and Application Properties of Polymethacrylate Coating Systems," in Aqueous Polymeric Coatings for Pharmaceutical Dosage Forms, ed. James McGinity, Marcel Dekker Inc., New York, pg 109-114.

Several derivatives of hydroxypropyl methylcellulose (HPMC) also exhibit pH dependent solubility and may be used in the invention for the modified release coating. As examples of such derivatives may be mentioned HPMC esters, for example hydroxypropyl methylcellulose phthalate (HPMCP), which rapidly dissolves in the upper intestinal tract and hydroxypropyl methylcellulose acetate succinate (HPMCAS) in which the presence of ionisable carboxyl groups causes the polymer to solubilize at high pH (> 5.5 for the LF grade and > 6.8 for the HF grade). These polymers are commercially available from Shin-Etsu Chemical Co. Ltd. As with other polymers described herein as useful for delayed release coatings, HPMC and derivatives (e.g. esters) may be combined with other polymers e.g. EUDRAGIT RL-30 D.

Other polymers may be used to provide a modified release coating in particular enteric, or pH-dependent, polymers. Such polymers can include phthalate, butyrate, succinate, and/or mellitate groups. Such polymers include, but are not limited to, cellulose acetate phthalate, cellulose acetate succinate, cellulose hydrogen phthalate, cellulose acetate trimellitate, hydroxypropylmethylcellulose phthalate, hydroxypropylmethyl cellulose acetate succinate, starch acetate phthalate, amylose acetate phthalate, polyvinyl acetate phthalate, and polyvinyl butyrate phthalate.

pH independent Polymer Modified Release Coatings

In a particular embodiment the modified release coating is or comprises a polymeric coating which is pH-independent in its dissolution profile and/or in its ability to release the cyclosporin A incorporated in the compositions of the invention. A pH-independent polymer modified release coating comprises a modified release polymer, optionally a plurality of modified release polymers, and one or more other optional components. The other components may serve to modulate the properties of the formulation Examples have already been given (e.g., Eudragit RS and RL).

Another example of a pH-independent polymeric modified release coating is a coating comprising ethylcellulose. It will be understood that an ethylcellulose composition for use in coating a dosage form may comprise, in addition to ethylcellulose and - in the case of a liquid composition - a liquid vehicle, one or more other components. The other components may serve to modulate the properties of the composition, e.g. stability or the physical properties of the coating such as the flexibility of the film coating. The ethylcellulose may be the sole controlled release polymer in such a composition. The ethylcellulose may be in an amount of at least 50%, at least 60%, at least 70%, at least 80%, at least 90% or at least 95% by weight of the dry weight of a coating composition for use in coating a dosage form. Accordingly, an ethylcellulose coating may include other components in addition to the ethylcellulose. The ethylcellulose may be in an amount of at least 50%, at least 60%, at least 70%, at least 80%, at least 90% or at least 95% by weight of the ethylcellulose coating. Suitably the ethyl cellulose coating further comprises a plasticizer as described below to improve the flexibility of the film and to improve the film-forming properties of the coating composition during application of the coating.

A particular ethylcellulose coating composition which may be applied to the compositions of the invention is a dispersion of ethylcellulose in a sub-micron to micron particle size range, e.g. from about 0.1 to 10 microns in size, homogeneously suspended in water with the aid of an emulsification agent, e.g. ammonium oleate. The ethylcellulose dispersion may optionally and preferably contain a plasticizer. Suitably plasticisers include for example dibutyl sebacate (DBS), diethylphthalate, triethyl citrate, tributyl citrate, triacetin, or medium chain triglycerides. The amount of plasticizer present in the coating composition will vary depending upon the desired properties coating. Typically the plasticizer comprises from 1 to 50%, for example about 8 to about 50% of the combined weight of the plasticizer and ethyl cellulose. Such ethylcellulose dispersions may, for example, be manufactured according to U.S. Pat. No. 4,502,888. One such ethylcellulose dispersion suitable for use in the present invention and available commercially is marketed under the trademark Surelease™, by Colorcon of West Point, Pa. USA. In this marketed product, the ethylcellulose particles are, e.g., blended with oleic acid and a plasticizer, then optionally extruded and melted. The molten plasticized ethylcellulose is then directly emulsified, for example in ammoniated water optionally in a high shear mixing device, e.g. under pressure. Ammonium oleate can be formed in situ, for instance to stabilize and form the dispersion of plasticized ethylcellulose particles. Additional purified water can then be added to achieve the final solids content. See also U.S. Pat. No. 4,123,403.

The trademark "Surelease™" is used hereinafter to refer to ethylcellulose coating materials, for example a dispersion of ethylcellulose in a sub-micron to micron particle size range, e.g. from about 0.1 to 10 microns in size, homogeneously suspended in water with the aid of an emulsification agent, e.g. ammonium oleate. In particular, the trademark "Surelease™" is used herein to refer to the product marketed by Colorcon under the Surelease™ trademark.

Surelease™ dispersion is an example of a combination of film-forming polymer, plasticizer and stabilizers which may be used as a coating to adjust rates of active principle release with reproducible profiles that are relatively insensitive to pH. The principal means of drug release is by diffusion through the Surelease™ dispersion membrane and is directly controlled by film thickness. Use of Surelease™ is particularly preferred and it is possible to increase or decrease the quantity of Surelease™ applied as coating in order to modify the dissolution of the coated composition. Unless otherwise stipulated, use of the term "Surelease" may apply to Surelease E-7-19020, E-7-19030, E-7-19040 or E-7-19050. An ethylcellulose coating formulation, for example Surelease E-7-19020, may comprise ethylcellulose blended with oleic acid and dibutyl sebacate, then extruded and melted. The molten plasticized ethylcellulose is then directly emulsified in ammoniated water in a high shear mixing device under pressure. Ammonium oleate is formed in situ to stabilize and form the dispersion of plasticized ethylcellulose particles. Additional purified water is then added to achieve the final solids content. An ethyl cellulose coating formulation, for example E-7-19030 additionally comprises colloidal anhydrous silica dispersed into the material. An ethyl cellulose coating formulation, for example E-7-19040,may comprise medium chain triglycerides instead of dibutyl sebacate, in particular, in particular in a formulation comprising colloidal anhydrous silica and oleic acid. An ethylcellulose coating formulation, for example Surelease E-7-19050, may derive from blending ethylcellulose with oleic acid before melting and extrusion. The molten plasticized ethylcellulose is then directly emulsified

in ammoniated water in a high shear mixing device under pressure. Ammonium oleate is formed in situ to stabilize and form the dispersion of plasticized ethylcellulose particles. However, formulations that comprise medium chain triglycerides, colloidal anhydrous silica and oleic acid are preferred. Surelease E-7-19040 is particularly preferred.

The invention also contemplates using combinations of ethylcellulose, e.g. a Surelease formulation with other coating components, for example sodium alginate, e.g. sodium alginate available under the trade name Nutrateric™.

In addition to the EUDRAGIT™ and Surelease™ polymers discussed above, where compatible, any combination of coating polymers disclosed herein may be blended to provide additional controlled- or targeted-release profiles.

The delayed release coating can further comprise at least one soluble excipient to increase the permeability of the polymeric material. These soluble excipients can also be referred to or are pore formers. Suitably, the at least one soluble excipient or pore former is selected from among a soluble polymer, a surfactant, an alkali metal salt, an organic acid, a sugar, and a sugar alcohol. Such soluble excipients include, but are not limited to, polyvinyl pyrrolidone, polyvinyl alcohol (PVA), polyethylene glycol, a water-soluble hydroxypropyl methyl cellulose, sodium chloride, surfactants such as, for example, sodium lauryl sulfate and polysorbates, organic acids such as, for example, acetic acid, adipic acid, citric acid, fumaric acid, glutaric acid, malic acid, succinic acid, and tartaric acid, sugars such as, for example, dextrose, fructose, glucose, lactose, and sucrose, sugar alcohols such as, for example, lactitol, maltitol, mannitol, sorbitol, and xylitol, xanthan gum, dextrins, and maltodextrins; and a polysaccharide susceptible of degradation by a bacterial enzyme normally found in the colon, for example polysaccharides include chondroitin sulphate, pectin, dextran, guar gum and amylase, chitosan etc. and derivatives of any of the foregoing. In some embodiments, polyvinyl pyrrolidone, mannitol, and/or polyethylene glycol can be used as soluble excipients.. The at least one soluble excipient can be used in an amount ranging from about 0.1% to about 15% by weight, based on the total dry weight of the polymer coating, for example from about 0.5% to about 10%, about 0.5% to about 5%, about 1% to about 3%, suitably about 2% based on the total dry weight of the polymer coating. The modified release coating may be free from HPMC.

The modifications in the rates of release, such as to create a delay or extension in release, can be achieved in any number of ways. Mechanisms can be dependent or independent of local pH in the intestine, and can also rely on local enzymatic activity to achieve the desired effect. Examples of modified-release formulations are known in the art and are described, for example, in U.S. Pat. Nos. 3,845,770; 3,916,899; 3,536,809; 3,598,123; 4,008,719; 5,674,533; 5,059,595; 5,591,767; 5,120,548; 5,073,543; 5,639,476; 5,354,556; and 5,733,566.

The addition to Surelease™ or other pH-independent polymer substance of a second polymer (e.g. a polysaccharide, especially a heteropolysaccharide) which is susceptible to degradation by colonic bacterial enzymes (and optionally or alternatively by pancreatic or other relevant enzymes), helps to provide targeted release of cyclosporin A to a site or sites within the GI tract where the second polymer is degraded. By varying the amount of second polymer added present in the coating the dissolution profile may be optimized to provide the required release of cyclosporin A from the composition.

In a particular embodiment the modified release coating provides for release of the cyclosporin A in at least the colon. Accordingly in one embodiment the coating comprises a combination of ethylcellulose (preferably a described above, and particularly formulated with an emulsification agent such as, for example, ammonium oleate and/or a plasticizer such as, for example, dibutyl sebacate or medium chain triglycerides) and a polysaccharide susceptible of degradation by a bacterial enzyme normally found in the colon. Such polysaccharides include chondroitin sulfate, pectin, dextran, guar gum and amylase, chitosan etc. and derivatives of any of the foregoing. Chitosan may be used in connection with obtaining a colon-specific release profile; additionally or alternatively, pectin may also be so used.

The use of polysaccharides by themselves for coating purposes has been tried with limited success. Most of the non-starch polysaccharides suffer from the drawback of lacking good film forming properties. Also, they tend to swell in the GI tract and become porous, resulting in the early release of the drug. Even amorphous amylose, which is resistant to degradation by pancreatic alpha amylase but capable of degradation by colonic bacterial enzymes has the disadvantage of swelling in aqueous media although this can be controlled by incorporating insoluble polymers, for example ethyl cellulose and/or acrylates into the amylose film. Amylose however is not water-soluble and although water-insoluble polysaccharides are not excluded, water-soluble polysaccharide (WSP) susceptible of bacterial enzymic degradation brings particularly advantageous results when used as a coating in accordance with this embodiment of the present invention. A particularly preferred polysaccharide in this embodiment of the present invention is pectin. Various kinds of pectin may be used including pectin of different grades available i.e. with differing degrees of methylation (DM), i.e. percentage of carbonyl groups esterified with methanol, for example pectins with a DM of more than 50%, known as High Methoxy (HM) Pectins or Low Methoxy (LM) pectins, or a pectin combination comprising an HM pectin and an LM pectin. It is also possible in this embodiment to use pectins having various degrees of acetylation (DAc). Taken together, the DM and DAc or the degree of substitution is known as Degree of Esterification (DE). pectins of various DE's may be used according to the invention. As an alternative to pectin, sodium alginate may be used as a polysaccharide according to an embodiment of the invention. However, other embodiments may conveniently include amylose and/or starch which contains amylose. Various grades of starch, containing different percentages of amylose may be used including for example Hylon V (National Starch Food Innovation) which has an amylose percentage of 56% or Hylon VII

which has an amylose percentage of 70%. The remaining percentage is amylopectin. The polysaccharides pectin, amylose and sodium alginate are particularly preferred for achieving colon delivery of the cyclosporin A.

It has been found that water-soluble polysaccharide, suitably pectin, can act as a former of pores in the coating otherwise provided by ethylcellulose (preferably Surelease™). By "pores" is not meant shaft-like holes from the surface to the core of the composition, rather areas of weakness or absence of coating occurring stochastically on and within the coating of the invention. As mentioned above, pore formation may also be achieved by inclusion other soluble excipients within the polymer coating to increase the permeability of the polymeric material.

Pore formers have been described before in connection with Surelease™ (see e.g. US 2005/0220878).

According to a particular embodiment of the invention the modified release coating comprises ethyl cellulose, e.g. Surelease™ and a water-soluble polysaccharide (WSP) wherein the proportion of Surelease™ to WSP is ideally in the range 90:10 to 99:1, preferably, 95:5 to 99:1, more preferably 97:3 to 99:1, for example about 98:2 based upon the dry weight of the coating.

Suitably in this embodiment and other embodiments described herein in which Surelease is used as a coating, the weight gain of the composition due to application of the coating comprising ethyl cellulose, (e.g. Surelease™ and WSP) is in the range of from 1 to 30% (for example from: 3% to 25%; 5% to 15%; 8% to 14%; 10% to 12%; 12% to 18%; or 16% to 18%, suitably the weight gain is about 11%, about 11.5%, or about 17%). It is particularly preferred that when a WSP is used in this embodiment, the WSP is pectin. Particularly favoured weight gains using coatings comprising ethyl cellulose e.g. Surelease™ are those in the range 5-12%, 8-12%, 5 to 10%, suitably about 8%, about 8.5%, about 9%, about 9.5%, about 10%, about 10.5%, about 11%, about 11.5% or about 12%.

Accordingly in an embodiment the modified release coating comprises ethyl cellulose and a water-soluble polysaccharide (particularly pectin) wherein the water-soluble polysaccharide (WSP) is present in an amount of 0.1% to about 10% by weight, based on the dry weight of the modified release coating. Suitably the WSP is present in an amount of from about 0.5% to about 10%, for example about 0.5% to about 5%, about 1% to about 3%, suitably about 2% based on the total dry weight of the modified release coating. In this embodiment the WSP is preferably pectin. In this embodiment the modified release composition suitably further comprises a plasticizer. Suitable plasticizers include these described above in relation to Surelease™. Suitably the weight gain of the composition due to application of the modified release coating in this embodiment is in the range of from 1 to 30% (for example from: 3% to 25%;1% to 20%; 5% to 15%; 8% to 14%; 10% to 12%; 12% to 18%; or 16% to 18%, suitably the weight gain is about 11%, about 11.5%, or about 17%).

## Sub-Coating

It has been found that sub-coating a core comprising cyclosporin A prior to applying a modified release coating provides unexpected advantages. The presence of a sub-coating has been found to enhance the dissolution properties of the modified release compositions according to the invention. In particular the presence of a sub-coating has been found to increase the rate of release of cyclosporin A from the composition and also to increase the amount of cyclosporin A released in a set time period compared to compositions prepared without using a sub-coating. These findings are unexpected, because would have been expected that the presence of a sub-coating in addition to a modified release outer coating would act to delay or inhibit release of drug from the composition and, at a given time, for there to be less drug released, because there is a thicker coating present. However, as illustrated in the Examples, contrary to these expectations both the extent and rate of release of cyclosporin A are increased compared to compositions without a sub-coating. Accordingly, modified release composition compositions according to the invention which comprise a sub-coat and a modified release coating outside the sub-coat, provide a unique dissolution profile. The presence of a sub-coating has also been found to reduce batch-to-batch variability, particularly when the core is in the form of a minibead. A sub-coating may therefore also reduce intra- and inter-patient variability as a result of a more consistent dissolution profile. The unique properties of sub-coated compositions according to the invention (particularly the dissolution profile) are expected to contribute to the favourable pharmacokinetic properties of the modified release compositions according to the invention.

Accordingly there is provided a modified release composition comprising cyclosporin A as claimed, wherein the composition further comprises a first coating and a second coating outside the first coating; and wherein

the first coating is or comprises a water-soluble cellulose ether or a water-soluble derivative of a cellulose ether; and the second coating is or comprises a modified release coating.

Suitably the first coating (sub-coating) is applied to a core comprising cyclosporin A. Still more particularly the core comprises a hydrogel forming polymer matrix and cyclosporin A. Such cores are described in more detail below. The first coating is suitably a coating on the outer surface of the core.

The first coating is water-soluble, suitably the first coating is soluble in the environment of the lower GI tract

following oral administration of the composition, for example the first coating is soluble in water at a pH of 5.5 or more, suitably at a pH of 6.5 or more.

The first coating is or comprises a water-soluble cellulose ether or a water-soluble derivative of a cellulose ether or a combination of two or more such materials. The water-soluble derivative of a cellulose ether may be or comprise a water-soluble ester of a cellulose ether. Accordingly in an embodiment the first coating is or comprises a water-soluble cellulose ether or a water-soluble ester of a cellulose ether. Preferably the first coating is or comprises a water-soluble cellulose ether. In some embodiments the first coating may be or comprise a water-soluble derivative of a cellulose ether, for example a water-soluble ester of a cellulose ether.

Suitably the material of the first coating (i.e. the sub-coating) is different to the modified release coating on the composition. For example, where the first coating is or comprises a water-soluble cellulose ether of derivative thereof, the major component(s) (e.g. more than 50%) of the modified release coating is or comprises a different polymer to that of the first coating. Accordingly, the first and second coatings suitably provide two layers of material as part of the composition. It is to be understood that when the modified release coating comprises a mixture of components, minor components of the outer modified release coating may the same as the material of the sub-coating. By way of example, when the first coating is or comprises HPMC and the modified release coating (second coating) comprises ethyl cellulose, the ethyl cellulose may optionally further comprise a minor amount (e.g. less than 50%,40%, 30% or 30%) of the first coating material, HPMC in this example. In such embodiments the sub-coat and the modified release coating are considered to be different.

The water-soluble cellulose ether may be a water-soluble cellulose ether selected from an alkyl cellulose, for example methyl cellulose, ethyl methyl cellulose; a hydroxyalkyl cellulose, for example hydroxyethyl cellulose (available as Cellosize™ and Natrosol™), hydroxypropyl cellulose (available as Klucel™) or hydroxymethyl cellulose; a hydroxyalkyl alkyl cellulose, for example hydroxyethyl methyl cellulose (HEMC), hydroxypropyl methyl cellulose (available as Methocel™, Pharmacoat™, Benecel™) or ethyl hydroxyethyl cellulose (EHEC); and a carboxyalkyl cellulose, for example carboxymethyl cellulose (CMC). Suitably the water-soluble cellulose ether may, for example be selected from methyl cellulose, hydroxyethyl cellulose, hydroxylpropyl cellulose and hydroxypropylmethyl cellulose.

The polymer of the first coat, for example a water-soluble cellulose ether, may be a low viscosity polymer which is suitable for application as a film or coating to the composition. The viscosity of the polymer may be from about 2 to about 60 mPa.s, for example a viscosity of: about 2 to about 20 mPa.s; about to 2 to about 8 mPa.s; more suitably a viscosity of about 4 to about 10 mPa.s, for example about 4 to about 6 mPa.s. Alternatively, the viscosity of the polymer may fall outside any or all of the just-mentioned ranges, for example be above 20 mPa.s. The viscosity of the polymer may be determined by measuring the viscosity of a 2% solution of the polymer in water at 20°C using a Ubbelode viscometer using ASTM standard methods (D1347 and D2363).

The first coat may be or comprise a water-soluble hydroxypropylmethyl cellulose (HPMC or hypromellose). HPMC is prepared by modifying cellulose to substitute hydroxy groups with methoxy and hydroxypropyl groups. Each anhydroglucose unit in the cellulose chain has three hydroxyl groups. The amount of substituent groups on the anhydroglucose units may be expressed as the degree of substitution. If all three hydroxyl groups on each unit are substituted, the degree of substitution is 3. The number of substituent groups on the ring determines the properties of the HPMC. The degree of substitution may also be expressed as the weight % of the methoxy and hydroxypropyl groups present. Suitably the HPMC has from about 19 to about 30% methoxy substitution and from about 7 to about 12% hydroxypropyl substitution. Particularly the HPMC has 25 to 30% methoxy substitution and 7 to 12% hydroxypropyl substitution. Suitably the HPMC is a low viscosity HPMC which is suitable for application as a film or coating to the composition. The viscosity of the HPMC is suitably from about 2 to 60 mPa.s, for example about 2 to about 20 mPa.s, more suitably a viscosity of about 4 to about 10 mPa.s . The viscosity of the HPMC is determined by measuring the viscosity of a 2% solution of the HPMC in water at 20°C using a Ubbelode viscometer using ASTM standard methods (D1347 and D2363). Such HPMC is available as for example Methocel™, for example Methocel™ E, including Methocel™ E5.

When the first coating is or comprises a water-soluble derivative of a cellulose ether, the derivative may, for example be a water-soluble ester of a cellulose ether. Water-soluble esters of cellulose ethers are well known and may comprise esters of a cellulose ether, formed with one or more suitable acylating agent(s). Acylation agents may be, for example suitable acids or acid anhydrides or acyl halides. Accordingly the ester of a cellulose ether may contain a single ester moiety or two or more ester moieties to give a mixed ester. Examples of water-soluble esters of cellulose ethers may be water-soluble phthalate, acetate, succinate, propionate or butyrate esters of a cellulose ether (for example HPMC). Suitably the water-soluble ester of a cellulose ether is a water-soluble phthalate, acetate-succinate, propionate, acetate-propionate or acetate-butyrate ester of a cellulose ether (for example HPMC).

In one embodiment the water-soluble ester of a cellulose ether may be or comprise a water-soluble ester of any of the water-soluble cellulose ethers described above in relation to the sub-coating.

Particular water-soluble esters of cellulose ethers are water-soluble esters of HPMC. Esters of HPMC which are soluble in water at a pH greater than 5.5 may be or comprise hydroxypropyl methylcellulose phthalate (HPMCP), or hydroxypropyl methylcellulose acetate succinate (HPMCAS) in which the presence of ionisable carboxyl groups causes

the polymer to solubilize at high pH (> 5.5 for the LF grade and > 6.8 for the HF grade). These polymers are commercially available from Shin-Etsu Chemical Co. Ltd.

The first coat may comprise or be hypromellose, e.g. it may be made of a mixture of hypromellose, titanium dioxide and polyethylene glycol; the first coat may comprise at least 50wt% hypromellose and optionally at least 75wt% hypromellose, e.g. at least 80wt% or at least 85wt% or 90wt% hypromellose. The coating material used to form the first coat may therefore comprise a dry weight percentage of hypromellose mentioned in the preceding sentence.

If it is desired for the first coat to use a mixture of hypromellose, titanium dioxide and polyethylene glycol, commercial products corresponding to such mixtures are available including Opadry White, a product commercialised by Colorcon. More generally, there may be mentioned various products commercialised under the trade name Opadry and Opadry II. Further non limiting examples include Opadry YS-1-7706-G white, Opadry Yellow 03B92357, Opadry Blue (03B90842). These compositions are available as dry film coating compositions that can be diluted in water shortly before use. Opadry and Opadry II formulations comprise a cellulosic film forming polymer (e.g., HPMC and/or HPC), and may contain polydextrose, maltodextrin, a plasticizer (e.g., triacetin, polyethylene glycol), polysorbate 80, a colorant (e.g., titanium dioxide, one or more dyes or lakes), and/or other suitable film-forming polymers (e.g., acrylate-methacrylate copolymers). Suitable OPADRY or OPADRY II formulations may comprise a plasticizer and one or more of maltodextrin, and polydextrose (including but not limited to a) triacetin and polydextrose or maltodextrin or lactose, or b) polyethylene glycol and polydextrose or maltodextrin). Particularly preferred commercial products are Opadry White (HPMC/HPC-based) and Opadry II White (PVA/PEG-based).

The first coating may also be applied as a simple solution comprising water and the polymer of the first coating. For example when the polymer is HPMC, for example Methocel, the first coating may be applied to the core as an aqueous solution or dispersion of the HPMC. Optionally the coating solution may include other solvents such as an alcohol. Alternatively the coating may be applied as a solution or dispersion in a volatile organic solvent.

Suitably the first coating is present in an amount corresponding to a weight gain of the composition due to the first coating of from 1 to 20% by weight based upon the weight of the composition prior to applying the first coating.

In another embodiment the first coating is present in an amount corresponding to a weight gain due to the first coating in a range selected from 9% to 20%, or particularly 10% to 15% by weight based upon the weight of the composition prior to applying the first coating.

Suitably the first coating (sub-coating) provides a coating thickness on the composition of from about 10μm to about 1mm, for example, from about 10 μm to about 500 μm, from about 50 μm to about 1 mm, or about from about 50 μm to about 500 μm. The thickness may therefore be from about 100 μm to about 1 mm, e.g. 100 μm to about 750 μm or about 100 μm to about 500 μm. The thickness may be from about 250 μm to about 1 mm, e.g. about 250 μm to about 750 μm or 250 μm to about 500 μm. . The thickness may be from about 500 μm to about 1 mm, e.g. about 750 μm to about 1 mm or about 500 μm to about 750 μm. The thickness may therefore be from about 10 μm to about 100 μm, e.g. from about 10 μm to about 50 μm or about 50 μm to about 100 μm.

It is preferred to dry the composition of the invention before the first coat is applied as is described in more detail below in relation to the coating process.

The second coating is outside the first coating and may be any of the modified release coatings described above. In particular, the second coating is or comprises a pH independent polymer modified release coating described above. For example the second coating may be or comprise an enteric coating or a pH independent coating. The second coating may comprise a mixture of polymers including a polymer degradable by bacterial or other enzymes. In a particular embodiment the second coating comprises ethyl cellulose (for example a Surelease™ coating). In another particular embodiment the second coating comprises ethyl cellulose and a water-soluble polysaccharide, in particular one susceptible to degradation by colonic bacteria, suitably pectin. Accordingly the second coating may comprise the Surelease-pectin mixture described above. The second coating may be or comprise ethyl cellulose (eg Surelease™) and a pore former, wherein the pore-former is a water-soluble excipient which acts to enhance the permeability of the coating when placed in an aqueous environment such as that found in the lower GI tract. Suitable pore formers include those described above. Suitable pore formers include those described above. In embodiments the second coating does not comprise a pore former, thus the second coating may not comprise pectin.

Accordingly in one embodiment of the invention there is provided a modified release composition as claimed, comprising a core, a first coating and a second coating outside the first coating; and wherein:

the core comprises a polymer matrix and cyclosporin A;

the first coating is or comprises a water-soluble cellulose ether, particularly hydroxypropylmethyl cellulose;

the second coating is or comprises a modified release coating, particularly a pH independent modified release coating;

the first coating is present in an amount corresponding to a weight gain due to the first coating in a range selected

from : (i) from 1% to 20%; (ii) from 8% to 12%, for example about 10%; (iii) from 4% to 6%, for example about 5%; or (iv) about 6% to about 10%, for example about 7%, about 7.5%, about 8%, about 8.5%, about 9% or about 9.5% by weight based upon the dry weight of the composition prior to applying the first coating; and wherein

the second coating is present in an amount corresponding to a weight gain of the composition due to the second coating selected from (a) from 5 to 40%; (b) from 10% to 12%, for example about 11% or about 11.5%; (c) from 16% to 18%, for example about 17%; or (d) from about 8% to about 12%, for example about 8.5%, about 9%, about 9.5%, about 10%, about 10.5% or about 11% by weight based upon the weight of the composition prior to applying the second coating.

Suitably in this embodiment the core comprises a hydrogel forming polymer matrix and cyclosporin A as described in more detail below.

The first and second coatings in this embodiment are suitably any of the first and second coatings described above or below. Accordingly it is intended that the coatings described in this section may be applied to any of the compositions described herein to provide a modified release coating if required. The coatings are particularly useful to provide a modified release coating to the cores comprising a polymer matrix and cyclosporin A described in this application.

The presence of a sub-coating, amongst other things, increases the amount of cyclosporin A released from the composition during dissolution compared to compositions without a subcoating. Accordingly there is provided a modified release composition comprising cyclosporin A, wherein the composition comprises a first coating (sub coating) and second coating (modified release coating) as described herein; wherein the first coating is present in an amount to provide a % release of the cyclosporin A that is higher than a % release of the cyclosporin A from a corresponding composition without the first coating throughout a time period from 8 hours to 18 hours, when measured in the two stage dissolution test described herein. For example the subcoated composition provides a higher % release in the period between 10 hours and 16 hours, suitably between 10 hours and 14 hours and more particularly at about 10 hours, about 12, hours about 14 hours or about 16 hours in the two stage dissolution test. The sub-coated composition of the invention may, for example, provide 2% or higher, 5% or higher, 10% or higher, 20% or higher, or 30% or higher more cyclosporin A release at a given time point during the two stage dissolution test compared to the same composition without the subcoating. For example 2 to 30%, particularly 2 to 20% more cyclosporin A. In this embodiment it is to be understood that reference to a higher % release refers to an absolute percentage increase. By way of an example if an uncoated composition releases 10% cyclosporin A at a particular time point and the coated composition releases 10% more cyclosporin A, this means that the coated composition releases 20% cyclosporin A at the same time point.

**Outer Barrier or Protective Coating**

The compositions described herein may comprise a protective coating outside the modified release coating. The protective coating may help to protect the modified release coating from damage resulting from, for example formulating the composition into a final dosage form, or during the handling, transport or storage of the composition. The protective coating is suitably applied to the outer surface of the composition. The protective coating may be applied directly to the modified release coating such that the protective coating is in contact with the modified release coating. The protective coating is suitably a water soluble coating which does not adversely affect the release of the cyclosporin A from the composition when in use. Suitably the protective coating is or comprises a water-soluble polymer. The protective coating may comprise a water-soluble cellulosic or PVA film-forming polymer. Suitably the protective coating may be or comprise Opadry (HPMC/HPCbased), Opadry II (PVA/PEG-based) or polyvinyl alcohol-polyethylene glycol graft copolymers (Kollicoat IR) as described herein. The protective coating may be present as a layer of from about 2 to about 50 $\mu$m. Suitably the protective coating is applied to give a weight-gain of from about 0.5 to about 10 %, based upon the weight of the composition prior to applying the protective coating.

**Polymer Matrix Core**

Suitably the composition of the invention comprises a core wherein the core comprises a cyclosporin A phase and a continuous phase or matrix phase to provide mechanical strength. In embodiments the cyclosporin A phase is or comprises a disperse phase within the continuous phase or matrix. The continuous phase or matrix phase suitably comprises a hydrogel-forming polymer matrix. The core may comprise a polymer matrix wherein the matrix-forming polymer is a hydrogel-forming polymer or a combination thereof. The core is coated with a sub-coating and a modified release coating as described above to provide a particular modified release profile.

The cyclosporin A is present as a disperse hydrophobic phase within the hydrogel-forming polymer matrix (continuous phase or aqueous phase) of the core. In the invention, the disperse phase comprises an oil phase and cyclosporin A. The cores may be prepared by dispersing the cyclosporin A phase within the aqueous phase to form a

colloid and then causing the composition to solidify (gel), thereby immobilising the cyclosporin A within the hydrogel-forming polymer matrix.

The core is a solid colloid, the colloid comprising a continuous phase and a disperse phase, wherein the continuous phase is or comprises the hydrogel forming polymer and the disperse phase is or comprises cyclosporin A. The disperse phase comprises an oil phase as a vehicle containing the cyclosporin A, for example containing it as a solution or a suspension. The vehicle is hydrophobic, and may comprise or be a solution of cyclosporin A or a suspension of cyclosporin A. The disperse phase may by way of example be liquid, semi-solid or solid.

The core has the characteristics of a dried colloid in which cyclosporin A is dispersed within the hydrogel-forming polymer matrix. Thus, the core has the form of a dried colloid, the colloid comprising a continuous phase and a disperse phase, wherein the continuous phase is or comprises the hydrogel-forming polymer and the disperse phase is or comprises cyclosporin A. The disperse phase comprises a vehicle containing the cyclosporin A, for example containing it as a solution or a suspension. The vehicle is hydrophobic, and may comprise or be a solution of cyclosporin A or a suspension of cyclosporin A. The disperse phase may by way of example be liquid, semi-solid or solid. The dried colloid may be a dried emulsion, i.e. the core has the characteristics of a dried colloid.

Such cores comprising a hydrogel-forming polymer and a disperse phase comprising cyclosporin A are described in more detail below.

**Continuous Phase Polymer Matrix (Aqueous Phase)**

This section of the specification relating to the polymer matrix recites amounts of constituents in terms of percent by weight of the formulation. In the context of this section of the specification, what is meant is percent by weight of the dry weight of the core, i.e. excluding coating(s).

It will be recalled that the core comprises a matrix or continuous phase and a disperse phase or discontinuous phase. Suitably the continuous phase of the core is or comprises a hydrogel-forming polymer. A hydrogel forming polymer is a polymer capable of forming a hydrogel. A hydrogel may be described as a solid or semi-solid material, which exhibits no flow when at rest, comprising a network (matrix) of hydrophilic polymer chains that span the volume of an aqueous liquid medium.

The core may comprise a hydrogel-forming polymer selected from the group consisting of: gelatin; agar; agarose; pectin; carrageenan; chitosan; alginate; starch; xanthan gum; gum Arabic; guar gum; locust bean gum; polyurethane; polyether polyurethane; cellulose; cellulose ester, cellulose acetate, cellulose triacetate; cross-bonded polyvinyl alcohol; polymers and copolymers of acrylic acid, hydroxyalkyl acrylates, hydroxyethyl acrylate, diethylene glycol monoacrylate, 2-hydroxypropylacrylate, 3-hydroxypropyl acrylate; polymers and copolymers of methacrylic acid, hydroxyethyl methacrylate, diethyleneglycol monomethacrylate, 2-hydroxypropyl methacrylate, 3-hydroxypropyl methacrylate, dipropylene glycol monomethylacrylate; vinylpyrrolidone; acrylamide polymers and copolymers, N-methylacrylamide, N-propylacrylamide; methacrylamide polymers and copolymers, N-isopropylmethacrylamide, N-2-hydroxyethylmethacrylamide; and vinyl pyrrolidone; and combinations thereof. In specific embodiments binary or tertiary etc combinations of any of the above substances are foreseen.

In a further embodiment the hydrogel-forming polymer is selected from the group consisting of gelatin, agar, a polyethylene glycol, starch, casein, chitosan, soya bean protein, safflower protein, alginates, gellan gum, carrageenan, xanthan gum, phthalated gelatin, succinated gelatin, cellulosephthalate-acetate, oleoresin, polyvinylacetate, hydroxypropyl methyl cellulose, polymerisates of acrylic or methacrylic esters and polyvinylacetate-phthalate and any derivative of any of the foregoing; or a mixture of one or more such a hydrogel forming polymers.

The hydrogel-forming polymer may also be referred to as a hydrocolloid i.e. a colloid system wherein the colloid particles are dispersed in water and the quantity of water available allows for the formation of a gel. In embodiments it is preferred to use reversible hydrocolloids preferably thermo-reversible hydrocolloids (e.g. agar, agarose, gelatin etc) as opposed to irreversible (single-state) hydrocolloids. Thermo-reversible hydrocolloids can exist in a gel and sol state, and alternate between states with the addition or elimination of heat. Gelatin, agar and agarose are thermo-reversible, rehydratable colloids and are particularly preferred. Gelatin derivatives such as, for example, succinated or phthalated gelatins are also contemplated. Thermoreversible hydrocolloids which may be used according to the invention, whether individually or in combination, include those derived from natural sources such as, for example, carrageenan (extracted from seaweed), gelatin (extracted from bovine, porcine, fish or vegetal sources), agar (from seaweed), agarose (a polysaccharide obtained from agar) and pectin (extracted from citrus peel, apple and other fruits). A non-animal based hydrocolloid may be preferred for certain applications e.g. administration to vegetarians or to individuals not wishing to ingest animal products for religious or health reasons. In relation to the use of carrageenan, reference is made to US patent application 2006/0029660 A1 (Fonkwe et al). The hydrogel-forming polymer may comprise or be a combination of gelatin with one or more other thermoreversible hydrocolloids, e.g. with one or more other of the thermoreversible hydrocolloids just listed. The hydrogel-forming polymer may comprise or be a combination of gelatin with agar; optionally, at least one further thermoreversible hydrocolloid may be included in the combination, for example one just listed.

Thermo-reversible colloids present a benefit over other hydrogel-forming polymers. Gelation or hardening of thermo-reversible colloids occurs by cooling the colloid, e.g. in a liquid cooling bath or by air flow. Gelation of other hydrogel-forming polymers, which is chemically driven, can lead to leakage of the composition contents into the gelation medium as the hardening process can take time to occur. Leakage of the content of the composition may lead to an inaccurate quantity of the active ingredient within the composition. Thermo-reversible colloids are also known as thermo-reversible gels, and it is therefore preferred that the hydrogel former be a thermoreversible gelling agent.

Another term which may be applied to hydrogel formers which are advantageous is "thermotropic": a thermotropic gelling agent (which the reader will infer is preferred as a hydrogel former used in the invention) is one caused to gel by a change in temperature and such gelling agents are able to gel more rapidly than those whose gelling is chemically induced, e.g. ionotropic gelling agents whose gelling is induced by ions, for example chitosan. In embodiments of the invention, therefore, the hydrogel former is a thermotropic gel-forming polymer or a combination of such polymers.

The manufacture of the composition to prepare a core may require that the hydrogel-forming polymer be present as a solution, which is preferably an aqueous solution. The hydrogel-forming polymer represents between 5% and 50%, preferably between 10% and 30%, still more preferably between 15% and 20% by weight of the aqueous phase during manufacture as described herein. In addition the hydrogel-forming polymer may comprise 8 to 35%, (for example 15-25%, preferably 17-18%) hydro-gel forming polymer; 65%-85% (preferably 77-82%) of water plus, optionally, from 1-5% (preferably 1.5 to 3%) sorbitol. When present surfactant (e.g. anionic surfactant) in the aqueous phase pre-mix may be present in an amount of 0.1 to 5% (preferably 0.5 to 4%) wherein all parts are by weight of the aqueous phase.

In embodiments the composition comprises at least 25%, suitably at least 40% by weight based upon the dry weight of the composition of the hydrogel-forming polymer. For example the hydrogel-forming polymer is present form 25 to 70%, for example 40 to 70% suitably 45 to 60% of the composition, wherein the % is by weight based upon the dry weight of the composition.

In embodiments the hydrogel-forming polymer is a pharmaceutically acceptable polymer.

In certain embodiments the hydrogel-forming polymer is gelatin. In certain embodiments the hydrogel-forming polymer comprises gelatin. In certain embodiments the gelatin comprises at least 40%, for example 40 to 70% suitably 45 to 60% of the composition, wherein the % is by weight based upon the dry weight of the composition.

The hydrogel-forming polymer may optionally comprise a plasticiser for example sorbitol or glycerine, or a combination thereof. In particular one or more plasticisers may be combined with gelatin.

In embodiments in which the hydrogel-forming polymer comprises or is, reference is hereby made to "Bloom strength", a measure of the strength of a gel or gelatin developed in 1925 by O. T. Bloom. The test determines the weight (in grams) needed by a probe (normally with a diameter of 0.5 inch) to deflect the surface of the gel 4 mm without breaking it. The result is expressed in Bloom (grades) and usually ranges between 30 and 300 Bloom. To perform the Bloom test on gelatin, a 6.67% gelatin solution is kept for 17-18 hours at 10°C prior to being tested.

When the hydrogel-forming polymer comprises or is gelatin the bloom strength of the gelatin may be in the range of 125 Bloom to 300 Bloom, 200 Bloom to 300 Bloom and preferably 250 Bloom to 300 Bloom. It should be appreciated that higher bloom strength gelatin can be replaced by lower bloom strength gelatin at higher concentrations.

According to the invention, in embodiments in which the hydrogel-forming polymer matrix comprises or is gelatin, the gelatin may be sourced by a variety of means. For example, it can be obtained by the partial hydrolysis of collagenous material, such as the skin, white connective tissues, or bones of animals. Type A gelatin is derived mainly from porcine skins by acid processing, and exhibits an isoelectric point between pH 7 and pH 9, while Type B gelatin is derived from alkaline processing of bones and animal (bovine) skins and exhibits an isoelectric point between pH 4.7 and pH 5.2. Type A gelatin is somewhat preferred. Gelatin for use in the invention may also be derived from the skin of cold water fish. Blends of Type A and Type B gelatins can be used in the invention to obtain a gelatin with the requisite viscosity and bloom strength characteristics for bead manufacture.

Lower temperature gelatin (or gelatin derivatives or mixtures of gelatins with melting point reducers) or other polymer matrices able to be solidified at lower temperatures (e.g. sodium alginate) may also be used. It is therefore believed that polymer which comprises or is low temperature gelatin is a preferred matrix polymer.

According to the invention, in embodiments in which the polymer comprises or is gelatin, the starting gelatin material is preferably modified before manufacture to produce "soft gelatin" by the addition of a plasticizer or softener to the gelatin to adjust the hardness of the composition of the invention. The addition of plasticizer achieves enhanced softness and flexibility as may be desirable to optimise dissolution and/or further processing such as, for example, coating. Useful plasticizers of the present invention for combination with gelatin or another hydrogel-forming polymer include glycerine (1,2,3-propanetriol), D-sorbitol (D-glucitol), sorbitol BP (a non-crystallizing sorbitol solution) or an aqueous solution of D-sorbitol, sorbitans (e.g. Andidriborb 85/70), mannitol, maltitol, gum arabic, triethyl citrate, tri-n-butyl citrate, dibutylsebacate. Other or similar low molecular weight polyols are also contemplated for example ethylene glycol and propylene glycol. Polyethylene glycol and polypropylene glycol may also be used although these are less preferred. Glycerine and D-sorbitol may be obtained from the Sigma Chemical Company, St. Louis, Mo. USA or Roquette, France. Some active agents and excipients included for other functions may act as plasticisers.

Softeners or plasticisers, if utilized, can be ideally incorporated in a proportion rising to 30%, preferably up to 20% and more preferably up to 10% by dry weight of the composition of the invention, even more preferably between 3 and 8%, and most preferably between 4% and 6%.

Although not essential, the hydrogel-forming polymer matrix may also optionally contain a disintegrant where it is particularly desired to enhance the rate of disintegration of the composition of the invention. Examples of disintegrants which may be included are alginic acid, croscarmellose sodium, crospovidone, low-substituted hydroxypropyl cellulose and sodium starch glycolate.

A crystallisation inhibitor (e.g. approximately 1% by dry weight of the composition) may also be included in the composition of the invention. An example is hydroxy propyl/methyl cellulose (HPC or HPMC, hypromellose etc) which may play other roles such as, for example, emulsifier.

In another embodiment, the hydrogel forming polymer matrix is chitosan which can exist in the form of biogels with or without additives as described e.g. in United States Patent 4,659,700 (Johnson & Johnson); by Kumar Majeti N.V. Ravi in Reactive and Functional Polymers, 46, 1, 2000; and by Paul et al. in ST.P. Pharma Science, 10, 5, 2000. Chitosan derivatives e.g. thiolated entities are also contemplated.

The hydrogel-forming polymer matrix may be a non-hydrocolloid gum. Examples are the cross-linked salts of alginic acid. For example, aqueous solutions of sodium alginate gums extracted from the walls of brown algae have the well-known property of gelling when exposed to diand trivalent cations. A typical divalent cation is calcium, often in the form of aqueous calcium chloride solution. It is preferred in this embodiment that the cross-linking or gelling have arisen through reaction with such a multivalent cation, particularly calcium.

The hydrogel-forming polymer matrix may have a low water content, therefore the composition may have a low water content. As described below during manufacture of a core the disperse phase comprising cyclosporin A is mixed with an aqueous solution of the hydrogel-forming polymer and composition is gelled, for example to provide cores which are minibeads. Suitably the cores are dried following formation to reduce the water content present in the core.

In certain embodiments the composition does not comprise compounds containing a disulphide bond. In embodiments the hydrogel-forming polymer does not comprise compounds containing a disulphide bond.

The hydrogel-forming polymer matrix forming the continuous phase of the core (aqueous phase) may further comprise a surfactant. Surfactants which may be used in the composition are described in the section "surfactants" below.

Surfactant which may be present in the continuous aqueous phase of the core include, for example, a surfactant selected from the group consisting of: cationic; amphoteric (zwitterionic); anionic surfactants, for example perfluoro-octanoate (PFOA or PFO), perfluoro-octanesulfonate (PFOS), sodium dodecyl sulfate (SDS), ammonium lauryl sulfate, and other alkyl sulfate salts, sodium laureth sulfate, also known as sodium lauryl ether sulfate (SLES) and alkyl benzene sulfonate; and non-ionic surfactants for example perfluorocarbons, polyoxyethyleneglycol dodecyl ether (e.g. Brij such as, for example, Brij 35), Myrj (e.g. Myrj 49, 52 or 59), Tween 20 or 80 (also known as Polysorbate) (Brij, Myrj and Tween products are available commercially from Croda), poloxamers which are nonionic triblock copolymers composed of a central hydrophobic chain of polyoxypropylene (polypropylene oxide)) flanked by two hydrophilic chains of polyoxyethylene (polyethylene oxide)), or a combination of the foregoing. In particular, the surfactant may be selected from, or comprise, anionic surfactants and combinations thereof, the anionic surfactants optionally being those mentioned in this paragraph. A particular class of surfactant comprises sulfate salts. A preferred anionic surfactant in the aqueous phase is SDS. Mixtures of further surfactants are also contemplated, e.g. mixtures comprising perfluorocarbons.

In embodiments of the invention, the core comprises a hydrophilic surfactant which, without being bound by theory, is believed at least partially to partition the aqueous phase (polymer matrix).

Such surfactants intended for such inclusion in the aqueous phase of the core are preferably readily diffusing or diffusible surfactants to facilitate manufacturing and processing of the composition of the invention. The surfactant may have an HLB of at least 10 and optionally of at least 15, e.g. at least 20, or at least 30 and optionally of 38-42, e.g. 40. Such surfactants can be of any particular type (ionic, non-ionic, zwitterionic) and may comprise as a proportion of dry weight of the composition from 0.1% to 6%, e.g. 0.1% to 5%. 0.1% to 4% or 0.1% to 3%, more preferably in a proportion of at least 1% and in particular between 1.0 and 4.5 or 5%, ideally within or just outside the 2-4% range, for example from 2 to 3% or approximately 2% or approximately 4%.

Unless otherwise stated or required, all percentages and ratios are by weight.

In one embodiment the anionic surfactant may be an anionic surfactant selected from alkyl sulfates, carboxylates or phospholipids, or combinations thereof.

The physical form of the surfactant at the point of introduction into the aqueous phase during preparation of the core plays a role in the ease of manufacture of the core. As such, although liquid surfactants can be employed, it is preferred to utilize a surfactant which is in solid form (e.g. crystalline, granules or powder) at room temperature, particularly when the aqueous phase comprises gelatin.

In general, mixtures of surfactants can be utilised e.g. to achieve optimum long term stability of the composition of the invention with shorter chain surfactants in general facilitating shorter term stability (an aid to processing) and longer chain surfactants facilitating longer term stability (an aid to shelf life). In some embodiments, shorter chain sur-

factants have up to $C_{10}$ alkyl (e.g. $C_6$-$C_{10}$ alkyl) as the hydrophobic portion of the surfactant whilst longer chain surfactants have $C_{10}$ or higher alkyl (e.g. $C_{10}$-$C_{22}$ alkyl) as the hydrophobic portion of the surfactant. It is envisaged that $C_{10}$ alkyl surfactants may facilitate processing or facilitate prolongation of shelf life, or both, depending on the identity of the other excipients and of the active principle(s). Higher alkyl may in particular implementations of the invention be $C_{11}$-$C_{22}$ or $C_{12}$-$C_{22}$ alkyl, and in some embodiments has a length of no greater than $C_{18}$.

**Disperse Phase**

The polymer matrix of the core described above (i.e. a hydrogel-forming polymer) further comprises a disperse phase. Suitably the disperse phase is or comprises cyclosporin A. In embodiments the disperse phase comprises cyclosporin A. In such embodiments the cyclosporin A is preferably soluble in the disperse phase. Embodiments wherein the cyclosporin A is soluble in the disperse phase are preferred, because such compositions release the cyclosporin A in a solubilised form, which may enhance the therapeutic effect of the drug at the site of release, for example by enhancing absorption into the colonic mucosa.

In embodiments the cyclosporin A is or is comprised in the disperse phase.

Suitably the disperse phase comprises an oil phase and optionally the oil phase is or comprises a liquid lipid and optionally a solvent miscible therewith. Cyclosporin A may be present in the oil phase. Suitably the cyclosporin A is soluble in the oil phase.

The disperse phase may comprise a combination of oils. The liquid lipid may be a short-, medium- or long-chain triglyceride composition, or a combination thereof. A medium chain triglyceride(s) (MCT) comprises one or more triglycerides of at least one fatty acid selected from $C_6$, $C_7$, $C_8$, $C_9$, $C_{10}$, $C_{11}$ and $C_{12}$ fatty acids. It will be understood that commercially available triglyceride, in particular MCT, compositions useful in the invention are mixtures derived from natural products and usually or always contain minor amounts of compounds which are not MCTs; the term "medium chain triglyceride composition" is therefore to be interpreted to include such compositions. A short chain triglyceride(s) comprises one or more triglycerides of at least one short chain fatty acid selected from $C_2$-$C_5$ fatty acids. A long chain triglyceride(s) comprises one or more triglycerides of at least one long chain fatty acid having at least 13 carbon atoms.

The liquid lipid may be or comprise triglycerides and/or diglycerides. Such glycerides may be selected from medium chain glycerides or short chain triglycerides or a combination thereof.

The liquid lipid may be a caprylic/caprictriglyceride, i.e. a caprylic/capric triglyceride composition (which it will be understood may contain minor amounts of compounds which are not caprylic/capric triglycerides).

Said solvent which is optionally included in an oil phase may be miscible with both the liquid lipid and with water. Examples of suitable solvents are 2-(2-ethoxyethoxy)ethanol available commercially under trade names Carbitol™, Carbitol cellosolve, Transcutol™, Dioxitol™, Poly-solv DE™, and Dowanal DE™; or the purer Transcutol™ HP (99.9). Transcutol P or HP, which are available commercially from Gattefosse, are preferred. Another possible co-solvent is polyethylene glycol). PEGs of molecular weight 190-210 (e.g. PEG 200) or 380-420 (e.g. PEG 400) are preferred in this embodiment. Suitable PEGs can be obtained commercially under the name "Carbowax" manufactured by Union Carbide Corporation although many alternative manufacturers or suppliers are possible.

The disperse phase, may represent from 10-85% by dry weight of the core.

As discussed above the disperse phase comprises an oil phase comprising any pharmaceutically suitable oil, e.g. a liquid lipid. The oil phase may be present as oil drops. In terms of dry weight of the core, the oil phase may comprise a proportion from 10% to 85%, e.g. 15% to 50%, for example 20% to 30% or from 35% to 45%. The term "oil" means any substance that is wholly or partially liquid at ambient temperature or close-to-ambient temperature e.g. between 10°C and 40°C or between 15°C and 35°C, and which is hydrophobic but soluble in at least one organic solvent. Oils include vegetable oils (e.g. neem oil) and petrochemical oils.

Oils which may be included in the oil phase include poly-unsaturated fatty acids such as, for example, omega-3 oils for example eicosapentanoic acid (EPA), docosohexaenoic acid (DHA), alpha-linoleic acid (ALA), conjugated linoleic acid (CLA). Preferably ultrapure EPA, DHA or ALA or CLA are used e.g. purity up to or above 98%. Omega oils may be sourced e.g. from any appropriate plant e.g. sacha inchi. Such oils may be used singly e.g. EPA or DHA or ALA or CLA or in any combination. Combinations of such components including binary, tertiary etc combinations in any ratio are also contemplated e.g. a binary mixture of EPA and DHA in a ratio of 1:5 available commercially under the trade name Epax 6000. The oil part of the oil phase may comprise or be an oil mentioned in this paragraph.

Oils which may be included in the oil phase are particularly natural triglyceride-based oils which include olive oil, sesame oil, coconut oil, palm kernel oil, neem oil. The oil may be or may comprise saturated coconut and palm kernel oil-derived caprylic and capric fatty acids and glycerin e.g. as supplied under the trade name Miglyol™ a range of which are available and from which one or more components of the oil phase of the invention may be selected including Miglyol™ 810, 812 (caprylic/capric triglyceride); Miglyol™ 818: (caprylic/capric/linoleictriglyceride); Miglyol™ 829: (caprylic/capric/succinic triglyceride; Miglyol™ 840: (propylene glycol dicaprylate/dicaprate). Note that Miglyol™ 810/812 are MCT compositions which differ only in $C_8$/$C_{10}$-ratio and because of its low $C_{10}$-content, the viscosity and cloud point of

Miglyol™ 810 are lower. The Miglyol™ range is available commercially from Sasol Industries. As noted above, oils which may be included in the oil phase need not necessarily be liquid or fully liquid at room temperature. Waxy-type oils are also possible: these are liquid at manufacturing temperatures but solid or semi-solid at normal ambient temperatures. The oil part of the oil phase may comprise or be an oil mentioned in this paragraph.

Alternative or additional oils which may be included in the oil phase according to the invention are other medium chain triglyceride compositions such as for example *Labrafac™ Lipophile* manufactured by Gattefosse in particular product number WL1349. Miglyol™ 810, 812 are also medium chain triglyceride compositions.

Accordingly the oil phase may be or comprise medium chain mono-di- or tri -glycerides.

The medium chain glyceride(s) (e.g. mono- di- or tri- glyceride(s)) mentioned herein are those which comprise one or more triglycerides of at least one fatty acid selected from fatty acids having 6, 7, 8, 9, 10, 11 or 12 carbon atoms, e.g. $C_8$-$C_{10}$ fatty acids.

The oil phase may further comprise one or more surfactants as described below under the section "surfactants". For example the oil phase may comprise one or more non-ionic or amphoteric surfactants. Particularly the oil phase may comprise a one or more non-ionic surfactant listed under "surfactants" below. The presence of a surfactant in the oil phase may also provide enhanced solubilisation of the cyclosporin A (i.e. act as a solubiliser) and/or may provide enhance emulsification when the disperse phase is mixed with the aqueous polymer phase during preparation of the core (i.e act as an emulsifier).

Surfactant in an oil phase may for example include polyethoxylated castor oils (polyethylene glycol ethers) which can be prepared by reacting ethylene oxide with castor oil. Commercial preparations may also be used as a surfactant/solubilizer e.g. those commercial preparations which contain minor components such as, for example, polyethyelene glycol esters of ricinoleic acid, polyethyelene glycols and polyethyelene glycol ethers of glycerol. A preferred example is Kolliphor™ EL, previously known as Cremophor™ EL. Another surfactant which may be present in an oil phase is for example a phospholipid.

In the present invention, the surfactant in the oil phase may be or comprise a non-ionic surfactant selected from sorbitan-based surfactants, PEG-fatty acids, glyceryl fatty acids, or poloxamers.

Within embodiments, the HLB of the oil is in the range 0-10 (optionally 1-8, e.g. 1-6 and sometimes 1-5).

In the present invention, the oil phase comprises an oil and a surfactant (suitably a nonionic surfactant) wherein the oil and the surfactant both have an HLB in the range 0-10. For example the oil has an HLB of 1-5, for example 1 to 4 or 1-2 and the surfactant has an HLB 2 -8, for example 3-7, 2-6, or 3-4).

Suitable oils with a low HLB (HLB less than 10) include medium chain triglycerides, linoleoyl macrogolglycerides (polyoxylglycerides), caprylocaproyl macrogolglycerides and caprylic/capric triglyceride. In terms of commercial products, particularly preferred oils in the lower HLB range are *Labrafac™ Lipophile (e.g.* 1349 *WL),* Captex 355 and Miglyol 810.

One example of a surfactant with high HLB includes polyethoxylated castor oils (polyethylene glycol ethers), for example the commercial product Kolliphor™ EL.

An oil phase not according to the invention may comprise a surfactant of high HLB and an oil of low HLB in a ratio of 1-4:1 by weight, e.g. 1.2-3.0:1 by weight, preferably 1.5-2.5:1 by weight and most preferably 1.8-2.2:1 by weight (high HLB: low HLB) that advantageously stabilizes the emulsion before and after immobilization of the oil droplets in the aqueous phase during the preparation of the cores. In this context "stabilize" means in particular that the embodiment improves dissolution and/or dispersion of the composition in vitro. In this embodiment "high" HLB is generally intended above 10, preferably from 10-14, more preferably between 12 and 13. By "low" HLB is generally intended below 10, preferably in the range 1 to 4, more preferably 1 to 2.

It is to be understood that the oil phase in the embodiments above may further comprise or more solvents (co-solvents), for example 2-(2-ethoxyethoxy)ethanol or low molecular weight PEG as mentioned above.

A particular oil phase not according to the invention comprises an oil (low HLB), a high HLB non-ionic surfactant and a co-solvent. For example the following three commercial products: Transcutol P or HP (as co-solvent), Miglyol 810 (as oil) and Kolliphor™ EL (surfactant). Miglyol has a low HLB and Kolliphor™ EL has a high HLB. An oil phase not according to the invention may therefore comprise or consist of a combination of the following and optionally a pharmaceutically active ingredient: 2-ethoxyethanol, an MCT and particularly a caprylic/capric triglyceride formulation, and a polyethoxylated castor oil.

The cyclosporin A is preferably soluble in the oil phase. As discussed below in relation to preparation of the core, the cyclosporin A is suitably dissolved in the oil phase and the oil phase in mixed with an aqueous phase comprising the hydrogel forming polymer.

The disperse phase (oil phase) may be or comprise a glyceride composition, optionally wherein the disperse phase is or comprises a fatty acid monoglyceride, diglyceride or triglyceride or a combination thereof, or the disperse phase is or comprises a caprylic/capric triglyceride composition.

The disperse phase comprises a surfactant. Suitable surfactants include surfactants comprising a hydrophobic chain and a hydrophilic chain can be selected from the group consisting of: macrogol esters; macrogol ethers; diblock copolymers; triblock copolymers; and amphiphilic polymers. Macrogol esters which are suitable for use in the present

invention are macrogol esters of fatty acids having at least 6 carbon atoms and optionally at least 10 carbon atoms, and particularly of at least 12 carbon atoms; some fatty acids have no more than 22 carbon atoms, for example $C_{10}$-$C_{20}$, $C_{12}$-$C_{20}$ or $C_{15}$-$C_{20}$ fatty acids. The fatty acids may be saturated or unsaturated but are in particular saturated. To be mentioned are macrogol 25 cetostearyl ether (Cremophor™ A25); macrogol 6 cetostearyl ether (Cremophor™ A6); macrogol glycerol ricinoleate 35 (Kolliphor™ EL); macrogol-glycerol hydroxystearate 40 (Kolliphor™ RH 40); macrogol-15-hydroxystearate (polyoxyl-15-hydroxystearate US Pharmacopoeia and National Formulary, European Pharmacopoeia, e.g. Kolliphor HS 15, previously known as Solutol™ HS 15). Examples of macrogol ethers which are suitable for use in the present invention are macrogol ethers of fatty alcohols having at least 6 carbon atoms and optionally at least 10 carbon atoms, and particularly of at least 12 carbon atoms; some fatty alcohols have no more than 22 carbon atoms, for example $C_{10}$-$C_{20}$, $C_{12}$-$C_{20}$ or $C_{15}$-$C_{20}$ fatty alcohols. The fatty alcohols may be saturate or unsaturated but are in one embodiment saturated. Kolliphor™ HS 15 is obtained by reacting 15 moles of ethylene oxide with 1 mole of 12-hydroxy stearic acid; the surfactant may therefore be or comprise a surfactant obtainable by (having the characteristics of a surfactant obtained by) reacting 10-25 moles of ethylene oxide with 1 mole of 12-hydroxy stearic acid; the number of moles of ethylene oxide may , from 12-25 and optionally from 15-20, e.g. 15 or 20. Only those with an HLB up to 10 are according to the invention.

Kolliphor™ HS 15 consists of polyglycol mono- and di-esters of 12-hydroxystearic acid and about 30% of free polyethylene glycol. The main components of the ester part have the following chemical structures:

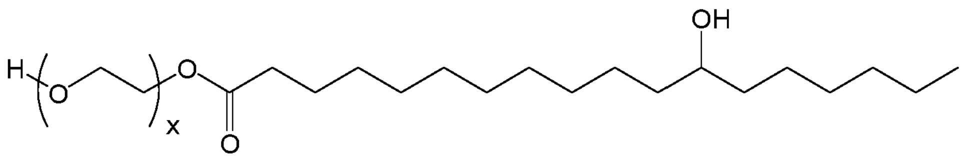

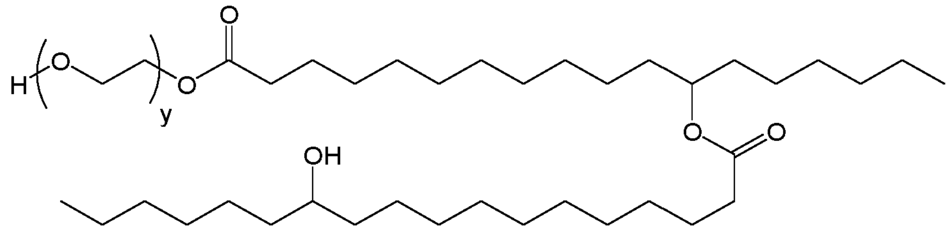

where x and y are integers and a small part of the 12-hydroxy group can be etherified with polyethylene glycol.

A disperse phase which is or comprises a surfactant may enhance the absorption of cyclosporin A into the tissue of the GIT, for example by forming self-assembly structures, such as micelles, which are associated with the cyclosporin A and thus present the drug to the mucosa tissue of the GI tract in a form which enhances uptake/absorption in the tissue.

The term "self-assembly structure" refers to any type of micelle, vesicle, microemulsion, lyotropic phase, laminar or other self-organised structure that forms spontaneously in the presence of an aqueous environment, or combination thereof. As is known, such self-assembly structures form when a self-assembly structure-forming substance, e.g. comprising or consisting of a surfactant, is present above a certain critical concentration. The term includes, for example, micelles, inverted micelles and liposomes, and combinations thereof. The self-assembly structures referred to in this specification may comprise, or be, micelles. More information on self-assembly structures can be found in "Dynamics of Surfactant Self-assemblies Micelles, Microemulsions, Vesicles and Lyotropic Phases" by Raoul Zana, particularly Chapter 1. The release of self-assembly structures from a bead or other composition of the invention may be determined by contacting the composition with water and observing for such structures using a suitable analytical method such as dynamic light scattering.

The oil phase may also include one or more volatile or non-volatile solvents, which may be the same or different from the solvent or co-solvent previously mentioned. Such solvents may for example remain in the composition of the invention following processing e.g. initial dissolution of the components present in the core, and have no particular function in the core composition. Alternatively, such solvents if present may function to maintain the cyclosporin a dissolved state (in solution) within the oil phase or to facilitate dispersion, egress etc. In other embodiments, the solvent may have partly or fully evaporated during processing and therefore be present in only minor quantities if at all. In a related embodiment, the solvent, particularly when a solvent which is both oil and water-soluble is used, may be partly or completely present in the aqueous phase of the core. An example of such a solvent is ethanol. Another example is Transcutol P or HP (2-(ethoxyethoxy)ethanol), which is already mentioned as a co-solvent.

A core not according to the present invention may comprise a hydrogel-forming polymer matrix which forms a

continuous phase and a disperse phase comprising cyclosporin A, a high HLB non-ionic surfactant compound, a low HLB oil, and optionally a co-solvent.

In the present invention, the core comprises a continuous phase which is or comprises a hydrogel-forming polymer and a disperse phase which is or comprises cyclosporin A and an oil phase, the oil phase comprising an oil and one or more surfactants, wherein the oil and the surfactant have an HLB of up to 10. The presence of a surfactant with an HLB of up to 10 has been found to provide advantageous effects during the manufacture of the composition by for example inhibiting crystallisation of cyclosporin from the oil phase when the disperse phase is mixed with the continuous phase to form a colloid, for example an oil in water emulsion. Such compositions form a further aspect of the invention.

The presence of a surfactant with an HLB of up to 10 in the oil phase may enhance the rate and or extent of release of cyclosporin A from the composition following oral administration. The presence of the surfactant may act to maintain a high proportion of the cyclosporin A in a solubilised form after it has been released from the composition into an aqueous medium such as that found in the lower GI tract, particularly the colon.

The composition comprises an orally administered composition comprising a core having the form of a solid colloid, the colloid comprising a continuous phase being or comprising a hydrogel forming polymer and a disperse phase being or comprising cyclosporin A, and an oil phase, the oil phase comprising an oil and one or more surfactants, wherein the surfactant has an HLB of up to 10, for example an HLB in the range 1-10. The composition is suitably a modified release composition. However, the core could be used to provide an instant release composition by, for example using the core without a modified release coating.

The HLB value of the surfactant present in the oil phase may be may be up to 8, up to 7, 1-8, 1-7, 1-5, 2-5, 1-4, 1-3, 1-2, 2-4, 3-4, 5-8, 6-8 or 6-7, for example the HLB value may be about 1, about 2, about 3, about 4, about 5, about 6 or about 7. The surfactant may be any surfactant having an HLB value with the ranges described above, for example any of the surfactants described herein under the section "surfactants" herein or elsewhere in the description and examples. The surfactant is suitably a non-ionic surfactant. The cyclosporin A may be soluble in the surfactant, for example the cyclosporin A may have a solubility of more than about 200mg/g in the surfactant. Thus, the surfactant may have a cyclosporin solubility of more than about 200 mg/g, optionally more than about 250 mg/g. The surfactant may have a cyclosporin solubility of from about 200 mg/g to about 500 mg/g, optionally from about 250 mg/g to about 500 mg/g, about 200 mg/g to about 400 mg/g, from about 225 mg/g to about 375 mg/g, from about 250 mg/g to about 375 mg/g, from about 200 mg/g to about 300 mg/g, from about 300 mg/g to about 400 mg/g, from about 250 mg/g to about 350 mg/g, from about 225 mg/g to about 275 mg/g, from about 350 mg/g to about 400 mg/g. Preferably, the surfactant has a cyclosporin solubility of from about 200 mg/g to about 400 mg/g or from about 225 mg/g to about 375 mg/g. Solubility of cyclosporin in a surfactant may be carried out following the protocol described in Development of a Self Micro-Emulsifying Tablet of Cyclosporine-A by the Liquisolid Compact Technique, Zhao et al (International Journal of Pharmaceutical Sciences and Research, 2011, Vol. 2(9), 2299-2308).

The surfactant may have an HLB of up to 6 and a cyclosporin solubility of from 200 mg/g to 400 mg/g. The surfactant may have an HLB value of 2-6 (optionally 3-6) and a cyclosporin solubility of from about 200 mg/g to about 400 mg/g. The surfactant may have an HLB value of 2-6 (optionally 3-6) and a cyclosporin solubility of from about 250 mg/g to about 400 mg/g. The surfactant may have an HLB value of 2-6 (optionally 3-6) and a cyclosporin solubility of from about 225 mg/g to about 275 mg/g. The surfactant may have an HLB value of 2-6 (optionally 3-6) and a cyclosporin solubility of from about 250 mg/g to about 350 mg/g.

The surfactant is or comprises a surfactant selected from: fatty acid glycerides, polyethylene glycol fatty acid esters, propylene glycol fatty acid esters, fatty acid lactic acid ester, sucrose fatty acid esters, polyethylene glycol fatty alcohol ethers, ethylene oxide-propylene oxide block co-polymers and polyoxyethylene ethers; wherein the surfactant has an HLB value of up to 10, up to 8, or particularly a HLB value described above for example 1 to 8, or 1 to 4.

The surfactant may be or comprise a surfactant selected from: fatty acid glycerides, polyethylene glycol fatty acid esters, propylene glycol fatty acid esters, fatty acid lactic acid esters or sucrose fatty acid esters, wherein the surfactant has an HLB value of up to 10, up to 8, or particularly a HLB value described above for example 1 to 8 or 1 to 4.

The surfactant may be or comprise a fatty acid glyceride, wherein the surfactant has an HLB value of up to 10, up to 8, or particularly a HLB value described above, for example 1 to 8 or 1 to 4.

The surfactant may be or comprise a sorbitan fatty acid ester, for example a sorbitan mono, di- or tri- fatty acid ester and wherein the surfactant has an HLB value described above, for example 1 to 8 or 1 to 4. The fatty acid may be or comprise for example one or more $C_{10}$-$C_{20}$, $C_{12}$-$C_{20}$ or $C_{15}$-$C_{20}$ fatty acids more particularly a $C_{16}$ or $C_{18}$ fatty acid. The fatty acids may be saturated or unsaturated. A particular surfactant is or comprises sorbitan trioleate (commercially available as Span 85), Another particular surfactant is or comprises sorbitan monopalmitate (commercially available as Span 40).

The surfactant may be or comprise polyethylene glycol fatty acid esters, suitably esters with for example one or more $C_{10}$-$C_{20}$, $C_{12}$-$C_{20}$ or $C_{15}$-$C_{20}$ fatty acid, which acid may be saturated or unsaturated. Suitably the surfactant is or comprises a mixture comprising polyethylene glycol fatty acid esters and fatty acid glycerides, wherein the fatty acid is a $C_{15}$-$C_{20}$ fatty acid, which may be saturated or unsaturated. A particular surfactant is or comprises a mixture of oleoyl

polyethylene glycol and oleoyl glycerides, for example oleoyl macrogol-6 glycerides (commercially available as Labrafil M1944CS).

The surfactant may be or comprise a polyglycerised fatty acid for example polyglyceryl dioleate. Accordingly the surfactant may act as an emulsifier and may be polyglyceryl-3 dioleate (for example products sold under the trade mark Plurol® Oleique).

The weight ratio of surfactant having a HLB value of up to 10 : oil may be from about 5:1 to about 1:5, from about 3:1 to about 1:2, from about 3:1 to about 1:1 or from about 2.5:1 to 1.5:1. Suitably the weight ratio may be about 1:1, about 2:1, about 2.5:1, about 3:1, about 1:1.5 or about 1:2. In aspects of the present invention, the ratio is 5:1 to 1.5:1 for oils in general with an HLB of up to 10.

The surfactant having a HLB value of up to 10 may be present in the composition in an amount of from about 5% to about 20%, from about 8% to about 15%, or from about 10% to about 14% by weight based upon the dry weight of the core. It is to be understood that reference to the "dry weight of the core" means the weight of the components present in the uncoated core other than water.

The oil may be any of the oils described herein, particularly the oils described in the section "Disperse Phase". The oil may be or comprise a short-, medium- or long- chain triglyceride composition, or a combination thereof. A medium chain triglyceride(s) (MCT) comprises one or more triglycerides of at least one fatty acid selected from $C_6$, $C_7$, $C_8$, $C_9$, $C_{10}$, $C_{11}$ and $C_{12}$ fatty acids. A particular oil phase is, or comprises a triglyceride based oil, such as those commercially available as Miglyol™, for example Miglyol™ 810, 812 (caprylic/capric triglyceride); Miglyol™ 818: (caprylic/capric/linoleic triglyceride); Miglyol™ 829: (caprylic/capric/succinic triglyceride).

The oil may be present in the composition in an amount of from about 2% to about 25%, from about 3% to about 20%, from about 3% to about 10% or from about 5% to about 10% by weight based upon the dry weight of the core.

The oil phase may also comprise a solvent. Suitable solvents are as described herein in relation to the disperse phase and are suitable miscible with both the oil and water. The solvent may be presently in the composition in an amount of form about 1% to 30%, for about 5% to about 30%, for about 10% to about 25%, or from about 12% to about 22% by weight based upon the dry weight of the core. A particular solvent is 2-(2-ethoxyethoxy)ethanol (available commercially as for example Transcutol™ P or HP).

The hydrogel-forming polymer may be or comprise one or more of the hydrogel-forming polymers described herein, particularly those described under "Continuous Phase Polymer Matrix". Suitably the hydrogel-forming polymer is or comprises a hydrogel-forming polymer selected from the group consisting of gelatin, agar, a polyethylene glycol, starch, casein, chitosan, soya bean protein, safflower protein, alginates, gellan gum, carrageenan, xanthan gum, phthalated gelatin, succinated gelatin, cellulosephthalate-acetate, oleoresin, polyvinylacetate, hydroxypropyl methyl cellulose, polymerisates of acrylic or methacrylic esters and polyvinylacetate-phthalate and any derivative of any of the foregoing; or a mixture of one or more such a hydrogel forming polymers. A particular hydrogel-forming polymer is selected from carrageenan, gelatin, agar and pectin, or a combination thereof, particularly gelatin and/or agar, more particularly gelatin. The hydrogel forming polymer is suitably present in the core in a gelled state such that the polymer forms a solid matrix within which the disperse phase is dispersed to provide for example a solid colloid. The hydrogel-forming polymer is preferably sufficiently gelled to provide a core which is sufficiently rigid to enable to be handled and further processed into a dosage form or to be coated with for example a modified release coating as described herein.

The hydrogel-forming polymer may be present in an amount of from about 20% to about 70%, about 20% to about 55%, about 25% to about 50%, about 30% to about 50%, or about 40% to about 45% by weight based upon the dry weight of the core.

The continuous phase may comprise a suitably plasticiser, particularly when the hydrogel-forming polymer is or comprises gelatin. A particular plasticiser is Sorbitol. When present the plasticiser may be present at for example up to about 20% or up to about 10%, suitably from about 3% to about 8%, or from about 4% to about 6% by weight based upon the dry weight of the core.

The continuous phase may comprise a surfactant. The surfactant present in the continuous phase is preferably different to the surfactant present in the oil phase. Suitable surfactants which may be present in the continuous phase are as described herein under the section "Continuous Phase Polymer Matrix". Accordingly particular surfactants which may be present in the continuous phase may be cationic, amphoteric (zwitterionic) or anionic surfactants. Suitably the surfactant present in the continuous phase is or comprises an anionic surfactant, more particularly a hydrophilic anionic surfactant. The surfactant in the continuous phase may be or comprise at least one surfactant selected from fatty acid salts, alkyl sulfates and bile salts, particularly an alkyl sulfate, for example a $C_{10}$-$C_{22}$ alkyl sulphate suitably sodium dodecyl sulphate. The surfactant present in the continuous phase, particularly anionic surfactant is present in the composition in an amount of from 0.1% to 6%, e.g. 0.1% to 5%. 0.1% to 4%, 0.1% to 3%, 1% to 4%, 1.5% to 4.5%, or 2.5% to 4.5% preferably in an amount 2-4% by weight based upon the dry weight of the core.

The cyclosporin A is suitably present in the composition in an amount for from about 5% to about 20%, from about 8% to about 15%, or from about 9% to about 14% % by weight based upon the dry weight of the core.

In a particular embodiment there is provided an orally administered modified release composition comprising

a core having the form of a solid colloid, the colloid comprising a continuous phase being or comprising a hydrogel forming polymer and a disperse phase;
wherein the disperse phase is or comprises:
cyclosporin A;

an oil being or comprising: a short-, medium- or long- chain triglyceride composition, or a combination thereof, for example a caprylic/capric triglyceride, a caprylic/capric/linoleic triglyceride; and a caprylic/capric/succinic triglyceride;

one or more non-ionic surfactants with an value HLB of up to 10, up to 8, up to 7, 1-8, 1-7, 1-5, 2-5, 1-4, 1-3, 1-2, 2-4, 3-4, 5-8, 6-8 or 6-7, for example about 1, about 2, about 3, about 4, about 5, about 6 or about 7; optionally wherein the surfactant is or comprises a fatty acid glyceride, a sorbitan fatty acid ester, or a polyethylene glycol fatty acid ester; and

optionally a solvent, wherein the solvent is miscible with the oil and with water, for example 2-(2-ethoxyethoxy)ethanol;

wherein the continuous phase is or comprises:

a hydrogel-forming polymer, for example a hydrogel forming polymer being or comprising carrageenan, gelatin, agar and pectin, or a combination thereof, optionally gelatin or agar or a combination thereof, more optionally the polymer of the a hydrogel forming polymer matrix is or comprises gelatin;

an anionic surfactant, optionally an anionic surfactant is selected from fatty acid salts, alkyl sulphates and bile salts, particularly an alkyl sulfate, for example a $C_{10}$-$C_{22}$ alkyl sulphate suitably, sodium dodecyl sulphate; and

optionally a plasticiser, for example sorbitol.

In another embodiment there is provided an orally administered modified release composition comprising a core having the form of a solid colloid, the colloid comprising a continuous phase being or comprising a hydrogel forming polymer and a disperse phase;
wherein the disperse phase is or comprises:
from about 8% to about 15% cyclosporin A;

from about 2% to about 20%, for example about 3% to about 10% of oil being or comprising a caprylic/capric triglyceride, a caprylic/capric/linoleic triglyceride; and a caprylic/capric/succinic triglyceride, preferably a caprylic/capric triglyceride;

one or more non-ionic surfactants with an value HLB of up to 10, up to 8, up to 7, 1-8, 1-7, 1-5, 2-5, 1-4, 1-3, 1-2, 2-4, 3-4, 5-8, 6-8 or 6-7, for example about 1, about 2, about 3, about 4, about 5, about 6 or about 7; optionally wherein the surfactant is or comprises a fatty acid glyceride, a sorbitan fatty acid ester, or a polyethylene glycol fatty acid ester, optionally wherein the non-ionic surfactant is present in an amount of from about 8% to about 15%; and

optionally from about 12% to about 22% solvent, wherein the solvent is miscible with the oil and with water, for example 2-(2-ethoxyethoxy)ethanol;

wherein the continuous phase is or comprises:

from about 30% to about 70%, for example about 30% to about 50% hydrogel-forming polymer, optionally wherein the hydrogel forming polymer is or comprises carrageenan, gelatin, agar and pectin, or a combination thereof, optionally gelatin or agar or a combination thereof, more optionally wherein the hydrogel forming polymer matrix is or comprises gelatin;

an anionic surfactant, optionally an anionic surfactant is selected from fatty acid salts, alkyl sulphates and bile salts, particularly an alkyl sulfate, for example a $C_{10}$-$C_{22}$ alkyl sulphate suitably sodium dodecyl sulphate, optionally wherein the anionic surfactant is present in an amount of from about 0.1% to about 5%, suitably from 2% to 4%; and

optionally up to about 10% plasticiser, for example sorbitol;

wherein all % are % by weight based upon the dry weight of the core.

In another embodiment there is provided an orally administered modified release composition comprising a core having the form of a solid colloid, the colloid comprising a continuous phase being or comprising a hydrogel forming polymer and a disperse phase;
wherein the disperse phase is or comprises:
from about 8% to about 15% cyclosporin A;

from about 3% to about 10% of oil being or comprising a caprylic/capric triglyceride;

one or more non-ionic surfactants with an value HLB of up to 7, for example 1-7, or 2-4 wherein the surfactant is or comprises a fatty acid glyceride, a sorbitan fatty acid ester, or a polyethylene glycol fatty acid ester, optionally wherein the non-ionic surfactant is present in an amount of from about 8% to about 15%; and

optionally from about 12% to about 22% solvent, wherein the solvent is miscible with the oil and with water, for example 2-(2-ethoxyethoxy)ethanol;

wherein the continuous phase is or comprises:

from about 30% to about 50% hydrogel-forming polymer selected from gelatin or agar or a combination thereof, optionally wherein the hydrogel forming polymer matrix is or comprises gelatin;

0.1% to about 5%, suitably from 2% to 4% anionic surfactant for example sodium dodecyl sulphate; and

optionally up to about 10% plasticiser, for example sorbitol;

wherein all % are % by weight based upon the dry weight of the core.

In a particular embodiment the core is in the form of a solid colloid, the colloid comprising a continuous phase and a disperse phase, wherein the continuous phase comprises the hydrogel forming polymer; wherein

the disperse phase is or comprises:

cyclosporin A;

a medium chain mono-, di- or tri-glyceride, for example a medium chain triglyceride, particularly caprylic/capric triglyceride;

a non-ionic surfactant (for example a polyethoxylated castor oil); and

a co-solvent (for example 2-(ethoxyethoxy)ethanol);

and wherein the continuous phase is or comprises:

a hydrogel forming polymer matrix which is or comprises a hydrocolloid selected from carrageenan, gelatin, agar and pectin, or a combination thereof optionally selected from gelatin and agar or a combination thereof, more optionally the polymer of the a hydrogel forming polymer matrix is or comprises gelatin;

optionally a plasticiser, for example a plasticiser selected from glycerin, a polyol for example sorbitol, polyethylene glycol and triethyl citrate or a mixture thereof, particularly sorbitol; and

an anionic surfactant, for example at least one surfactant selected from fatty acid salts, alkyl sulphates and bile salts, particularly an alkyl sulfate, for example sodium dodecyl sulfate.

In a further specific embodiment the core comprises a hydrogel forming polymer matrix comprising gelatin in an amount of 300 to 700 mg/g, the core further comprising cyclosporin A, medium chain mono-, di- or tri-glycerides (for example a medium chain triglyceride, particularly caprylic/capric triglyceride) in an amount of 20 to 200 mg/g, and the core further comprises the following components:

co-solvent (for example 2-(ethoxyethoxy)ethanol) in an amount of 150 to 250 mg/g;
non-ionic surfactant in an amount of 80 to 200 mg/g; and
anionic surfactant in an amount of 15 to 50 mg/g,

wherein weights are based upon the dry weight of the core.

Suitably in the embodiment of the above paragraph the cyclosporin A may be present in an amount of 60 to 150 mg/g, for example 80 to 120 mg/g or particularly 80 to 100 mg/g. The nonionic and anionic surfactants are as defined herein, for example an anionic surfactant selected from alkyl sulfates, carboxylates or phospholipids (particularly SDS); or a non-ionic surfactant selected from sorbitan-based surfactants, PEG-fatty acids, or glyceryl fatty acids or poloxamers. A particular non-ionic surfactant is a polyethoxylated castor oil (for example Kolliphor™ EL).

Out of the embodiments disclosed in the above paragraphs, only those according to claims 1 and 2 are according to the invention. The cores described above comprising hydrogel-forming polymer matrix and cyclosporin A are suitably coated as described herein to provide a modified release composition according to the invention. A particular coating for these embodiments is a coating comprising a first coating (sub-coating) which is or comprises a water-soluble cellulose ether, particularly hydroxypropylmethyl cellulose;

a second coating outside the first coating which is or comprises a modified release coating, particularly a pH independent modified release coating, more especially a coating comprising ethyl cellulose (e.g. Surelease™) still more particularly a coating comprising ethyl cellulose and a water-soluble polysaccharide such as pectin (e.g. a Surelease™ pectin coating as described herein); and wherein
the first coating is present in an amount corresponding to a weight gain due to the first coating in a range selected from : (i) from 1% to 20%; (ii) from 8% to 12%, for example about 10%; or (iii) from 4% to 6%, for example about 5% by weight based upon the weight of the composition prior to applying the first coating; and wherein
the second coating is present in an amount corresponding to a weight gain of the composition due to the second coating selected from (a) from 5 to 40%; (b) from 10% to 12%, for example about 11% or about 11.5%; or (c) from 16% to 18%, for example about 17% by weight based upon the weight of the composition prior to applying the second coating.

The compositions described herein are optionally further coated with a suitable outer protective coating as described above.

**Surfactant**

The composition may contain one or more surfactant, for example surfactants may be present in the core (including in the hydrogel-forming polymer matrix, and in the disperse phase or both). Surfactants may also be present in one or more of the coatings applied to the core.

Suitable surfactants can be anionic, cationic, zwitterionic, or non-ionic. In the description and claims of this specification, the term "surfactant" is employed as a contraction for "surface active agent". For the purposes of this description and claims, it is assumed that there are four major classifications of surfactants; therefore the surfactant may be: anionic, cationic, non-ionic, and amphoteric (zwitterionic). The non-ionic surfactant remains whole, has no charge in aqueous solutions, and does not dissociate into positive and negative ions. Anionic surfactants are water-soluble, have a negative charge and dissociate into positive and negative ions when placed in water. The negative charge lowers the surface tension of water and acts as the surface-active agent. Cationic surfactants have a positive charge, and also dissociate into positive and negative ions when placed in water. In this case, the positive ions lower the surface tension of the water and act as the surfactant. The amphoteric (zwitterionic) surfactant assumes a positive charge in acidic solutions and performs as a cationic surfactant, or it assumes a negative charge in an alkaline solution and acts as an anionic surfactant.

The surfactant(s) may be selected from: anionic surfactants and combinations thereof; from non-ionic surfactants and combinations thereof; and from combination of an anionic surfactant (e.g. a single such surfactant or a plurality thereof) and a non-ionic surfactant (e.g. a single such surfactant or a plurality thereof).

Surfactants can also be classified according to their hydrophilic-lipophilic balance (HLB) which is a measure of the degree to which the surfactant is hydrophilic or lipophilic, determined by calculating values for the different regions of the molecule, as described (originally for non-ionic surfactants) by Griffin in 1949 and 1954 and later by Davies. The methods apply a formula to the molecular weight of the whole molecule and of the hydrophilic and lipophilic portions to give an arbitrary (semi-empirical) scale up to 40 although the usual range is between 0 and 20. An HLB value of 0 corresponds to a completely hydrophobic molecule, and a value of 20 would correspond to a molecule made up completely of hydrophilic components. The HLB value can be used to predict the surfactant properties of a molecule:

| HLB Value | Expected properties |
|---|---|
| 0 to 3 | antifoaming agent |
| from 4 to 6 | W/O emulsifier |
| from 7 to 9 | wetting agent |
| from 8 to 18 | an O/W emulsifier |
| from 13 to 15 | typical of detergents |
| 10 to 18 | solubiliser or hydrotrope |

Although HLB numbers are assigned to surfactants other than the non-ionic, for which the system was invented, HLB numbers for anionic, cationic, non-ionic, and amphoteric (zwitterionic) surfactants can have less significance and often represent a relative or comparative number and not the result of a mathematical calculation. This is why it is possible to have surfactants above the "maximum" of 20. HLB numbers can however be useful to describe the HLB requirement of a desired application for a given emulsion system in order to achieve good performance.

Non-ionic Surfactants

The surfactant may be or comprise at least one surfactant selected from the following nonionic surfactants.

PEG-fatty acid monoester surfactants, PEG-fatty acid diester surfactants, PEG-fatty acid monoester and diester surfactant mixtures, PEG glycerol fatty acid esters, transesterified products of oils and alcohols, lower alcohol fatty acid esters, polyglycerised fatty acids, propylene glycol fatty acid esters, mono and diglyceride surfactants, sterol and sterol derivative surfactants, PEG-sorbitan fatty acid esters, sorbitan fatty acid esters, polyethylene glycol alkyl ethers, sugar ester surfactants, polyethylene glycol alkyl phenol surfactants, POE-POP block copolymers, fatty acid salts, bile salts, phospholipids, phosphoric acid esters, carboxylates, acyl lactylates, sulfates and sulfonates, and cationic surfactants.

A PEG-fatty acid mono ester surfactant for example PEG 4-100 monolaurate, PEG 4-100 monooleate, PEG 4-100 monostearate, PEG-laurate, PEG-oleate, PEG stearate, and PEG ricinoleate. A PEG-fatty acid diester surfactant for example PEG dilaurate; PEG dioleate, PEG distearate, PEG dipalmitate. A mixture of PEG-fatty acid mono- and diesters.

A PEG glycerol fatty acid ester for example PEG glyceryl laurate, PEG glyceryl stearate, PEG glyceryl oleate.

PEG-sorbitan fatty acid esters for example PEG sorbitan laurate, PEG sorbitan monolaurate, PEG sorbitan monopalmitate, PEG sorbitan monostearate, PEG sorbitan tristearate, PEG sorbitan tetrastearate, PEG sorbitan monooleate, PEG sorbitan oleate, PEG sorbitan trioleate, PEG sorbitan tetraoleate, PEG sorbitan monoisostearate, PEG sorbitol hexaoleate, PEG sorbitol hexastearate.

Propylene glycol fatty acid esters for example propylene glycol monocaprylate, propylene glycol monolaurate, propylene glycol oleate, propylene glycol myristate, propylene glycol monostearate, propylene glycol hydroxy stearate, propylene glycol ricinoleate, propylene glycol isostearate, propylene glycol monooleate, propylene glycol dicaprylate/dicaprate, propylene glycol dioctanoate, propylene glycon caprylate/caprate, propylene glycol dilaurate, propylene glycol distearate, propylene glycol dicaprylate, propylene glycol dicaprate.

A sorbitan fatty acid ester for example sorbitan monolaurate, sorbitan monopalmitate, sorbitan monooleate, sorbitan monostearate, sorbitan trioleate, sorbitan sesquioleate, sorbitan tristearate, sorbitan monoisostearate, sorbitan sesquistearate.

Lower alcohol fatty acid esters for example ethyl oleate, isopropy myristate, isopropyl palmitate, ethyl linoleate, isopropyl linoleate.

Polyoxyethylene-polyoxypropylene block copolymers for example poloxamer 105, poloxamer 108, poloxamer 122, poloxamer 123, poloxamer 124, poloxamer 181, poloxamer 182, poloxamer 183, poloxamer 184, poloxamer 185, poloxamer 188, poloxamer 212, poloxamer 215, poloxamer 217, poloxamer 231, poloxamer 234, poloxamer 235, poloxamer 237, poloxamer 238, poloxamer 282, poloxamer 284, poloxamer 288, poloxamer 331, poloxamer 333, poloxamer 334, poloxamer 335, poloxamer 338, poloxamer 401, poloxamer 402, poloxamer 403, poloxamer 407.

Polyglycerised fatty acids for example polyglyceryl stearate, polyglyceryl oleate, polyglyceryl isostearate, polyglyceryl laurate, polyglyceryl ricinoleate, polyglyceryl linoleate, polyglyceryl pentaoleate, polyglyceryl dioleate, polyglyceryl distearate, polyglyceryl trioleate, polyglyceryl septaoleate, polyglyceryl tetraoleate, polyglyceryl decaisostearate, polyglyceryl decaoleate, polyglyceryl monooleate, dioleate, polyglyceryl polyricinoleate.

PEG alkyl ethers for example PEG oleyl ether, PEG lauryl ether, PEG cetyl ether, PEG stearyl ether.

PEG alkyl phenols for example PEG nonyl phenol, PEG octyl phenol ether.

Transesterification products of alcohol or polyalcohol with natural or hydrogenated oils for example PEG castor oil, PEG hydrogenated castor oil, PEG corn oil, PEG almond oil, PEG apricot kernel oil, PEG olive oil, PEG-6 peanut oil, PEG hydrogenated palm kernel oil, PEG palm kernel oil, PEG triolein, PEG corn glycerides, PEG almond glycerides, PEG trioleate, PEG caprylic/capric triglyceride, lauroyl macrogol glyceride, stearoyl macrogol glyceride, mono, di, tri, tetra esters of vegetable oils and sorbitol, pentaerythrityl tetraisostearate, pentaerythrityl distearate, pentaerythrityl tetraoleate, pentaerythrityl tetrastearate, pentaerythrityl tetracaprylate/tetracaprate, pentaerythrityl tetraoctanoate.

Oil-soluble vitamins for example vitamins A, D, E, K, and isomers, analogues, and derivatives thereof. The derivatives include, for example, organic acid esters of these oil-soluble vitamin substances, for example the esters of vitamin E or vitamin A with succinic acid. Derivatives of these vitamins include tocopheryl PEG-1000 succinate (Vitamin E TPGS) and other tocopheryl PEG succinate derivatives with various molecular weights of the PEG moiety, for example PEG 100-8000.

Sterols or sterol derivatives (e.g. esterified or etherified sterols as for example PEGylated sterols) for example cholesterol, sitosterol, lanosterol, PEG cholesterol ether, PEG cholestanol, phytosterol, PEG phytosterol.

Sugar esters for example sucrose distearate, sucrose distearate/monostearate, sucrose dipalmitate, sucrose monostearate, sucrose monopalmitate, sucrose monolaurate, alkyl glucoside, alkyl maltoside, alkyl maltotrioside, alkyl glycosides, derivatives and other sugar types: glucamides.

Carboxylates (in particular carboxylate esters) for example ether carboxylates, succinylated monoglycerides, sodium stearyl fumarate, stearoyl propylene glycol hydrogen succinated, mono/diacetylated tartaric acid esters of mono- and diglycerides, citric acid esters of mono-, diglycerides, glyceryl-lacto esters of fatty acids; acyl lactylates: lactylic esters of fatty acids, calcium/sodium stearoyl-2-lactylate calcium/sodium stearoyl lactylate, alginate salts, propylene glycol alginate.

A fatty acid monoglyceride, diglyceride or triglyceride or a combination thereof.

**Anionic Surfactants**

Anionic surfactants may be selected from following anionic surfactants.

Fatty acid salts and bile salts for example sodium caproate, sodium caprylate, sodium caprate, sodium laurate, sodium myristate, sodium myristolate, sodium palmitate, sodium palmitoleate, sodium oleate, sodium ricinoleate, sodium linoleate, sodium linolenate, sodium stearate, sodium lauryl sulfate, sodium tetradecyl sulfate, sodium lauryl sarcosinate, sodium dioctyl sulfosuccinate; sodium cholate, sodium taurocholate, sodium glycocholate, sodium deoxycholate, sodium taurodeoxycholate, sodium glycodeoxycholate, sodium ursodeoxycholate, sodium chenodeoxycholate, sodium taurochenodeoxycholate, sodium glyco chenodeoxycholate, sodium cholylsarcosinate, sodium N-methyl taurocholate

Phospholipids for example egg/soy lecithin, cardiolipin, sphingomyelin, phosphatidylcholine, phosphatidyl ethanolamine, phosphatidic acid, phosphatidyl glycerol, phosphatidyl serine.

Phosphoric acid esters having the general formula $RO\text{-}PO_3^{-}M^{+}$ where the R group is an ester forming group, e.g. an alkyl, alkenyl or aryl group optionally substituted by a PEG moiety through which the alkyl, alkenyl or aryl group is coupled to the phosphate moiety. R may be a residue of a long chain (e.g. >C9) alcohol or a phenol. Specific examples include diethanolammonium polyoxyethylene-10 oleyl ether phosphate, esterification products of fatty alcohols or fatty alcohol ethoxylates with phosphoric acid or anhydride.

Sulfates and sulfonates (in particular esters thereof) for example ethoxylated alkyl sulfates, alkyl benzene sulfones, $\alpha$-olefin sulfonates, acyl isethionates, acyl taurates, alkly glyceryl ether sulfonates, octyl sulfosuccinate disodium, disodium undecylenamideo-MEA-sulfosuccinate, alkyl phosphates and alkyl ether phosphates.

Particular anionic surfactants include alkyl sulfates, for example. $C_{10}$-$C_{22}$ alkyl sulfates such as sodium dodecyl sulfate.

The anionic surfactant may be perfluoro-octanoate (PFOA or PFO), perfluoro-octanesulfonate (PFOS), sodium dodecyl sulfate (SDS), ammonium lauryl sulfate, and other alkyl sulfate salts, sodium laureth sulfate, also known as sodium lauryl ether sulfate (SLES) and alkyl benzene sulphonate. A particular class of surfactant comprises alkyl sulfate salts. A preferred anionic surfactant is SDS.

Cationic Surfactants

Cationic surfactants may be selected from the following cationic surfactants.

Hexadecyl triammonium bromide, dodecyl ammonium chloride, alkyl benzyldimethylammonium salts, diisobutyl phenoxyethoxydimethyl benzylammonium salts, alkylpyridinium salts; betains (trialkylglycine): lauryl betaine (N-lauryl,N,N-dimethylglycine); ethoxylated amines: polyoxyethylene-15 coconut amine, alkyl -amines/ diamines/ quaternaty amines and alkyl ester.

Emulsifiers

The surfactant may act as an emulsifier such surfactants include non-ionic emulsifiers, for example selected from: a mixture of triceteareth-4 phosphate, ethylene glycol palmitostearate and diethylene glycol palmitostearate (for example sold under the trade mark SEDFOS™ 75); sorbitan esters, e.g. sorbitan monooleate, sorbitan monolaurate, sorbitan monpalmitate, sorbitan monostearate (for example products sold under the trade mark Span™), PEG-8 beeswax e.g. sold under the trade mark Apifil®; a mixture of cetyl alcohol, ceteth-20 and steareth-20 (for example Emulcire™ 61 WL 2659); a mixture of glyceryl monostearate EP/NF and PEG-75 palmitostearate (for example Gelto™ 64); a mixture of PEG-6 stearate and PEG-32 stearate (for example Tefose® 1500); a mixture of PEG-6 palmitostearate, ethylene glycol palmitostearate, and PEG-32 palmitostearate (e.g. Tefose® 63); triglycerol diisostearate (for example products sold under the trade mark Plurol Diisostearique®); polyglyceryl-3 dioleate (for example products sold under the trade mark Plurol® Oleique).

**Other Excipients**

The modified release composition optionally contains one or more of the following additional substances or categories of substances. For example, the composition may contain a protectant such as, for example, a proteolytic enzyme inhibitor or a protector against acid degradation or both (e.g. an alkali for example sodium hydroxide); an adhesive entity such as, for example, a muco- or bio-adhesive; excipients to maximize solubility of the cyclosporin A; excipients to maximize permeability of the cyclosporin A in the GIT. Typical excipients for enhancing the permeability of the epithelial barrier include but are not limited to sodium caprate, sodium dodecanoate, sodium palmitate, SNAC, chitosan and derivatives thereof, fatty acids, fatty acid esters, polyethers, bile salts, phospholipids, alkyl polyglucosides, hydroxylase inhibitors, antioxidants (e.g. ascorbic acid) and/or nitric oxide donors. The preceding list is of particular interest to enhance permeability in the ileum.

To enhance permeability in the colon, typical excipients include, but not limited to sodium caprate, sodium dodecanoate, sodium palmitate, SNAC, chitosan and derivatives thereof, fatty acids, fatty acid esters, polyethers, bile salts, phospholipids, alkyl polyglucosides, hydroxylase inhibitors, antioxidants and/or nitric oxide donors, including nitric oxide donor groups covalently attached to various active pharmaceutical ingredients.

The composition may further comprise excipients to enhance the therapeutic potential of the cyclosporin A in the ileum and colon including, but not limited to absorption limiters, essential oils such as, for example, omega 3 oils, natural plant extracts such as, for example, neem, ionexchange resins, bacteria degradable conjugation linkers such as, for example, azo bonds, polysaccharides such as, for example, amylose, guar gum, pectin, chitosan, inulin, cyclodextrins, chondroitin sulphate, dextrans, guar gum and locust bean gum, nuclear factor kappa B inhibitors, acids such as, for example, fumaric acid, citric acid and others, as well as modifications thereof.

The composition may further comprise excipients to reduce systemic side effects associated with absorption in the GIT, such as the small intestine, including, but not limited to, antioxidants, such as, for example, curcuminoids, flavanoids or more specifically including curcumin, beta-carotene, $\alpha$-tocopherol, ascorbate or lazaroid.

The composition may further or separately comprise antioxidants (such as, for example, ascorbic acid or BHT - butyl hydroxy toluene) taste-masking or photosensitive components or photoprotective components. Antioxidants may be incorporated in the aqueous phase (e.g. hydrophilic antioxidants) or in the disperse phase of the core (e.g. hydrophobic antioxidants such as, for example, vitamin E) for example up to 1% by weight, preferably between 0.01 and 0.50% by weight, more preferably between 0.10 to 0.20% by weight.

The composition may further comprise immune-enhancing nutrients such as vitamins A/B/C/E; carotenoids/beta-carotene and iron, manganese, magnesium, selenium or zinc. Such nutrients may be present in composition, or for example if it is in the form of a bead, the nutrients may be included in the coating.

The composition may also include other well know excipients used in pharmaceutical compositions including colorants, taste masking agents, diluents, fillers, binders etc. The presence of such optional additional components will of course depend upon the particular dosage form adopted.

**Shape, Size and Geometry**

The composition of the invention can be formed into a limitless number of shapes and sizes. In the section below describing the process for making the composition, various methods are given including pouring or introducing a fluid dispersion into a mould where it hardens or can be caused to harden. Thus the composition can be created in whichever form is desired by creating an appropriate mould (e.g. in the shape of a disc, pill or tablet). However, it is not essential to use a mould. For example, the composition may be formed into a sheet e.g. resulting from pouring a fluid dispersion onto a flat surface where it hardens or can be caused to harden.

Preferably, the composition may be in the form of spheres or spherical-like shapes made as described below.

Preferably, the composition of the invention is in the form of substantially spherical, seamless minibeads. The absence of seams on the minibead surface is an advantage e.g. in further processing, for example coating, since it allows more consistent coating, flowability etc. The absence of seams on the minibeads also enhances consistency of dissolution of the beads.

The preferred size or diameter range of minibeads according to the invention can be chosen to avoid retention in the stomach upon oral administration of the minibeads. Larger dosage forms are retained for variable periods in the stomach and pass the pyloric sphincter only with food whereas smaller particles pass the pylorus independently of food. Selection of the appropriate size range (see below) thus makes the therapeutic effect post-dosing more consistent. Compared to a single large monolithic oral format such as, for example, a traditional compressed pill, a population of beads released into the GI tract (as foreseen by the dosage form of the present invention) permits greater intestinal lumen dispersion so enhancing absorption via exposure to greater epithelial area, and achieves greater topical coating in certain parts of the GI tract for example the colon). Reduction of residence time in the ileo-caecal junction is another potential advantage.

The composition of the invention is preferably monolithic meaning internally (i.e. crosssectionally) homogeneous, excluding a possible thin skin of matrix material and excluding any coating layers.

The minibeads provided for by the composition of the present invention generally range in diameter from 0.5 mm to 10 mm with the upper limit preferably 5 mm, e.g. 2.5 mm A particularly convenient upper limit is 2mm or 1.7mm. The lower limit can preferably be 1mm, e.g. 1.2mm, more preferably from 1.3mm, most preferably from 1.4mm. In one embodiment the diameter is from 0.5 to 2.5mm, for example from 1mm to 3mm, 1mm to 2mm, 1.2mm to 3mm or 1.2mm to 2mm. The minibeads may have a diameter of no more than 2.5mm, irrespective of their minimum size. The beads may have a diameter of no more than 2mm, irrespective of their minimum size.

A minibead as described herein may have an aspect ratio of no more than 1.5, e.g. of no more than 1.3, for example of no more than 1.2 and, in particular, of from 1.1 to 1.5, 1.1 to 1.3 or, 1.1 to 1.2. A population of minibeads as described herein, e.g. at least 10 beads, may have an average aspect ratio of no more than 1.5, e.g. of no more than 1.3, for example of no more than 1.2 and, in particular, of from 1 to 1.5, 1 to 1.3 or 1 to 1.2. The aspect ratios mentioned in this paragraph optionally apply to coated minibeads and optionally apply to uncoated minibeads. Average aspect ratio is suitably determined for a population of minibeads, e.g. at least 10 minibeads, using a particle size analyser, for example an Eyecon™ particle characteriser of Innopharma Labs, Dublin 18, Ireland.

The minibeads of the disclosure may, therefore, have a size as disclosed above and an aspect ratio of from 1 to 1.5. The beads of the disclosure may have a size as disclosed above and an aspect ratio of no more than 1.3, for example of no more than 1.2 and, in particular, of from 1.1 to 1.5, 1.1 to 1.3 or, 1.1 to 1.2.

Bead size (diameter) may be measured by any suitable technique, for example microscopy, sieving, sedimentation, optical sensing zone method, electrical sensing zone method or laser light scattering. For the purposes of this specification, bead size is measured by analytical sieving in accordance with USP General Test <786> Method I (USP 24-NF 18, (U.S. Pharmacopeial Convention, Rockville, MD, 2000), pp. 1965-1967).

In embodiments, minibeads of the invention are monodisperse. In other embodiments, minibeads of the invention are not monodisperse. By "monodisperse" is meant that for a population of beads (e. g. at least 100, more preferably at least 1000) the minibeads have a coefficient of variation (CV) of their diameters of 35% or less, optionally 25% or less, for example 15% or less, such as e.g. of 10% or less and optionally of 8% or less, e.g. 5% or less. A particular class of polymer beads has a CV of 25% or less. CV when referred to in this specification is defined as 100 times (standard deviation) divided by average where "average" is mean particle diameter and standard deviation is standard deviation in particle size. Such a determination of CV is performable using a sieve.

The invention includes minibeads having a CV of 35% and a mean diameter of 1 mm to 2 mm, e.g. 1.5 mm. The invention also includes minibeads having a CV of 20% and a mean diameter of 1 mm to 2 mm, e.g. 1.5 mm, as well as minibeads having a CV of 10% and a mean diameter of 1 mm to 2 mm, e.g. 1.5 mm. In one class of embodiments, 90% of minibeads have a diameter of from 0.5 mm to 2.5 mm, e.g. of from 1 mm to 2 mm.

**Dosage Forms**

The modified release composition of the invention is prepared as an orally administrable dosage form suitable for pharmaceutical use. In those embodiments where the composition is in the form of a minibead, the present invention provides for a dosage form comprising a plurality of the minibeads for example as a capsule, a tablet, a sprinkle or a sachet.

In embodiments the dosage form comprising a population of beads may be presented in a single unit dosage form e.g. contained in a single hard gel or HPMC capsule which releases the beads e.g. in the stomach. Alternatively the beads may be presented in a sachet or other container which permits the beads to be sprinkled onto food or into a drink or to be administered via a feeding tube for example a naso-gastric tube or a duodenal feeding tube. Alternatively, the beads may be administered as a tablet for example if a population of beads is compressed into a single tablet as described below. Alternatively, the beads may be filled e.g. compressed into a specialist bottle cap or otherwise fill a

space in a specialised bottle cap or other element of a sealed container (or container to be sealed) such that e.g. on twisting the bottle cap, the beads are released into a fluid or other contents of the bottle or vial such that the beads are dispersed (or dissolve) with or without agitation in such contents. An example is the Smart Delivery Cap manufactured by Humana Pharma International (HPI) S.p.A, Milan, Italy.

The dosage form may be formulated in such a way so that the beads of the invention can be further developed to create a larger mass of beads e.g. via compression (with appropriate oil or powder-based binder and/or filler known to persons skilled in the art. The larger (e.g. compressed) mass may itself take a variety of shapes including pill shapes, tablet shapes, capsule shapes etc. A particular problem which this version of the bead embodiment solves is the "dead space" (above the settled particulate contents) and/or "void space" (between the particulate content elements) typically found in hard gel capsules filled with powders or pellets. In such pellet- or powder-filled capsules with dead/void space, a patient is required to swallow a larger capsule than would be necessary if the capsules contained no such dead space. The beads of this embodiment of the invention may readily be compressed into a capsule to adopt the inner form of whichever capsule or shell may be desired leaving much reduced, e.g. essentially no, dead/void space. Alternatively the dead or void space can be used to advantage by suspending beads in a vehicle such as, for example, an oil which may be inert or may have functional properties such as, for example, permeability enhancement or enhanced dissolution or may comprise an active ingredient being the same or different from any active ingredients in the bead. For example, hard gelatin capsules may be filled with a liquid medium combined with uncoated and/or coated beads. The liquid medium may be one or more of the surfactant phase constituents described herein or it may be one or more surfactants. Particularly preferred but non-limiting examples are corn oil, sorbitane trioleate (sold under the trade mark SPAN 85), propylene glycol dicaprylocaprate (sold under the trade mark Labrafac), 2-(2-ethoxyethoxy)ethanol (sold under the trade mark Transcutol P or HP) and polysorbate 80 (sold under the trade mark Tween 80).

In a representative embodiment the bead of the dosage form is prepared as described herein for example by mixing together at least the following materials: a hydrogel-forming polymer; and cyclosporin A, suitably cyclosporin A dissolved in a hydrophobic material, i.e. an oil to form a dispersion of the cyclosporin A in the hydrogel-forming polymer. The dispersion is immobilized within the solidified bead by ejection from a single orifice nozzle into a suitable cooling liquid. Following removal of the drying liquid the bead is coated with a modified release coating (suitably with a sub-coat under the modified release coating), the coated bead is the filled into a capsule suitable for pharmaceutical use, for example a hard-gel, gelatin or HPMC capsule.

Suitably the dosage form is prepared as a unit dosage form containing from for oral administration comprising from 0.1 mg to 1000 mg, optionally from 1mg to 500 mg, for example 10mg to 300 mg, or 25 to 250 mg suitably about 25mg, about 35 mg, about 50mg, about 75mg, about 100 mg, about 150 mg, about 180 mg, about 200 mg, about 210 mg or about 250 mg cyclosporin A.

**Determination of Contents and Distribution of Compositions**

The identity and/or distribution of one or more of the components of a composition according to the invention can be determined by any method known to those skilled in the art. The distribution of one or more components of a composition can, for example, be determined by nearinfrared (NIR) chemical imaging technology. NIR chemical imaging technology can be used to generate images of the surface or cross section of a composition, for example a minibead. The image produced by this technique shows the distribution of one or more components of the composition. In addition to NIR chemical imaging technology, the distribution of one or more components of a composition such as minibead, for example, be determined by time-of-flight secondary ion mass spectrometry (ToFSIMS). ToFSIMS imaging can reveal the distribution of one or more components within the composition. The images produced by ToFSIMS analysis or NIR analysis can show the distribution of components across a surface of the composition or a cross section of the composition. The methods described in this paragraph are applicable, for example, to compositions comprising a polymer matrix, e.g. a dried, colloid, solution or dispersion.

**Manufacturing Processes**

Various methods may be used to prepare the modified release compositions of the invention.

In those embodiments where the modified release composition comprises cyclosporin A in a water-insoluble polymer matrix a basic method for making the composition is to mix a fluid form of the matrix material, for example a water-insoluble polymer matrix material (e.g. poly(amides), poly(amino-acids), hyaluronic acid; lipo proteins; poly(esters), poly(orthoesters), poly(urethanes) or poly(acrylamides), poly(glycolic acid), poly(lactic acid) and corresponding co-polymers (poly(lactideco-glycolide acid; PLGA); siloxane, poly siloxane; dimethylsiloxane/methylvinylsiloxane copolymer; poly(dimethylsiloxane/methylvinylsiloxane/¬methylhydrogensiloxane) dimethylvinyl or trimethyl copolymer; silicone polymers; alkyl silicone; silica, aluminium silicate, calcium silicate, aluminium magnesium silicate, magnesium silicate, diatomaceous silica etc. as described more generally elsewhere herein), with cyclosporin A to form mixture that may

take the form of a suspension, solution or a colloid. The mixture is processed to form the composition, for example a minibead. For example the composition may be shaped into the desired form using a molding or hot-melt extrusion process to form beads.

Methods for preparing cores comprising cyclosporin A and a water-soluble polymer matrix are described below. Generally, cores are coated with a modified release coating (and sub-coating) to give the final modified release composition of the invention.

Generally, the manufacturing processes described herein comprise mixing of liquids. Such mixing processes must be performed at temperatures at which the substances to be mixed in the liquid state are in liquid form. For example, thermoreversible gelling agents must be mixed at a temperature where they are in the liquid state, for example at a temperature of 50 to 75°C, for example 50 to 70°C, or 55-75°C, e.g. 60-70°C and in particular embodiments about 55°C or 65°C in the case of mixing compositions comprising aqueous gelatin. Similarly other components of the composition may need to be heated to melt the component for example waxes or surfactants which may be used in the disperse phase.

Cores comprising a hydrogel-forming polymer and cyclosporin A as disclosed herein may be made by mixing materials comprising for example water, a hydrogel-forming polymer and a surfactant to form an aqueous continuous phase, and mixing a disperse phase. At least one of the aqueous phase and the disperse phase comprises cyclosporin A. Suitably both phases may be a clear liquid before they are mixed together. For example, the disperse phase may comprise cyclosporin A (for example a disperse phase comprising an oil, an optional surfactant, cyclosporin A and a surfactant) with the aqueous phase to form a colloid. The colloid may have the form of an emulsion or microemulsion wherein the cyclosporin A disperse phase is dispersed in the aqueous continuous phase. The hydrogel-forming polymer is then caused or allowed to gel. Suitably, the process includes formulating or processing the core composition into a desired form, e.g. a minibead, which forming process may comprise moulding but preferably comprises ejecting the aqueous colloid through a single orifice nozzle to form droplets which are caused or allowed to pass into a cooling medium, e.g. a water-immiscible cooling liquid, in which the droplets cool to form for e.g. minibeads.

The mixing of the materials may comprise mixing an aqueous pre-mix (or aqueous phase) and a disperse phase pre-mix (e.g. oil phase pre-mix), wherein the aqueous pre-mix comprises water and water-soluble substances whilst the disperse phase pre-mix may comprise for example a vehicle containing an active ingredient. The vehicle may be a hydrophobic liquid, for example a liquid lipid, or it may be or comprise a material, for example a surfactant, for forming self-assembly structures. In particular, a disperse phase pre-mix may comprise cyclosporin A, oil and other oil soluble components for example surfactant and optional solvents. The pre-mixes may contain one or more surfactants suitable for the phase they are to form, as previously mentioned.

The aqueous pre-mix comprises, or usually consists of, a solution in water of water-soluble constituents, namely the hydrogel-forming polymer and water-soluble excipient(s). The aqueous pre-mix may include a plasticiser for the hydrogel-forming polymer, as described elsewhere in this specification. The aqueous pre-mix may include a surfactant, e.g. to increase polymer viscosity and improve emulsification and thereby help prevent precipitation of active agent during processing. SDS is an example of such a surfactant. In any event, the constituents of the aqueous pre-mix may be agitated for a period sufficient to dissolve/melt the components, for example, from 1 hour to 12 hours to form the completed aqueous pre-mix.

The disperse phase pre-mix may comprise cyclosporin A as a dispersion or preferably a solution in a vehicle as described above, for example in a liquid comprising an oil and/or surfactant as described above. For example the oil phase pre-mix may therefore be a liquid lipid, for example a medium chain triglyceride (MCT) composition, the medium chain triglyceride(s) being one or more triglycerides of at least one fatty acid selected from $C_6$-$C_{12}$ fatty acids and cyclosporin A. Suitably the oil phase pre-mix is stirred at ambient temperature to form a solution of the cyclosporin A in the oil. In some embodiments, the components of the oil phase pre-mix are mixed (or otherwise agitated) for a period of, for example, 10 minutes to 3 hours to form the pre-mix.

The two pre-mixes may be combined and agitated, for example for a period of a few seconds to an hour, for example from 30 seconds to 1 hour, suitably 5 mins to an hour, to form a dispersion of the disperse phase in an aqueous hydrogel-forming polymer, which dispersion may then be further processed to form the final formulation. The two pre-mixes may be combined into the dispersion by agitation in a mixing vessel; they may additionally or alternatively be combined in a continuous flow mixer.

The basic method for making a core comprising cyclosporin A and hydrogel-forming polymer matrix, therefore, is to mix a liquid form (preferably a solution) of the hydrogel-forming polymer (or mixture of polymers) with the cyclosporin A (and other disperse phase components) to form a dispersion in the polymer, which later in the process forms a hydrogel. The method normally comprises mixing together an aqueous polymer phase pre-mix and a disperse phase pre-mix. Taking account of the final composition required (as described elsewhere herein), the disperse phase pre-mix and the fluidic hydrogel-forming polymer (i.e. the solution or suspension of hydrogel-forming polymer) may be mixed in a weight ratio of from 1:1 to 1:10, particularly 1:4 to 1:9, e.g. 1:5 to 1:8, preferably approximately 1:7. In general, only gentle stirring of the components is required using a magnetic or mechanical system e.g. overhead stirrer as would be familiar to a person skilled in the art to achieve a dispersion of the disperse phase in the aqueous phase to form a colloid (which

may be in the form of for example an emulsion or micro emulsion in which the aqueous hydrogel is the continuous phase). Continuous stirring is preferred. Mixing may also be achieved using an in-line mixing system. Any appropriate laboratory stirring apparatus or industrial scale mixer may be utilized for this purpose for example the Magnetic Stirrer (manufactured by Stuart) or Overhead Stirrer (by KNF or Fisher). It is preferred to set up the equipment in such a way as to minimise evaporation of contents such as, for example, water. In one embodiment of the process of the invention, it is preferred to utilise a closed system for stirring in order to achieve this aim. In-line mixing may be particularly suitable for closed system processing. Suitably mixing of the two components takes place at a temperature of 50 to 70°C, or 55-75°C, e.g. 60-70°C.

The mixing of the two phases results in a colloid wherein the aqueous hydrogel-forming polymer is an aqueous continuous phase and the component(s) not soluble in the aqueous phase, including cyclosporin A are a disperse phase. The colloid may have the form of an emulsion or microemulsion.

The amount of the surfactant in the disperse phase pre-mix may be selected such that upon combination of the disperse phase pre-mix with the aqueous pre-mix the surfactant concentration in the combined mixture exceeds the CMC for the surfactant used such that micelles are formed in the aqueous phase comprising the hydrogel-forming polymer. Depending on the concentration of surfactant used self-assembly structures other than micelles may also form. The CMC for a particular surfactant may be determined using well known methods, for example as described in Surfactants and Polymers in Aqueous Solutions Second Edition, Chapter 2, Holmberg et al. In embodiments mixing of the aqueous and disperse phase which is or comprises a surfactant may result in the formation of a clear liquid, for example a microemulsion, in which the aqueous phase comprising the hydrogel-forming polymer is the continuous phase. Microemulsions are a thermodynamically stable dispersion of self-assembly structures in the aqueous phase, the size of the self-assembly structures being sufficiently small to give a transparent appearance. The size of the self-assembly structures present as the disperse phase resulting from the mixing of the aqueous and surfactant phases may be from about 0.5 nm to 200 nm, for example about 1 nm to 50 nm, or about 5 nm to 25 nm. The size of the self-assembly structures formed and other characteristics such as the optical isotropicity of the composition (for example a microemulsion) may be determined using well known techniques such as dynamic light scattering.

Where the polymer matrix substantially consists of gelatin with the addition of sorbitol, the aqueous phase of polymer matrix is prepared by adding the appropriate quantities of sorbitol (and surfactant if desired) to water, heating to approximately 50 to 75°C, for example 60-75°C until in solution and then adding gelatin although the precise order and timing of addition is not critical. A typical "gelatin solution" comprises 8 to 35%, (for example 15-25%, preferably 17-18%) gelatin; 65%-85% (preferably 77-82%) of water plus, optionally, from 1-5% (preferably 1.5 to 3%) sorbitol. When present surfactant (e.g. anionic surfactant) in the aqueous phase pre-mix may be present in an amount of 0.1 to 5% (preferably 0.5 to 4%) wherein all parts are by weight of the aqueous phase.

Optionally the processing temperature required for standard gelatin can be reduced to a desirable target temperature e.g. 37°C by use of lower melting-point gelatin (or gelatin derivatives or mixtures of gelatins with melting point reducers) or other polymer matrix material such as, for example, sodium alginate. If gelatin droplets are being formed by machine extrusion and immediately cooled e.g. in a cooling bath, additional appropriate inlet tubing can be used to introduce an oil phase containing cyclosporin A at ambient temperature into the hotter fluid gelatin solution (and the mixture can be immediately homogenized) very shortly before ejection from a beading nozzle or other dropletting process such that the duration of exposure of the cyclosporin A to the higher temperature gelatin is limited so reducing the degree of any heat-dependent degradation of the active ingredient. This process may use any appropriate device such as, for example, a homogenizer, e.g. a screw homogenizer, in conjunction with an extrusion-type apparatus as described for example in WO 2008/132707 (Sigmoid Pharma).

The colloid is formed by combining the disperse phase pre-mix with the liquid aqueous phase with stirring as described above. The resultant colloidal dispersion then has the composition of a solidified core described above but with liquid water still present in the core composition.

Optionally the cyclosporin A may be added after mixing the aqueous phase and other components of the disperse phase of the type comprising a vehicle in addition to the cyclosporin A, however, it is preferred that the cyclosporin A is added together with any other components of the disperse phase as a pre-mix.

The resulting colloid is then poured or introduced into a mould or other vessel or poured onto sheets or between sheets or delivered dropwise (or extruded) into another fluid such that the polymer matrix-containing aqueous phase, on solidification, takes the form of the mould, vessel, sheet or droplet/bead intended. It is preferred to progress to mould-forming e.g. beading without delay.

Solidification (gelling) can occur in a variety of ways depending on the polymer of the matrix, for example by changing the temperature around the mould, vessel, sheet, droplet/bead etc or by applying a solidification fluid or hardening solution so that the moulded shape is gelled or solidified. In certain embodiments both temperature change and application of a solidifying fluid or hardening solution are employed together or simultaneously.

In the preferred embodiment in which the core comprising cyclosporin A takes the form of minibeads, the minibeads may be formed for example by dropping the colloid dropwise into a fluid which effects solidification. Where

the viscosity of the composition to be beaded reaches a certain point, drop formation becomes more difficult and specialised apparatus is then preferred.

By use of the term "dry", it is not sought to imply that a drying step is necessary to produce the dry core (although this is not excluded) rather that the solid or solidified aqueous external phase is substantially free of water or free of available water. Solidification of the aqueous phase (external phase) may have arisen through various means including chemically (e.g. by cross-linking) or physically (e.g. by cooling or heating). In this respect, the term "aqueous phase" is nevertheless employed in this document to denote the external (continuous) phase of the core even though water, in certain embodiments, is largely absent from (or trapped within the cross-linked matrix of) the core. The external phase of the core is however water-soluble and dissolves in aqueous media.

In the case where solidification can be achieved by raising or reducing temperature, the temperature of the solidification fluid can be adapted to achieve solidification of the core at a desired rate. For example, when gelatin is used as the hydrogel-forming polymer, the solidification fluid is at a lower temperature than the temperature of the emulsion thus causing solidification i.e. gelling of the polymer matrix. In this case, the solidification fluid is termed a cooling fluid.

In the case where solidification can be achieved chemically, e.g. by induction of crosslinking on exposure to a component of the solidification fluid, the concentration of such component in the solidification fluid and/or its temperature (or other characteristic or content) can be adjusted to achieve the desired rate and degree of solidification. For example, if alginate is chosen as the polymer matrix, one component of the solidification fluid may be a calcium-containing entity (such as, for example, calcium chloride) able to induce cross-linking of the alginate and consequent solidification. Alternatively, the same or similar calcium-containing entity may be included (e.g. dispersed) in the aqueous phase of the fluid emulsion prior to beading and triggered to induce cross-linking e.g. by applying a higher or lower pH to a solidification fluid into which droplets of emulsion fall dropwise or are introduced. Such electrostatic cross-linking can be varied as to the resulting characteristics of the minibead by control of calcium ion availability (concentration) and other physical conditions (notably temperature).The solidification fluid may be a gas (for example air) or a liquid or both. For example, when gelatin is used as the hydrogel-forming polymer matrix, the solidification fluid can be initially gaseous (e.g. droplets passing through cooling air) and then subsequently liquid (e.g. droplets passing into a cooling liquid). The reverse sequence may also be applied while gaseous or liquid cooling fluids alone may also be used. Alternatively, the fluid may be spray-cooled in which the emulsion is sprayed into a cooling gas to effect solidification.

In the case of gelatin or other water-soluble polymer (or polymer mixture) destined to form an immobilization matrix, it is preferred that the solidification fluid be a non-aqueous liquid (such as, for example, medium chain triglycerides, mineral oil or similar preferably with low HLB to ensure minimal wetting) which can conveniently be placed in a bath (cooling bath) to receive the droplets of the colloid as they solidify to form the minibeads of the core. Use of a non-aqueous liquid allows greater flexibility in choice of the temperature at which cooling is conducted.

Where a liquid cooling bath is employed, it is generally maintained at less than 20°C, preferably maintained in the range 5-15°C, more preferably 8-12°C when standard gelatin is used as the hydrogel-forming polymer. If a triglyceride is chosen as the cooling fluid in the cooling bath, a preferred example is Miglyol 810 from Sasol.

If alginate is selected as the polymer matrix, a typical method of making minibeads involves dropwise addition of a 3% sodium alginate solution in which oil droplets are dispersed as described above into a 4°C crosslinking bath containing 0.1 M calcium chloride to produce calcium alginate (this method can be referred to as "diffusion setting" because the calcium is believed to diffuse into the minibeads to effect cross-linking or setting). Using a syringe pump, or Inotech machine, droplets can be generated or extruded (eg. at 5 mL/h if a pump is used) through a sterile needle or other nozzle (described elsewhere herein) which can be vibrating as discussed elsewhere herein. Airflow of between 15 and 20 L/min through 4.5 mm tubing can be applied downwards over the needle to reduce droplet size if desired. Newly formed minibeads can then be stirred in the calcium chloride bath for up to an hour. If carrageenan is used as the polymer matrix both salt and reduction in temperature e.g. by dropping into cooling oil may be used to obtain solidification.

An alternative approach when using alginate is internal gelation in which the calcium ions are dispersed in the aqueous phase prior to their activation in order to cause gelation of hydrocolloid particles. For example, this can be achieved by the addition of an inactive form of the ion that will cause crosslinking of the alginate, which is then activated by a change in e.g. pH after sufficient dispersion of the ion is complete (see Glicksman, 1983a; Hoefler, 2004). This approach is particularly useful where rapid gelation is desired and/or where the diffusion approach may lead to loss of drug by diffusion thereof into the crosslinking bath.

Where another ionotropic polymer is used than alginate, suitable analogous processes may be used to those described herein in relation to alginate.

Following shape-forming, moulding or beading, the resultant shapes or forms may be washed then dried if appropriate. In the case of minibeads solidified in a solidification fluid, an optional final step in the method of production described above therefore comprises removal of the solidified minibeads from the solidification fluid. This may be achieved e.g. by collection in a mesh basket through which the solidification fluid (e.g. medium chain triglycerides) is drained and

the minibeads retained and is preferably conducted without delay e.g. as soon as the minibeads have formed or within 5, 10, 15, 20, 25 or 30 minutes of their formation. Excess solidification fluid may then be removed using a centrifuge (or other apparatus or machine adapted to remove excess fluid) followed by drying of the minibeads to remove water or free water and/or removal of some or all of any additional solvent e.g. ethanol or isopropyl alcohol used to dissolve or facilitate dissolution of the active principle in preceding steps optionally followed by washing (e.g. using ethyl acetate) and a subsequent "drying" step to remove excess solvent (e.g. ethyl acetate). Isopropyl alcohol is an example of a solvent which is preferably removed later in processing to reduce residues in the oil or aqueous phase. Drying can be achieved by any suitable process known in the art such as use of a drum drier (e.g. Freund Drum dryer which may be part of the Spherex equipment train if used) with warm air at between 15°C and 25°C, preferably around 20°C leading to evaporation or entrainment of the water by the air. Alternatively, drying may be carried out using of a fluid bed drier (e.g. Glatt GPCG 1.1) with warm air between 40°C and 60°C. Use of gelatin as the polymer matrix (e.g. as principal constituent of the aqueous immobilisation phase) in most cases requires a drying step and for minibeads this is preferably achieved by drying in air as above described. The resultant composition (the composition of the invention) is essentially dry as described in more detail above.

In general, the minibeads may be generated by the application of surface tension between the liquid dispersion (the mixture of the aqueous and surfactant phases) and an appropriate solidification fluid such as, for example, gas or liquid in order to create the spherical or substantially spherical shape of the ultimate minibeads.

Alternatively, the minibeads may be produced through ejection or extrusion of the liquid dispersion through an orifice or nozzle with a certain diameter and optionally subject to selected vibration (using selected frequencies) and/or gravitational flow. Examples of apparatus which may be used to form the minibeads include encapsulation prilling, drop pelletising, spray cooling or spray congealing apparatus, for example, the Freund Spherex, ITAS/Lambo, Globex, Inotech,GEA Niro, Droppo, Buchi, Gelpell processing equipment. Operation of the Spherex apparatus manufactured by Freund as may be desired to manufacture minibeads according to the present invention is described in US patent 5,882,680 (Freund). It is preferred to select a vibrational frequency in the region of 2-200 Hz suitably 10-15 Hz although the ultimate choice (and separately the amplitude of vibration selected) depends on the viscosity of the dispersion to be beaded. If the polymer matrix is chosen to solidify at lower temperature, it may be appropriate to maintain the lines to the orifice/nozzle at a certain temperature to maintain the fluidity of the solution. Suitably the colloid is ejected through a single-orifice nozzle, e.g. having a diameter of from 0.1 mm to 5 mm (for example 0.5-5 mm), to form drops which are then caused or allowed to fall into a cooling oil or other hardening medium and allowed to harden to form seeds, after which the seeds are recovered from the cooling oil and dried.

Therefore, a process for manufacturing a core comprising cyclosporin A in a polymer matrix comprises: forming an aqueous pre-mix which comprises water and water-soluble/dispersible materials (including therefore a hydrogel-forming polymer) and a disperse pre-mix (e.g. an oil phase pre-mix) which comprises cyclosporin A and optionally a vehicle and other excipients (e.g. oil(s) and oil soluble/dispersible materials), and combining the two pre-mixes to form a colloid (disperse phase) within an aqueous phase comprising the hydrogel-forming polymer. The colloid may then be formed into a shaped unit, for example a minibead to provide the core comprising the cyclosporin A. More particularly the manufacture of a core comprising cyclosporin A and a polymer matrix (suitably a hydrogel-forming polymer matrix may comprise:

(i) forming an aqueous phase pre-mix comprising a solution in water of water-soluble constituents (i.e. of a hydrogel forming polymer, any water-soluble excipient(s), as described elsewhere herein);
(ii) forming a disperse phase pre-mix typically comprising a dispersion or preferably a solution of cyclosporin A in a liquid, optionally where the liquid is an oil (and optionally together with other disperse phase constituents (e.g. surfactant, solvents etc as described elsewhere herein));
(iii) mixing the aqueous phase pre-mix (i) and the disperse phase pre-mix (ii) to form a colloid;
(iv) ejecting the colloid through a nozzle to form droplets;
(v) causing or allowing the a hydrogel forming polymer to gel or solidify to form a water-soluble polymer matrix; and
(vi) drying the solid.

Some manufacturing processes comprise steps (A) to (D) below or, alternatively, a manufacturing process may comprise a single one or any combination of steps (A) to (D).

<u>(A) Exemplary Preparation of Aqueous Phase:</u>

Aqueous phase components are added to water, e.g. purified water, under agitation e.g. sonication or stirring. The temperature is gradually increased, for example to 60-70° C and in particular 65°C, to achieve complete dissolution of the solids. The aqueous phase components include a hydrogel forming polymer, e.g. gelatin or agar and optionally one or more other excipients, for example D-sorbitol (a plasticiser) and surfactant (for example SDS). Possible aqueous

phase components are described elsewhere herein.

The gelatin may be Type A gelatin. In some less preferred implementations, the gelatin is Type B. The gelatin may have a Bloom strength of 125-300, optionally of 200-300, for example of 250-300, and in particular 275. The components of the aqueous phase may be agitated for a period of, for example, from 1 hour to 12 hours to complete preparation of the aqueous phase (aqueous pre-mix).

(B) Exemplary Preparation of Disperse Phase:

Cyclosporin A is mixed with other disperse phase components (for example an oil, surfactant and co-solvent) under agitation e.g. sonication or stirring, suitably at ambient temperature to disperse or preferably dissolve the cyclosporin A.

(C) Exemplary Mixing of the two phases

The aqueous phase and the disperse phase are mixed. The two phases may be mixed in a desired weight; for example, the weight ratio of disperse phase to aqueous phase may be from 1:1 to 1:10, e.g. from 1:4 to 1:9 and optionally from 1:5 to 1:8 such as about 1:5 or about 1:7. The resulting colloid is agitated, e.g. sonicated or stirred, at a temperature of 60-70°C and in particular 65°C, to achieve a homogeneous dispersion, then the homogenous dispersion is formed into minibeads. In particular, the homogenous dispersion is ejected through a single orifice nozzle to form droplets which fall into a cooling medium. The nozzle is suitably vibrated to facilitate droplet formation. The nozzle may be vibrated at a frequency of 2-200 Hz and optionally 15-50 Hz. Alternatively the dispersion (colloid) can simply be ejected from the nozzle without vibration to form droplets.

The cooling medium may for example be air or an oil; the oil is suitably physiologically acceptable as, for example, in the case of medium chain triglycerides e.g. Miglyol 810N. The cooling medium may be at a cooling temperature often of less than 15°C, for example of less than 10°C but above 0°C. In some embodiments the cooling temperature is 8-10°C. The nozzle size (diameter) is typically from 0.5 to 7.5mm, e.g. from 0.5 to 5mm and optionally from 0.5 to 4mm. In some embodiments, the nozzle diameter is from 1 to 5mm for example from 2 to 5mm, and optionally from 3 to 4mm, and in particular may be 3.4mm.

The flow rate through a 3.4mm nozzle is 5 to 35 g/min and optionally 10 to 20 g/min and for nozzles of different sizes may be adjusted suitably for the nozzle area.

(D) Exemplary Processing of minibeads

Cooled minibeads are recovered, for example they may be recovered from cooling oil after a residence time of 15-60 minutes, for example after approximately 30 minutes. Beads recovered from a cooling liquid (e.g. oil) may be centrifuged to eliminate excess cooling liquid, and then dried. Suitably, drying is carried out at room temperature, for example from 15-25°C and optionally from 20-25°C. The drying may be performed in a drum drier, for example for a period from 6 to 24 hours, e.g. of about 12 hours in the case of minibeads dried at room temperature. The dried minibeads may be washed, suitably with a volatile non-aqueous liquid at least partially miscible with water, e.g. they may be washed with ethyl acetate. The washed minibeads may be dried at room temperature, for example from 15-25°C and optionally from 20-25°C. The drying may be performed in a drum drier, for example for a period from 6 to 48 hours, e.g. of about 24 hours in the case of minibeads dried at room temperature. Drying may be achieved by any suitable means, for example using a drum dryer, suitably under vacuum; or by simply passing warm air through the batch of minibeads, or by fluidising the minibeads in a suitable equipment with warm air, for example if a fluid bed dryer. Following drying, the minibeads are passed through a 1 to 10 mm, optionally 2 to 5 mm to remove oversized beads and then through a sieve with a pore size of 0.5 to 9 mm optionally 1 to 4 mm to remove undersized beads.

It can be appreciated that it is possible to recycle the minibeads that are rejected by the sieving process.

As a further aspect there is provided a composition obtainable by (having the characteristic of) any of the processes described herein. It is to be understood that the processes described herein may therefore be used to provide any of the specific cores described in embodiments herein by dispersing the appropriate components which form the disperse phase of the core in the appropriate components which form the aqueous continuous matrix phase of the core.

The preceding paragraphs describe the formation of uncoated cores comprising cyclosporin A in for example a hydrogel-forming polymer matrix. The cores are suitably coated to provide a modified release composition. Suitably the cores are first coated with a subcoat and is then further coated with a modified release coating. Suitable sub coats and modified release coatings are any of those described herein. Optionally the composition is further coated with an optional outer protective coating as described herein. The coating(s) may be applied using well known methods, for example spray coating as described below to give the desired sub coat and modified release coating weight gains.

With regard to one of the methods described above (ejection of emulsion through an optionally vibrating nozzle)

with two concentric orifices (centre and outer), the outer fluid may form a coating (outside the minibead)as described herein. The Spherex machine manufactured by Freund (see US patent 5,882,680 to Freund) is preferably used. Other similar ejection or extrusion apparatus may also be used, for example the ejection apparatus described hereinbefore.

Use of the Spherex machine achieves very high monodispersity. For example, in a typical 100g, batch 97g of minibeads were between 1.4 to 2 mm diameter or between 1 and 2 mm. Desired size ranges can be achieved by methods known in the art for rejecting/screening different sized particles. For example, it is possible to reject/screen out the larger/smaller minibeads by passing a batch first through e.g. a 2 mm mesh and subsequently through a 1.4 mm mesh.

The 1.4 to 2mm diameter range is a good size if it is desired to spray coat the minibeads (if smaller, the spray of the coating machine may bypass the minibead; if too large, the minibeads may be harder to fluidise which is necessary to achieve consistent coating).

**Coating Process**

The coating process can be carried out by any suitable means such as, for example, by use of a coating machine which applies a solution of a polymer coat (as described above in particular) to the composition. Polymers for coating are either provided by the manufacturer in ready-made solutions for direct use or can be made up before use following manufacturers' instructions.

Coating is suitably carried out using a fluid bed coating system such as a Wurster column to apply the coating(s) to the cores. Appropriate coating machines are known to persons skilled in the art and include, for example, a perforated pan or fluidized-based system (including top spray, bottom spray and radial spray variants). Specific examples include the GLATT, Vector (e.g. CF 360 EX), ACCELACOTA, Diosna, O'Hara and/or HICOATER processing equipment. To be mentioned is the MFL/01 Fluid Bed Coater (Freund) used in the "Bottom Spray" configuration.

Typical coating conditions are as follows:

| Process Parameter | Values |
|---|---|
| Fluidising airflow (m3/h) | 20-60 (preferably 30-60) |
| Inlet air temperature (°C) | 20 - 65 |
| Exhaust air temperature (°C) | 20 - 42 |
| Product temperature (°C) | 20 - 45 (preferably 40 to 42) |
| Atomizing air pressure (bar) | Up to 1.4 e.g. 0.8-1.2 |
| Spray rate (g/min) | 2-10 and 3-25 RPM |

Suitably the coating is applied as a solution or dispersion of the polymers (and other components) of the coating. Generally the coatings are applied as an aqueous, solution of dispersion, although other solvent systems may be used if required. The coating dispersion is applied to the cored as a spray in the fluid bed coater to give the required coating weight gain. Generally the coating process is carried out at a temperature which maintains the cores at a temperature of from 35 to 45°C, preferably 40 to 42°C.

After applying the coating, the composition may be dried, for example by drying at 40 to 45°C.

The disclosure further provides a product having the characteristics of a composition obtained as described herein, a product defined in terms of its characteristics being defined by the characteristics of the composition to the exclusion of the method by which it was made.

As mentioned herein the processes described may be used to provide any of the compositions described in the various embodiments herein. By way of example there is provided a modified release composition of the invention comprising a core and a modified release coating wherein the core comprises a hydrogel forming polymer matrix comprising gelatin, cyclosporin A, medium chain mono-di- or tri-glycerides, a co-solvent and surfactant, the core having the characteristics of a core obtained by the process comprising steps (i) to (vi) described above for forming the core, wherein the aqueous phase pre-mix in step (i) of the process comprises gelatin and surfactant (suitably an anionic surfactant), and the disperse phase pre-mix in step (ii) of the process comprises medium chain mono-di- and/or tri-glycerides, cyclosporin A, surfactant (suitably a non-ionic surfactant) and cosolvent (for example 2-(2-ethoxyethoxy)ethanol e.g. Transcutol P); and wherein the core is coated with a first coating (sub coating) comprising a water-soluble cellulose ether or a water-soluble derivative of a cellulose ether and the sub-coated core is coated with a modified release coating; wherein the first coating (subcoating) and the modified release coating are any of those described herein.

In the cores described herein to which the following characteristics are applicable, e.g. in the immediately preceding paragraph, the following characteristics may be present:

gelatin may be present in an amount of in an amount of 300 to 700 mg/g;

the medium chain mono-, di- or tri-glycerides (for example caprylic/capric triglyceride) may be present in an amount of 20 to 200 mg/g;

co-solvent (for example 2-(ethoxyethoxy)ethanol) may be present in an amount of 150 to 250 mg/g;

non-ionic surfactant (for example sorbitan-based surfactants, PEG-fatty acids, or glyceryl fatty acids or poloxamers or particularly a polyethoxylated castor oil for example Kolliphor™ EL) may be present in an amount of 80 to 200 mg/g;

anionic surfactant (for example, alkyl sulfates, carboxylates or phospholipids (particularly SDS)) may be present in an amount of 15 to 50 mg/g; and

cyclosporin A may be present in an amount of from 60 to 150 mg/g, suitably 80 to 100mg/g, for example 81 to 98 mg/g;

wherein all weights are based upon the dry weight of the core before coating.

The core is coated with a first coating (sub-coating) and a modulated release coating outside the first coating; wherein the first coating is or comprises a water-soluble cellulose ether or a water-soluble derivative thereof, particularly hydroxypropylmethyl cellulose; the first coating being present in an amount corresponding to a weight gain due to the first coating in a range selected from: (i) from 1% to 20%; (ii) from 8% to 12%, for example about 10%; (iii) from 4% to 6%, for example about 5%; %; or (iv) about 6% to about 10%, for example about 7%, about 7.5%, about 8%, about 8.5%, about 9% or about 9.5% by weight based upon the weight of the core prior to applying the first coating; and wherein preferably, any modified release coating, especially in the embodiments of the immediately preceding paragraphs, is or comprises a pH independent modified release coating, more especially a modified release coating comprising ethyl cellulose (e.g. Surelease™) still more particularly a modified release coating comprising ethyl cellulose and a water-soluble polysaccharide, for example pectin (e.g. a Surelease™ pectin coating as described herein); and wherein the second coating (modified release coating) is present in an amount corresponding to a weight gain of the composition due to the second coating selected from (a) from 5 to 40%; (b) from 10% to 12%, for example about 11% or about 11.5%; (c) from 16% to 18%, for example about 17%; or (d) from about 8% to about 12%, for example about 8.5%, about 9%, about 9.5%, about 10%, about 10.5% or about 11% by weight based upon the weight of the composition prior to applying the second coating.

In the embodiments of the above paragraphs, only the compositions according to claims 1 and 2 are according to the invention.

## Applications

The modified release compositions of the invention provide a unique combination of pharmacokinetic profile and cyclosporin A release which results in low systemic exposure to cyclosporin A, whilst providing high levels of cyclosporin A in the lower GI tract, particularly in the colon. Such compositions release the cyclosporin A in an active form for example as a solution, which provides enhanced absorption of cyclosporin A in the local tissue of the lower GI tract. When the composition is used in the form of minibeads, the minibeads are advantageously dispersed along large sections of the GI tract following oral administration and are therefore expected provide a more uniform exposure to cyclosporin to large sections of for example the colon.

Accordingly the modified release compositions according to the invention are expected to be useful in the local treatment or prevention of a condition of the GIT. In particular the composition of the invention may be useful in the prevention or treatment of inflammatory conditions affecting the lower GI tract, particularly conditions affecting the colon.

The composition of the invention is administered orally. The dose required will vary depending upon the specific condition being treated and the stage of the condition. Generally the composition will be administered to provide a dose of cyclosporin A of from 0.1 to 100mg , for example a dose of 1 to 500 mg or particularly a dose of 25 to 250mg cyclosporin A, for example a dose of 37.5 mg, 75 mg or 150 mg. The composition is suitably administered as a single daily dose. Optionally the composition may be administered twice per day, for example 37.5 mg, 75 mg or 150 mg twice per day.

In one aspect of the invention there is provided a modified release composition comprising cyclosporin A as described herein for use in the treatment or prophylaxis of an inflammatory bowel disease, Crohn's disease, ulcerative colitis, graft-versus-host disease, gastrointestinal graft-versus-host disease, myasthenia gravis, irritable bowel syndrome (e.g. with constipation, diarrhea and/or pain symptoms), celiac disease, stomach ulcers, diverticulitis, pouchitis, proctitis, mucositis, chemotherapy-associated enteritis, radiation-associated enteritis, short bowel disease, or chronic diarrhea, gastroenteritis, duodenitis, jejunitis, peptic ulcer, Curling's ulcer, appendicitis, colitis, diverticulosis, endometriosis, colorectal carcinoma, adenocarcinoma, inflammatory disorders such as diversion colitis, ischemic colitis, infectious colitis,

chemical colitis, microscopic colitis (including collagenous colitis and lymphocytic colitis), atypical colitis, pseudomembraneous colitis, fulminant colitis, autistic enterocolitis, indeterminate colitis, jejunoiletis, ileitis, ileocolitis or granulomatous colitis, the prevention of rejection following bone marrow transplantation, psoriasis, atopic dermatitis, rheumatoid arthritis, nephrotic syndrome, primary sclerosing cholangitis, familial adenomatous polyposis, or perinanal Crohn's, including perianal fistulae.

In one aspect of the invention there is provided a modified release composition of the invention for use in the treatment or prophylaxis of an inflammatory bowel disease, irritable bowel syndrome, Crohn's disease, ulcerative colitis, celiac disease, graft-versus-host disease, gastrointestinal graft-versus-host disease, gastroenteritis, duodenitis, jejunitis, ileitis, peptic ulcer, Curling's ulcer, appendicitis, colitis, pseudomembraneous colitis, diverticulosis, diverticulitis, pouchitis, endometriosis, colorectal carcinoma or adenocarcinoma.

In one embodiment the modified release composition of the invention is for use in the treatment of inflammatory bowel disease. The main forms of inflammatory bowel disease are Crohn's disease and ulcerative colitis. Accordingly the composition of the invention may be for use the treatment of Crohn's disease and/or ulcerative colitis.

The modified release composition of the invention may be for use in the treatment or prevention of irritable bowel syndrome (e.g. with constipation, diarrhea and/or pain symptoms), celiac disease, stomach ulcers, diverticulitis, pouchitis, proctitis, mucositis, radiation-associated enteritis, short bowel disease, or chronic diarrhea, gastroenteritis, duodenitis, jejunitis, peptic ulcer, Curling's ulcer, appendicitis, colitis, diverticulosis, endometriosis, colorectal carcinoma, adenocarcinoma, inflammatory disorders such as diversion colitis, ischemic colitis, infectious colitis, chemical colitis, microscopic colitis (including collagenous colitis and lymphocytic colitis), atypical colitis, pseudomembraneous colitis, fulminant colitis, autistic enterocolitis, indeterminate colitis, jejunoiletis, ileitis, ileocolitis, granulomatous colitis, fibrosis, graft-versus-host disease, gastrointestinal graft-versus-host disease, HIV prophylaxis and treatment (for example HIV enteropathy) or gastrointestinal enteropathies.

Crohn's disease may affect the entire GI tract including the colon. However, ulcerative colitis is a condition which affects only the colon and the rectum. Accordingly, the specific pharmacokinetic and cyclosporin A release profile provided by the modified release composition according to the invention are expected to be beneficial for use in the treatment or prevention of a range of inflammatory gastrointestinal diseases affecting the colon such as ulcerative colitis.

The composition of the invention primarily releases cyclosporin A in the colon. However, drug may also be released higher in the GI tract and accordingly the composition may also be for use in the treatment or prevention of conditions which affect other parts of the lower GI tract, for example Crohn's disease, irritable bowel syndrome (e.g. with constipation, diarrhea and/or pain symptoms), celiac disease, stomach ulcers, diverticulitis, pouchitis, proctitis, mucositis, radiation-associated enteritis, short bowel disease, chronic diarrhea, gastroenteritis, duodenitis, jejunitis, peptic ulcer, Curling's ulcer, appendicitis, diverticulosis, endometriosis, colorectal carcinoma, adenocarcinoma, inflammatory disorders such as, jejunoiletis, ileitis, ileocolitis, celiac disease, fibrosis, graft-versus-host disease, gastrointestinal graft-versus-host disease, HIV prophylaxis and treatment (for example HIV enteropathy) or enteropathies.

Gastrointestinal Graft-Versus-Host-Disease (GI-GVHD) is a life-threatening condition and one of the most common causes for bone marrow and stem cell transplant failure. In patients with GI-GVHD it is the donor cells that begin to attack the patient's body - most frequently the gut, liver and skin. Patients with mild-to-moderate GI GVHD typically develop symptoms of anorexia, nausea, vomiting and diarrhoea. If left untreated, GI GVHD can progress to ulcerations in the lining of the GI tract, and in its most severe form, can be fatal. Accordingly, in one embodiment the modified release composition is for use in the treatment or prophylaxis of Gastrointestinal Graft-Versus-Host-Disease (GI-GVHD).

In a further embodiment there is provided a modified release composition of the invention for use in the treatment or prophylaxis of celiac disease.

In a further embodiment there is provided a modified release composition of the invention for use in the treatment or prophylaxis of ulcerative colitis.

Also provided is a composition modified release composition of the invention is for use in the treatment of neurodegenerative diseases (for example Parkinson's disease, Alzheimer's disease or vascular dementia) or paediatric diseases, including, but not limited to ulcerative colitis, Crohn's disease and GvHD.,

## Examples

### Example 1: Preparation of Minibead Modified Release Compositions (not according to the present invention)

Formulations I, II III and a Comparative Formulation were prepared using the process described below.

**Formulation I:** "Medium" coating level (10% weight gain Opadry subcoat; 11% weight gain Surelease™/Pectin overcoat)

| | Component | % |
|---|---|---|
| Core | Cyclosporin A | 8.8 |
| | Miglyol 810 N | 3.8 |
| | Transcutol HP | 13.5 |
| | Kolliphor™ EL | 7.6 |
| | SDS | 3.3 |
| | Sorbitol | 4.7 |
| | Gelatin | 40.3 |
| Sub-Coat | Opadry | 8.2 |
| Overcoat | Surelease™ (solid contents) | 9.7 |
| | Pectin | 0.2 |

**Formulation II:** "High" coating level (10% weight gain Opadry subcoat; 17% weight-gain Surelease™/Pectin overcoat)

| | Component | % |
|---|---|---|
| Core | Cyclosporin A | 8.4 |
| | Miglyol 810 N | 3.6 |
| | Transcutol HP | 12.8 |
| | Kolliphor™ EL | 7.2 |
| | SDS | 3.1 |
| | Sorbitol | 4.4 |
| | Gelatin | 38.3 |
| Sub-coat | Opadry | 7.8 |
| Overcoat | Surelease™ (solid contents) | 14.2 |
| | Pectin | 0.3 |

**Formulation III:** 5% Opadry subcoat; 11.5 % Surelease™/Pectin overcoat)

| | Component | % |
|---|---|---|
| Core | Cyclosporin A | 9.2 |
| | Miglyol 810 N | 3.9 |
| | Transcutol HP | 14.0 |
| | Kolliphor™ EL | 7.9 |
| | SDS | 3.4 |
| | Sorbitol | 4.9 |
| | Gelatin | 42.1 |
| Sub-coat | Opadry | 4.3 |

(continued)

| | Component | % |
|---|---|---|
| Overcoat | Surelease™ (solid contents) | 10.1 |
| | Pectin | 0.2 |

**Comparative Formulation (No Surelease™ Pectin overcoat)**

| | Component | % |
|---|---|---|
| Core | Cyclosporin A | 9.8 |
| | Miglyol 810 N | 4.2 |
| | Transcutol HP | 14.9 |
| | Kolliphor™ EL | 8.4 |
| | SDS | 3.6 |
| | Sorbitol | 5.2 |
| | Gelatin | 44.8 |
| Sub-coat | Opadry | 9.1 |

Core Manufacture

The cores in the form of seamless minibeads were prepared using Spherex process as follows.

An aqueous phase was prepared by mixing sodium dodecyl sulfate (SDS) and D-sorbitol with purified water under constant stirring. Gelatin was then added to this solution and gentle heat applied to approximately 60-70° C to achieve complete melting of the gelatin.

An oil phase was prepared by mixing together Transcutol HP, Kolliphor™ EL and Miglyol 810 with stirring at room temperature to form a solution. Cyclosporin A was added and mixed until a clear solution was obtained. The oil phase was mixed with the heated aqueous phase in a ratio of approximately 1:7. The resulting mixture was stirred at 60-70°C to achieve homogeneity.

The resulting mixture was then fed (via temperature controlled tubing) through a vibrating nozzle, with single nozzle outlet with a diameter of 3mm. Seamless minibeads were formed as the solution flowed through the vibrating nozzle into a cooling chamber of constantly flowing medium chain triglyceride (Miglyol 810) cooling oil at a temperature of 10°C.

The minibeads were removed from the cooling oil and placed in a centrifuge to remove the excess oil. Following centrifugation, drying was initiated in a Freund drum dryer with a set refrigerator temperature of 10°C and a heater temperature of 20°C and a drying drum rotation speed at 15 RPM. When the beads were observed to be freely rotating in the drying drum, they were considered to be dry.

The minibeads were washed with ethyl acetate and then dried for a further 24h under the same conditions set out above. The dried minibeads were then sieved to remove oversize and undersize beads resulting in minibeads cores 1mm-2mm in diameter.

**Sub-Coating**

The minibead cores were loaded into a fluid bed coater (Wurster column) and coated with an Opadry White dispersion (Opadry White 20A28380 Ex. Colorcon). During coating the cores were maintained at a temperature of between 40°C and 42°C, by adjusting the fluid bed process parameters such as inlet air temperature and inlet air volume. Coating was continued until the required subcoat weight gain (5 or 10%) was reached. The resulting subcoated minibeads were dried for 5 minutes at 40°C in the coater.

**Over Coating (Modified Release Coating)**

Pectin was added to purified water in a stainless steel vessel and mixed to obtain a solution. Surelease™ was slowly added to the vessel whilst maintaining mixing to provide the required pectin concentration in the Surelease™ for the overcoat. The resulting coating suspension was then applied onto the surface of the sub-coated minibeads until the desired weight gain of Surelease™ pectin overcoat was reached. The over-coated minibeads were then dried in the coater for an hour at 40 to 45°C

**Example 2 Human Pharmacokinetic Study (not according to the present invention) Study Objectives:**

Objective 1: To compare the rate and extent of absorption of cyclosporin-A following administration of Comparative Formulation (fast-release capsule; Test 1), Formulation I (medium-release capsule; Test 2), and Formulation II (slow-release capsule; Test 3) with Neoral™ immediate- release capsule (reference), administered as a single 75 mg dose under fasting conditions.

Objective 2: To evaluate the amount of unchanged cyclosporin-A excreted in the faeces after administration of the Comparative Formulation (fast-release capsule; test 1), Formulation I (medium-release capsule; Test 2), Formulation II (slow-release capsule; Test 3) versus Neoral, administered as a single 75 mg dose under fasting conditions.

**Study Design:**

A single centre, randomised, single-dose, open-label, 4-period, 4-sequence crossover comparative BA study, performed under fasting conditions. Subjects were confined to the Clinical Facility from at least 10 hours prior to drug administration until after approximately 28 hours post-dose (however, the subject was allowed to leave the clinical facility if he had defecated on the morning of Day 2), in each period. The treatment phases were separated by a washout periods of 7 days

**Subjects:**

Enrolled and randomised: 18 (12 females and 6 males) Withdrew consent: 0
Withdrawal: 1 (was withdrawn) Completed all 4 periods: 16
Safety population: 18
Pharmacokinetic (PK) population: 18

**Diagnosis and Main Criteria for Inclusion:**

Subjects had to be healthy, adult, 18 years of age and older, body mass index (BMI) >18.5 and <30.0 kg/m2. All subjects had to be in compliance with the inclusion and exclusion criteria described in the protocol and were judged eligible for enrolment in this study, based on medical and medication histories, demographic data (including sex, age, race, ethnicity), body measurements (body weight [kg], height [cm], and BMI [kg/m2]), vital signs measurements (blood pressure, pulse rate, respiratory rate, and oral temperature), 12-lead electrocardiogram, physical examination, purified protein derivative (PPD) skin test, urine drug screen, urine pregnancy test (female subjects), and clinical laboratory tests (haematology, biochemistry, urinalysis, Human Immunodeficiency Virus, Hepatitis C antibodies, and Hepatitis B surface antigen).

**Treatment**

Subjects were treated using the compositions summarised in Table 1.

Table 1

| **Treatment Identification:** | **Test 1 (Treatment A)** | **Test 2 (Treatment B)** | **Test 3 (Treatment C)** | **Referenc e (Treatment D) (Neoral)** |
|---|---|---|---|---|
| **Strength:** | 25 mg | 25 mg | 25 mg | 25 mg |

(continued)

| Treatment Identification: | Test 1 (Treatment A) | Test 2 (Treatment B) | Test 3 (Treatment C) | Referenc e (Treatment D) (Neoral) |
|---|---|---|---|---|
| **Dosage Form:** | Comparati ve Formulatio n (fast-release capsule) | Formulation I (medium-release capsule) | Formulation II (slow-release capsule) | Neoral™ capsule |
| **Dose Administered:** | 3 x 25 mg | 3 x 25 mg | 3 x 25 mg | 3 x 25 mg |
| **Route of Administration:** | oral | oral | Oral | oral |

**Duration of Treatment:**

A single oral dose of cyclosporin-A as a 3 x 25 mg capsules was administered in each study period. The treatment phases were separated by washout periods of 7 days.

**Sampling Points:**

Blood sample collection:

For Treatments A and D, blood samples were collected prior to drug administration and 0.333, 0.667,1.00, 1.25, 1.50, 1.75, 2.00, 2.50, 3.00, 4.00, 6.00, 8.00, 12.0, 16.0, and 24. 0-hour post-dose in each period.

For Treatments B and C, blood samples were collected prior to drug administration and 1.00, 1.50, 2.00, 2.50, 3.00, 4.00, 5.00, 6.00, 8.00, 10.0, 12.0, 14.0, 16.0, 20.0, and 24. 0-hour post-dose in each period.

Faeces Samples:

Subjects were recommended to defecate upon arrival at the clinical facility on Day -1 (this sample was collected and one aliquot was collected as blank matrix) and were requested to collect their faeces from 12 to 28 hours after dosing.

**Pharmacokinetic Results and Statistical Analysis**

**Data Sets Analyzed**

Of the 18 subjects who were dosed, all completed at least 1 period. In accordance with the study protocol, data from all subjects completing at least 1 period and for whom the PK profile could be adequately characterised were used for PK and statistical analyses (N=18). Note that only subjects who completed at least 2 periods were included in the analysis of variance (ANOVA) (N=18).

**Demographics and Other Baseline Characteristics**

Descriptive statistics of the subjects included in the PK analyses are presented in Tables 2, 3 and 4:

Table 2: Descriptive Statistics of Demographic Data for Subjects Included in the Pharmacokinetic Population (N=17) of Test 1 (Treatment A) and Reference (Treatment D)

| Parameter | Age (years) | Height (cm) | Weight (kg) | BMI ($kg/m^2$) |
|---|---|---|---|---|
| Mean ± SD | 47 ± 13 | 163.9 ± 8.5 | 69.34 ± 10.61 | 25.70 ± 2.44 |
| Range | 25 - 68 | 148.0 - 178.5 | 50.80 - 86.50 | 20.87 - 28.90 |
| Median | 46 | 161.5 | 70.00 | 26.28 |
| BMI: Body mass index; SD: Standard deviation. | | | | |

Table 3: Descriptive Statistics of Demographic Data for Subjects Included in the Pharmacokinetic Population (N=17) of Test 2 (Treatment B)

| Parameter | Age (years) | Height (cm) | Weight (kg) | BMI ($kg/m^2$) |
|---|---|---|---|---|
| Mean ± SD | 49 ± 12 | 164.4 ± 8.7 | 70.15 ± 10.55 | 25.83 ± 2.36 |
| Range | 30 - 68 | 148.0 - 178.5 | 50.80 - 86.50 | 20.87 - 28.90 |
| Median | 49 | 161.5 | 72.30 | 26.28 |
| BMI: Body mass index; SD: Standard deviation. | | | | |

Table 4: Descriptive Statistics of Demographic Data for Subjects Included in the Pharmacokinetic Population (N=18) of Test 3 (Treatment C)

| Parameter | Age (years) | Height (cm) | Weight (kg) | BMI ($kg/m^2$) |
|---|---|---|---|---|
| Mean ± SD | 48 ± 13 | 164.4 ± 8.4 | 69.71 ± 10.41 | 25.69 ± 2.37 |
| Range | 25 - 68 | 148.0 - 178.5 | 50.80 - 86.50 | 20.87 - 28.90 |
| Median | 48 | 162.3 | 71.15 | 26.26 |
| BMI: Body mass index; SD: Standard deviation. | | | | |

## Statistical Methods and Analysis

The actual clock time for dosing and the actual clock time for each collection time were recorded using the electronic data capture. For all sampling times, the actual sampling times were calculated as the difference between the actual clock time of dosing and the sample collection time, rounded to the closest minute. Therefore, the difference between the scheduled and the actual sampling time was considered acceptable if it was less than 30 seconds. When the difference exceeded this time limit, the actual sampling times (rounded off to three decimal digits) were used to calculate pharmacokinetic parameters, except for pre-dose samples, which were always reported as zero (0.000), regardless of time deviations. Scheduled sampling times are presented in concentration tables and graphs in the pharmacokinetic section of the report.

Pharmacokinetic and statistical analyses were performed using Pharsight™ Knowledgebase Server™ (PKS) version 4.0.2 and WinNonlin™ 5.3. These software perform non-compartmental analyses of pharmacokinetic parameters and statistical analyses (via SAS version 9.2) according to current regulatory recommendations.

The number of observations (N), mean, standard deviation (SD), coefficient of variation (CV (%)), range (min. and max.), median and geometric mean were calculated for whole blood concentrations of cyclosporin for each sampling time and treatment, as well for feces concentrations of cyclosporin for each regions of the feces (beginning, middle and end) and treatment. These descriptive statistics were also presented for $AUC_{0\text{-}t}$ (ng·hr/mL), $AUC_{0\text{-}inf}$ (ng·hr/mL), $C_{max}$(ng/mL), Residual area (%), $T_{max}$ (hr), $T½_{el}$ (hr), $K_{el}$ (1/hr), $K_{el\ Lower}$ (hr), and $K_{el\ Upper}$ (hr). The calculation of these pharmacokinetic parameters is explained below.

## Maximum Observed Concentration and Time of Observed Peak Concentration

$C_{max}$, the maximum observed concentration, and $T_{max}$, the time to reach that peak concentration, were determined for each subject and for each treatment.

## Half-Life and Elimination Rate Constant

To calculate the elimination rate constant ($K_{el}$), regression analyses were performed on the natural log (Ln) of whole blood concentration values (y) versus time (x). Calculations were made between a time point where log-linear elimination phase begins ($K_{el\ Lower}$) and the time at which the last concentration above the limit of quantitation ($K_{el\ Upper}$) occurred. The $K_{el}$ was taken as the slope multiplied by (-1) and the apparent half-life ($T_{½\ el}$) as (ln 2)/$K_{el}$.

**$K_{el\ Lower}$ and $K_{el\ Upper}$**

$K_{el\ Lower}$, the time point where In-linear $K_{el}$ calculation begins, and $K_{el\ Upper}$, the sampling time of the last quantifiable concentration used to estimate the $K_{el}$, were determined by the scientist for each subject and for each treatment. Whenever possible, at least 4 non-zero observations during the terminal elimination phase were used to calculate the $K_{el}$. A minimum of 3 observations was used if fewer than 4 observations were available. If the constant ($K_{el}$) could not be measured (e.g.: fewer than 3 non-zero concentrations in the terminal elimination phase) or the determination coefficient ($r^2$ value) from the regression of the In-linear elimination phase was less than 64% (or 0.64) for some subjects (or r value positive or less than 80% or 0.80 in absolute value), then the parameters related to the elimination were not calculated for that individual pharmacokinetic profile. Other parameters from that subject were valid and were therefore reported.

**Areas Under the Concentration-Time Curves**

$AUC_{0-t}$ was calculated using the linear trapezoidal rule.
The $AUC_{0-inf}$ was calculated as:

$$AUC_{0-t} + \frac{C_t}{K_{el}}$$

Where: Ct = the fitted last non-zero concentration for that treatment, $AUC_{0-t}$ = the AUC from time zero to the time of the last non-zero concentration for that treatment and $K_{el}$ = the elimination rate constant.
Residual area (%) was calculated as (1 - ($AUC_{0-t}$/ $AUC_{0-inf}$)) x 100, and was used to calculate the percentage of extrapolated area under the curve.

**Statistical Analysis**

For cyclosporin-A, analysis of variance was performed on the In-transformed data of $AUC_{0-t}$, $AUC_{0-inf}$, and $C_{max}$. ANOVA was also carried out on the untransformed data of $T_{½\ el}$ and $K_{el}$. All ANOVAs were performed with the SAS (version 9.2 for Windows) General Linear Models Procedure (GLM) The model included sequence, subject within sequence, period and treatment as factors. The sequence effect was tested using subjects within sequence effect as the error term. The treatment and period effects were tested against the residual mean square error. All sums of squares (Types I, II, III, and IV) were reported. Probability (p) values were derived from Type III sums of squares. A non-parametric test (Friedman's Signed-Rank test) was carried out to compare the $T_{max}$ between treatments. For all analyses, effects were considered statistically significant if the probability associated with 'F' was less than 0.05. When the difference between treatments was statistically significant, Duncan's Multiple Range Test was used to determine which treatments were significantly different. Based on pairwise comparisons of the In-transformed $AUC_{0-t}$, $AUC_{0-inf}$, and $C_{max}$ data, the ratios of the least-squares means (treatments A/D, B/D, C/D, A/B, A/C, and B/C), calculated according to the formula "e (X-Y) X 100", as well as the 90% geometric confidence intervals for In-transformed $AUC_{0-t}$, $AUC_{0-inf}$, and $C_{max}$ were determined. Finally, the inter- and intra-subject CVs were also determined.

**Analysis of Pharmacokinetics and Statistical Issues**

**Adjustment of Factors in the ANOVA**

As described above, the ANOVA model included sequence, subject within sequence, period and treatment as factors. These factors were chosen as they are the sources of variation that are assumed to have an effect on the pharmacokinetic parameters.

**Pharmacokinetic Analysis**

The pharmacokinetic parameters for this study were $AUC_{0-t}$, $AUC_{0-int}$, $C_{max}$, Residual area, $T_{max}$, $K_{el}$, and $T_{½\ el}$ for cyclosporin-A.
The mean concentration-time profile for cyclosporin-A for each treatment (N = 17 for treatments A, B and D and N=18 for treatment C) is shown in Figure 1 on both linear and log scales.

The mean profiles for both the test and reference formulations are plotted based on the mean whole blood concentration levels calculated per time point. Therefore, the maximum concentrations observed in the mean data figures may not reflect the mean $C_{max}$, as the $C_{max}$ and the time of maximum concentration ($T_{max}$) vary between individuals. Mean pharmacokinetic values are summarized in the Table 5.

Table 5: Summary of pharmacokinetic parameters for cyclosporin-A for each treatment

| Mean ± SD (CV%) | Whole Blood Cyclosporin-A | | | |
|---|---|---|---|---|
| | Cyclosporin-A (Test 1) Comparative Formulation | Cyclosporin-A (Test 2) Formulation I | Cyclosporin-A (Test 3) Formulation II | Neoral |
| N | 17 | 17 | 18 | 17 |
| $AUC_{0-t}$ | 1212.52 ± 297.62 | 609.89 ± 280.15 | 408.49 ± 231.01 | 1582.20 ± 358.09 |
| (ng•hr/mL) | (24.55) | (45.93) | (56.55) | (22.63) |
| $AUC_{0-inf}$ | 1257.83 ± 312.14 | 672.07 ± 296.71 | 474.37 ± 247.93 | 1639.78 ± 371.52 |
| (ng•hr/mL) | (24.82) | (44.15) | (52.27) | (22.66) |
| $C_{max}$ | 321.33 ± 87.61 | 138.28 ± 63.54 | 82.81 ± 48.01 | 594.66 ± 117.01 |
| (ng/mL) | (27.27) | (45.95) | (57.98) | (19.68) |
| Residual Area | 3.55 ± 0.71 | 10.72 ± 8.10 | 15.38 ± 12.69 | 3.52 ± 0.77 |
| (%) | (20.12) | (75.50) | (82.52) | (21.87) |
| $T_{max}$[a] | 2.00 | 5.00 | 5.00 | 1.25 |
| (hr) | (1.25 - 3.00) | (5.00 - 8.00) | (5.00 - 10.0) | (1.00 - 1.75) |
| $K_{el}$ | 0.1105 ± 0.0113 | 0.0863 ± 0.0259 | 0.0822 ± 0.0232 | 0.1037 ± 0.0103 |
| (1/hr) | (10.25) | (30.01) | (28.20) | (9.97) |
| $T_{½ el}$ | 6.33 ± 0.61 | 8.72 ± 2.76 | 9.49 ± 4.55 | 6.75 ± 0.77 |
| (hr) | (9.70) | (31.66) | (47.96) | (11.43) |
| [a] Median (Min - Max) | | | | |

In Table 5 the AUC and Cmax values are the mean value ± standard deviation (SD)

ANOVA performed on the In-transformed data for $AUC_{0-t}$, $AUC_{0-inf}$, and $C_{max}$ detected a statistically significant (p-values < 0.05) difference between treatments for these parameters. The Duncan's Multiple Range Test was also performed on these parameters. The p-values for treatment, period and sequence effects as well as the difference between treatments as per Duncan Test are summarized in Table 6 for these parameters for treatments A,B, C and D.

Table 6: p-values for $AUC_{0-t}$, $AUC_{0-inf}$, and $C_{max}$ for cyclosporin-A

| Parameter | p-values | | | Duncan Test |
|---|---|---|---|---|
| | Treatment | Period | Sequence | |
| $AUC_{0-t}$ | <0.0001 | 0.1047 | 0.3764 | (A = D) > B > C |
| $AUC_{0-inf}$ | <0.0001 | 0.0526 | 02954 | D > A > B > C |
| $C_{max}$ | <0.0001 | 0.2615 | 0.3340 | D > A > B > C |

The least-squares means ratios (A/B), the 90% geometric confidence intervals, intra- and inter-subject CVs were also determined for $AUC_{0-t}$, $AUC_{0-inf}$, and $C_{max}$, respectively. These results are summarized in the Table 7.

Table 7: Ratios, 90% geometric confidence intervals for $AUC_{0-t}$, $AUC_{0-inf}$, and $C_{max}$ for cyclosporin-A (N = 17 for treatments A, B and D and N=18 for treatment C)

| | | | 90% Geometric C.I.[2] | | | |
|---|---|---|---|---|---|---|
| Parameter | Treatment Comparisons | Ratio[1] | Lower | Upper | Intra-Subject CV | Inter-Subject CV |
| | | | | | | |
| $AUC_{0-t}$ | Test-1 (A) - Test-2(B) | 226.02% | 176.65% | 289.20% | 44.06% | 35.58% |
| | Test-1 (A) - Test-3(C) | 363.49% | 285.57% | 462.68% | | |
| | Test-1 (A) - Reference(D) | 76.96% | 60.35% | 98.13% | | |
| | Test-2(B) - Test-3(C) | 160.82% | 126.39% | 204.64% | | |
| | Test-2(B) - Reference(D) | 34.05% | 26.61% | 43.57% | | |
| | Test-3(C) - Reference(D) | 21.17% | 16.63% | 26.96% | | |
| | | | | | | |
| $AUC_{0-inf}$ | Test-1 (A) - Test-2(B) | 209.49% | 169.71% | 258.58% | 37.18% | 34.21% |
| | Test-1 (A) - Test-3(C) | 314.72% | 256.10% | 386.75% | | |
| | Test-1 (A) - Reference(D) | 76.97% | 62.54% | 94.73% | | |
| | Test-2(B) - Test-3(C) | 150.23% | 122.29% | 184.56% | | |
| | Test-2(B) - Reference(D) | 36.74% | 29.76% | 45.36% | | |
| | Test-3(C) - Reference(D) | 24.46% | 19.89% | 30.06% | | |
| | | | | | | |
| $C_{max}$ | Test-1 (A) - Test-2(B) | 281.15% | 198.39% | 398.44% | 65.29% | 29.24% |
| | Test-1 (A) - Test-3(C) | 503.53% | 357.94% | 708.35% | | |
| | Test-1 (A) - Reference(D) | 53.54% | 37.96% | 75.51% | | |
| | Test-2(B) - Test-3(C) | 179.10% | 127.37% | 251.83% | | |
| | Test-2(B) - Reference(D) | 19.04% | 13.43% | 26.99% | | |
| | Test-3(C) - Reference(D) | 10.63% | 7.55% | 14.97% | | |

(continued)

| | | | 90% Geometric C.I.[2] | | | |
|---|---|---|---|---|---|---|
| Parameter | Treatment Comparisons | Ratio[1] | Lower | Upper | Intra-Subject CV | Inter-Subject CV |
| | | | | | | |

[1] Calculated using least-squares means.
[2] 90% Geometric Confidence Interval using In-transformed data.

The mean Residual area was lower than 20% for all treatments. However, 3 subjects out of 18 (16.7%) had a residual area above 20% for the test 2 and test 3 products (for Treatment B, No. 7 = 36.57%, No. 18 = 25.34% and for Treatment C, No. 8 = 38.49%, No. 18 = 57.82%).

Friedman's Test performed on the $T_{max}$ data detected a statistically significant difference between treatments for this parameter (p-value < 0.05). ANOVA performed on the untransformed $T_{½\ el}$ and $K_{el}$ data detected a statistically significant difference between treatments for these parameters (p-values < 0.05)

**Pharmacokinetic and Statistical Conclusions**

A statistically significant difference between treatments was detected using ANOVA for In-transformed $AUC_{0-t}$, $AUC_{0-inf}$, and $C_{max}$ and untransformed $K_{el}$ and $T_{½\ el}$. According to Duncan's Multiple Range Test, the differences in PK parameters between formulations were as follows: (A = D) > B > C for $AUC_{0-t}$ and D > A > B > C for $AUC_{0-inf}$, and $C_{max}$. A statistically significant difference was detected between treatments for $T_{max}$ using Friedman's test.

The mean Residual area was lower than 20% for all treatments. According to the EMA guideline of the investigation of bioequivalence (London, 20 January 2010, Doc. Ref.: CPMP/QWP/EWP/1401/98 Rev. 1), if the Residual area is greater than 20% in more than 20% of the observations then the validity of the study may need to be discussed. In the current study, only 3 subjects out of 18 (16.7%) had a residual area above 20% for the test 2 and test 3 products (for Treatment B, No. 7 = 36.57%, No. 18 = 25.34% and for Treatment C, No. 8 = 38.49%, No. 18 = 57.82%). Therefore it can be concluded that the duration of sampling was sufficient for cyclosporin-A. The intra-subject CVs for $AUC_{0-t}$, $AUC_{0-inf}$, and $C_{max}$ were respectively 44.06%, 37.18%, and 65.29% for cyclosporin-A.

The results of this study suggest that the test-1 (Comparative Formulation), which is the closest to the Neoral™ reference product PK profile, has a lower rate and extent of absorption when compared to the Neoral™ reference as demonstrated with the ratios below 77% for AUCs and below 54% for $C_{max}$. The Formulation I (test-2 medium coating) and Formulation II (test-3 high coating) have a much lower rate and extent of absorption when compared to the Neoral™ reference as demonstrated with the ratios below 37% for AUCs and below 20% for $C_{max}$.

**Determination of cyclosporin-A and its metabolites, AM9 and AM4N, in faecal samples**

***Method:***

Faecal samples collected during the PK trial were analysed by RP-LC-MS/MS with LLE sample clean up as described previously (Fang, et al, Analysis of cyclosporine A and its metabolites in rat urine and faeces by liquid chromatography-tandem mass spectrometry. Journal of Chromatography B. 878(15-16): p. 1153-1162; and Binkhathlan et al, Development of a liquid chromatography-mass spectrometry (LC/MS) assay method for the quantification of PSC 833 (Valspodar) in rat plasma. Journal of Chromatography B, 2008. 869(1-2): p. 31-37.
Samples were analysed for the presence of cyclosporin A (Cyc A) and its metabolites AM9 and AM4N.

**Liquid-Liquid Extraction Method:**

***Sample Extraction***

To an extraction tube 100μL of faecal sample 50μL of Cyc C, 500ng/mL, (ISTD), 150μL of ACN/Water (70/30, v/v), 500μL of water and 2mL of *t*-BME was added. Samples were vortexed, mixed on a blood tube mixer for 5 minutes and centrifuged at 3200g for 5 minutes. The organic layer was removed with a glass pasteur pipette and 1.1mL of solvent was transferred to conical bottomed glass LC autosampler vials (Chromacol). The vials were evaporated to dryness using a Genevac EZ-2 evaporator at ambient temperature, without light. The samples were reconstituted in 100□L of ACN/water, 70/30, v/v, with 20□L injected in duplicate by the autosampler.

***Standard Curve Extraction***

A standard curve was prepared by serial dilution (1 in 5) of each analyte in ACN/water (70/30, v/v), with a highest concentration of 2μg/mL. Of each analyte dilution, 50μL was spiked into blank faecal sample (n=3 for each concentration) along with 50μL of Cyc C, 500ng/mL, 500μL of water and 2mL of *t*-BME. The samples were extracted as described above.

**LC-MS**

***MRM, chromatography and ESI conditions***

The multi reaction monitoring transitions of each analyte, Cyc A, AM4N, AM9 and Cyc C (internal standard) were determined, with respect to precursor ion, product ion, optimum fragmentor voltage and optimum collision energy. Separation of the three analytes and the ISTD was achieved on a Prodigy C18 column (150mm×4.6mm i.d., 5μm particle size) with a SecurityGuard C18 guard column (4mm×3.0mm i.d.) both from Phenomenex, UK. Mobile phase ACN/20mM Ammonium Formate pH 5 (83/17, v/v) was run at a flow rate of 0.8mL/min with isocractic elution. The column temperature was maintained at 60°C and the autosampler was maintained 4°C. The complete chromatographic run time of each sample was 10 minutes, with the first 2.7minutes diverted to waste. The retention times were AM9 3.4 mins, AM4N 4.2 mins, Cyc C 4.9 mins and Cyc A 6.2minutes.

Ionisation was achieved with an ESI source operated in positive mode. The ionisation temperature was 350°C, gas flow rate was 11L/min and nebulizer pressure was 345kPa (50psi). Nitrogen was used as the ionisation source gas and ultrapure nitrogen as the collision cell gas.

**Data Analysis**

All data was analysed using Masshunter Quantification software and exported to Excel.

The standard curve was plotted using a log-log plot of mass and peak area ratio (PAR). PAR is calculated from the integrated peak area of the analyte divided by the peak area of the internal standard. A log-log plot was selected as it counter-acts the bias of the regression line, and tends to make the determination of lower drug concentration values more accurate. Standard curves were linear across the range used. As the expected levels in samples was unknown, a linear range from 3.2ng/mL to 1000ng/mL for each analyte.

The limit of detection (LOD) and limit of quantification (LOQ) are determined by examining the signal to noise ratio of the peaks, as calculated by the Masshunter Quantification software. An S/N of 3 is accepted for LOD and an S/N of 5 is accepted for LOQ.

***Results:***

***Quantification***

The level of cyclosporin A and its metabolites were quantified based on the peak area ratio (analyte/ISTD). A log-log plot of the mass in tube vs the peak area ratio was plotted and the resulting equation used to calculate the mass in tube of the candidate samples. The quantified mass is factored up to give quantification as ng/mL of sample.

The concentration of cyclosporin A and the metabolites AM4N, AM9 is shown in Table 8 and illustrated in Figure 2.

Table 8:

| % | cyclosporin A | AM9 | AM4N | Total metabolites | Ratio cyclosporin A / Total metabolites |
|---|---|---|---|---|---|
| **Formulations** | | | | | |
| Comparative | 73.8 | 14.2 | 12.0 | 26.2 | 2.8:1 |
| Formulation I | 86.9 | 7.6 | 5.5 | 13.1 | 6.6:1 |
| Formulation II | 91.5 | 5.1 | 3.4 | 8.5 | 11:1 |
| Neoral | 37.1 | 36.7 | 26.2 | 62.9 | 0.6:1 |

The faecal analysis shows that treatments with Formulations I and II resulted in lower concentrations of the cyclosporin metabolites. The concentration of cyclosporin A in the faecal samples from the treatments using Formulations

II and II was significantly higher than treatment with the Neoral™ indicating that these compositions provide high levels of cyclosporin A in the lower GI tract.

**Example 3 Pharmacokinetic Study in a Pig Model (not according to the present invention)**

The pharmacokinetic properties of Formulation III described in Example 1 was compared orally administered Neoral™ and intravenously administered Sandimmun. All doses were administered to provide 2 mg/kg cyclosporin A.

**Method**

**Pig Cannulation and Surgery**

Male pigs (Landrace) weighing 18 ± 2 kg were pre medicated with ketamine (26.4 mg/kg) and azaperone (3.2 mg/kg) administered by intramuscular (i.m.) injection. Following sedation, an intravenous (i.v.) cannula was inserted into the ear vein for induction of general anaesthesia using ketamine - midazolam mixture (3.3:0.2 mg/kg, i.v.). A sterile catheter (1.2 × 2.0 mm, Vygon) was surgically inserted into the jugular vein, while the proximal end of the catheter was tunnelled subcutaneously to the back of the neck and secured in place with surgical thread (Sofsilk™, Covidien). The catheter was flushed with heparinised saline and the neck wound was closed with sterile polypropylene sutures (Surgipro™, Covidien). Carprofen 25mg subcutaneous (s.c.) was administered peri-operatively as an anti-inflammatory analgesic. The pigs were returned to the recovery pens for 24 hours, where they had access to food and water, prior to oral CsA administration.

**Procedure for Orally Dosed Cyclosporin A (Formulation III and Neoral)**

The pigs were fasted overnight and dosed independently prior to oral administration of Neoral™ and the Formulation III minibeads. The animals were mildly sedated with ketamine (5.3 mg/kg) and xylazine (1 mg/kg) and weighed. Soft gelatin capsules were used for pre-microemulsion (preME) formulations i.e. (Neoral™) and hard gelatin capsules were used for the Formulation III minibeads. The animals were then given an oral dose (2 mg/kg cyclosporin A) of Formulation III or Neoral. Immediately after dosing, 50 ml of water was orally supplied via a syringe. Whole blood samples (4 ml) were collected before cyclosporin administration and at 0.5, 1, 1.5, 2, 2.5, 3, 3.5, 4, 5, 6, 8, 10, 12, and 24 hours after administration and frozen at -20°C in EDTA tubes (BD Vacutainer™) until analysis.

**Procedure for Intravenous Cyclosporin A Dosing (Sandimmun)**

Sandimmun™ (50 mg/ml concentrate ex.Novartis) was given via an ear cannula directly into the vein using the following procedure. The pigs were pre-medicated with ketamine (26.4 mg/kg) and azaperone (3.2 mg/kg) prior to cannulation. Sandimmun™ concentrate was diluted 1:20 with 0.9% w/v saline before administration and given by slow iv infusion over 5 minutes to provide a dose of 2 mg/kg. Whole blood samples (4 ml) were taken before Sandimmun™ administration and at 0.05, 0.15, 0.30, 0.45, 1, 1.5, 2, 3, 4, 5, 8, 10, 12, and 24 hours after administration and frozen at - 20°C in EDTA tubes (BD Vacutainer™) until analysis.

**Luminal & tissue cyclosporin collection**

At the end of the pharmacokinetic study, the pigs were killed by intravenous injection of 2 ml Pentobarbital sodium (200 mg/ml) followed by 10 ml potassium chloride (KCL). The animals were eviscerated and the gastrointestinal tract (GIT) was removed and placed on a sterilised surgical table. Occluding ligatures were applied at the distal and proximal ends of the upper small intestine (SIU), lower small intestine (SIL), caecum (CAC), ascending colon (AC), transverse colon (TC), descending colon (DC), and rectum (RT)

The luminal content of each section was removed and placed in 50 ml sterilised collection tubes.

Approximately 15 cm of tissue at each section was excised and placed in sterilised collection tubes. All samples were frozen at -20°C until analysis.

*Blood and Luminal Contents Sample Preparation*

1 ml samples of whole blood were added to 200 μl of 1M sodium hydroxide (NaOH) (Sigma-Aldrich) and 1.70 ml of deionised water (Sigma-Aldrich) and vortexed for about 1 minute. The solution was extracted into 2 × 4 ml diethyl ether:methanol mixture (95:5) and mixed for 10 mins using an IKA Vibrax™ shaker (VWR) followed by centrifugation at 4,000 rpm for 5 minutes. The organic layer was removed and evaporated under a stream of nitrogen at 40°C. The residue

left over was reconstituted in 250 μl of acetonitrile : water (50 : 50). The solution was washed with 1 ml of hexane (Sigma-Aldrich), mixed for 5 mins and centrifuged at 10,000 rpm for 5 mins. 100 μl of the injection solution was injected into the HPLC for analysis. The extraction and hexane wash procedures, identical to those for the blood samples, were repeated for the gastrointestinal luminal samples.

**Tissue Sample Preparation**

Gastrointestinal tissue samples (~ 1 g) were cleaned in 100 ml phosphate buffered saline (PBS) (Sigma-aldrich). The samples were quick frozen using liquid Nitrogen and pulverised using a pestle and mortar to form fine tissue samples. The fine tissue samples were homogenised for a further 2 minutes at 30,000 rpm using a polytron PT 1600E homogeniser (Kinematica AG). 200 μl of 1M NaOH and 1.7 ml deionised water was added to the samples. The extraction and hexane wash procedures, identical to those described for the blood and luminal samples, were subsequently carried out.

**Isolation of transverse colon tissue subsections**

A sample of the transverse colon was removed and cleaned in 100 ml phosphate buffered saline. The mucosa and submucosal layers (~ 0.5 g) were scraped using a glass slide and transferred to a sterilised collection tube containing 200 μl of 1M NaOH and 1.7 ml deionised water. Approximately 0.5 g of the remaining tissue, composed of the circular and longitudinal muscles (muscularis externa), was excised, quick frozen in liquid Nitrogen and pulverised using a pestle and mortar to form a fine tissue sample. This was subsequently added to 200 μl of 1M NaOH and 1.7 ml deionised water and homogenised for 2 minutes at 30,000 rpm using a polytron PT 1600E homogeniser (Kinematica AG). The extraction and hexane wash procedures, identical to those described for whole tissue samples, were subsequently carried out.

**Cyclosporin A Analysis**

*Protocol for HPLC Analysis*

Blood, tissue and luminal CsA was analysed using an UV - HPLC method as previously described (Hermann et al.,Journal of Pharmaceutical and Biomedical Analysis,30 (2002) 1263-1276). HPLC was performed using acetonitrile/water (50:50 v/v) mobile phase with a Zorbax™ Eclipse Plus $C_{18}$ column (4.6 × 150 mm, Agilent Technologies), preceded by a 4-mm × 3-mm guard column (Phenomenex, UK). The temperature of the analytical and guard columns was maintained at 80 $^0$C in a column heater (Agilent Technologies). The mobile phase was pumped at a flow rate of 2 ml/min using a gradient method. The injection volume of each sample was 100 μl, with an equilibration delay of 1 min before the next sample was injected. UV detection was carried out at 214 nm. The limit of quantification (LOQ) was 40 ng/ml.

*Data analysis*

Data are expressed as means ± standard error of the mean (SEM) (n = 3). Statistical variations were assessed using repeated measures or one way analysis of variance (ANOVA). Results were considered significant at $P < 0.05$. Post-hoc pair-wise multiple comparison of the means was performed using the Bonferroni test. The maximum blood CsA concentration ($C_{max}$) and the time ($T_{max}$) to reach $C_{max}$ were both calculated as the highest measured blood CsA concentration at the time of sampling . The area under the concentration - time curve ($AUC_{0\text{-}24\ hrs}$) was calculated using the trapezoidal method. Bioavailability (F) and blood elimination half - life ($T_{1/2}$) were also carried out using standard equations described in Bauer LA. Applied clinical pharmacokinetics: McGraw-Hill Medical; 2001. Data treatment and statistics were performed using SPSS statistics 20.0 software.

***Whole Blood PK Profile***

The Pharmacokinetic parameters for cyclosporin A measured in the blood are summarised in Table 9.

| | **Sandimmun™ iv** | **Neoral™ IR** | **Formulation III** |
|---|---|---|---|
| **Dose (mg/kg)** | 2 | 2 | 2 |
| **$T_{max}$ (hr)** | n/a | 2.17 ± 0.15 | 3.5 ± 0.38 |
| **$C_{max}$ (ng/ml)** | 1379.50 ± 294.14 | 284.18 ± 86.43 | 28.89 ± 18.66 |

(continued)

| | Sandimmun™ iv | Neoral™ IR | Formulation III |
|---|---|---|---|
| **$AUC_{0-24}$ (ng*hr/ml)** | 1573.23 ± 373.31 | 904.54 ± 217.33 | 121.24 ± 30.34* |
| **$F_{abs}$ (%)** | 100 | 57.50 | 7.71 |
| *p ≤ 0.05 | | | |

Figure 3 shows the pharmacokinetic blood profile of Formulation III compared to Neoral™ and Sandimmun (i.v) formulations dosed at 2 mg/kg. Data represents mean ± SEM (n = 3).

***Cyclosporin A Tissue Concentrations***

The *Post-mortem* concentration of cyclosporin A in gastrointestinal tissue sections at specific locations along the GI tract taken 24 hours after a single oral dose of 2 mg/kg cyclosporin A for each of Formulation III, Neoral™ and Samdimmun are shown in Table 10 and illustrated in Figure 4. Formulation III

Table 10

| | Sandimmun™ iv | Neoral™ IR | Formulation III |
|---|---|---|---|
| Duodenum (DML) | 17.5 (± 8.7) | 78.7 (± 56.7) | 0 (± 0) |
| Ileum (ILM) | 17.6 (± 7.9) | 69.2 (± 42.7) | 26.7 (± 11.5) |
| Caecum (CAC) | 24.9 (± 12.1) | 146.4 (± 120.9) | 225.4 (± 54.1) |
| Proximal colon (PCN) | 40.6 (± 10.5) | 70.0 (± 27.6) | 351.1 (± 99.1) |
| Transverse colon (TCN) | 28.8 (± 12.9) | 93.9 (± 49.5) | 568.0 (± 151.8) |
| Distal colon (DCN) | 55.6 (± 4.2) | 162.6 (± 28.3) | 388.7 (± 139.4) |
| Rectum (RTM) | 57.6 (± 12.0) | 140.8 (± 68.4) | 179.1 (± 60.9) |

Table 10 shows the mean ± Standard Error of the Mean (SEM)

Figure 4 shows statistically significantly higher concentrations of cyclosporin A in the proximal colon, transverse colon and distal colon for Formulation III compared to Sandimmun and Neoral.

The ratios of mean cyclosporin A concentration in colonic tissue for Formulation III : Neoral™ is shown in Table 11 at specific locations along the GI tract taken 24 hours after a single oral dose of 2 mg/kg in the pig study.

Table 11

| | Formulation III :Neoral™ IR |
|---|---|
| Proximal colon (PCN) | 5:1 |
| Transverse colon (TCN) | 6:1 |
| Distal colon (DCN) | 2.4:1 |

Table 11 shows that the composition of the invention gave significantly higher cyclosporin A concentration in the proximal colon, transverse colon and distal colon tissues compared to administration of Neoral.

***Cyclosporin A Luminal Concentrations***

The *Post-mortem* concentration of cyclosporin A in gastrointestinal luminal content at specific locations along the GI tract taken 24 hours after a single oral dose of 2 mg/kg cyclosporin A for each of Formulation III, Neoral™ and Samdimmun™ are shown in Table 12 and Figure 5.

Table 12

| | Sandimmun™ iv | Neoral™ IR | Formulation III |
|---|---|---|---|
| Duodenum (DML) | 44.3 (± 30.2) | 214.8 (± 96.1) | 59.2 (± 20.9) |
| Ileum (ILM) | 81.0 (± 56.2) | 316.1 (± 101.0) | 424.0 (± 210.0) |
| Caecum (CAC) | 148.1 (± 70.4) | 361.9 (± 72.2) | 877.9 (± 114.3) |
| Proximal colon (PCN) | 199.8 (± 63.2) | 489.6 (± 147.2) | 1696.2 (± 400.1) |
| Transverse colon (TCN) | 292.8 (± 83.8) | 903.4 (± 344.0) | 2616.2 (± 1036.5) |
| Distal colon (DCN) | 370.9 (± 141.7) | 925.5 (± 314.3) | 4524.1 (± 1224.5) |
| Rectum (RTM) | 432.5 (± 108.9) | 1160.2 (± 190.8) | 2897.7 (± 504.1) |

Table 11 shows the mean ± Standard Error of the Mean (SEM)

Figure 5 shows statistically significantly higher concentrations of cyclosporin A in the proximal colon, transverse colon, distal colon and rectal lumen contents for Formulation III compared to Sandimmun and Neoral.

Analysis of the data in Table 12 gave the ratios of cyclosporin A in the luminal contents for Formulation III : Neoral™ shown in Table 13.

Table 13

| | Formulation III : Neoral™ |
|---|---|
| Transverse colon (TCN) | 2.9:1 |
| Distal colon (DCN) | 4.9:1 |
| Rectum (RTM) | 2.5:1 |

Table 13 shows that the composition of the invention gave significantly higher cyclosporin A concentration in the luminal content in the transverse colon, distal colon and rectum compared to administration of Neoral.

***Cyclosporin A concentration in transverse tissue layers of colonic tissue***

The concentration of cyclosporin A in the mucosa, submucosa and muscularis externa of a section of transverse colon tissue was measured 24 hours after dosing the pigs with the three formulations at 2 mg/kg (i.e. Neoral, Sandimmun and Formulation III). The results are shown in Table 14 and illustrated in Figure 6.

Table 14

| | Sandimmun™ iv | Neoral™ | Formulation III |
|---|---|---|---|
| Average Mucosa | 24.3 (± 3.1) | 19.4 (± 2.0) | 147.5 (± 39.6) |
| Average Submucosa | 36.5 (± 2.1) | 20.0 (± 4.3) | 104.9 (± 24.1) |
| Average muscularis externa | 46.3 (± 6.7) | 27.9 (± 11.2) | 60.4 (± 7.8) |

Table 14 and Figure 6 show that the concentration of cyclosporin A in the mucosa and sub-mucosa of the colonic tissue was much higher for Formulation III compared to Neoral™ and Sandimmun. The data in Table 14 and Figure 6 suggests there is a directionality of supply of the cyclosporin to the colonic tissue. Cyclosporin A from Formulation III is delivered to the inner colonic tissue from the lumen; whereas cyclosporin is delivered primarily via the outer mesentery tissue for Neoral™ and Sandimmun (see Figure 7).

Table 15 shows the relative ratios of cyclosporin A resulting from Formulation III : Neoral™ in the mucosa, sub-mucosa and muscularis externa tissues based upon the data in Table 14.

Table 15

| | Formulation III: Neoral™ in transverse colon tissue |
|---|---|
| Average Mucosa | 7.6:1 |

(continued)

| | Formulation III: Neoral™ in transverse colon tissue |
|---|---|
| Average Submucosa | 5:1 |
| Average muscularis externa | 2:1 |

Table 15 illustrates the relatively high cyclosporin A concentration on the inner colonic tissue compared to administration of Neoral. A comparison of cyclosporin A between the mucosa and muscularis externa is shown in Table 16

Table 16

| | Formulation III | Neoral™ |
|---|---|---|
| Ratio of Average Mucosa : Average muscularis externa | 2.4:1 | 1:1.4 |

Table 16 further illustrates the directional supply of cyclosporin A to the transverse colon tissue in the pig model. Formulation III provides a high internal tissue concentration in the mucosa relative to the muscularis externa. In contrast the relative concentration of cyclosporin from Neoral™ was higher in the outer muscularis externa compared to the inner mucosa tissue. The high local cyclosporin A concentration in the luminal contents provided by compositions of the invention (see Figure 5, and Tables 12 and 13) may drive higher levels of cyclosporin A into the inner mucosa and submucosa compared to administration of Neoral.

**Example 4 In-Vitro Dissolution (not according to the present invention)**

The in-vitro dissolution profile of Formulations I, II and II described in Example 1 was measured using a two stage dissolution test. The dissolution testing was carried out in accordance with USP <711> Dissolution using Apparatus II (paddle apparatus) operated with a paddle speed of 75 rpm and with the dissolution medium at a temperature of 37°C ± 0.5°C. In the first stage of the test the dissolution medium was 750ml of 0.1N HCl simulating the gastric environment. At the start of the test (t=0) the sample was placed in the dissolution medium. After 2 hours an aliquot of the medium is taken for subsequent analysis and immediately (suitably within 5 minutes) the second stage of the dissolution test is initiated. In the second stage of the test 250 ml of 0.2M tribasic sodium phosphate containing 2% sodium dodecyl sulfate (SDS) is added to the dissolution medium and the pH adjusted to 6.8 ± 0.05 using 2N NaOH or 2N HCl as required giving a dissolution medium volume of 1000 ml during the second stage of the test.

Samples of the dissolution medium were taken at the following time points during the second stage of the test: 4 hours; 6 hours; 12 hours; and 24 hours from the start of the test (i.e. from t=0 at the start of the first stage).

The sample taken at the end of the first stage (2 hours) and the samples from the second stage were analysed for cyclosporin A using Reverse Phase HPLC with UV detection at 210nm.

The amount of dissolved cyclosporin A in the dissolution medium expressed as a % based upon the original cyclosporin content in the test formulation (the % released) for Formulations I, II and III at the sample points are summarised in Table 17

Table 17

| Time (hr) | % release from Formulation I | % release from Formulation II | % release from Formulation III |
|---|---|---|---|
| 2 | 0 | 0 | 0 |
| 4 | 33 | 20 | 31 |
| 6 | 63 | 53 | 55 |
| 12 | 93 | 86 | 93 |
| 24 | 97 | 96 | 99 |

**Example 5 Effect of HPMC Sub-Coating on In-Vitro Release From Mini-beads with an Ethyl Cellulose:Pectin Modified Release Coating (not according to the present invention)**

Minibeads (5a to 5f) having the composition shown in Table 18 were prepared using an analogous method to

that described in Example 1.

Table 18

| Component | 5a EXP 11/085 (%) | 5b EXP 11/227 (%) | 5c EXP 111268 (%) | 5d EXP 11/241 (%) | 5e EXP 11/283 (%) | 5f Non-subcoat (%) |
|---|---|---|---|---|---|---|
| Cyclosporin A | 8.8 | 9.2 | 9.4 | 9.5 | 9.2 | 9.9 |
| Miglyol 810 N | 3.8 | 3.9 | 4.0 | 4.0 | 4.0 | 4.2 |
| Transcutol HP | 13.4 | 13.9 | 14.4 | 14.4 | 14.1 | 15.1 |
| Kolliphor™ EL | 7.6 | 7.8 | 8.1 | 8.1 | 7.9 | 8.4 |
| SDS | 3.3 | 3.4 | 3.5 | 3.5 | 3.4 | 3.7 |
| Sorbitol | 4.7 | 4.8 | 5.0 | 5.0 | 4.9 | 5.2 |
| Gelatin | 40.3 | 41.8 | 43.1 | 43.2 | 42.1 | 45.2 |
| Opadry* / Methocel E5** | 8.2* | 5.3* | 2.6** | 2.4* | 4.5** | N/A |
| Surelease™ (solid contents) | 9.7 | 9.7 | 9.7 | 9.7 | 9.7 | 8.1 |
| Pectin | 0.2 | 0.2 | 0.2 | 0.2 | 0.2 | 0.2 |

The Opadry was Opadry White 20A28380 (supplied by Colorcon Limited)
Methocel E5 (HPMC, supplied by Colorcon Limited)
Each minibead had a ethyl cellulose: pectin outer modified release coating (Surelease™:Pectin) which provided a weight gain of 11%, except 5f, which had a weight gain of 9% (Surelease™:Pectin).
The beads of Examples 5a, 5b and 5d had an Opadry subcoat (HPMC dispersion)
The beads of Examples 5c and 5e had a Methocel E5 subcoating (HPMC).
The % weight gain of the various coatings is summarised in Table 19

Table 19

| | % weight gain of Opadry | % weight gain of Methocel | % weight gain of Surelease™/Pectin |
|---|---|---|---|
| Example 5a | 10% | NA | 11% |
| Example 5b | 6.3% | NA | 11% |
| Example 5c | NA | 3% | 11% |
| Example 5d | 2.7% | NA | 11% |
| Example 5e | NA | 5.3% | 11% |
| Example 5f | NA | NA | 9% |

The in-vitro dissolution profile of the minibeads was measured using the two stage dissolution test described in Example 4. The results are shown in Figure 8, which shows that more of the cyclosporin A was released from the HPMC sub-coated minibeads (5a to 5e) compared to the non-subcoated minibeads (5f)

Figure 8 shows that the HPMC subcoated minbeads released more cyclosporin A and at a faster rate than the minibeads without the HPMC sub-coat. The minibeads without the sub-coat had less Surelease™:Pectin than the sub-coated minibeads (9% compared to 11%). A higher coating weight gain would be expected to delay release however, unexpectedly; the reverse was true for the sub-coated minbeads.

**Example 6: Reduction in inter-batch variability of in-vitro dissolution profile (not according to the present invention)**

Three separate batches of the two minibead Formulations A and B shown in Table 19 were prepared using an analogous method to that described in Example 1. Formulation A had an HPMC sub-coating (Opadry), giving a 5%

weight gain and an outer Surelease™ pectin coating (11.5% weight gain). Formulation B had no HPMC sub-coating and an outer Surelease™ pectin coating (11.5% weight gain).

Table 20

| Component | Formulation A with subcoat (%) | Formulation B without subcoat (%) |
|---|---|---|
| Cyclosporin A | 9.2 | 9.9 |
| Miglyol 810 N | 3.9 | 4.2 |
| Transcutol HP | 14.0 | 15.1 |
| Kolliphor™ EL | 7.9 | 8.4 |
| SDS | 3.4 | 3.7 |
| Sorbitol | 4.9 | 5.2 |
| Gelatin | 42.1 | 45.2 |
| Opadry | 4.3 | N/A |
| Surelease™ (solid contents) | 10.1 | 8.1 |
| Pectin | 0.2 | 0.2 |

The dissolution profile for minibeads from each batch was measured using the two stage dissolution test described in Example 4. The results are shown in Figure 9, which shows that the HPMC sub-coated minibeads had an improved batch to batch variability in dissolution profile compared to minibeads without an HPMC subcoating.

**Example 7: Minibead Compositions (only compositions with surfactants and coatings as** in the **claims are according to the invention)**

Minibeads having the compositions shown in Table 22 were prepared using an analogous method to that described in Example 1 under "Core Manufacture" except the oil phase to aqueous phase ratio was 1:5 in the compositions of Table 22. Mixing of the oil phase and the aqueous phase resulted in a liquid mixture with the composition shown in Table 21. The "surfactant" of Table 21 and Table 22 was one of the surfactants listed in Table 23. Minibeads with a composition of Table 22 were prepared for all of the surfactants of Table 23 except for Labrafil M 1944 CS. It is expected that minibeads could be formed with a liquid composition comprising Labrafil M 1944 CS by varying the oil to aqueous phase ratio or by increasing the viscosity of the liquid composition.

Table 21

| Component | % w/w |
|---|---|
| Cyclosporin | 4.1 |
| Transcutol HP | 6.2 |
| Surfactant* | 4.3 |
| Miglyol 810 | 2.1 |
| Type A Gelatin | 14.3 |
| Sorbitol | 1.7 |
| SDS | 1.1 |
| Purified Water | 66.2 |

Table 22

| Component | % w/w |
|---|---|
| Cyclosporin | 12.1 |

(continued)

| Component | % w/w |
|---|---|
| Transcutol HP | 18.3 |
| Surfactant* | 12.9 |
| Miglyol 810 | 6.2 |
| Type A Gelatin | 42.3 |
| Sorbitol | 5.0 |
| SDS | 3.2 |

Table 23 shows the surfactants of the compositions of Table 21 and 22. The table also shows the results of a crystallisation test carried out on the liquid compositions comprising each of the surfactants.

Crystallisation Test

Emulsions were obtained with a composition disclosed in Table 21 for each of the surfactants listed in Table 23 with stirring at 250-350rpm. Samples of the emulsion were taken at 30 minute intervals and viewed under a microscope at 50x or 100x magnification. The time when crystals appeared in the sample is shown in Table 23.

Table 23

| Surfactant | HLB | Crystallization time (h) |
|---|---|---|
| Span 85 | 1.8 | 3 |
| Labrafil M 1944 CS | 4 | 1 |
| Span 40 | 6.7 | 1.5 |
| Plurol Oleique CC 497 | 6 | 1 |
| Labrafil M 2130 CS | 4 | 0.5 |
| Cremophor EL | 14 | 0.5 |

The cores described in Table 22 could be coated to provide an over coat and optionally a sub-coat, using the coatings and coating methods analogous to those described in Example 1.

**Example 8: Multiple dose, multi-stage study to evaluate the safety, tolerability, pharmacokinetics and colon tissue distribution of cyclosporin modified release capsules (CyCol®) compared to intravenous cyclosporin (Sandimmun™) in healthy male volunteers**

The clinical study described below may be carried out to demonstrate the pharmacokinetic properties of a modified release composition according to the invention. References to "CyCol®" in this example is a reference to a modified release composition according to the invention.

**Investigational Drug Administration**

Stage 1: CyCol®: 75 mg OD for 7 days

CyCol®: 75 mg BID* for 7 days

Sandimmun® IV 2mg/kg as a 24 hour infusion (2mg/kg/day)

Stage 2: CyCol®: 37.5 mg OD **OR** 150 mg OD for 7 days

Stage 3: CyCol®: 37.5 mg BID* **OR** 150 mg BID* for 7 days

* A single dose will be administered on Day 7 (in the morning).

**STUDY OBJECTIVES**

Primary Objectives:

- To characterise the whole blood pharmacokinetics of CyCol® following single and multiple oral doses, and compare to a single Sandimmun® IV administration pharmacokinetic profile in healthy male subjects.

- To evaluate the colonic mucosa concentrations of cyclosporin and it's metabolites following multiple oral doses of CyCol® and compare to concentrations following a single Sandimmun® IV administration.

Secondary Objectives:

- To obtain safety and tolerability information following multiple oral doses of CyCol® at the selected dosing regimens in healthy male subjects.

Exploratory Objectives:

- To evaluate the amount of unchanged cyclosporin and it's metabolites excreted in the faeces after administration of multiple doses of CyCol® and compare to amounts following a single Sandimmun® IV administration.

**STUDY DESIGN**

An open label, multiple-dose, multi-stage pharmacokinetic (PK) study. A maximum of 40 healthy adult male volunteers aged between 18 and 55 years will be enrolled at a single clinical research unit. For each CyCol® group dosing will last 7 days, whilst the Sandimmun® IV group will have a single dose over 24 hours (2 consecutive 12 hour infusions).

The first stage of the study will involve three parallel study groups. Subjects will receive Sandimmun® IV (2mg/kg) administered as an infusion over 24 hours (2mg/kg/day), CyCol® 75mg OD for 7 days or CyCol® 75mg BID for 7 days (single morning dose only on Day 7). Following review of these data alternative CyCol® dosing regimens may be explored in a sequential manner to a possible maximum dose of 150mg BID.

On the morning of Day 1, following an overnight fast, subjects will either start a 24 hour infusion (2 consecutive 12 hour infusions) of Sandimmun® IV at a dose equivalent to 2mg/kg/day, or receive CyCol® 75mg as an oral capsule. Subjects in the twice daily CyCol® dosing group will receive their second dose in the evening approximately 12 hours after the morning dose and after their 12 hour PK sample.

Blood samples for PK analysis will be collected from all subjects on Day 1 at 0 (i.e. pre dose), 2, 3, 4, 5, 6, 8, 10, 12, 16, 20 and 24 hours post dose (start of infusion for Sandimmun® IV group).

For those in the Sandimmun® IV group blood samples will also be collected at 2, 4, 6 and 8 hours after the completion of the infusion on Day 2.

For those in the CyCol® groups trough PK samples will be obtained pre-morning dose on Day 4. For those receiving CyCol® once daily additional PK samples will be obtained at 6, 12 and 16 hours post the morning dose on Day 6, whilst for those receiving CyCol® twice daily additional PK samples will be obtained at 6 and 12 hours post the morning dose (prior to evening dose) on Day 6, and 4 hours post the evening dose. On Day 7, all subjects in the CyCol® groups will have blood samples for PK obtained at 0 (i.e. pre-dose), 2, 4, 6, 8, and 12 hours post dose.

For all subjects, faecal sample collection will be requested from Day 0 until discharge from the unit at the end of the study. For those subjects receiving Sandimmun® IV samples collected during the infusion should be kept separate from those collected after completion of the infusion. A representative sample will be taken from each bowel movement and sent to the bioanalytical laboratory to determine amounts of unchanged cyclosporin and relative concentrations of its metabolites AM9, AM4N and AM1.

A sigmoidoscopy will be performed in an unprepared (except for air and water) bowel on Day 2 in the Sandimmun® IV group within the last hour of the infusion (infusion must be ongoing). Subjects dosed with CyCol® shall have a sigmoidoscopy performed on Day 7, within 4 to 6 hours of the morning/last dose.

During the sigmoidoscopy procedure a minimum of 5 biopsies will be obtained. Biopsies, approximately 1 cm apart, will be obtained from ideally as close as possible to the sigmoid colon. In the event that access to the sigmoid colon is restricted, 5 biopsies (1 cm apart) will be obtained from the rectum. Following collection of biopsy samples, 3 intracolonic faecal samples will be taken from the region of the biopsy collection site to test for cyclosporin concentrations.

**CRITERIA FOR EVALUATION**

- Whole blood: Cyclosporin concentrations and parameters:

    - Day 1: $C_{max}$, $T_{max}$, $AUC_{0-t}$, $AUC_{inf}$, and $T_{1/2}$
    - Steady State: $C_{max}$, $T_{max}$ and $AUC_{0-T}$

- Faeces: amount of unchanged cyclosporin and relative concentrations of its metabolites AM9, AM4N and AM1

- Colonic mucosa: Cyclosporin and metabolite concentrations

- Safety: adverse events, vital signs measurements, 12-lead ECGs, clinical safety laboratory measurements.

**CLINICAL PROCEDURES**

***Flexible Sigmoidoscopy***

Sigmoidoscopies will be performed in an unprepared bowel (except for air and water).

Subjects receiving Sandimmun® IV will be required to have the sigmoidoscopy performed within the last hour of their infusion (infusion must be ongoing).

Subjects receiving CyCol® will be required to have the sigmoidoscopy performed within 4 to 6 hours of the Day 7 morning/last dose.

***Biopsies***

Standard pinch biopsy forceps will be used to obtain the colonic mucosa biopsies. Each biopsy will be approximately 5mm in size. A total of 5 biopsies, approximately 1 cm apart will be obtained from as close to the sigmoid colon as possible.

In the event that access to the sigmoid colon is limited, the 5 biopsies will be obtained from the rectum.

Each biopsy should be rinsed with saline, blot dried and then transferred to a pre-weighed collection tube. The tube will then be weighed to enable determination of the biopsy's weight. The biopsy, without any further preparation or processing will be transferred to a cryovial and stored at - 70°C prior to analysis for cyclosporin and its metabolites in the tissue. Prior to analysis the tissue sample will be washed with of N-acetyl cysteine to remove the mucose layer from the surface of the tissue sample such that the concentration of CyA/metabolites measured in the tissue is the concentration present in the mucosal and epithelial tissues. The washings containing the mucose may also be analysed for CyA/metabolites.

**Pharmacokinetic Samples**

Blood samples for PK analysis will be collected from all subjects on Day 1 at 0 (pre dose), 2, 3, 4, 5, 6, 8, 10, 12, 16, 20 and 24 hours post dose (start of infusion for Sandimmun® IV group).

For those in the Sandimmun® IV group blood samples will also be collected at 2, 4, 6 and 8 hours after the completion of the infusion on Day 2.

For those in the CyCol® groups trough PK samples will be obtained pre-morning dose on Day 4. For those receiving CyCol® once daily additional PK samples will be obtained at 6, 12 and 16 hours post the morning dose on Day 6, whilst for those receiving CyCol® twice daily additional PK samples will be obtained at 6 and 12 hours post the morning dose (prior to evening dose) on Day 6, and 4 hours post the evening dose. On Day 7, all subjects in the CyCol® groups will have blood samples for PK obtained at 0 (i.e. pre-dose), 2, 4, 6 8, and 12 hours post dose.

Actual sampling times will be used for statistical analyses and so each time must be recorded accurately.

**Faecal Samples**

Subjects will be recommended to defecate on Day 0 prior to the administration of the study drug. This sample will be collected and one aliquot will be collected as a blank matrix.

From Day 0 through to completion of the study, subjects will be requested to collect their faeces. For those subjects receiving Sandimmun® IV samples collected during the infusion should be kept separate from those collected after completion of the infusion.

Time and date of each sample will be recorded. Each sample will be collected and weighed. Faecal samples will be homogenised (mixed) as soon as possible following collection and with 20mL of distilled water as needed to obtain a homogeneous sample with a consistency similar to a milkshake or thick cream. Samples may be stored at temperatures of 2-8oC if not homogenised after collection. Additional water may be added to the sample to achieve this consistency. Record the collection time of the sample and any volume of distilled water added to the faecal sample during mixing. No further weighing is required. One aliquot of approx 5g will be taken from the homogenised sample, without any other processing or preparation will be transferred to a 10mL clean pre-labelled screw cap container. The pre-labelled screw top container will be frozen at -70oC or below and stored at this temperature on dry ice prior to analysis.

The three intracolonic samples (approximately 500mg- 1g) will be collected and stored in individual containers without any additives at -70°C prior to analysis.

**Pharmacokinetic Analysis**

Pharmacokinetic parameters of AUC, Cmax, Tmax, T1/2 and Kel will be determined from the collected data using analogous methods to those described in Example 2.

**Colonic Tissue Analysis**

Concentrations of cyclosporin and its metabolites (AM1, AM9 and AM4N) in colonic tissue will be determined using the following protocol:

**Principle**

Liquid-liquid extraction with internal standardisation and HPLC separation using a C18-column, followed by MS/MS detection.

**Internal Standard** - D12 - Cyclosporin A

**Sample Matrix** - Human Tissue

Calibration standards and quality control samples are prepared in 50% EtOH.

**Solutions**

The IS stock solution and respective dilutions are prepared by using DMSO/MeOH (1/1). The internal standard (IS) working solution is prepared by dilution of the IS stock solution or one of its dilutions with DMSO/MeOH (1/1), and should have a concentration of ~50 ng/mL

**Storing of Samples and Solutions**

Samples / solutions should be stored at -20°C to -80°C

**Sample Handling and Sample Preparation for Analysis**

| Step | Thawing / transfer procedure (step by step) | |
|---|---|---|
| 1 | The following thawing procedures are possible:<br>• Thawing at approximately 20 to 25°C in a water bath for approx. 10 minutes<br>• Thawing air exposed at approximately 20 to 25°C for at least 30 minutes (depends on sample volume) | |
| 2 | If applicable: Vortexing for 30 seconds | |
| 3a | Cal. Stds. & QCs: | Transfer of **1000 μL** of each sample into a sample vial |
| 3b | Study sample: weight: approx.. 2 - 20 mg | |
| 4 | Re-freezing of original samples between -20°C and -80°C<br>Unless used for immediate preparation -> freezing of transferred samples between -20°C and -80°C | |

**Chromatographic and Auto-Sampler Parameters**

| Parameter | Scheduled range / description | |
|---|---|---|
| Mobile phase solvent ***A*** | 10 mM Ammonium acetate in water | |
| Mobile phase solvent ***B*** | ACN/THF (8/2) | |
| Mobile phase solvent loading pump | 10 mM Ammonium acetate in water | |
| Chromatographic run | 0.0 - 4.5 min linear gradient: | 40 % B → 52 % B |
| | 4.5 - 6.0 min linear gradient: | 52 % B → 85 % B |
| | 6.0 - 6.01 min linear gradient: | 85 % B → 0 % B |
| | 6.01 - 7.0 min isocratic: | 0 % B |
| Flow | 0.8 mL/min | |
| Injection volume | 10 μL | |
| Pre-column / Column | Luna C18, 4 × 2 mm / ACE3AQ; 100 × 2.1 mm, 3 μm (ACT, UK) | |
| Column temperature | 80°C | |
| **Parameter** | **Scheduled range / description** | |
| Cooling set point (T) | 25°C | |

**Detection**

| Parameter | Scheduled range / description |
|---|---|
| MS Ionisation mode | ESI |
| MS polarity | Positive |
| MS detection mode | MRM |
| Vaporizer temperature | 600°C |
| Ionisation voltage | 5.5 kV |

(continued)

| Parameter | Scheduled range / description |
|---|---|
| Gas 1 | Pressure = 75 psi |
| Gas 2 | Pressure = 75 psi |
| Curtain gas | pressure = 40 psi |
| Lateral position | 5 units ± 2 units (default) |
| Vertical position | 4 units ± 2 units (ESI default) |
| Quadrupole resolution | low → low |
| Transitions | 1203.0 ± 0.3 → 99.9 ± 0.3 m/z: Cyclosporin A (CE: 125 eV, CXP: 16V) |
| | 1215.0 ± 0.3 → 99.9 ± 0.3 m/z: D12-Cyclosporin A (CE: 125 eV, CXP: 16V) |
| | 1219.0 ± 0.3 → 224.0 ± 0.3 m/z: AM1 (CE: 65 eV, CXP: 15V) |
| | 1219.0 ± 0.3 → 99.9 ± 0.3 m/z: AM9 (CE: 125 eV, CXP: 16V) |
| | 1189.0 ± 0.3 → 224.0 ± 0.3 m/z: AM4N (CE: 65 eV, CXP: 15V) |
| DP (declustering potential) | 130 V ± 20 V |

**Acceptance Criteria for Chromatograms**

| | Parameter | Scheduled range / acceptance criteria / description |
|---|---|---|
| AM1 | Retention time for SST | 4.2 min ± 0.5 min |
| AM4N | Retention time for SST | 5.4 min ± 0.5 min |
| AM9 | Retention time for SST | 4.4 min ± 0.5 min |

***Calibration Standards and Quality Control Samples:***

***Preparation of blank samples and processed matrix.***

- Preparation as described below, but taking DMSO / MeOH (1/1) instead of IS working solution.

| Step | Preparation procedure (step by step) |
|---|---|
| I | [if not stored /available as ***1000 μL aliquots*** already -> see transfer above] |
| II | [if frozen -> thawing at 20°C to 25°C in a water bath for approx.. 5 min] |
| 1 | Addition of 25 μL of internal standard working solution |
| 2 | Addition of 4 mL of DIPE |
| 3 | ***Conversion Point:*** Extraction by shaking the test tubes vigorously for approx. 5 minutes using a DVX-2500 Multi-tube Vortexer (1700 rpm; cycle: 5 seconds run, 1 second pause time) |

(continued)

| Step | Preparation procedure (step by step) |
|---|---|
| 4 | Centrifugation (phase separation) at 4000 rpm for 2 minutes |
| 5 | Storage at -75°C for about 10 minutes |
| 6 | Decanting of the organic, liquid phase into a centrifuge vial |
| 7 | Evaporation of the organic phase using compressed air (Turbovap) at about 40°C for 14 minutes |
| 8 | Addition of 50 μL of 50 % EtOH |
| 9 | Vortexing for approx. 2 minutes using a DVX-2500 Multi-tube Vortexer (2500 rpm; cycle: 5 seconds run, 1 second pause time) |
| 10 | Centrifugation at 4000 rpm for 1 minute |

***Carry-over samples:***

- Transfer of approx. 100 μL 50% EtOH into appropriate auto-sampler vials

***Matrix samples (human tissue) - PART A:***

| Step | Preparation procedure (step by step) |
|---|---|
| I | [if not stored /available as ***approx.. 2 - 20 mg aliquots*** already -> see transfer above] |
| II | [if frozen -> thawing at 20°C to 25°C in a water bath for approx.. 5 min] |
| 1 | Addition of 500 μL 2 % N-Acetyl-L-Cysteine in water |
| 2 | Vortexing for approx. 10 min using a DVX-2500 Multi-tube Vortexer (**1000 rpm**) |
| 3 | Centrifugation (phase separation) at 13000 rpm for 2 minutes using biofuge pico |
| 4 | Decanting of the liquid phase into a sample vial (volume: approx. 10 mL) |
| 4a | **Caution: The remaining residue will be prepared separately (described in part B)** |
| 5 | Addition of 500 μL EtOH to the liquid phase |
| 9 | Addition of 25 μL of internal standard working solution |
| 10 | Addition of 4 mL of DIPE |
| 11 | ***Conversion Point:***<br>Extraction by shaking the test tubes vigorously for approx. 5 minutes using a DVX-2500 Multi-tube Vortexer (1700 rpm; cycle: 5 seconds run, 1 second pause time) |
| 12 | Centrifugation (phase separation) at 4000 rpm for 2 minutes |
| 13 | Storage at -75°C for about 10 minutes |
| 14 | Decanting of the organic, liquid phase into a centrifuge vial |
| 15 | Evaporation of the organic phase using compressed air (Turbovap) at about 40°C for 14 minutes |
| 16 | Addition of 50 μL of 50 % EtOH |
| 17 | Vortexing for approx. 2 minutes using a DVX-2500 Multi-tube Vortexer (2500 rpm; cycle: 5 seconds run, 1 second pause time) |
| 18 | Centrifugation at 4000 rpm for 1 minute |

• ***Matrix samples (human tissue) - PART B (Samples are taken from part A, step 4a):***

| Step | Preparation procedure (step by step) |
|---|---|
| 1 | Addition of 500 μL 50 % EtOH to the remaining residue |
| 2 | Addition of 25 μL of internal standard working solution |
| 3 | Destroying of the tissue by using an ultrasonic processor (cycle: 0.5 s, max. amplitude) for 30 s |
| 4 | Decanting of the liquid phase into a sample vial (volume: approx. 10 mL) |
| 5 | Addition of 500 μL 50 % EtOH to the remaining residue to the remaining residue |
| 6 | Vortexing for approx. 1 min using a DVX-2500 Multi-tube Vortexer (2500 rpm; cycle: 5 seconds run, 1 second pause time) |
| 7 | Decanting of the liquid phase including all tissue into the **same sample vial as used in step 4** |
| 8 | Addition of 4 mL of Diisopropylether (DIPE) |
| 9 | ***Conversion Point:*** Extraction by shaking the test tubes vigorously for approx. 5 minutes using a DVX-2500 Multi-tube Vortexer (1700 rpm; cycle: 5 seconds run, 1 second pause time) |
| 10 | Centrifugation (phase separation) at 4000 rpm for 2 minutes |
| 11 | Storage at -75°C for about 10 minutes |
| 12 | Decanting of the organic, liquid phase into a centrifuge vial |
| 13 | Evaporation of the organic phase using compressed air (Turbovap) at about 40°C for 14 minutes |
| 14 | Addition of 50 μL of 50 % EtOH |
| 15 | Vortexing for approx. 2 minutes using a DVX-2500 Multi-tube Vortexer (2500 rpm; cycle: 5 seconds run, 1 second pause time) |
| 16 | Centrifugation at 4000 rpm for 1 minute |

Regression and Statistics

Based on calibration standards the calibration curve fitting will be established using the data processing software by means of peak area ratios (analyte / internal standard). Analyte concentrations will be evaluated using an internal standard method.

**Faecal Analysis**

The amount of unchanged cyclosporin and relative concentrations of its metabolites AM9, AM4N and AM1 will be measured using analogous methods to those described above.

Results with Sandimmun™ **IV (2mg/kg)**

The concentration of cyclosporin (CyA) and its metabolites (AM1, AM4N and AM9) in the faecal and colonic tissue for those subjects treated with IV Sandimmun™ in accordance with the trial protocol described above are shown in Table 24.

Table 24

| | |
|---|---|
| CyA in intracolonic faeces | 1654 ng/g |
| CyA colonic tissue* | 834 ng/g |
| CyA in blood (taken simultaneously with the tissue biopsy) | 145 ng/ml |

(continued)

| | |
|---|---|
| Blood $AUC_{0-24hr}$ | 8836 ng.h/ml |
| Ratio CyA conc. in intracolonic faeces: CyA conc. in colonic tissue | 1.98 |
| Ratio CyA conc. in intracolonic faeces: CyA conc. In blood at time tissue sample was obtained | 5.75 |
| Whole blood $AUC_{0-24hr}$:CyA concentration in colonic tissue | 10.6 |
| * tissue samples were washed with N-acetyl cysteine to remove the mucose prior to analysis. | |

Intracolonic feaces:Colonic Tissue Ratio

The concentration of cyclosporin (CyA) in intracolonic faeces: concentration of CyA in colonic tissue for those subjects treated with a single 2 mg/kg/day IV Sandimmun™ in accordance with the trial protocol described above was approximately 2:1.

As described in the trial protocol above, the tissue biopsies obtained from an unprepared (except for air and water) bowel on Day 2 in the Sandimmun® IV group during the last hour of the IV infusion).

It is expected that the modified release compositions according to the invention will provide significantly higher concentrations of cyclosporin in the faeces compared to the IV administration of cyclosporin resulting in a high concentration gradient between the intracolonic faeces and the colonic tissue. Compositions according to the invention are expected to provide ratios of about 50:1 to 500:1 0:1, for example about 100:1 to 300:1, or particularly about 150:1 to 250:1. Compositions according to the invention are expected to show much lower systemic exposure to cyclosporin compared to IV administration of Sandimmun. Accordingly the concentrations of cyclosporin metabolites resulting from oral administration of the compositions according to the invention (at a dose of 75 mg once or twice per day as described above) are expected to be lower than the metabolite concentrations resulting from IV administration of Sandimun™

CyA Concentration in Colonic Tissue : CyA in Blood Ratio

The concentration of cyclosporin in colonic tissue: the concentration of cyclosporin in whole blood following IV administration of 2 mg/kg of Sandimmun ™ IV was 5.75:1.

The tissue biopsies were obtained as described above obtained from an unprepared (except for air and water) bowel on Day 2 in the Sandimmun® IV group within the last hour of the infusion (infusion must be ongoing).

The cyclosporin A concentration in the blood in the above ratio was the concentration of cyclosporin present in the blood at the time the tissue biopsy was obtained

It is expected that the modified release compositions according to the invention will provide significantly higher ratios of cyclosporin in the colonic tissue : cyclosporin concentration in the blood compared to the IV administration of cyclosporin. Compositions according to the invention are expected to provide tissue: blood ratios of about 10:1 to about 200:1 , for example about 20:1 to about 100:1, or from about 20:1 to about 40:1.

## Claims

**1.** A modified release composition for oral administration comprising a core having the form of a solid colloid, the colloid comprising a continuous phase comprising a hydrogel forming polymer and a disperse phase comprising cyclosporin A and an oil phase, the oil phase comprising an oil and one or more surfactants, wherein:

the oil and surfactant have an HLB of up to 10;
the oil comprises medium chain triglyceride;
the surfactant comprises a surfactant selected from: fatty acid glycerides, polyethylene glycol fatty acid esters, propylene glycol fatty acid esters, fatty acid lactic acid ester, sucrose fatty acid esters, sorbitan fatty acid esters, polyethylene glycol fatty alcohol ethers, ethylene oxide-propylene oxide block co-polymers and polyoxyethylene ethers;
the composition comprises a first coating and a second coating outside the first coating;

and wherein

(i) the first coating comprises a water-soluble cellulose ether or a water-soluble derivative of a cellulose ether,

wherein the first coating is present in an amount corresponding to a weight gain of the composition due to the first coating of from 1% to 20% by weight based upon the weight of the composition prior to applying the first coating; and
(ii) the second coating comprises a modified release coating to control or modulate release of the cyclosporin A from the composition selected from a controlled release polymer, a sustained release polymer, an enteric polymer, a pH independent polymer, a pH dependent polymer and a polymer specifically susceptible to degradation by bacterial enzymes in the gastrointestinal tract, or a combination of two or more such polymers.

**2.** A modified release composition for oral administration comprising a core having the form of a solid colloid, the colloid comprising a continuous phase comprising a hydrogel forming polymer and a disperse phase comprising cyclosporin A and an oil phase, the oil phase comprising an oil and one or more surfactants, wherein:

the oil and surfactant have an HLB of up to 10;
the weight ratio of surfactant: oil is from 5:1 to 1.5:1;
the surfactant comprises a surfactant selected from: fatty acid glycerides, polyethylene glycol fatty acid esters, propylene glycol fatty acid esters, fatty acid lactic acid ester, sucrose fatty acid esters, sorbitan fatty acid esters, polyethylene glycol fatty alcohol ethers, ethylene oxide-propylene oxide block co-polymers and polyoxyethylene ethers;
the composition comprises a first coating and a second coating outside the first coating;

and wherein

(i) the first coating comprises a water-soluble cellulose ether or a water-soluble derivative of a cellulose ether, wherein the first coating is present in an amount corresponding to a weight gain of the composition due to the first coating of from 1% to 20% by weight based upon the weight of the composition prior to applying the first coating; and
(ii) the second coating comprises a modified release coating to control or modulate release of the cyclosporin A from the composition selected from a controlled release polymer, a sustained release polymer, an enteric polymer, a pH independent polymer, a pH dependent polymer and a polymer specifically susceptible to degradation by bacterial enzymes in the gastrointestinal tract, or a combination of two or more such polymers.

**3.** The composition according to any of the preceding claims, wherein the composition releases less than 20% of the cyclosporin A after 2 hours; releases 10 to 40% of the cyclosporin A at 4 hours and releases at least 50% of the cyclosporin A at 12 hours, when measured in a two stage dissolution test using a USP Apparatus II with a paddle speed of 75 rpm and a dissolution medium temperature of 37°C; wherein for the first 2 hours of the dissolution test the dissolution medium is 750 ml of 0.1 N HCl, and at 2 hours 250 ml of 0.2M tribasic sodium phosphate containing 2% SDS is added to the dissolution medium and the pH is adjusted to pH 6.8.

**4.** The composition according to any one of the preceding claims, wherein the surfactant has a HLB value selected from: up to 8, e.g. of 1-8.

**5.** The composition of any of claims 1 to 3 wherein the oil has an HLB of 1-5 and the surfactant has an HLB of 2-8.

**6.** The composition of claim 4 or claim 5, wherein the surfactant is non-ionic.

**7.** The composition of any of claims 4 to 6, wherein the surfactant comprises a surfactant selected from: fatty acid glycerides, polyethylene glycol fatty acid esters , propylene glycol fatty acid esters , fatty acid lactic acid esters or sucrose fatty acid esters.

**8.** The composition of any of claims 4 to 7, wherein the surfactant
comprises a surfactant selected from: fatty acid glycerides, polyethylene glycol fatty acid esters, propylene glycol fatty acid esters and fatty acid lactic acid esters.

**9.** The composition of any of claims 4 to 8, wherein the surfactant comprises a fatty acid glyceride.

**10.** The composition of any of claims 4 to 9, wherein the oil comprises a triglyceride selected from caprylic/capric triglyceride; caprylic/capric/linoleic triglyceride; and caprylic/capric/succinic triglyceride.

**11.** The composition of any preceding claim, wherein the disperse phase comprises 20-30% by dry weight of the core.

**12.** The composition of any preceding claim, wherein cyclosporin A is dissolved in the disperse phase.

**13.** The composition of any preceding claim, wherein the hydrogel forming polymer matrix comprises a reversible hydrocolloid.

**14.** The composition of any preceding claim, wherein the hydrogel forming polymer matrix comprises gelatin, agar, a polyethylene glycol, starch, casein, chitosan, soya bean protein, safflower protein, alginates, gellan gum, carrageenan, xanthan gum, phthalated gelatin, succinated gelatin, cellulose phthalate-acetate, oleoresin, polyvinylacetate, hydroxypropyl methyl cellulose, polymerisates of acrylic or methacrylic esters and polyvinylacetate-phthalate; or a mixture of two or more such hydrogel forming polymers.

**15.** The composition of claim 13, wherein the hydrogel forming polymer matrix comprises a hydrocolloid selected from carrageenan, gelatin, agar and pectin, or a combination thereof.

**16.** The composition of claim 13 or claim 14, wherein the hydrogel forming polymer is gelatin.

**17.** The composition of any of claims 14 to 16, wherein the continuous phase comprises a plasticiser, particularly sorbitol.

**18.** The composition of any preceding claim, wherein the modified release coating comprises ethyl cellulose.

**19.** The composition according to any preceding claim, wherein the modified release coating further comprises a pore forming material.

**20.** The composition according to any preceding claim, wherein the modified release coating further comprises a water-soluble polysaccharide.

**21.** The composition of any preceding claim, wherein the first coating comprises a water-soluble cellulose ether.

**22.** The composition according to claim 21, wherein the first coating comprises hydroxypropylmethyl cellulose.

**23.** The composition according to any preceding claim, wherein the first coating is present in an amount corresponding to a weight gain of the composition due to the first coating of from 1% to 15% by weight based upon the weight of the composition prior to applying the first coating.

**24.** The composition according to any of claims 1 to 22, wherein the first coating is present in an amount corresponding to a weight gain due to the first coating in a range selected from: 1% to 6%, 1% to 4%, 4% to 6%, 6% to 10%, 9% to 15% and 12% to 15% by weight based upon the weight of the composition prior to applying the first coating.

**25.** The composition according to any preceding claims, wherein the second coating is present in an amount corresponding to a weight gain of the composition due to the second coating of from 5% to 20%, optionally from 7% to 15%, by weight based upon the weight of the composition prior to applying the second coating.

**26.** The composition according to any of claims 1 to 22, wherein the first coating is present in an amount corresponding to a weight gain due to the first coating in a range of from 1% to 15% by weight based upon the weight of the composition prior to applying the first coating; and wherein
the second coating is present in an amount corresponding to a weight gain of the composition due to the second coating selected from (a) from 5 to 40%; (b) from 10% to 12%or (c) from 16% to 18%by weight based upon the weight of the composition prior to applying the second coating.

**27.** The composition of claim 26, wherein the first coating is present in an amount corresponding to a weight gain due to the first coating in a range selected from (a) from 8% to 12%; or (b) from 4% to 6% by weight based upon the weight of the composition prior to applying the first coating.

**28.** The composition of claim 26 or claim 27, wherein the first coating comprises hydroxypropylmethyl cellulose and the second coating comprises ethyl cellulose.

**29.** A composition selected from

(i) the composition according to any of the preceding claims wherein the composition is in the form of a minibead;
(ii) a composition comprising a multiplicity of such minibeads.

**30.** The composition according to claim 29 wherein the largest cross sectional dimension of the minibead is from 1 mm to 5 mm, and/or the minibead is spheroidal and has an aspect ratio of no more than 1.5.

**31.** The composition of any of the preceding claims wherein the composition comprises an outer water-soluble protective coating, for example an outer coating comprising a water-soluble HPMC.

**32.** A composition according to any of the preceding claims formulated into a unit dosage form for oral administration comprising from 0.1 mg to 1000 mg, optionally 25 to 250 mg, cyclosporin A.

**33.** The composition of any preceding claim for use:

(A) as a medicament;
(B) in the treatment of an inflammatory bowel disease, Crohn's disease, ulcerative colitis, graft-versus-host disease, gastrointestinal graft-versus-host disease, myasthenia gravis, irritable bowel syndrome, celiac disease, stomach ulcers, diverticulitis, pouchitis, proctitis, mucositis, chemotherapy-associated enteritis, radiation-associated enteritis, short bowel disease, chronic diarrhea, gastroenteritis, duodenitis, jejunitis, peptic ulcer, Curling's ulcer, appendicitis, colitis, diverticulosis, endometriosis, colorectal carcinoma, adenocarcinoma, diversion colitis, ischemic colitis, infectious colitis, chemical colitis, microscopic colitis (including collagenous colitis and lymphocytic colitis), atypical colitis, pseudomembraneous colitis, fulminant colitis, autistic enterocolitis, indeterminate colitis, jejunoiletis, ileitis, ileocolitis or granulomatous colitis, prevention of rejection following bone marrow transplantation, psoriasis, atopic dermatitis, rheumatoid arthritis, nephrotic syndrome, primary sclerosing cholangitis, familial adenomatous polyposis, or perianal Crohn's, including perianal fistulae;
(C) in the treatment of an inflammatory condition of the GIT; or
(D) in the treatment of a condition of the GIT selected from irritable bowel syndrome, celiac disease, stomach ulcers, diverticulitis, pouchitis, proctitis, mucositis, radiation-associated enteritis, short bowel disease, or chronic diarrhea, gastroenteritis, duodenitis, jejunitis, peptic ulcer, Curling's ulcer, appendicitis, colitis, diverticulosis, endometriosis, colorectal carcinoma, adenocarcinoma, inflammatory disorders such as diversion colitis, ischemic colitis, infectious colitis, chemical colitis, microscopic colitis (including collagenous colitis and lymphocytic colitis), atypical colitis, pseudomembraneous colitis, fulminant colitis, autistic enterocolitis, indeterminate colitis, jejunoiletis, ileitis, ileocolitis, granulomatous colitis, fibrosis, graft-versus-host disease, gastrointestinal graft-versus-host disease, HIV or enteropathies.

## Patentansprüche

**1.** Zusammensetzung mit modifizierter Freisetzung zur oralen Verabreichung, umfassend einen Kern in Form eines festen Kolloids, wobei das Kolloid eine kontinuierliche Phase, die ein Hydrogel bildendes Polymer umfasst, und eine dispergierte Phase, die Cyclosporin A umfasst, und eine Ölphase umfasst, wobei die Ölphase ein Öl und ein oder mehrere Tenside umfasst, wobei:

das Öl und das Tensid einen HLB-Wert von bis zu 10 aufweisen;
das Öl mittelkettiges Triglycerid umfasst,
das Tensid ein Tensid umfasst, das aus Folgenden ausgewählt ist: Fettsäureglyceriden, Polyethylenglykol-Fettsäureestern, Propylenglykol-Fettsäureestern, Fettsäuremilchsäureestern, Saccharose-Fettsäureestern, Sorbitan-Fettsäureestern, Polyethylenglykol-Fettalkoholethern, Ethylenoxid-Propylenoxid-Block-Copolymeren und Polyoxyethylenethern;
die Zusammensetzung eine erste Beschichtung und eine zweite Beschichtung außerhalb der ersten Beschichtung umfasst, und wobei

(i) die erste Beschichtung einen wasserlöslichen Celluloseether oder ein wasserlösliches Derivat eines Celluloseethers umfasst, wobei die erste Beschichtung in einer Menge vorhanden ist, die einer Massenzunahme der Zusammensetzung aufgrund der ersten Beschichtung von 1% bis 20% Massenanteil, bezogen auf die Masse der Zusammensetzung vor einem Aufbringen der ersten Beschichtung, entspricht, und

(ii) die zweite Beschichtung eine Beschichtung mit modifizierter Freisetzung umfasst, um eine Freisetzung des Cyclosporin A aus der Zusammensetzung zu steuern oder zu modulieren, und die aus einem Polymer mit gesteuerter Freisetzung, einem Polymer mit verzögerter Freisetzung, einem magensaftresistenten Polymer, einem pH-unabhängigen Polymer, einem pHabhängigen Polymer und einem Polymer, das spezifisch für einen Abbau durch bakterielle Enzyme im Gastrointestinaltrakt anfällig ist, oder einer Kombination von zwei oder mehreren solcher Polymere ausgewählt ist.

**2.** Zusammensetzung mit modifizierter Freisetzung zur oralen Verabreichung, umfassend einen Kern in Form eines festen Kolloids, wobei das Kolloid eine kontinuierliche Phase, die ein Hydrogel bildendes Polymer umfasst, und eine dispergierte Phase, die Cyclosporin A umfasst, und eine Ölphase umfasst, wobei die Ölphase ein Öl und ein oder mehrere Tenside umfasst, wobei:

das Öl und das Tensid einen HLB-Wert von bis zu 10 aufweisen;
das Massenverhältnis von Tensid:Öl von 5:1 bis 1,5:1 beträgt;
das Tensid ein Tensid umfasst, das aus Folgenden ausgewählt ist: Fettsäureglyceriden, Polyethylenglykol-Fettsäureestern, Propylenglykol-Fettsäureestern, Fettsäuremilchsäureestern, Saccharose-Fettsäureestern, Sorbitan-Fettsäureestern, Polyethylenglykol-Fettalkoholethern, Ethylenoxid-Propylenoxid-Block-Copolymeren und Polyoxyethylenethern;
die Zusammensetzung eine erste Beschichtung und eine zweite Beschichtung außerhalb der ersten Beschichtung umfasst, und wobei

(i) die erste Beschichtung einen wasserlöslichen Celluloseether oder ein wasserlösliches Derivat eines Celluloseethers umfasst, wobei die erste Beschichtung in einer Menge vorhanden ist, die einer Massenzunahme der Zusammensetzung aufgrund der ersten Beschichtung von 1% bis 20% Massenanteil, bezogen auf die Masse der Zusammensetzung vor einem Aufbringen der ersten Beschichtung, entspricht, und
(ii) die zweite Beschichtung eine Beschichtung mit modifizierter Freisetzung umfasst, um eine Freisetzung des Cyclosporin A aus der Zusammensetzung zu steuern oder zu modulieren, und die aus einem Polymer mit gesteuerter Freisetzung, einem Polymer mit verzögerter Freisetzung, einem magensaftresistenten Polymer, einem pH-unabhängigen Polymer, einem pHabhängigen Polymer und einem Polymer, das spezifisch für einen Abbau durch bakterielle Enzyme im Gastrointestinaltrakt anfällig ist, oder einer Kombination von zwei oder mehreren solcher Polymere ausgewählt ist.

**3.** Zusammensetzung gemäß einem der vorhergehenden Ansprüche, wobei die Zusammensetzung weniger als 20% des Cyclosporin A nach 2 Stunden freisetzt, 10 bis 40% des Cyclosporin A nach 4 Stunden freisetzt und mindestens 50% des Cyclosporin A nach 12 Stunden freisetzt, wenn dies in einem zweistufigen Auflösungstest unter Verwendung einer USP-Vorrichtung II mit einer Paddelgeschwindigkeit von 75 U/min und einer Temperatur des Lösungsmediums von 37 °C gemessen wird; wobei für die ersten 2 Stunden des Auflösungstests das Lösungsmedium 750 ml 0,1 N HCl ist und nach 2 Stunden 250 ml 0,2M dreibasisches Natriumphosphat, das 2% SDS enthält, dem Lösungsmedium zugegeben wird und der pH auf pH 6,8 eingestellt wird.

**4.** Zusammensetzung gemäß irgendeinem der vorhergehenden Ansprüche, wobei das Tensid einen HLB-Wert aufweist, der aus Folgendem ausgewählt ist: bis zu 8, z. B. 1-8.

**5.** Zusammensetzung nach einem der Ansprüche 1 bis 3, wobei das Öl einen HLB-Wert von 1-5 aufweist und das Tensid einen HLB-Wert von 2-8 aufweist.

**6.** Zusammensetzung nach Anspruch 4 oder Anspruch 5, wobei das Tensid nichtionisch ist.

**7.** Zusammensetzung nach einem der Ansprüche 4 bis 6, wobei das Tensid ein Tensid umfasst, das aus Folgenden ausgewählt ist: Fettsäureglyceriden, Polyethylenglykol-Fettsäureestern, Propylenglykol-Fettsäureestern, Fettsäuremilchsäureestern oder Saccharose-Fettsäureestern.

**8.** Zusammensetzung nach einem der Ansprüche 4 bis 7, wobei das Tensid ein Tensid umfasst, das aus Folgenden ausgewählt ist: Fettsäureglyceriden, Polyethylenglykol-Fettsäureestern, Propylenglykol-Fettsäureestern und Fettsäuremilchsäureestern.

**9.** Zusammensetzung nach einem der Ansprüche 4 bis 8, wobei das Tensid ein Fettsäureglycerid umfasst.

**10.** Zusammensetzung nach einem der Ansprüche 4 bis 9, wobei das Öl ein Triglycerid umfasst, das aus Capryl-/Caprintriglycerid; Capryl-/Caprin-/Linoltriglycerid; und Capryl-/Caprin-/Succintriglycerid ausgewählt ist.

**11.** Zusammensetzung nach einem vorhergehenden Anspruch, wobei die dispergierte Phase 20-30% der Trockenmasse des Kerns umfasst.

**12.** Zusammensetzung nach einem vorhergehenden Anspruch, wobei Cyclosporin A in der dispergierten Phase gelöst ist.

**13.** Zusammensetzung nach einem vorhergehenden Anspruch, wobei die Hydrogel bildende Polymermatrix ein reversibles Hydrokolloid umfasst.

**14.** Zusammensetzung nach einem vorhergehenden Anspruch, wobei die Hydrogel bildende Polymermatrix Gelatine, Agar, ein Polyethylenglykol, Stärke, Casein, Chitosan, Sojabohnenprotein, Saflorprotein, Alginate, Gellangummi, Carrageen, Xanthan, phthalierte Gelatine, succinierte Gelatine, Cellulosephthalat-Acetat, Oleoresin, Polyvinylacetat, Hydroxypropylmethylcellulose, Polymerisate von Acryl- oder Methacrylsäureestern und Polyvinylacetat-Phthalat; oder ein Gemisch von zwei oder mehr solcher Hydrogel bildenden Polymere umfasst.

**15.** Zusammensetzung nach Anspruch 13, wobei die Hydrogel bildende Polymermatrix ein Hydrokolloid umfasst, das aus Carrageen, Gelatine, Agar und Pektin oder einer Kombination davon ausgewählt ist.

**16.** Zusammensetzung nach Anspruch 13 oder 14, wobei das Hydrogel bildende Polymer Gelatine ist.

**17.** Zusammensetzung nach einem der Ansprüche 14 bis 16, wobei die kontinuierliche Phase einen Weichmacher, insbesondere Sorbitol, umfasst.

**18.** Zusammensetzung nach einem vorhergehenden Anspruch, wobei die Beschichtung mit modifizierter Freisetzung Ethylcellulose umfasst.

**19.** Zusammensetzung gemäß einem vorhergehenden Anspruch, wobei die Beschichtung mit modifizierter Freisetzung ferner ein porenbildendes Material umfasst.

**20.** Zusammensetzung gemäß einem vorhergehenden Anspruch, wobei die Beschichtung mit modifizierter Freisetzung ferner ein wasserlösliches Polysaccharid umfasst.

**21.** Zusammensetzung nach einem vorhergehenden Anspruch, wobei die erste Beschichtung einen wasserlöslichen Celluloseether umfasst.

**22.** Zusammensetzung gemäß Anspruch 21, wobei die erste Beschichtung Hydroxypropylmethylcellulose umfasst.

**23.** Zusammensetzung gemäß einem vorhergehenden Anspruch, wobei die erste Beschichtung in einer Menge vorhanden ist, die einer Massenzunahme der Zusammensetzung aufgrund der ersten Beschichtung von 1% bis 15% Massenanteil, bezogen auf die Masse der Zusammensetzung vor einem Aufbringen der ersten Beschichtung, entspricht.

**24.** Zusammensetzung gemäß einem der Ansprüche 1 bis 22, wobei die erste Beschichtung in einer Menge vorhanden ist, die einer Massenzunahme aufgrund der ersten Beschichtung in einem Bereich entspricht, der aus Folgendem ausgewählt ist: 1% bis 6%, 1% bis 4%, 4% bis 6%, 6% bis 10%, 9% bis 15% und 12% bis 15% Massenanteil, bezogen auf die Masse der Zusammensetzung vor einem Auftragen der ersten Beschichtung.

**25.** Zusammensetzung gemäß einem der vorhergehenden Ansprüche, wobei die zweite Beschichtung in einer Menge vorhanden ist, die einer Massenzunahme der Zusammensetzung aufgrund der zweiten Beschichtung von 5% bis 20%, optional von 7% bis 15% Massenanteil, bezogen auf die Masse der Zusammensetzung vor einem Aufbringen der zweiten Beschichtung, entspricht.

**26.** Zusammensetzung gemäß einem der Ansprüche 1 bis 22, wobei die erste Beschichtung in einer Menge vorhanden ist, die einer Massenzunahme aufgrund der ersten Beschichtung in einem Bereich von 1% bis 15% Massenanteil, bezogen auf die Masse der Zusammensetzung vor einem Aufbringen der ersten Beschichtung, entspricht, und wobei

die zweite Beschichtung in einer Menge vorhanden ist, die einer Massenzunahme der Zusammensetzung aufgrund der zweiten Beschichtung entspricht, die aus (a) von 5 bis 40%; (b) von 10% bis 12% oder (c) von 16% bis 18% Massenanteil, bezogen auf die Masse der Zusammensetzung vor einem Aufbringen der zweiten Beschichtung, ausgewählt ist.

**27.** Zusammensetzung nach Anspruch 26, wobei die erste Beschichtung in einer Menge vorhanden ist, die einer Massenzunahme aufgrund der ersten Beschichtung in einem Bereich entspricht, der aus (a) von 8% bis 12%; oder (b) von 4% bis 6% Massenanteil, bezogen auf die Masse der Zusammensetzung vor einem Aufbringen der ersten Beschichtung, ausgewählt ist.

**28.** Zusammensetzung nach Anspruch 26 oder Anspruch 27, wobei die erste Beschichtung Hydroxypropylmethylcellulose umfasst und die zweite Beschichtung Ethylcellulose umfasst.

**29.** Zusammensetzung, ausgewählt aus

(i) der Zusammensetzung gemäß einem der vorhergehenden Ansprüche, wobei die Zusammensetzung in Form eines Mini-Beads vorliegt;
(ii) einer Zusammensetzung, die eine Vielzahl solcher Mini-Beads umfasst.

**30.** Zusammensetzung gemäß Anspruch 29, wobei die größte Querschnittsabmessung des Mini-Beads von 1 mm bis 5 mm beträgt und/oder das Mini-Bead kugelförmig ist und ein Seitenverhältnis von nicht mehr als 1,5 aufweist.

**31.** Zusammensetzung nach einem der vorhergehenden Ansprüche, wobei die Zusammensetzung eine äußere wasserlösliche Schutzbeschichtung umfasst, beispielsweise eine äußere Beschichtung, die eine wasserlösliche HPMC umfasst.

**32.** Zusammensetzung gemäß einem der vorhergehenden Ansprüche, formuliert in eine Einheitsdosierungsform zur oralen Verabreichung, die von 0,1 mg bis 1000 mg, optional 25 bis 250 mg, Cyclosporin A umfasst.

**33.** Zusammensetzung nach einem vorhergehenden Anspruch zur Verwendung:

(A) als ein Arzneimittel;
(B) bei der Behandlung einer entzündlichen Darmerkrankung, von Morbus Crohn, Colitis ulcerosa, einer Graft-versus-Host-Krankheit, gastrointestinalen Graft-versus-Host-Krankheit, Myasthenia gravis, von Reizdarmsyndrom, Zöliakie, Magengeschwüren, Divertikulitis, Pouchitis, Proktitis, Mukositis, einer Chemotherapie-assoziierten Enteritis, strahlenassoziierten Enteritis, Kurzdarmsyndrom, chronischer Diarrhö, Gastroenteritis, Duodenitis, Jejunitis, eines peptischen Geschwürs, Curling-Geschwür, Blinddarmentzündung, Colitis, Divertikulose, Endometriose, Kolorektalkarzinom, Adenokarzinom, Diversionscolitis, ischämischen Colitis, infektiösen Colitis, chemischen Colitis, mikroskopischen Colitis (einschließlich kollagener Colitis und lymphozytärer Colitis), atypischen Colitis, pseudomembranösen Colitis, fulminanten Colitis, autistischen Enterocolitis, Colitis indeterminata, Jejunoileitis, Ileitis, Ileocolitis oder granulomatösen Colitis, Prävention einer Abstoßung nach Knochenmarktransplantation, Psoriasis, atopischen Dermatitis, rheumatoiden Arthritis, nephrotischem Syndrom, primären sklerosierenden Cholangitis, familiären adenomatösen Polyposis oder perianalem Crohn, einschließlich perianaler Fisteln;
(C) bei der Behandlung eines entzündlichen Zustands des GIT; oder
(D) bei der Behandlung einer eines Erkrankung des GIT, die ausgewählt ist aus Reizdarmsyndrom, Zöliakie, Magengeschwüren, Divertikulitis, Pouchitis, Proktitis, Mukositis, strahlenassoziierter Enteritis, Kurzdarmsyndrom oder chronischer Diarrhö, Gastroenteritis, Duodenitis, Jejunitis, peptischen Geschwür, Curling-Geschwür, Blinddarmentzündung, Colitis, Divertikulose, Endometriose, Kolorektalkarzinom, Adenokarzinom, entzündlichen Erkrankungen wie Diversionscolitis, ischämischer Colitis, infektiöser Colitis, chemischer Colitis, mikroskopischer Colitis (einschließlich kollagener Colitis und lymphozytärer Colitis), atypischer Colitis, pseudomembranöser Colitis, fulminanter Colitis, autistischer Enteroclitis, Colitis indeterminata, Jejunoileitis, Ileitis, Ileocolitis, granulomatöser Colitis, Fibrose, Graft-versus-Host-Krankheit, gastrointestinaler Graft-versus-Host-Krankheit, HIV oder Enteropathien.

## Revendications

1. Composition à libération modifiée pour administration orale comprenant un coeur comportant la forme d'un colloïde solide, le colloïde comprenant une phase continue comprenant un polymère formant hydrogel et une phase dispersée comprenant de la cyclosporine A et une phase huileuse, la phase huileuse comprenant une huile et un ou plusieurs tensioactifs, dans laquelle :

    l'huile et le tensioactif comportent un HLB allant jusqu'à 10 ;
    l'huile comprend un triglycéride à chaîne moyenne ;
    le tensioactif comprend un tensioactif choisi parmi : les glycérides d'acides gras, les esters d'acides gras de polyéthylène glycol, les esters d'acides gras de propylène glycol, un ester d'acide lactique d'acide gras, les esters d'acides gras du saccharose, les esters d'acides gras du sorbitane, les éthers d'alcools gras de polyéthylène glycol, les copolymères séquencés oxyde d'éthylène-oxyde de propylène et les éthers polyoxyéthyléniques ;
    la composition comprend un premier enrobage et un second enrobage à l'extérieur du premier enrobage ; et dans laquelle

    (i) le premier enrobage comprend un éther cellulosique hydrosoluble ou un dérivé d'éther cellulosique hydrosoluble, ledit premier enrobage étant présent en une quantité correspondant à un gain de poids de la composition dû au premier enrobage de 1 % à 20 % en poids sur la base du poids de la composition avant application du premier enrobage ; et
    (ii) le second enrobage comprend un enrobage à libération modifiée pour contrôler ou moduler la libération de la cyclosporine A de la composition choisi parmi un polymère à libération contrôlée, un polymère à libération prolongée, un polymère entérique, un polymère indépendant du pH, un polymère dépendant du pH et un polymère spécifiquement sensible à la dégradation par les enzymes bactériennes dans le tractus gastro-intestinal, ou une combinaison de deux de ces polymères ou plus.

2. Composition à libération modifiée pour administration orale comprenant un coeur comportant la forme d'un colloïde solide, le colloïde comprenant une phase continue comprenant un polymère formant hydrogel et une phase dispersée comprenant de la cyclosporine A et une phase huileuse, la phase huileuse comprenant une huile et un ou plusieurs tensioactifs, dans laquelle :

    l'huile et le tensioactif comportent un HLB allant jusqu'à 10 ;
    le rapport massique tensioactif huile vaut de 5:1 à 1,5:1 ;
    le tensioactif comprend un tensioactif choisi parmi : les glycérides d'acides gras, les esters d'acides gras de polyéthylène glycol, les esters d'acides gras de propylène glycol, un ester d'acide lactique d'acide gras, les esters d'acides gras du saccharose, les esters d'acides gras du sorbitane, les éthers d'alcools gras de polyéthylène glycol, les copolymères séquencés oxyde d'éthylène-oxyde de propylène et les éthers polyoxyéthyléniques ;
    la composition comprend un premier enrobage et un second enrobage à l'extérieur du premier enrobage ; et dans laquelle

    (i) le premier enrobage comprend un éther cellulosique hydrosoluble ou un dérivé d'éther cellulosique hydrosoluble, ledit premier enrobage étant présent en une quantité correspondant à un gain de poids de la composition dû au premier enrobage de 1 % à 20 % en poids sur la base du poids de la composition avant application du premier enrobage ; et
    (ii) le second enrobage comprend un enrobage à libération modifiée pour contrôler ou moduler la libération de la cyclosporine A de la composition choisi parmi un polymère à libération contrôlée, un polymère à libération prolongée, un polymère entérique, un polymère indépendant du pH, un polymère dépendant du pH et un polymère spécifiquement sensible à la dégradation par les enzymes bactériennes dans le tractus gastro-intestinal, ou une combinaison de deux de ces polymères ou plus.

3. Composition selon l'une quelconque des revendications précédentes, ladite composition libérant moins de 20 % de la cyclosporine A après 2 heures ; libérant 10 à 40 % de la cyclosporine A à 4 heures et libérant au moins 50 % de la cyclosporine A à 12 heures, lorsqu'elle est mesurée dans un essai de dissolution à deux étapes à l'aide d'un appareil USP II avec une vitesse de palette de 75 rpm et une température de milieu de dissolution de 37°C ; pendant les 2 premières heures de l'essai de dissolution le milieu de dissolution étant 750 ml de HCl à 0,1 N, et à 2 heures 250 ml de phosphate de sodium tribasique à 0,2 M contenant 2 % de SDS étant ajoutés au milieu de dissolution et

le pH étant ajusté au pH de 6,8.

4. Composition selon l'une quelconque des revendications précédentes, ledit tensioactif comportant une valeur de HLB choisie parmi : allant jusqu'à 8, par ex. de 1 à 8.

5. Composition selon l'une quelconque des revendications 1 à 3, ladite huile comportant un HLB de 1 à 5 et le tensioactif comportant un HLB de 2 à 8.

6. Composition selon la revendication 4 ou 5, ledit tensioactif étant non-ionique.

7. Composition selon l'une quelconque des revendications 4 à 6, ledit tensioactif comprenant un tensioactif choisi parmi : les glycérides d'acides gras, les esters d'acides gras de polyéthylène glycol, les esters d'acides gras de propylène glycol, les esters d'acide lactique d'acide gras ou les esters d'acides gras du saccharose.

8. Composition selon l'une quelconque des revendications 4 à 7, ledit tensioactif comprenant un tensioactif choisi parmi : les glycérides d'acides gras, les esters d'acides gras de polyéthylène glycol, les esters d'acides gras de propylène glycol et les esters d'acides lactiques d'acides gras.

9. Composition selon l'une quelconque des revendications 4 à 8, ledit tensioactif comprenant un glycéride d'acide gras.

10. Composition selon l'une quelconque des revendications 4 à 9, ladite huile comprenant un triglycéride choisi parmi un triglycéride caprylique/caprique ; un triglycéride caprylique/caprique/linoléique ; et un triglycéride caprylique/caprique/succinique.

11. Composition selon l'une quelconque des revendications précédentes, ladite phase dispersée comprenant 20 à 30 % en poids sec du coeur.

12. Composition selon l'une quelconque des revendications précédentes, ladite cyclosporine A étant dissoute dans la phase dispersée.

13. Composition selon l'une quelconque des revendications précédentes, ladite matrice polymère formant hydrogel comprenant un hydrocolloïde réversible.

14. Composition selon l'une quelconque des revendications précédentes, ladite matrice polymère formant hydrogel comprenant de la gélatine, de la gélose, un polyéthylène glycol, de l'amidon, de la caséine, du chitosane, de la protéine de soja, de la protéine de carthame, des alginates, de la gomme gellane, de la carraghénane, de la gomme xanthane, de la gélatine phtalatée, de la gélatine succinatée, du phtalate-acétate de cellulose, de l'oléorésine, de l'acétate de polyvinyle, de l'hydroxypropylméthylcellulose, des polymérisats d'esters acryliques ou méthacryliques et d'acétate-phtalate de polyvinyle ; ou un mélange de deux de ces polymères formant hydrogel ou plus.

15. Composition selon la revendication 13, ladite matrice polymère formant hydrogel comprenant un hydrocolloïde choisi parmi la carraghénane, la gélatine, la gélose et la pectine, ou une combinaison de celles-ci.

16. Composition selon la revendication 13 ou 14, ledit polymère formant hydrogel étant de la gélatine.

17. Composition selon l'une quelconque des revendications 14 à 16, ladite phase continue comprenant un plastifiant, en particulier du sorbitol.

18. Composition selon l'une quelconque des revendications précédentes, ledit enrobage à libération modifiée comprenant de l'éthylcellulose.

19. Composition selon l'une quelconque des revendications précédentes, ledit enrobage à libération modifiée comprenant en outre un matériau porogène.

20. Composition selon l'une quelconque des revendications précédentes, ledit enrobage à libération modifiée comprenant en outre un polysaccharide hydrosoluble.

21. Composition selon l'une quelconque des revendications précédentes, ledit premier enrobage comprenant un éther

cellulosique hydrosoluble.

**22.** Composition selon la revendication 21, ledit premier enrobage comprenant de l'hydroxypropylméthylcellulose.

**23.** Composition selon l'une quelconque des revendications précédentes, ledit premier enrobage étant présent en une quantité correspondant à un gain de poids de la composition dû au premier enrobage de 1 % à 15 % en poids sur la base du poids de la composition avant application du premier enrobage.

**24.** Composition selon l'une quelconque des revendications 1 à 22, ledit premier enrobage étant présent en une quantité correspondant à un gain de poids dû au premier enrobage dans une plage choisie parmi : 1 % à 6 %, 1 % à 4 %, 4 % à 6 %, 6 % à 10 %, 9 % à 15 % et 12 % à 15 % en poids sur la base du poids de la composition avant application du premier enrobage.

**25.** Composition selon l'une quelconque des revendications précédentes, ledit second enrobage étant présent en une quantité correspondant à un gain de poids de la composition dû au second enrobage de 5 % à 20 %, éventuellement de 7 % à 15 %, en poids sur la base du poids de la composition avant application du second enrobage.

**26.** Composition selon l'une quelconque des revendications 1 à 22, ledit premier enrobage étant présent en une quantité correspondant à un gain de poids dû au premier enrobage dans une plage de 1 % à 15 % en poids sur la base du poids de la composition avant application du premier enrobage ; et
ledit second enrobage étant présent en une quantité correspondant à un gain de poids de la composition dû au second enrobage choisi parmi (a) de 5 à 40% ; (b) de 10 % à 12 % ou (c) de 16 % à 18 % en poids sur la base du poids de la composition avant application du second enrobage.

**27.** Composition selon la revendication 26, ledit premier enrobage étant présent en une quantité correspondant à un gain de poids dû au premier enrobage dans une plage choisie parmi (a) de 8 % à 12 % ; ou (b) de 4 % à 6 % en poids sur la base du poids de la composition avant application du premier enrobage.

**28.** Composition selon la revendication 26 ou 27, ledit premier enrobage comprenant de l'hydroxypropylméthylcellulose et ledit second enrobage comprenant de l'éthylcellulose.

**29.** Composition choisie parmi

(i) la composition selon l'une quelconque des revendications précédentes, ladite composition étant sous la forme d'une minibille ;
(ii) une composition comprenant une pluralité de ces minibilles.

**30.** Composition selon la revendication 29, la dimension transversale la plus grande de la minibille allant de 1 mm à 5 mm, et/ou la minibille étant sphéroïdale et comportant un rapport de forme inférieur ou égal à 1,5.

**31.** Composition selon l'une quelconque des revendications précédentes, ladite composition comprenant un enrobage externe de protection hydrosoluble, par exemple un enrobage externe comprenant un HPMC hydrosoluble.

**32.** Composition selon l'une quelconque des revendications précédentes formulée en forme posologique unitaire pour administration orale comprenant de 0,1 mg à 1000 mg, éventuellement 25 à 250 mg, de cyclosporine A.

**33.** Composition selon l'une quelconque des revendications précédentes pour utilisation :

(A) comme médicament ;
(B) dans le traitement d'une maladie intestinale inflammatoire, la maladie de Crohn, la colite ulcéreuse, une réaction de greffe contre hôte, une maladie gastrointestinale de greffe contre hôte, une myasthénie grave, le syndrome du côlon irritable, la maladie coeliaque, les ulcères de l'estomac, la diverticulite, la pochite, la proctite, la mucosité, une entérite associée à une chimiothérapie, une entérite associée aux rayonnements, la maladie de l'intestin court, une diarrhée chronique, la gastroentérite, la duodénite, la jéjunite, un ulcère peptique, un ulcère de Curling, l'appendicite, la colite, la diverticulose, l'endométriose, un carcinome colorectal, un adénocarcinome, la colite de diversion, la colite ischémique, la colite infectieuse, la colite chimique, la colite microscopique (y compris la colite collagène et la colite lymphocytaire), la colite atypique, la colite pseudomembraneuse, la colite fulminante, l'entérocolite autistique, la colite indéterminée, la jéjuno-iléite, l'iléite, l'iléocolite ou

la colite granulomateuse, la prévention d'un rejet suite à une transplantation de moelle osseuse, le psoriasis, la dermatite atopique, la polyarthrite rhumatoïde, le syndrome néphrotique, la cholangite sclérosante primitive, la polypose adénomateuse familiale ou la maladie de Crohn périanale, y compris les fistules périanales ;
(C) dans le traitement d'un état inflammatoire du tractus gastro-intestinal ; ou
(D) dans le traitement d'un état du tractus gastro-intestinal choisi parmi le syndrome du côlon irritable, la maladie coeliaque, les ulcères de l'estomac, la diverticulite, la pochite, la proctite, la mucosité, une entérite associée aux rayonnements, la maladie de l'intestin court, ou une diarrhée chronique, la gastroentérite, le duodénite, la jéjunite, un ulcère peptique, un ulcère de Curling, l'appendicite, la colite, la diverticulose, l'endométriose, un carcinome colorectal, un adénocarcinome, les troubles inflammatoires tels que la colite de diversion, la colite ischémique, la colite infectieuse, la colite chimique, la colite microscopique (y compris la colite collagène et la colite lymphocytaire), la colite atypique, la colite pseudomembraneuse, la colite fulminante, l'entérocolite autistique, la colite indéterminée, la jéjuno-iléite, l'iléite, l'iléocolite, la colite granulomateuse, la fibrose, une réaction de greffe contre hôte, une maladie gastrointestinale de greffe contre hôte, le VIH ou les entéropathies.

(a) Linear Scale

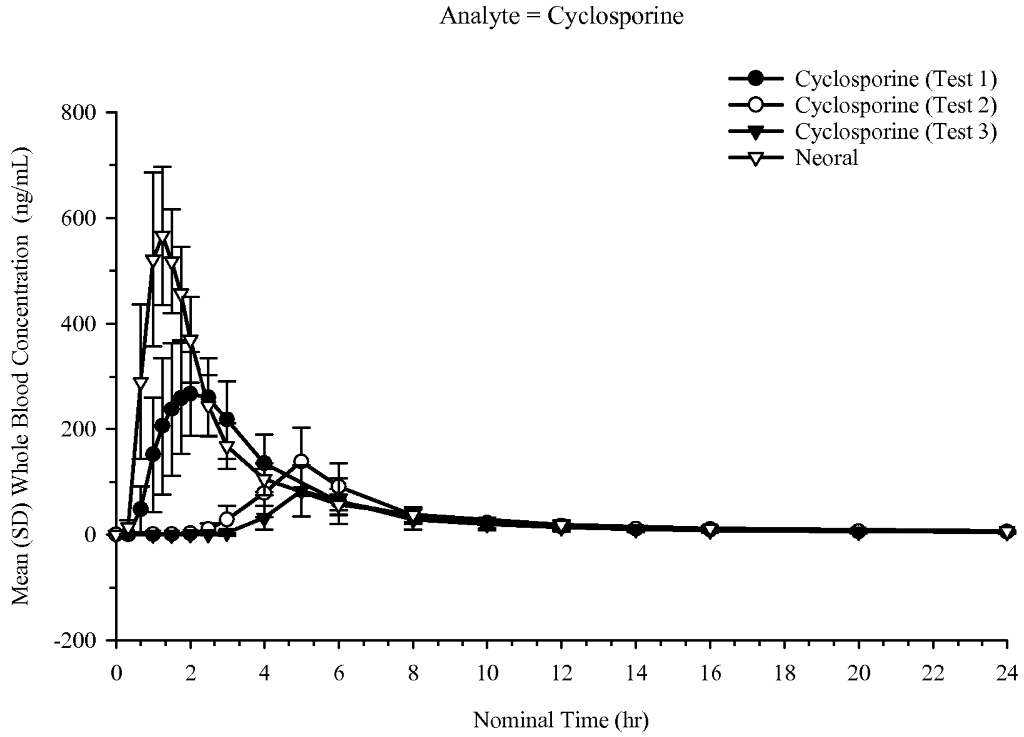


(b) Log Scale

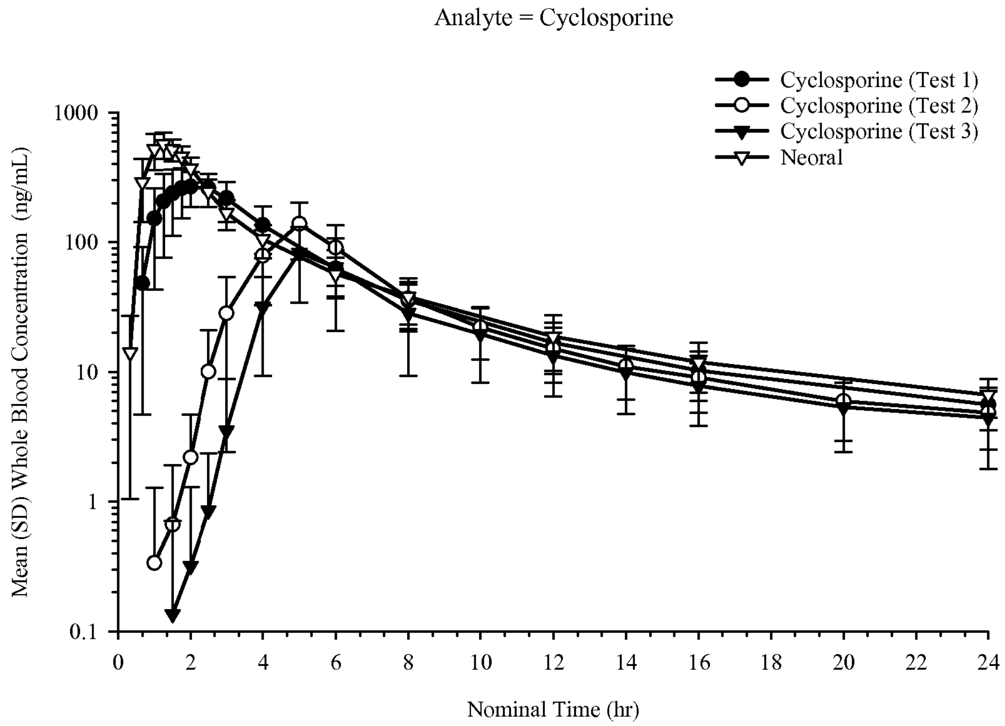


Figure 1

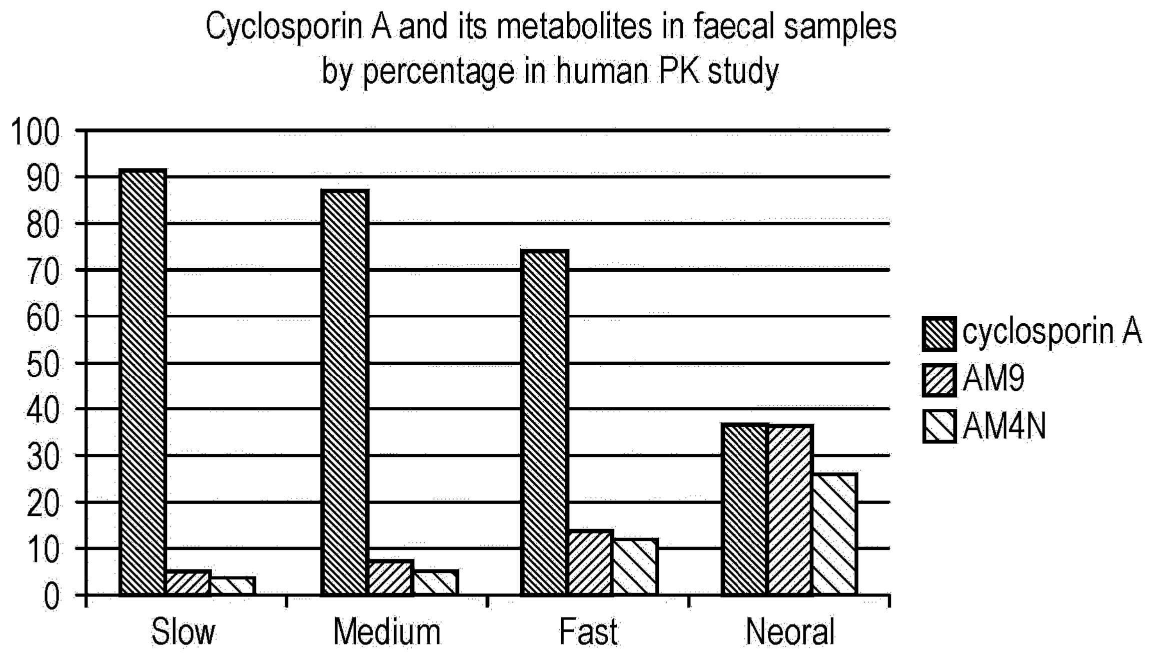


Figure 2

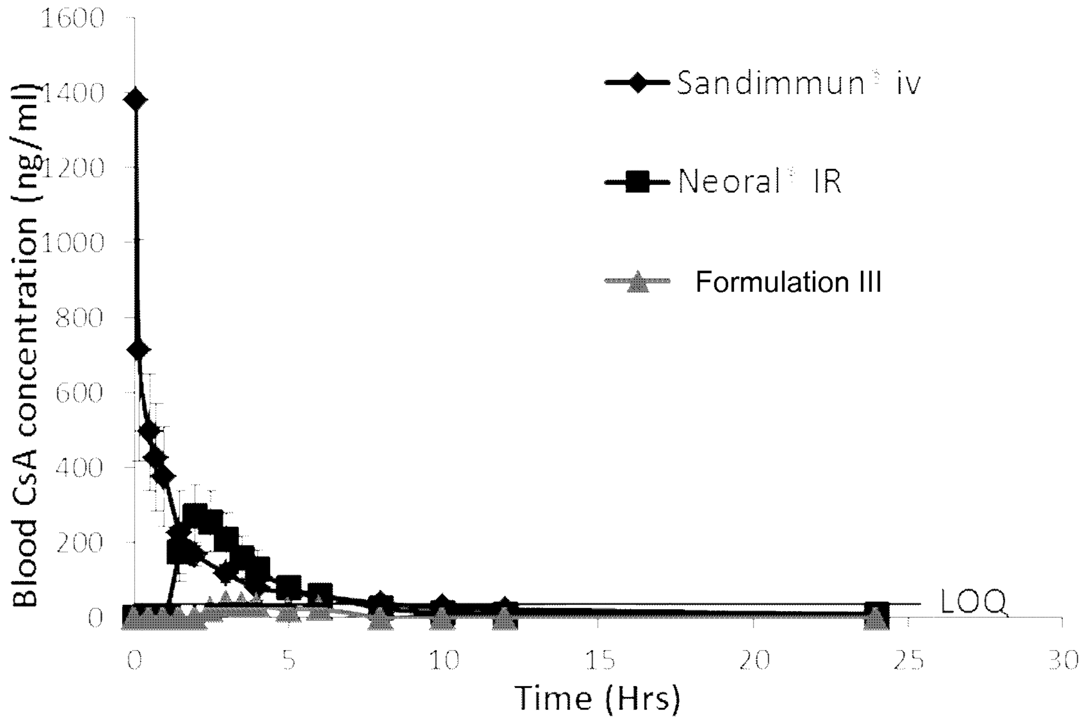


Figure 3

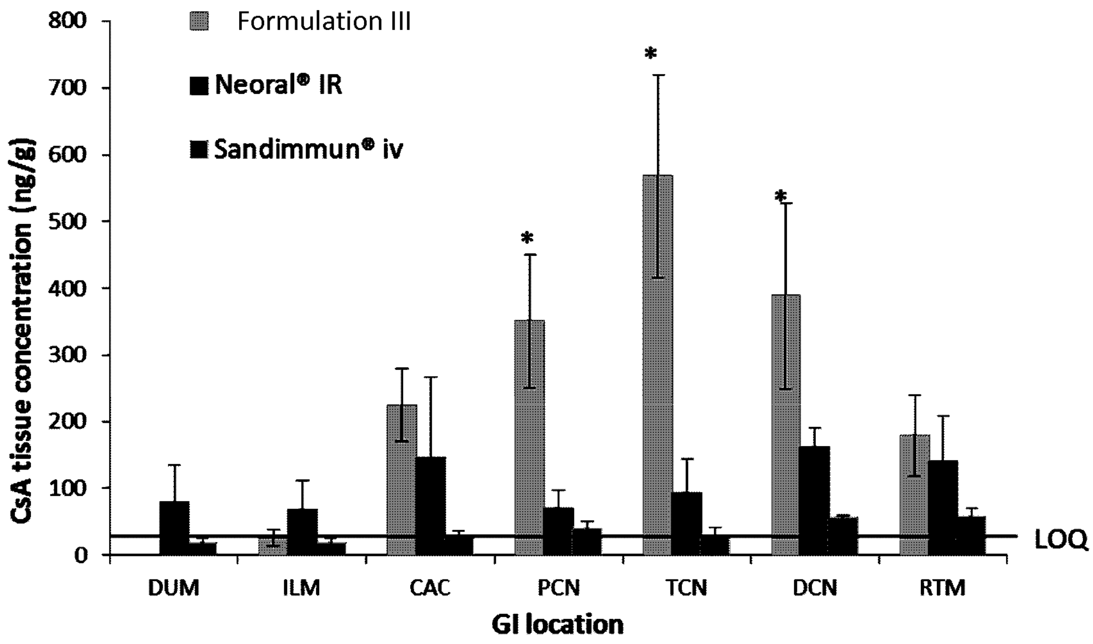


*p ≤ 0.05

Figure 4

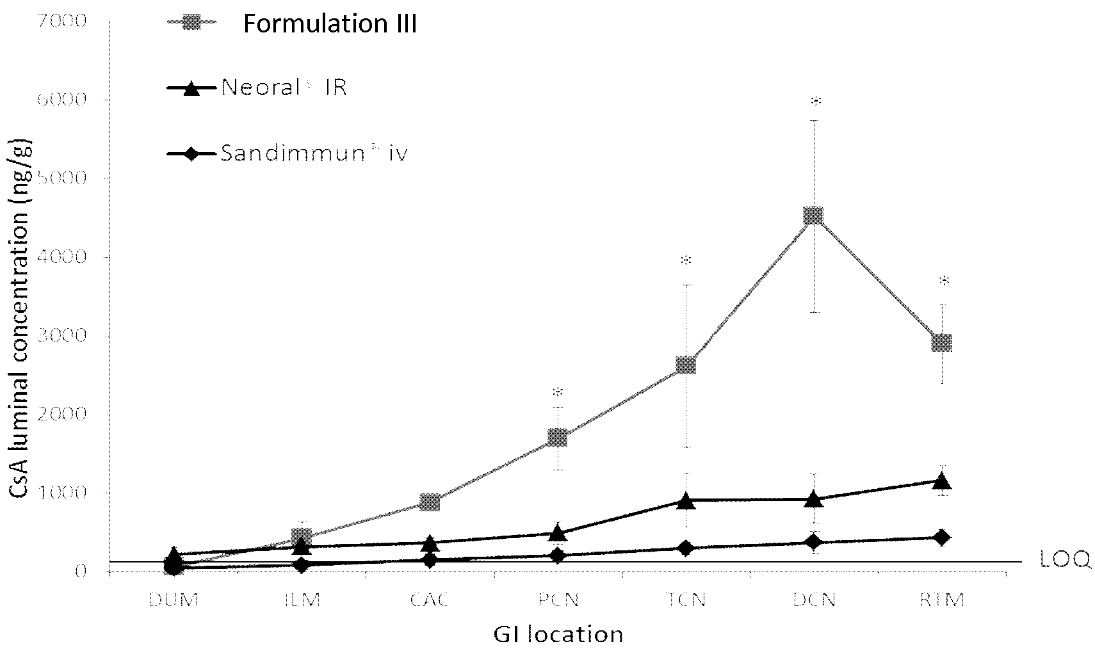


* p ≤ 0.05

Figure 5

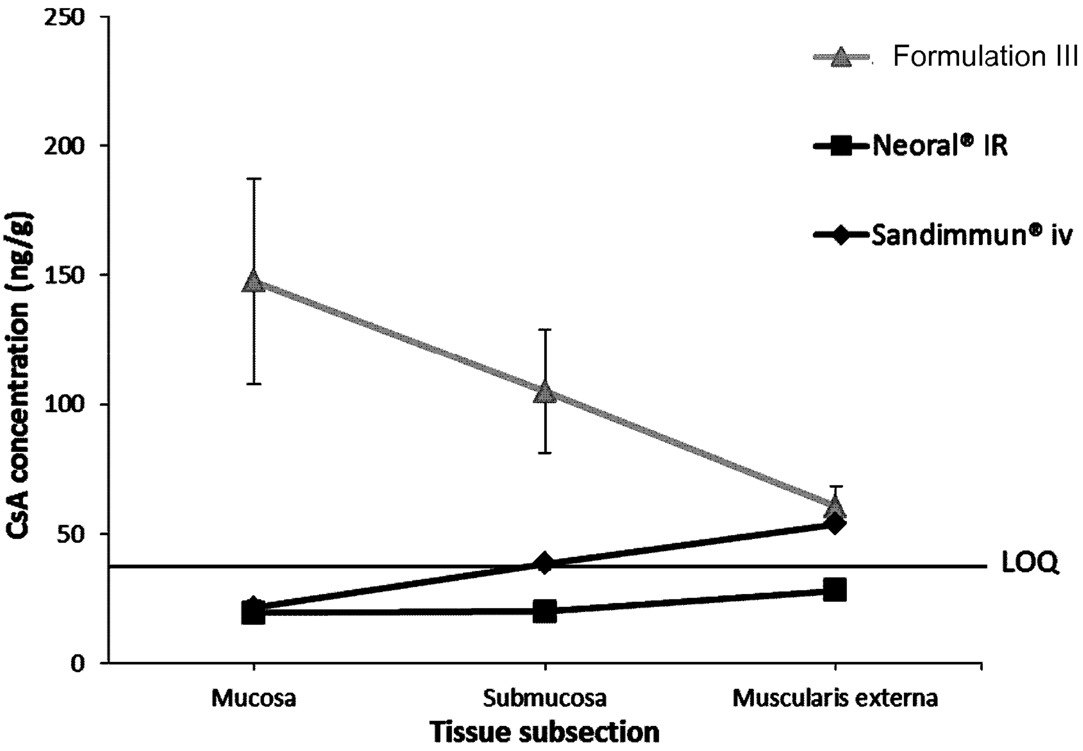


Figure 6

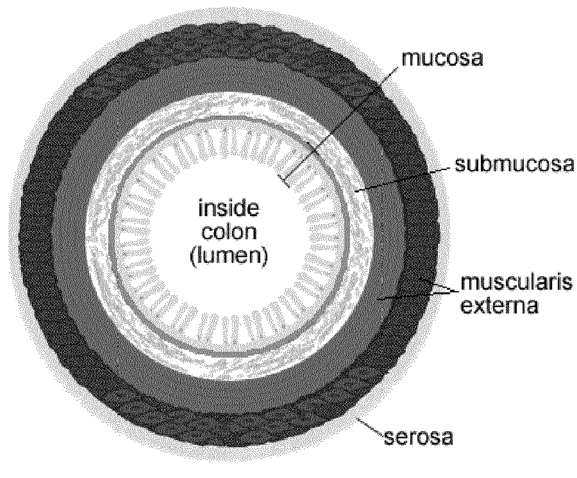


Figure 7

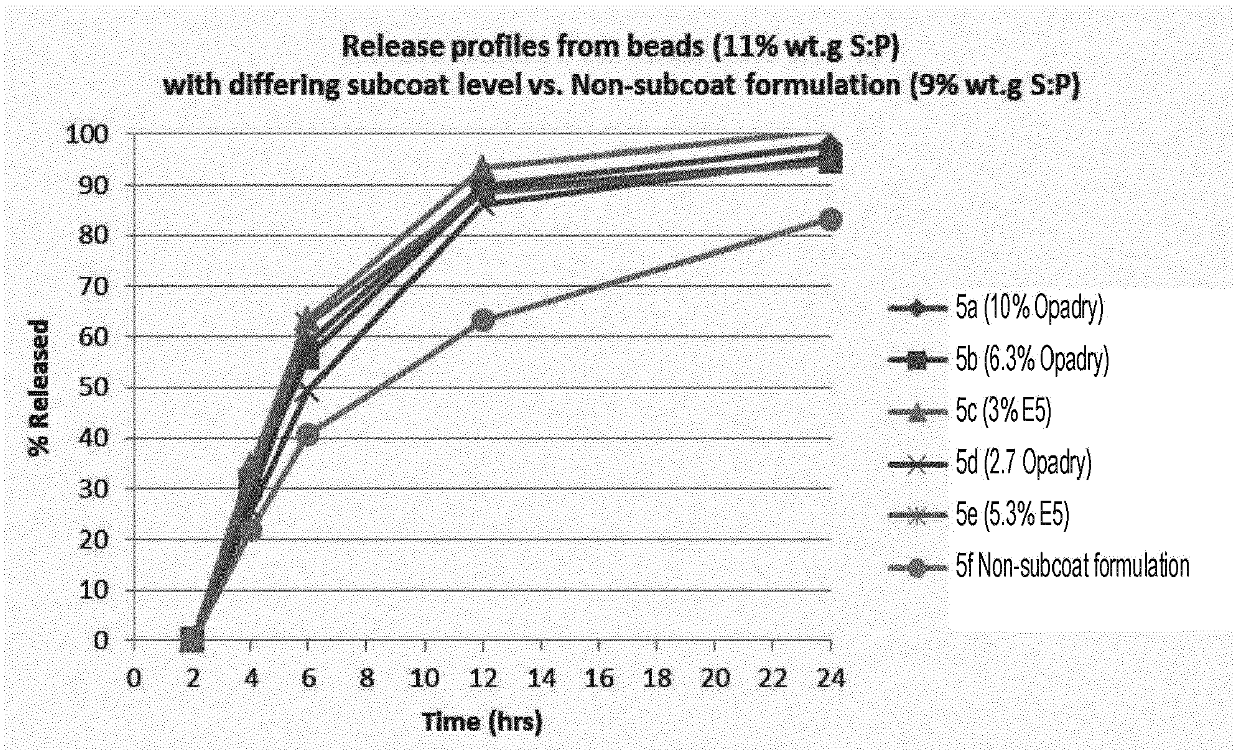


Figure 8

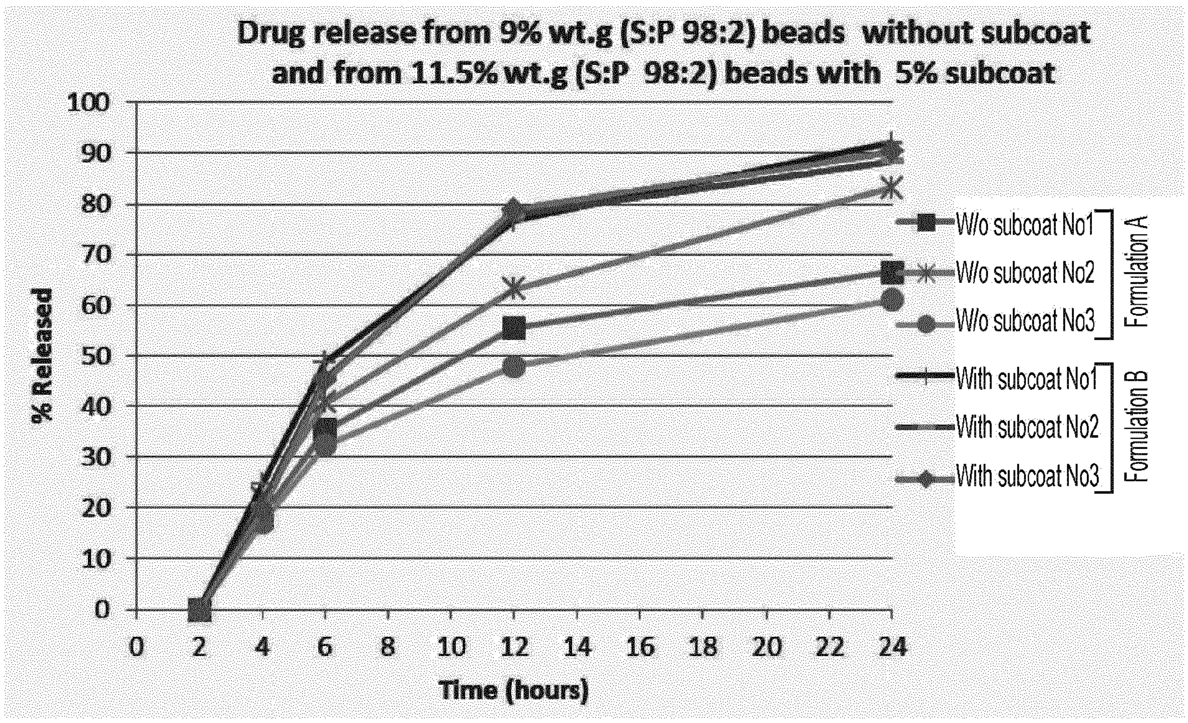


Figure 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008122965 A
- WO 2010133609 A
- US 4502888 A
- US 4123403 A
- US 3845770 A
- US 3916899 A
- US 3536809 A
- US 3598123 A
- US 4008719 A
- US 5674533 A
- US 5059595 A
- US 5591767 A
- US 5120548 A
- US 5073543 A
- US 5639476 A
- US 5354556 A
- US 5733566 A
- US 20050220878 A
- US 20060029660 A1, Fonkwe
- US 4659700 A
- WO 2008132707 A
- US 5882680 A, Freund


### Non-patent literature cited in the description


- **LANDFORD et al.** *Ann Intern Med,* 1998, vol. 128, 1021-1028
- **SANDBORN WJ.** a critical review of cyclosporin therapy in inflammatory bowel disease. *Inflamm Bowel Dis,* 1995, vol. 1, 48-63
- **LICHTIGER et al.** preliminary report (cyclosporine in the treatment of severe ulcerative colitis). *Lancet,* 1990, vol. 336, 16-19
- **COHEN et al.** Intravenous cyclosporine in ulcerative colitis (a five-year experience). *Am J Gastroenterol,* 1999, vol. 94, 1587-1592
- **FEUTREN et al.** Risk factors for cyclosporine-induced nephropathy in patients with auto-immune diseases, International kidney biopsy registry of cyclosporine for autoimmune diseases. *N Engl J Med,* 1992, vol. 326, 1654-1660
- **WIJDICKS et al.** Neurotoxicity in liver transplant recipients with cyclosporine immunosuppression. *Neurology,* 1995, vol. 45, 1962-1964
- **PORTER et al.** Cyclosporine-associated hypertension, National High Blood Pressure Education Program. *Arch Intern Med,* 1990, vol. 150, 280-283
- **SANDBORN et al.** *J Clin Pharmacol,* 1991, vol. 31, 76-80
- **RANZI T et al.** *Lancet,* 1989, vol. 2, 97
- **LICHTIGER et al.** *N. Engl J Med,* 1994, vol. 330, 1841-1845
- Remington, The Science and Practice of Pharmacy
- **SARA E. ROSENBAUM.** Basic Pharmacokinetics and Pharmacodynamics: An integrated Textbook and Computer Simulations. John Wiley& Sons, 2011
- **HERMANN et al.** *Journal of Pharmaceutical and Biomedical Analysis,* 2002, vol. 30, 1263-1276
- Chemistry and Application Properties of Polymethacrylate Coating Systems. Aqueous Polymeric Coatings for Pharmaceutical Dosage Forms. Marcel Dekker Inc, 109-114
- **KUMAR MAJETI N.V. RAVI.** *Reactive and Functional Polymers,* 2000, vol. 46, 1
- **PAUL et al.** *ST.P. Pharma Science,* 2000, vol. 10 (5
- **ZHAO et al.** Development of a Self Micro-Emulsifying Tablet of Cyclosporine-A by the Liquisolid Compact Technique. *International Journal of Pharmaceutical Sciences and Research,* 2011, vol. 2 (9), 2299-2308
- **HOLMBERG.** Surfactants and Polymers in Aqueous Solutions
- **FANG et al.** Analysis of cyclosporine A and its metabolites in rat urine and faeces by liquid chromatography-tandem mass spectrometry. *Journal of Chromatography B,* vol. 878 (15-16), 1153-1162
- **BINKHATHLAN et al.** Development of a liquid chromatography-mass spectrometry (LC/MS) assay method for the quantification of PSC 833 (Valspodar) in rat plasma. *Journal of Chromatography B,* 2008, vol. 869 (1-2), 31-37
- **BAUER LA.** Applied clinical pharmacokinetics. McGraw-Hill Medical, 2001